(19) Europäisches Patentamt
European Patent Office
Office européen des brevets

(11) EP 3 470 073 A1

# (12) EUROPEAN PATENT APPLICATION

(43) Date of publication:
**17.04.2019 Bulletin 2019/16**

(21) Application number: **18190631.4**

(22) Date of filing: **03.04.2013**

(51) Int Cl.:
***A61K 31/711*** *(2006.01)*
***C12N 5/0797*** *(2010.01)*
***A61P 25/28*** *(2006.01)*
***A61P 11/00*** *(2006.01)*
***A61P 9/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**03.04.2012 GB 201205972**
**19.07.2012 GB 201212848**
**12.02.2013 GB 201302468**

(62) Document number(s) of the earlier application(s) in accordance with Art. 76 EPC:
**13716352.3 / 2 833 893**

(71) Applicant: **Reneuron Limited**
**Guildford**
**Surrey GU2 7AF (GB)**

(72) Inventors:
- **SINDEN, John**
  **Guildford, Surrey GU2 7AF (GB)**
- **STEVANATO, Lara**
  **Guildford, Surrey GU2 7AF (GB)**
- **CORTELING, Randolph**
  **Guildford, Surrey GU2 7AF (GB)**

(74) Representative: **Elkington & Fife LLP**
**Prospect House**
**8 Pembroke Road**
**Sevenoaks, Kent TN13 1XR (GB)**

Remarks:
- Claims filed after the date of receipt of the divisional application (Rule 68(4) EPC).
- This application was filed on 24-08-2018 as a divisional application to the application mentioned under INID code 62.

## (54) STEM CELL MICROPARTICLES

(57) This invention relates to stem cell microparticles, their use and production, in particular neural stem cell microparticles and their use in therapy. The stem cell microparticle is typically an exosome or microvesicle and may be derived from a neural stem cell line. The neural stem cell line may be a conditionally-immortalised stem cell line such as CTX0E03 (deposited at the ECACC with Accession No. 04091601).

EP 3 470 073 A1

## Description

### Field of the Invention

This invention relates to stem cell microparticles, their use and production thereof, in particular neural stem cell microparticles and their use in therapy.

### Background of the Invention

Stem cells have the ability to self-renew and to differentiate into functionally different cell types. They have the potential to be a powerful tool in the growing field of Regenerative Medicine, in particular regenerative therapy requiring tissue replacement, regeneration or repair (Banerjee et al. 2011). However, there are drawbacks to the use of stem cells in therapy: there is a need for a consistent and substantial supply of stem cells with functional and phenotypic stability and the associated high costs and time delay caused by cell generation, storage, transport and handling; there is a requirement for immunological compatibility to avoid rejection of the stem cells by the recipient; and there are complex regulatory issues related to potential safety risks of tumour or ectopic tissue formation. Further, despite the therapeutic efficacy of stem cell transplantation, there is no convincing evidence for a direct long-term effect of the transplanted stem cells, for example through engraftment and differentiation into reparative or replacement cells.

Neural stem cells (NSCs) are self-renewing, multipotent stem cells that generate neurons, astrocytes and oligodendrocytes (Kornblum, 2007). The medical potential of neural stem cells is well-documented. Damaged central nervous system (CNS) tissue has very limited regenerative capacity so that loss of neurological function is often chronic and progressive. Neural stem cells (NSCs) have shown promising results in stem cell-based therapy of neurological injury or disease (Einstein et al. 2008). Implanting neural stem cells (NSCs) into the brains of post-stroke animals has been shown to be followed by significant recovery in motor and cognitive tests (Stroemer et al. 2009). It is not completely understood how NSCs are able to restore function in damaged tissues but it is now becoming increasingly recognised that NSCs have multimodal repairing properties, including site-appropriate cell differentiation, pro-angiogenic and neurotrophic activity and immunomodulation promoting tissue repair by the native immune system and other host cells (Miljan & Sinden, 2009, Horie et al., 2011). It is likely that many of these effects are dependent on transient signalling from implanted neural stem cells to the host milieu, for example NSCs transiently express proinflammatory markers when implanted in ischaemic muscle tissue damage which directs and amplifies the natural pro-angiogenic and regulatory immune response to promote healing and repair (Hicks et al., unpublished data). In chronic stroke brain, NSCs also have a substantial neurotrophic effect. For example, they promote the repopulation of the stoke-damaged striatal brain tissue with host brain derived doublecortin positive neroblasts (Hassani, O'Reilly, Pearse, Stroemer et al., PLoS One. 2012;7(11)).

Furthermore, on the basis of a large body of NSC restorative effects in animal models with chronic stroke, a clinical trial using neural stem cells is being carried out by ReNeuron Limited (Surrey, UK), to trial the treatment of disabled stroke patients using its "CTX0E03" conditionally-immortalised cortex-derived neural stem cells (Clinicaltrials.gov Identifier: NCT01151124).

Mesenchymal stem cells (MSCs) are lineage-restricted stem cells which have the potential to differentiate into mesenchymal cell types only, namely of the adipocytic, chondrocytic and osteocytic lineages (Pittenger et al 1999; Ding et al. 2011). MSCs (also referred to as Mesenchymal Stromal Cells and Mesenchymal Progenitor Cells) are derived from a variety of sources including bone marrow, blood, adipose and other somatic tissues. The therapeutic potential of MSCs, however, is more directed towards the application of their pro-angiogenic and immune modulating properties as undifferentiated cells. Production of human MSCs is limited by the inability of these cells to expand in numbers stably beyond approximately 15-20 population doublings.

Mesenchymal stem cell-conditioned medium (MSC-CM) has a therapeutic efficacy similar to that of MSCs themselves, suggesting a paracrine mechanism of MSC-based therapy (Timmers et al. 2007). WO-A-2009/105044 discloses that particles known as exosomes, secreted by MSCs, comprise at least one biological property of the MSCs and suggests the use of these MSC particles in therapy, while Théry et al. 2011 provides a general review of exosomes and other similar secreted vesicles. Whereas some of the drawbacks of using stem cells directly as therapeutic agents are overcome by using the mesenchymal stem cell-derived exosomes (e.g. storage, transport and handling), the problem remains of providing a consistent and substantial supply of functionally and phenotypically stable stem cells to produce the exosomes. For therapeutic use, the exosomes preferably need to be produced on a large scale. In the absence of a stem cell line, replenishment of the cells through repeated derivation from a source of stem cells is required, which incurs recurring costs for testing and validation of each new batch. Furthermore, the diseases and disorders that can be treated by MSCs may be limited.

There remains a need for improved stem cell-based therapies.

## Summary of the Invention

The present invention is based on the surprising finding that neural stem cells contain microparticles that are therapeutically useful.

It has also been found that it is possible to alter the production of microparticles by stem cells by the addition of components to the culture medium, by culturing the stem cells under hypoxic conditions, or by co-culture with other cell types, thereby providing an improved method of producing stem cell microparticles.

A first aspect of the invention provides a neural stem cell microparticle. The microparticle may be an exosome, microvesicle, membrane particle, membrane vesicle, exosome-like vesicle, ectosome-like vesicle, ectosome or exovesicle. Typically, the microparticle is an exosome. The microparticle may be derived from a neural stem cell that has been cultured in an environment that allows stem cell differentiation. The microparticle may be isolated from partially-differentiated neural stem cells. In one embodiment, an environment that allows stem cell differentiation is a multi-compartment bioreactor, typically where the cells are cultured for more than seven days. The microparticle may be derived from a neural stem cell line. In some embodiments, the neural stem cell line may be the "CTX0E03" cell line, the "STR0C05" cell line, the "HPC0A07" cell line or the neural stem cell line disclosed in Miljan et al Stem Cells Dev. 2009. In some embodiments, the microparticle is derived from a stem cell line that does not require serum to be maintained in culture. The microparticle may have a size of between 30 nm and 1000 nm, or between 30 and 200 nm, or between 30 and 100 nm, as determined by electron microscopy; and/or a density in sucrose of 1.1-1.2 g/ml. The microparticle may comprise RNA. The RNA may be mRNA, miRNA, and/or any other small RNA. The microparticle may comprise one, two, three or four of hsa-miR-1246, hsa-miR-4492, hsa-miR-4488 and hsa-miR-4532. The microparticle may comprise one or more lipids, typically selected from ceramide, cholesterol, sphingomyelin, phosphatidylserine, phosphatidylinositol, phosphatidylcholine. The microparticle may comprise one or more tetraspanins, typically CD63, CD81, CD9, CD53, CD82 and/or CD37. The microparticle may comprise one or more of TSG101, Alix, CD109, thy-1 and CD133. The microparticle may comprise at least 10 of the proteins present in Table 19 or Table 21. The microparticle may comprise at least one biological activity of a neural stem cell or a neural stem cell-conditioned medium. At least one biological activity may be a tissue regenerative activity. The microparticle of the invention is typically isolated or purified.

A second aspect of the invention provides a neural stem cell microparticle for use in therapy. The therapy may be regenerative therapy requiring tissue replacement, regeneration or repair, for example where the therapy requires angiogenesis, neurogenesis and/or neuroprotection. The therapy may be for a neurological disease, disorder or deficit. The therapy may improve functional and/or cognitive recovery. The therapy may be of stroke, peripheral arterial disease, neuropathy or any other disease or disorder that requires tissue regeneration, revascularisation or local anti-inflammatory action, including:

(i) Neurological disorder, disease or deficit, such as Parkinson's disease, Alzheimer's disease, Stroke, or ALS;
(ii) Lysosomal storage disorders;
(iii) Cardiovascular disorders, such as Myocardial Infarction, congestive heart failure, Peripheral Arterial Disease, diabetic ulcers, wound healing;
(iv) Diseases of the lung, including Idiopathic Pulmonary Fibrosis, Respiratory Distress Syndrome, Chronic Obstructive Pulmonary Disease, Idiopathic Pulmonary Hypertension, Cystic Fibrosis and Asthma;
(v) Metabolic or inflammatory disorders, such as Diabetes (I or II), rheumatoid arthritis, osteoarthritis, lupus, Crohn's disease, Inflammatory Bowel Disease, or Graft versus Host Disease;
(vi) Psychiatric disorders, such as Depression, Bipolar disorder, Schizophrenia or an Autistic syndrome disorder such as Autism, Asperger's syndrome or Rett Syndrome;
(vii) Blindness-causing diseases of the retina, such as Age-related macular degeneration, Stargardt disease, diabetic retinopathy, retinitis pigmentosa; and
(viii) Demyelinating diseases, such as multiple sclerosis, cerebral palsy, central pontine myelinolysis, tabes dorsalis, transverse myelitis, Devic's disease, progressive multifocal leukoencephalopathy, optic neuritis, leukodystrophies, Guillain-Barre syndrome, Anti-MAG peripheral neuropathy and Charcot-Marie-Tooth disease.

In one embodiment, the microparticle is an exosome and therapy is of a disease or condition requiring tissue replacement, regeneration or repair. In another embodiment, the microparticle is a microvesicle and the therapy is of a disease requiring angiogenesis or a neurological disease, disorder or deficit.

The therapy may also be a prophylactic therapy to induce tolerance, typically immunotolerance, in a host that is subsequently, concurrently or simultaneously to receive the stem cells from which the microparticle is derived. The administration of one or more doses of microparticles of the invention to a patient, prior to or concurrent with administration of a stem cell therapy, can be used to reduce the risk of an adverse immune response, i.e. "rejection", of the stem cell therapy.

A third aspect of the invention provides the use of a neural stem cell microparticle in the manufacture of a medicament for the treatment of a disease.

A fourth aspect of the invention provides a method of producing a stem cell microparticle, typically a neural stem cell microparticle. The method may comprise culturing the stem cells in an environment that allows stem cell differentiation and collecting the microparticles that are produced by the cells. The microparticles may be isolated from partially-differentiated neural stem cells. The stem cells may be cultured under conditions that allow the efficient removal of metabolic waste. In one embodiment, an environment that allows stem cell differentiation is culture in a multi-compartment bioreactor, typically for a prolonged period of time (for example more than seven days). The method may comprise isolating a microparticle from a stem cell-conditioned medium. The stem cell-conditioned medium may comprise one or more additive components or agents which stimulate the release of microparticles by the stem cells into the medium. The one or more components may be selected from transforming growth factor-beta (TGF-$\beta$), interferon-gamma (IFN-$\gamma$) and/or tumour necrosis factor-alpha (TNF-$\alpha$). The microparticles may be isolated from stem cell-conditioned medium wherein the stem cells were cultured under hypoxic conditions. The microparticles may be isolated from stem cell-conditioned medium produced by stem cells co-cultured with a different cell type, typically endothelial cells, in order to create the NSC niche environment.

A fifth aspect of the invention provides a microparticle obtainable by a method according to the fourth aspect of the invention.

A sixth aspect of the invention provides a composition comprising a neural stem cell microparticle and a pharmaceutically acceptable excipient, carrier or diluent.

A seventh aspect of the invention provides a method of screening for an agent that alters the production of a microparticle by a stem cell, comprising contacting a stem cell with a candidate agent and observing whether the rate of production of microparticles by the contacted stem cell increases or decreases compared to a control.

An eighth aspect of the invention provides a kit for use in a method for producing a stem cell microparticle, comprising: (a) a medium suitable for culturing stem cells; (b) a stem cell; (c) optionally the one or more components of the fourth aspect of the invention; (d) optionally a stem cell microparticle suitable for use as a control; (e) optionally a detection agent suitable for specific detection of the produced microparticles; and (f) instructions for producing the stem cell microparticle using the kit.

A ninth aspect of the invention provides a composition comprising two, three or all four of hsa-miR-1246, hsa-miR-4492, hsa-miR-4488 and hsa-miR-4532. This composition is optionally a pharmaceutical composition, comprising a pharmaceutically-acceptable carrier, diluent, vehicle and/or excipient. The pharmaceutical composition is suitable for use in therapy, typically in the same therapies as the microparticles of the invention, as noted above.

## Brief Description of the Drawings

Figure 1 depicts electron micrographs of CTX0E03 conditionally-immortalised neural stem cells producing microparticles. Panels A-E show intracellular multivesicular bodies (MVBs) containing exosomes between 30nm and 50nm in diameter and Panel F shows microvesicles >100nm in diameter released from neural stem cells through a process of budding at the cell membrane.

Figure 2 is an outline protocol for the identification, characterisation and production of microparticles from stem cells.

Figure 3 shows Human angiogenesis ELISA strip optical density read out performed on CTX0E03 conditioned and un-conditioned medium.

Figure 4A shows the amount of protein (measured by BCA assay) extracted from 15ml of media containing microparticles purified from the Integra system compared to normal culture conditions (3 days T175). Figure 4B shows the FACS detection (at 2ug/ml, 1:250) of (i) CD63 in Integra cultured CTX0E03 exosomes (top left panel) and microvesicles (top right panel) and (ii) CD81 in Integra cultured CTX0E03 exosomes (bottom left panel) and microvesicles (bottom right panel).

Figure 5 shows the amount of isolated total RNA measured at 260/280nm extracted from 15ml of media containing microparticles purified by filtration from the Integra system compared to normal culture conditions (3 days T175).

Figure 6A shows the results of a wound closure/scratch assay representing the migration activity of normal human dermal fibroblasts (NHDF) cultured in CTX0E03 conditioned media or upon the addition of purified CTX0E03 exosomes. Figure 6B shows the results of a scratch assay after 72 hours, comparing the effect of 10$\mu$g CTX0E03 exosomes to basal conditions (without exosomes). Figure 6C shows the % of healed areas for basal conditions,

2μg/ml exosomes, 6 μg/ml exosomes, 20 μg/ml exosomes and an LSGS (low serum growth supplement) positive control. The top panel of Figure 6C shows exosomes isolated from CTX0E03 cells cultured for 2 weeks in the Integra Celline system and the bottom panel of Figure 6C shows exosomes isolated from CTX0E03 cells cultured for 6 weeks in the Integra Celline system. Figure 6D compares CTX0E03 cells to a negative control (saline) in an *in vivo* injection wound healing assay.

Figure 7 shows the quantity of purified exosomes obtained per culture medium from standard CTX0E03 (T175) cultures vs the Integra CELLine system at the 3 week time point.

Figure 8A shows the concentration of exosomes harvested from two different flasks after 1 week, 2 weeks and 3 weeks of CTX0E03 Integra CELLine culture system. Figure 8B shows the concentration of exosomes harvested from a single Integra CELLine flask during a 6 week continuous culture of CTX0E03 cells.

Figure 9 shows the fold change of expression levels of various mRNA markers measured in CTX0E03 cells cultured for 3 weeks in the Integra CELLine system compared to standard ("control") CTX0E03 (T175) cultures.

Figure 10 shows the fold up and down regulation of various miRNAs in exosomes obtained from CTX0E03 cells cultured for 3 weeks in Integra bioreactor culture and microparticles obtained from standard CTX0E03 (T175) cultures, assessed against a baseline expression level in CTX0E03 cells in standard (T175) culture.

Figure 11 depicts the miRNA profiles obtained from deep sequencing of miRNA from CTX0E03 cells ("CTX"), microvesicles ("MV") and exosomes ("EXO") cultured under standard (T175) conditions. Figure 11a and 11b show results from two cultures.

Figure 12 shows the effect of hNSC microvesicles on angiogenesis of HUVECs. Figure 12A is a photograph showing the clear increase in tube formation observed when microvesicles are added (right hand panels) compared to basal HUVECs. Figures 12B and 12C show the increase in total tube length provided by the hNSC microvesicles at various concentrations (0.05μg, 0.1 μg, 0.3μg - Figure 12B; and 0.6μg/ml - Figure 12C).

Figure 13 shows the effect of hNSC microvesicles on neurite outgrowth in PC-12 cells.

Figure 14 is an electropherogram showing the total RNA content profile in CTX0E03 cells, exosomes and microvesicles as determined by Agilent RNA bioanalyser.

Figure 15 is a schematic presentation of the percentage of coding genes fully overlapping exon, and non-coding transcripts located with intron or intergenic sequences (produced by running NGS BAM files against GENCODE sequence data set).

Figure 16 depicts the top ranking preferentially shuttled novel miRNAs in exosomes and MV compared to CTX0E03 producer cells.

Figure 17 shows the results of NanoSight analysis undertaken to determine the particle size and concentration of CTX0E03 exosomes (Figure 17A) and microvesicles (Figure 17B) cultured in the Integra Celline system for 1, 2, 3, 4, 5 and 6 weeks

Figure 18 shows Venn diagrams comparing the proteomic data from CTX0E03 exosomes and microvesicles (18A and 18B), and comparing neural stem cell exosomes with mesenchymal stem cell exosomes (18C and 18D). Figure 18A illustrates the number of unique proteins within CTX0E03 exosomes and microvesicles, isolated from week 2 Integra culture system. Figure 18B compares the biological processes associated with the identified proteins within the CTX0E03 exosomes and microvesicles. Figure 18C compares the CTX0E03 neural stem cell exosome proteome to a Mesenchymal Stem Cell exosome proteome, and Figure 18D compares the biological processes associated with the identified proteins in the MSC derived exosomes with the neural stem cell derived exosomes.

Figure 19 shows the 30 biological processes found to be associated with NSC derived exosomes and not mesenchymal stem cell exosomes.

## Detailed Description of the Invention

The present inventors have surprisingly identified microparticles in neural stem cells. These microparticles retain some of the functions of the neural stem cells from which they are derived and are typically therapeutically useful for the same treatments as the neural stem cells. The microparticles are advantageous over the corresponding stem cells because they are smaller and less complex, thereby being easier to produce, maintain, store and transport, and have the potential to avoid some of the regulatory issues that surround stem cells. The microparticles can be produced continuously, by isolation from conditioned media, for example in a bioreactor such as a multi-compartment bioreactor, which allows for large scale production and the provision of an "off-the-shelf" therapy. The multi-compartment bioreactor is typically a two-compartment bioreactor.

It has further been found that, surprisingly, culturing stem cells (of any type, not limited to neural stem cells) in an environment that allows the stem cells to begin to differentiate, increases dramatically the yield of microparticles produced.

The inventors have surprisingly observed that culturing stem cells (of any type, not limited to neural stem cells) in a multi-compartment bioreactor, results in partial differentiation of the stem cells, into stem cells in a more differentiated form. This differentiation in culture does not require the addition of an agent to induce differentiation. This differentiation typically requires a culture period of at least one week, at least two weeks or at least three weeks. The changes to the stem cells that occur in culture in a multi-compartment bioreactor are reflected by the microparticles produced by the cultured stem cells. Therefore, by culturing stem cells in a multi-compartment bioreactor, it is possible to induce differentiation of the cells. Accordingly, microparticles from partially differentiated stem cells can be produced by harvesting microparticles from stem cells cultured in a multi-compartment bioreactor, typically for at least one week, at least two weeks, at least three weeks, at least four weeks, at least five weeks or at least six weeks. Optionally, the NSCs have been cultured for no more than ten weeks. In one embodiment, the invention provides a method of producing microparticles by isolating the microparticles from partially-differentiated neural stem cells.

The inventors have also found that it is possible to induce the secretion of microparticles from stem cells. This finding, which also is not limited to neural stem cells and can be used for the production of microparticles from any stem cell, allows for an improved yield of microparticles to be obtained from a stem cell culture. Several agents have been identified that enhance the secretion of microparticles to different degrees, which has the further advantage of being able to control the amount of microparticles that are secreted. Culturing stem cells under hypoxic conditions also improves microparticle production. Further, it has been found that co-culturing a stem cell with a different cell type, in particular an endothelial cell type can beneficially alter the microparticles that are produced by the stem cell.

In a further embodiment, the invention provides microparticles, typically exosomes, produced by serum-free stem cells. Serum is required for the successful culture of many cell lines, but contains many contaminants including its own exosomes. As described below, the inventors have produced microparticles from stem cells that do not require serum for successful culture.

### Neural Stem Cell Microparticles

The invention provides, in one aspect, microparticles obtainable from a neural stem cell. A neural stem cell microparticle is a microparticle that is produced by a neural stem cell. Typically, the microparticle is secreted by the neural stem cell. More typically, the microparticle is an exosome or a microvesicle. Microparticles from other cells, such as mesenchymal stem cells, are known in the art.

A "microparticle" is an extracellular vesicle of 30 to 1000 nm diameter that is released from a cell. It is limited by a lipid bilayer that encloses biological molecules. The term "microparticle" is known in the art and encompasses a number of different species of microparticle, including a membrane particle, membrane vesicle, microvesicle, exosome-like vesicle, exosome, ectosome-like vesicle, ectosome or exovesicle. The different types of microparticle are distinguished based on diameter, subcellular origin, their density in sucrose, shape, sedimentation rate, lipid composition, protein markers and mode of secretion (i.e. following a signal (inducible) or spontaneously (constitutive)). Four of the common microparticles and their distinguishing features are described in Table 1, below.

*Table 1: Various Microparticles*

| Microparticle | Size | Shape | Markers | Lipids | Origin |
|---|---|---|---|---|---|
| **Microvesicles** | 100-1000nm | Irregular | Integrins, selectins, CD40 ligand | Phosphatidylserine | Plasma membrane |

(continued)

| Microparticle | Size | Shape | Markers | Lipids | Origin |
|---|---|---|---|---|---|
| **Exosome-like vesicles** | 20-50nm | Irregular | TNFRI | No lipid rafts | MVB from other organelles |
| **Exosomes** | 30-100nm; (<200nm) | Cup shaped | Tetraspanins (e.g. CD63, CD9) Alix, TSG101, ESCRT | Cholesterol, sphingomyelin, ceramide, lipid rafts, phosphatidylserine | Multivesicular endosomes |
| **Membrane particles** | 50-80nm | Round | CD133, no CD63 | Unknown | Plasma membrane |

Microparticles are thought to play a role in intercellular communication by acting as vehicles between a donor and recipient cell through direct and indirect mechanisms. Direct mechanisms include the uptake of the microparticle and its donor cell-derived components (such as proteins, lipids or nucleic acids) by the recipient cell, the components having a biological activity in the recipient cell. Indirect mechanisms include microvesicle-recipient cell surface interaction, and causing modulation of intracellular signalling of the recipient cell. Hence, microparticles may mediate the acquisition of one or more donor cell-derived properties by the recipient cell. It has been observed that, despite the efficacy of stem cell therapies in animal models, the stem cells do not appear to engraft into the host. Accordingly, the mechanism by which stem cell therapies are effective is not clear. Without wishing to be bound by theory, the inventors believe that the microparticles secreted by neural stem cells play a role in the therapeutic utility of these cells and are therefore therapeutically useful themselves.

The microparticles and stem cells of the invention are isolated. The term "isolated" indicates that the microparticle, microparticle population, cell or cell population to which it refers is not within its natural environment. The microparticle, microparticle population, cell or cell population has been substantially separated from surrounding tissue. In some embodiments, the microparticle, microparticle population, cell or cell population is substantially separated from surrounding tissue if the sample contains at least about 75%, in some embodiments at least about 85%, in some embodiments at least about 90%, and in some embodiments at least about 95% microparticles and/or stem cells. In other words, the sample is substantially separated from the surrounding tissue if the sample contains less than about 25%, in some embodiments less than about 15%, and in some embodiments less than about 5% of materials other than the microparticles and/or stem cells. Such percentage values refer to percentage by weight. The term encompasses cells or microparticles which have been removed from the organism from which they originated, and exist in culture. The term also encompasses cells or microparticles which have been removed from the organism from which they originated, and subsequently re-inserted into an organism. The organism which contains the re-inserted cells may be the same organism from which the cells were removed, or it may be a different organism.

Neural stem cells naturally produce microparticles by a variety of mechanisms, including budding of the plasma membrane (to form membrane vesicles and microvesicles) and as a result of the fusion of intracellular multivesicular bodies (which contain microparticles) with the cell membrane and the release of the microparticles into the extracellular compartment (to secrete exosomes and exosome-like vesicles).

The neural stem cell that produces the microparticles of the invention can be a fetal, an embryonic, or an adult neural stem cell, such as has been described in US5851832, US6777233, US6468794, US5753506 and WO-A-2005121318. The fetal tissue may be human fetal cortex tissue. The cells can be selected as neural stem cells from the differentiation of induced pluripotent stem (iPS) cells, as has been described by Yuan et al. (2011) or a directly induced neural stem cell produced from somatic cells such as fibroblasts (for example by constitutively inducing Sox2, Klf4, and c-Myc while strictly limiting Oct4 activity to the initial phase of reprogramming as recently by Their *et al,* 2012). Human embryonic stem cells may be obtained by methods that preserve the viability of the donor embryo, as is known in the art (e.g. Klimanskaya *et al.,* 2006, and Chung *et al.* 2008). Such non-destructive methods of obtaining human embryonic stem cell may be used to provide embryonic stem cells from which microparticles of the invention can be obtained. Alternatively, microparticles of the invention can be obtained from adult stem cells, iPS cells or directly-induced neural stem cells. Accordingly, microparticles of the invention can be produced by multiple methods that do not require the destruction of a human embryo or the use of a human embryo as a base material.

Typically, the neural stem cell population from which the microparticles are produced, is substantially pure. The term "substantially pure" as used herein, refers to a population of stem cells that is at least about 75%, in some embodiments at least about 85%, in some embodiments at least about 90%, and in some embodiments at least about 95% pure, with respect to other cells that make up a total cell population. For example, with respect to neural stem cell populations, this term means that there are at least about 75%, in some embodiments at least about 85%, in some embodiments at least about 90%, and in some embodiments at least about 95% pure, neural stem cells compared to other cells that make up a total cell population. In other words, the term "substantially pure" refers to a population of stem cells of the present invention that contain fewer than about 25%, in some embodiments fewer than about 15%, and in some embodiments fewer than about 5%, of lineage committed cells in the original unamplified and isolated population prior to subsequent culturing and amplification.

A neural stem cell microparticle comprises at least one lipid bilayer which typically encloses a milieu comprising lipids, proteins and nucleic acids. The nucleic acids may be deoxyribonucleic acid (DNA) and/or ribonucleic acid (RNA). RNA may be messenger RNA (mRNA), micro RNA (miRNA) or any miRNA precursors, such as pri-miRNA, pre-miRNA, and/or small nuclear RNA (snRNA).

A neural stem cell microparticle retains at least one biological function of the stem cell from which it is derived. Biological functions that may be retained include the ability to promote angiogenesis and/or neurogenesis, the ability to effect cognitive improvement in the brain of a patient that has suffered a stroke, or the ability to accelerate blood flow recovery in peripheral arterial disease. For example, CTX0E03 cells are known to inhibit T cell activation in a PBMC assay and, in one embodiment, the microparticles of the invention retain this ability to inhibit T cell activation in a PBMC assay. PBMC assays are well-known to the skilled person and kits for performing the assay are commercially available.

Example 8, Table 2 and Figure 6 demonstrate that CTX0E03 stem cell exosomes retain the ability to close a wound in a "scratch" model of wound healing. The results in Figure 6A show that the migration activity of normal human dermal fibroblasts (NHDF) cultured in CTX0E03 conditioned media is almost the same as the migration activity observed on the addition of purified exosomes. Accordingly, one biological function that microparticles of the invention may retain is the ability to stimulate migration activity of normal human dermal fibroblasts (NHDF).

Example 8 also shows that microvesicles of the invention are able to stimulate angiogenesis of primary HUVECs and to stimulate neurite outgrowth of PC-12 cells. Accordingly, a biological function that microparticles of the invention may retain is the ability to stimulate angiogenesis of primary HUVECs and/or to stimulate neurite outgrowth of PC-12 cells.

The proteomic analysis in Example 13 indicates that neural stem cell exosomes comprise biological functions associated with the production, packaging, function and degradation of genetic material. Accordingly, in one embodiment, exosomes of the invention retain these functions, typically one or more of RNA polymerase function, RNA degradation function, ribosome function and spliceosome function.

The microparticle obtained from the neural stem cell has a diameter of 1000nm or less. Typically, the microparticle of the invention will have a diameter of 200nm or less, for example 100nm or less. As noted in Table 1 above, microvesicles have a diameter of 100nm to 1000nm. Exosomes are typically defined as having a diameter of 30-100nm, but more recent studies confirm that exosomes can also have a diameter between 100nm and 200nm, (e.g. Katsuda *et al*, Proteomics 2013 and Katsuda *et al,* Scientific Reports 2013). Accordingly, exosomes typically have a diameter between 30nm and 150nm. Membrane particles have a diameter of 50nm to 80nm and exosome-like particles have a diameter of 20nm-50nm. The diameter can be determined by any suitable technique, for example electron microscopy or dynamic light scattering. The term microparticle includes, but is not limited to: membrane particle, membrane vesicle, microvesicle, exosome-like vesicle, exosome, ectosome-like vesicle, ectosome or exovesicle.

Figure 1 panels A-E show the presence in neural stem cells of MVB's containing exosomes between 30-50nm in diameter, while panel F shows microvesicles >100nm in diameter. Table 20 and Figure 17 (below) show that typical neural stem cell exosomes were measured to have a diameter ranging from approximately 70nm to approximately 150nm, which is consistent with the size of exosomes (from mesenchymal stem cells) described in the art. Accordingly, exosomes of the invention typically have a diameter between 30nm and 200nm, more typically between 50nm and 150nm. As noted above, exosomes are typically positive for the Alix marker (UNIPROT Accession No. Q8WUM4).

Figure 1F and Table 20 shows the observed size of typical neural stem cell microvesicles, with a mode diameter of approximately 150nm - 200nm, or a median diameter of approximately 180nm - 350nm. Accordingly, microvesicles of the invention typically have a diameter between 100 and 1000nm, more typically between 150nm and 350nm.

Some microparticles of the invention express the CD133 surface marker. Other microparticles of the invention do not express the CD133 surface marker.

"Marker" refers to a biological molecule whose presence, concentration, activity, or phosphorylation state may be detected and used to identify the phenotype of a cell.

Exosomes are endosome-derived lipid microparticles of typically 30-100nm diameter and sometimes between 100nm and 200nm diameter, that are released from the cell by exocytosis. Exosome release occurs constitutively or upon induction, in a regulated and functionally relevant manner. During their biogenesis, exosomes incorporate a wide range of cytosolic proteins (including chaperone proteins, integrins, cytoskeletal proteins and the tetraspanins) and genetic material. Consequently, exosomes are considered to be inter-cellular communication devices for the transfer of proteins, lipids and genetic material between cells, in the parent cell microenvironment and over considerable distance. Although the invention is not bound by this theory, it is possible that the exosomes are responsible for the efficacy of the neural stem cells. Therefore, exosomes from neural stem cells are themselves expected to be therapeutically efficacious.

*Microparticles designed to have desired functions*

Microparticles retain at least some of the functions of the stem cells that produce them. Therefore, it is possible to design microparticles by manipulating the stem cell (which can be any stem cell type and is not limited to neural stem cells, although the neural stem cell microparticles of the invention are expressly included as an embodiment) to possess one or more desired functions, typically protein or miRNA. The manipulation will typically be genetic engineering, to introduce one or more exogenous coding, non-coding or regulatory nucleic acid sequences into the stem cell. For example, if an exosome containing VEGF and/or bFGF is desired, then the exosome-producing stem cell can be transformed or transfected to express (high levels of) VEGF and/or bFGF, which would then be incorporated into the microparticles produced by that stem cell. Similarly, iPS cells can be used to produce microparticles, and these cells can be designed to produce the proteins and nucleic acids (e.g. miRNA) that are required in the microparticles produced by the iPS cells. The invention therefore provides ad hoc microparticles, from any stem cell type, that contain a function that is not naturally present in the stem cell from which is produced, i.e. the microparticles (e.g. exosomes) contain one or more exogenous protein or nucleic acid sequences, are not naturally-occurring and are engineered.

In one embodiment, isolated or purified microparticles are loaded with one or more exogenous nucleic acids, lipids, proteins, drugs or prodrugs which are intended to perform a desired function in a target cell. This does not require manipulation of the stem cell and the exogenous material can optionally be directly added to the microparticles. For example, exogenous nucleic acids can be introduced into the microparticles by electroporation. The microparticles can then be used as vehicles or carriers for the exogenous material. In one embodiment, microparticles that have been isolated from the cells that produced them are loaded with exogenous siRNA, typically by electroporation, to produce microparticles that can be deployed to silence one or more pathological genes. In this way, microparticles can be used as vehicles to deliver one or more agents, typically therapeutic or diagnostic agents, to a target cell. An example of this is a neural stem cell exosome comprising exogenous siRNA capable of silencing one or more pathological genes.

*Microparticle Marker*

The invention provides a population of isolated neural stem cell microparticles, wherein the population essentially comprises only microparticles of the invention, i.e. the microparticle population is pure. In many aspects, the microparticle population comprises at least about 80% (in other aspects at least 85%, 90%, 91%, 92%, 93%, 94%, 95%, 96%, 97%, 98%, 99%, 99.5%, 99.9% or 100%) of the microparticles of the invention.

The isolated neural stem cell microparticle of the invention is characterised in that it has a distinctive expression profile for certain markers and is distinguished from microparticles from other cell types. When a marker is described herein, its presence or absence may be used to distinguish the microparticle. For example, the term "may comprise" or "may express" also discloses the contrary embodiment wherein that marker is not present, e.g. the phrase "the microparticle may comprise one or more tetraspanins, typically CD63, CD81, CD9, CD53, CD82 and/or CD37" also describes the contrary embodiment wherein the microparticle may not comprise one or more tetraspanins, typically CD63, CD81, CD9, CD53, CD82 and/or CD37.

The neural stem cell microparticle of the invention is typically considered to carry a marker if at least about 70% of the microparticles of the population, e.g. 70% of the membrane particles, membrane vesicles, microvesicles, exosome-like vesicles, exosomes, ectosome-like vesicles, ectosomes or exovesicles show a detectable level of the marker. In other aspects, at least about 80%, at least about 90% or at least about 95% or at least about 97% or at least about 98% or more of the population show a detectable level of the marker. In certain aspects, at least about 99% or 100% of the population show detectable level of the markers. Quantification of the marker may be detected through the use of a quantitative RT-PCR (qRT-PCR) or through fluorescence activated cell sorting (FACS). It should be appreciated that this list is provided by way of example only, and is not intended to be limiting. Typically, a neural stem cell microparticle of the invention is considered to carry a marker if at least about 90% of the microparticles of the population show a detectable level of the marker as detected by FACS.

The markers described herein are considered to be expressed by a cell of the population of the invention, if its expression level, measured by qRT-PCR has a crossing point (Cp) value below or equal to 35 (standard cut off on a qRT-PCR array). The Cp represents the point where the amplification curve crosses the detection threshold, and can also be reported as crossing threshold (ct).

In one embodiment, the invention relates to microparticles produced by a neural stem cell population characterised in that the cells of the population express one or more of the markers Nestin, Sox2, GFAP, βIII tubulin, DCX, GALC, TUBB3, GDNF and IDO. In another embodiment, the microparticle is an exosome and the population of exosomes expresses one or more of DCX (doublecortin - an early neuronal marker), GFAP (Glial fibrillary acidic protein - an astrocyte marker), GALC, TUBB3, GDNF and IDO.

The neural stem cell microparticles of the invention may express one or more protein markers at a level which is lower or higher than the level of expression of that marker in a mesenchymal stem cell microparticle of the same species. Protein markers that are expressed by the CTX0E03 cell microparticles are identified herein and below. In some embodiments, the microparticles may express a protein marker at a level relative to $\alpha$ tubulin or other such control protein(s). In some embodiments, the microparticles of the invention may express that protein at a level of at least +/- 1.2 fold change relative to the control protein, typically at least +/-1.5 fold change relative to the control protein, at least +/-2 fold change relative to the control protein or at least +/-3 fold change relative to the control protein. In some embodiments, the microparticles may express a protein marker at a level of between $10^{-2}$ and $10^{-6}$ copies per cell relative to $\alpha$ tubulin or other control protein. In some embodiments, the microparticles of the invention may express that protein at a level of between $10^{-2}$ and $10^{-3}$ copies per cell relative to $\alpha$ tubulin or other control protein.

The neural stem cell microparticles of the invention may express one or more miRNAs (including miRNA precursors) at a level which is lower or higher than the level of expression of that miRNA (including miRNA precursors) in a mesenchymal stem cell microparticle of the same species. miRNA markers that are expressed by the CTX0E03 cell microparticles are identified below. In some embodiments, the microparticles of the invention may express the marker miRNA at a level of least +/- 1.5 fold change, typically at least +/- 2 fold change or at least +/- 3 fold change (calculated according to the $\Delta\Delta$ct method, which is well-known) relative to U6B or 15a, or any other miRNA reference gene, also referred to as an internal control gene.

The neural stem cell microparticles of the invention may express one or more mRNAs at a level which is lower or higher than the level of expression of that mRNA in a mesenchymal stem cell microparticle of the same species. In some embodiments, the microparticles of the invention may express the marker mRNA at a level of least +/- 1.5 fold change, typically at least +/- 2 fold change or at least +/- 3 fold change (calculated according to the $\Delta\Delta$ct method) relative to ATP5B or YWHAZ, or any other reference gene, also referred to as an internal control gene.

Exosomes of the invention typically express specific integrins, tetraspanins, MHC Class I and/or Class II antigens, CD antigens and cell-adhesion molecules on their surfaces, which may facilitate their uptake by specific cell types. Exosomes contain a variety of cytoskeletal proteins, GTPases, clathrin, chaperones, and metabolic enzymes (but mitochondrial, lysosomal and ER proteins are excluded, so the overall profile does not resemble the cytoplasm). They also contain mRNA splicing and translation factors. Finally, exosomes generally contain several proteins such as HSP70, HSP90, and annexins that are known to play signalling roles yet are not secreted by classical (ER-Golgi) mechanisms.

The lipid bilayer of an exosome is typically enriched with cholesterol, sphingomyelin and ceramide. Exosomes also express one or more tetraspanin marker proteins. Tetraspanins include CD81, CD63, CD9, CD53, CD82 and CD37. Exosomes can also include growth factors, cytokines and RNA, in particular miRNA. Exosomes typically express one or more of the markers TSG101, Alix, CD109, thy-1 and CD133. Alix (Uniprot accession No. Q8WUM4), TSG101 (Uniprot accession No. Q99816) and the tetraspanin proteins CD81 (Uniprot accession No. P60033) and CD9 (Uniprot accession No. P21926) are characteristic exosome markers.

Alix is an endosomal pathway marker. Exosomes are endosomal-derived and, accordingly, a microparticle positive for this marker is characterised as an exosome. Exosomes of the invention are typically positive for Alix. Microvesicles of the invention are typically negative for Alix.

*Microparticle proteome*

Tables 18 and 20 list all proteins detected by mass spectrometry in exosomes and microvesicles, respectively, isolated from CTX0E03 cells cultured for two weeks in an Integra Celline multicompartment bioreactor. In one embodiment, exosomes of the invention comprise at least 70%, at least 80%, at least 90%, at least 95%, at least 99% or at least 99.5% of the proteins listed in Table 18. Similarly, microvesicles of the invention typically comprise at least 70% at least 80%, at least 90%, at least 95%, at least 99% or at least 99.5% of the proteins listed in Table 20. In a further embodiment, the proteome of a microvesicle or exosome of the invention is least 70%, at least 80%, at least 90%, at least 95%, at least 99% or at least 99.5% identical to the proteome provided in Table 18 (exosome) or Table 20 (microvesicle). When determining the protein content of a microparticle or exosome, mass spectrometry is typically used, for example the LC/MS/MS method described in Example 13.

Tables 19 and 21 show the 100 most abundant proteins detected by mass spectrometry in exosomes and microvesicles, respectively, isolated from CTX0E03 cells cultured for two weeks in an Integra Celline multicompartment bioreactor. Typically, an exosome of the invention comprises the first ten proteins listed in Table 19, more typically the first 20, the first 30, the first 40 or the first 50 proteins listed in Table 19. Similarly, a microparticle of the invention typically comprises the first ten proteins listed in Table 21, more typically the first 20, the first 30, the first 40 or the first 50 proteins listed in Table 21. In one embodiment, an exosome of the invention comprises all 100 proteins listed in Table 19. In one embodiment, a microvesicle of the invention comprises all 100 proteins listed in Table 21. Typically, the 100 most abundant proteins in an exosome or microvesicle of the invention contain at least 70 of the proteins identified in Table 19 (exosome) or Table 21 (microparticle). More typically, the 100 most abundant proteins in an exosome or microvesicle of the invention contain at least 80, at least 90, at least 95, 96, 97, 98 or 99, or all 100 of the proteins identified in Table 19 (exosome) or Table 21 (microparticle).

*Microparticle miRNA content*

Example 12 (and the related Figure 11) shows the results of deep sequencing of miRNA present in CTX0E03 cells, microvesicles and exosomes produced by these cells. This Example shows that, surprisingly, the number of different miRNA species present in the microparticles is greatly reduced compared to the number of different miRNA species present in the cells; the microparticles contain fewer than 120 different miRNAs whereas the cells contain between 450 and 700 miRNA species. The microparticles contain a majority of hsa-miR-1246.

The data in Example 12 also show that the microparticles are characterised by four main miRNA species, namely hsa-miR-1246, hsa-miR-4492, hsa-miR-4488 and hsa-miR-4532. These four miRNAs are the only miRNAs present at a read count of greater than 1000 in the microparticles; these four miRNAs are present in massive excess compared to the other miRNAs in the microparticles. This is in contrast to the profile in the cells, which contain a much greater number of miRNAs present at high (read count greater than 1000) or very high (read count greater than 10,000) levels. Although not bound by theory, the inventors propose that hsa-miR-1246, hsa-miR-4492, hsa-miR-4488 and hsa-miR-4532 are selectively trafficked (or otherwise incorporated) into the microparticles and are thought to play a role in the function of the microparticles.

Typically, in one embodiment microparticles, e.g. exosomes, of the invention contain one, two, three or all four of hsa-miR-1246, hsa-miR-4492, hsa-miR-4488 and hsa-miR-4532. Each of these miRNA markers is typically present at a read count (optionally determined using the deep sequence technique described in Example 12) of at least 1000 per microparticle. hsa-miR-1246 may optionally have a read count of at least 2000, 5000, 10,000, 20,000, or 25,000 per microparticle. Hsa-miR-4492 may optionally have a read count of at least 2000, 3000, 4000 or 5000 per microparticle. Hsa-miR-4532 may optionally have a read count of at least 2000 or 3000 per microparticle.

In one embodiment, each of hsa-miR-1246, hsa-miR-4492, hsa-miR-4488 and/or hsa-miR-4532 is present in the microparticle, e.g. exosome, at a higher read count than is present in the cell that produced the microparticle. In particular, miR-1246 typically has a read count in the microparticle at least twice the read count in the cell, more typically at least 4, 5, 6, 7, or 8 times the read count in the cell, and optionally 10, 15 or 20 times the read count in the cell.

In one embodiment, microparticles of the invention contain hsa-let-7a-5p, has-miR-92b-3p, hsa-miR-21-5p, hsa-miR-92a-3p, hsa-miR-10a-5p, hsa-100-5p and/or hsa-99b-5p at a lower read count than is present in the cell that produced the microparticle. Typically, each of these miRNAs has a read count of less than 1000 in the microparticles of the invention, more typically less than 100, for example less than 50. Optionally, microparticles of the invention contain hsa-let-7a-5p at a read count of less than 50 or less than 25.

In one embodiment, microparticles of the invention contain fewer than 150 types of miRNA (i.e. different miRNA species) when analysed by deep sequencing, typically fewer than 120 types of miRNA.

In one embodiment, hsa-miR-1246 is the most abundant miRNA in the microparticles of the invention (optionally determined using the deep sequence technique described in Example 12). Typically, at least 40% of the total count of miRNA in microparticles (e.g. microvesicles and exosomes) of the invention is hsa-miR-1246. Typically, at least 50% of the total count of miRNA in exosomes of the invention is hsa-miR-1246.

hsa-miR-4492 is typically the second-most abundant miRNA in the microparticles of the invention. Typically, at least 3% of the total count of miRNA in microparticles (e.g. microvesicles and exosomes) of the invention is hsa-miR-4492. More typically, at least 4% of the total count of miRNA in microparticles (e.g. microvesicles and exosomes) of the invention is hsa-miR-4492.

Typically, at least 2% of the total count of miRNA in microparticles (e.g. microvesicles and exosomes) of the invention is hsa-miR-4532.

Typically, at least 1% of the total count of miRNA in microparticles (e.g. microvesicles and exosomes) of the invention is hsa-miR-4488.

In one embodiment microparticles of the invention contain one or both of hsa-miR-4508, hsa-miR-4516 at a level at least 0.1% of the total miRNA content of the particle.

One or more of hsa-miR-3676-5p, hsa-miR-4485, hsa-miR-4497, hsa-miR-21-5p, hsa-miR-3195, hsa-miR-3648, hsa-miR-663b, hsa-miR-3656, hsa-miR-3687, hsa-miR-4466, hsa-miR-4792, hsa-miR-99b-5p and hsa-miR-1973 may be present in the microparticles of the invention.

Typically, each of hsa-let-7a-5p and hsa-100-5p is present at less than 1%, more typically less than 0.1% or less than 0.05% of the total miRNA count in microparticles of the invention.

In a typical exosome of the invention, at least 50% of the total count of miRNA is hsa-miR-1246, and less than 0.1% of the total miRNA count is hsa-let-7a-5p.

In one embodiment, at least 90% of the total count of miRNA in microparticles of the invention comprises hsa-miR-1246, hsa-miR-4492, hsa-miR-4488 and hsa-miR-4532. Typically, at least 95% or 96% of the total count of miRNA in microparticles of the invention comprises hsa-miR-1246, hsa-miR-4492, hsa-miR-4488 and hsa-miR-4532. Less than 10% of the total miRNA content of these microparticles is an miRNA that is not hsa-miR-1246, hsa-miR-4492, hsa-miR-4488 and hsa-miR-4532.

Combinations of the miRNA embodiments discussed above are provided. For example, a microparticle of the invention typically contains each of hsa-miR-1246, hsa-miR-4492, hsa-miR-4488 and hsa-miR-4532 at a read count of at least 1000 and contains each of hsa-let-7a-5p, hsa-miR-92b-3p, hsa-miR-21-5p, hsa-miR-92a-3p, hsa-miR-10a-5p, hsa-100-5p and hsa-99b-5p at a read count of less than 100. Typically, at least 90% or at least 95% of the total miRNA in these microparticles is hsa-miR-1246, hsa-miR-4492, hsa-miR-4488 and hsa-miR-4532.

A microparticle (e.g. microvesicle or exosome) of the invention typically has hsa-miR-1246 as the most abundant miRNA and hsa-miR-4492 is the second-most abundant miRNA. In this embodiment, at least 40% of the total count of miRNA in microparticles (e.g. microvesicles and exosomes) of the invention is hsa-miR-1246 and at least 3% of the total count of miRNA in the microparticle is hsa-miR-4492. At least 2% of the total count of miRNA in these microparticles is hsa-miR-4532 and at least 1% of the total count of miRNA in these microparticles is hsa-miR-4488. Each of hsa-let-7a-5p and hsa-100-5p is present at less than 0.1% of the total miRNA count in these microparticles.

Plotting the deep sequencing results in the exosomes and microvesicles as relative fold change compared to the cells confirms that hsa-miR-1246, hsa-miR-4492, hsa-miR-4488 and hsa-miR-4532 are significantly upregulated in the exosomes and microvesicles compared to the cells. This comparison also shows that miRNA hsa-miR-3195 is the miRNA that is most upregulated, in both exosomes and microvesicles. Although the absolute reads of hsa-miR-3195 are in the range of -40 for exosomes and microvesicles, there is no hsa-miR-3195 detected in the cells. Accordingly, hsa-miR-3195 is uniquely found in the exosomes and microvesicles of the invention and, in one embodiment, an exosome or microvesicle of the invention comprises hsa-miR-3195.

In one embodiment, microparticles of the invention comprise one or more of the following miRNA precursors:

AC079949.1 (SEQ ID NO:738)
GGCCGCGCCCCGTTTCCCAGGACAAAGGGCACTCCGCACCGGACCCTGGTCCCAGCG;

AP000318.1 (SEQ ID NO: 739)
CCCACTCCCTGGCGCCGCTTGTGGAGGGCCCAAGTCCTTCTGATTGAGGCCCAACCCGTGGAAG;

AL161626.1 (SEQ ID NO:740)
CGCCGGGACCGGGGTCCGGGGCGGAGTGCCCTTCCTCCTGGGAAACGGGGTGCGGC;

AC004943.1 (SEQ ID NO:741)

GCTTCACGTCCCCACCGGCGGCGGCGGCGGTGGCAGTGGCGGCGGCGGCGGTGGCGGCGGCGGCGGCGGCG
GCTC;

and
AL121897.1 (SEQ ID NO:742)

GCCGCCCCCGCCGCCGCCGCCGCCGCCGCCGCCGCCGCCGCCCGCTTTCGGCTCGGGCCTCAGGTGAGTCGGAG
GGGCCGGGCGCC

In one embodiment, microparticles of the invention comprise one, two or three of the following mature miRNAs derived from the precursors listed above (as detailed in part D of Example 12):

ggcggagugcccuucuuccugg (derived from AL161626.1-201) (SEQ ID NO:743)
ggagggcccaaguccuucugau (derived from AP000318.1-201) (SEQ ID NO:744)
gaccaggguccggugcggagug (derived from AC079949.1-201) (SEQ ID NO:745)

These 5 miRNA precursors and 3 mature miRNAs have not previously been isolated and each sequence is therefore also provided as a new sequence *per se.* Accordingly, in one aspect, the invention provides a composition comprising one or more of the miRNA precursors AC079949.1, AP000318.1, AL161626.1, AC004943.1 and AL121897.1. In another embodiment, the invention provides a composition comprising one or more of the mature miRNAs ggcggagugcccuucuuccugg (derived from AL161626.1-201), ggagggcccaaguccuucugau (derived from AP000318.1-201) and gaccaggguccggugcggagug (derived from AC079949.1-201). Optionally, the composition is a pharmaceutical composition comprising one or more of the miRNA precursors and/or one or more of the mature miRNAs and a pharmaceutically-acceptable carrier or diluent. As noted in Example 12, these miRNAs and precursors appear to be selectively shuttled into the exosomes and microvesicles and so may be at least partially responsible for the function of the microparticles.

Example 12 also shows that neural stem cell microparticles comprise a variety of non-coding RNA species. In one embodiment, microparticles of the invention comprise one or more of ribosomal RNA, small nucleolar RNA, small nuclear RNA, microRNA, large intergenic non-coding RNA and miscellaneous other RNA (e.g. RMRP, vault RNA, metazoan SRP and/or RNY).

Example 4 shows miRNAs present in microparticles produced by the CTX0E03 cells and having a Cp below 35 as determined by a qRT-PCR array. Typically, in one embodiment microparticles of the invention contain 1, 2, 3, 4, 5, 6, 7, 8, 9, 10, 15, 20, 25, 30, 35, 40, 45, 50, 60 or more, or all, of the following miRNAs (identified according by name according to Ambros *et al* and accessible at www.mirbase.org):

| |
|---|
| hsa-let-7a |
| hsa-let-7b |
| hsa-let-7c |
| hsa-let-7d |
| hsa-let-7e |
| hsa-let-7f |
| hsa-let-7g |
| hsa-let-7i |
| hsa-miR-100 |
| hsa-miR-101 |
| hsa-miR-103a |
| hsa-miR-106b |
| hsa-miR-10a |
| hsa-miR-10b |
| hsa-miR-124 |
| hsa-miR-125a-5p |
| hsa-miR-125b |
| hsa-miR-126 |
| hsa-miR-127-5p |
| hsa-miR-128 |
| hsa-miR-129-5p |
| hsa-miR-130a |
| hsa-miR-132 |
| hsa-miR-134 |
| hsa-miR-137 |
| hsa-miR-141 |
| hsa-miR-146b-5p |
| hsa-miR-150 |
| hsa-miR-155 |
| hsa-miR-15a |
| hsa-miR-15b |

(continued)

| |
|---|
| hsa-miR-16 |
| hsa-miR-17 |
| hsa-miR-181 a |
| hsa-miR-182 |
| hsa-miR-183 |
| hsa-miR-185 |
| hsa-miR-18a |
| hsa-miR-18b |
| hsa-miR-192 |
| hsa-miR-194 |
| hsa-miR-195 |
| hsa-miR-196a |
| hsa-miR-205 |
| hsa-miR-20a |
| hsa-miR-20b |
| hsa-miR-21 |
| hsa-miR-210 |
| hsa-miR-214 |
| hsa-miR-218 |
| hsa-miR-219-5p |
| hsa-miR-22 |
| hsa-miR-222 |
| hsa-miR-23b |
| hsa-miR-24 |
| hsa-miR-26a |
| hsa-miR-301 a |
| hsa-miR-302a |
| hsa-miR-302c |
| hsa-miR-33a |
| hsa-miR-345 |
| hsa-miR-375 |
| hsa-miR-378 |
| hsa-miR-424 |
| hsa-miR-7 |
| hsa-miR-9 |
| hsa-miR-92a |
| hsa-miR-93 |
| hsa-miR-96 |
| hsa-miR-99a |

In one embodiment, the CTX0E03 microparticles contain 1, 2, 3, 4, 5, 6, 7, 8, 9, 10, 15, 20, 25, 30 or more of the following miRNAs (which are selected from the list above):

| |
|---|
| hsa-let-7g |
| hsa-miR-101 |
| hsa-miR-10a |
| hsa-miR-10b |
| hsa-miR-126 |
| hsa-miR-128 |
| hsa-miR-129-5p |
| hsa-miR-130a |
| hsa-miR-134 |
| hsa-miR-137 |
| hsa-miR-155 |
| hsa-miR-15a |
| hsa-miR-15b |
| hsa-miR-16 |
| hsa-miR-17 |
| hsa-miR-182 |
| hsa-miR-183 |
| hsa-miR-185 |
| hsa-miR-18b |
| hsa-miR-192 |
| hsa-miR-194 |
| hsa-miR-195 |
| hsa-miR-20a |
| hsa-miR-20b |
| hsa-miR-210 |
| hsa-miR-218 |
| hsa-miR-301 a |
| hsa-miR-302a |
| hsa-miR-302c |
| hsa-miR-345 |
| hsa-miR-375 |
| hsa-miR-378 |
| hsa-miR-7 |
| hsa-miR-9 |
| hsa-miR-93 |
| hsa-miR-96 |
| hsa-miR-99a |

*Proteins detected by a dot-blot*

Example 5 shows proteins present in microparticles produced by the CTX0E03 cells, as detected by a dot-blot. Typically, microparticles of the invention contain 1, 2, 3, 4, 5, 6, 7, 8, 9, 10 or all of the following proteins:

| |
|---|
| EDA-A2 |
| Galectin-3 |
| IGFBP-2 |
| IGFBP-rp1/IGFBP-7 |
| IL-1a |
| LECT2 |
| MCP-1 |
| SPARC |
| TIMP-1 |
| Thrombospondin-1 |
| VEGF |

Galectin-3 and Thrombospondin-1 are also identified as present in exosomes and microvesicles in Example 13. TIMP-1 is identified in Example 13 as being present in exosomes.

Example 5 also shows that the microparticles produced by the CTX0E03 cells may also express 1, 2, 3, 4 or 5 of the following proteins:

| |
|---|
| EGF-R/ErbB1 |
| MDC |
| Endostatin |
| Follistatin |
| Csk |

EGF-R and Csk are also identified as present in exosomes and microvesicles in Example 13.

Galectin-3, SPARC, TIMP-1, Thrombospondin-1, VEGF, MDC and Endostatin are known to be modulate angiogenesis. Accordingly, microparticles containing one or more of these proteins are useful in treating diseases or disorders requiring modulation of angiogenesis.

IL-1a, LECT2, MCP-1 and Csk are known to modulate inflammation. Accordingly, microparticles containing one or more of these proteins are useful in treating diseases or disorders requiring modulation of inflammation.

Microparticles containing one or more of (i) Galectin-3, SPARC, TIMP-1, Thrombospondin-1, VEGF, MDC and Endostatin, and one or more of (ii) IL-1a, LECT2, MCP-1 and Csk, may be useful for treating diseases or disorders requiring modulation of angiogenesis and inflammation.

*Neural Stem cells in multi-compartment bioreactor culture*

As shown in Example 10 and Figure 9 below, after multi-compartment bioreactor culture for three weeks, neural stem cells express a number of markers at significantly higher levels than neural stem cells cultured according to standard procedure in a standard single-compartment T175 flask. In one embodiment, microparticles of the invention are isolated from NSCs that have been cultured, typically in a multi-compartment bioreactor, for at least two weeks, typically at least three weeks, at least four weeks, at least five weeks or at least six weeks. Optionally, the NSCs have been cultured for no more than ten weeks, e.g. between 2 and 10 weeks, between 3 and 10 weeks, between 4 and 10 weeks, between 5 and 10 weeks or between 6 and 10 weeks.

CTX0E03 neural stem cells cultured for three weeks in a multi-compartment bioreactor express DCX, GALC, GFAP, TUBB3, GDNF and IDO at a higher level than neural stem cells cultured in a standard single-compartment T175 cell culture. Accordingly neural stem cells that have been cultured in a multi-compartment bioreactor, typically for a week or more, ten days or more, two weeks or more, or at least three weeks, four weeks, five weeks or more, may express one or more of DCX, GALC, GFAP, TUBB3, GDNF and IDO. Cells cultured in a two-compartment bioreactor typically show increased expression of one or more of DCX, GALC, GFAP, TUBB3, GDNF and IDO compared to the stem cells cultured under standard conditions. The expression level of these markers in the multi-compartment bioreactor-cultured cells is typically significantly higher than in the cells cultured in a standard single-compartment T175 culture flask. Typically, a stem cell cultured in a multi-compartment bioreactor expresses one or more of DCX1, GALC, GFAP, TUBB3, GDNF or IDO at a level least 2 fold higher than in CTX0E03 cells cultured in a T-175 flask according to standard culture procedure. In one embodiment, microparticles, typically exosomes, are obtained from neural stem cells that show increased expression of one or more of DCX, GALC, GFAP, TUBB3, GDNF and IDO compared to the stem cells cultured under standard conditions. For example, microparticles can be obtained from freshly filtered conditioned medium collected from Integra CeLLine bioreactor cultured neural stem cells.

The upregulated markers include DCX (doublecortin - an early neuronal marker), GFAP (Glial fibrillary acidic protein - an astrocyte marker), GALC, TUBB3, GDNF and IDO. CTX0E03 cells are able to differentiate into 3 different cell types: neurons, astrocytes and oligodendrocytes. The high levels of DCX and GFAP after three weeks in a multi-compartment bioreactor indicates that the cultured stem cells have partially differentiated and have entered the neuronal (DCX+ cells) and/or astrocytic (GFAP+ cells) lineage. Accordingly, in one embodiment the invention provides a microparticle produced by a neural stem cell population that expresses (i) one or more markers associated with a neuronal lineage, typically DCX and/or (ii) one or more markers associated with an astrocytic lineage, typically GFAP. In another embodiment, the invention provides neural stem cell microparticles, typically exosomes, that express (i) one or more markers associated with a neuronal lineage, typically DCX and/or (ii) one or more markers associated with an astrocytic lineage, typically GFAP. These cells, or the microparticles (typically exosomes) derived from these cells, express DCX and/or GFAP at a higher level than the corresponding stem cells in standard (T-175) culture. Typically, these cells or microparticles express DCX and/or GFAP at a level at least 2 fold more than the stem cells, more typically at least 2.5 fold more than the corresponding stem cells in standard culture, at least 5 fold more than the corresponding stem cells in standard culture, at least 7.5 fold more than the corresponding stem cells in standard culture or at least 10 fold more than the corresponding stem cells in standard culture. For expression of DCX, the fold change in the cells or microparticles compared to the corresponding stem cells in standard (T-175) culture can optionally be at least 20 fold, at least 50 fold, at least 100 fold, at least 500 fold or at least 1000 fold more than the standard stem cells.

The term "bioreactor" is to be given its usual meaning in the art, i.e. an apparatus used to carry out a bioprocess. The bioreactors described herein are suitable for use in stem cell culture. Simple bioreactors for cell culture are single compartment flasks, such as the commonly-used T-175 flask (e.g. the BD Falcon™ 175 $cm^2$ Cell Culture Flask, 750 ml, tissue-culture treated polystyrene, straight neck, blue plug-seal screw cap, BD product code 353028). Bioreactors can have multiple compartments, as is known in the art. These multi-compartment bioreactors typically contain at least two compartments separated by one or more membranes or barriers that separate the compartment containing the cells from one or more compartments containing gas and/or culture medium. Multi-compartment bioreactors are well-known in the art. An example of a multi-compartment bioreactor is the Integra CeLLine bioreactor, which contains a medium compartment and a cell compartment separated by means of a 10 kDa semi-permeable membrane; this membrane allows a continuous diffusion of nutrients into the cell compartment with a concurrent removal of any inhibitory waste product. The individual accessibility of the compartments allows to supply cells with fresh medium without mechanically interfering with the culture. A silicone membrane forms the cell compartment base and provides an optimal oxygen supply and control of carbon dioxide levels by providing a short diffusion pathway to the cell compartment. Any multi-compartment bioreactor may be used according to the invention.

Example 11, Table 3 and Figure 10 show that the miRNA content of exosomes produced by neural stem cells that have been cultured in a multi-compartment bioreactor, for three weeks, is different from the miRNA content of stem cells cultured in standard T-175 flasks and from microparticles produced by the neural stem cells cultured in a single-compartment T175 culture flask for three weeks. In one embodiment, the invention provides a microparticle, typically an exosome, wherein at least two, three, four, five, six or seven miRNAs are up or down regulated compared to in the corresponding stem cells cultured in standard T-175 flasks, as calculated by Fold Regulation (see Example 11). The Fold Regulation of each miRNA is optionally at least two-fold up or down.

It can be seen from Figure 6C and Example 8 that exosomes isolated from NSCs show particularly surprising efficacy when the NSCs have been cultured for several weeks. Accordingly, in one embodiment, exosomes of the invention are isolated from NSCs that have been cultured, typically in a multi-compartment bioreactor, for at least two weeks, typically at least three weeks, at least four weeks, at least five weeks or at least six weeks. Optionally, the NSCs have been cultured for no more than ten weeks, e.g. between 2 and 10 weeks, between 3 and 10 weeks, between 4 and 10 weeks, between 5 and 10 weeks or between 6 and 10 weeks.

In one embodiment, neural stem cell exosomes of the invention express one, two, three, four, five, six or seven of the following miRNAs at a higher level than is expressed in the corresponding stem cells cultured in standard T-175 flasks, as calculated by Fold Regulation (where an asterisk indicates an miRNA where at least a two-fold regulation increase is preferred):

| |
|---|
| hsa-miR-146b-5p* |
| hsa-let-7c* |
| hsa-miR-99a* |
| hsa-miR-132* |
| hsa-miR-378* |
| hsa-miR-181 a* |
| hsa-let-7b* |

In one embodiment, neural stem cell exosomes of the invention express one, two, three, four, five, six, seven, eight, nine, ten or more of the following miRNAs at a lower level than is expressed in the corresponding stem cells cultured in standard T-175 flasks, as calculated by Fold Regulation (where an asterisk indicates an miRNA where at least a two-fold regulation decrease is preferred):

| |
|---|
| hsa-miR-7* |
| hsa-miR-106b* |
| hsa-miR-101* |
| hsa-miR-302a* |
| hsa-miR-301 a* |
| hsa-miR-183* |
| hsa-miR-219-5p* |
| hsa-miR-18a* |
| hsa-miR-15a* |
| hsa-miR-182* |
| hsa-miR-33a* |
| hsa-miR-96* |
| hsa-miR-18b* |

In a further embodiment, NSC exosomes of the invention comprise (i) an increased level of at least one, two, three, four, five, six or seven of the miRNAs indicated above as being increased in exosomes compared to the corresponding cells in standard culture and (ii) a decreased level of at least one, two, three, four, five, six, seven, eight, nine, ten or more or more of the miRNAs indicated above as being decreased in exosomes compared to the corresponding cells in standard culture. For example, a neural stem cell exosome may contain a fold-regulation increase in three or more or more of the miRNAs indicated above as being increased in exosomes compared to the corresponding cells in standard culture and a fold-regulation decrease in three or more of the miRNAs indicated above as being decreased in exosomes compared to the corresponding cells in standard culture. In another exemplary embodiment, a neural stem cell exosome may contain a fold-regulation increase in five or more of the miRNAs indicated above as being increased in exosomes compared to the corresponding cells in standard culture and a fold-regulation decrease in five or more of the miRNAs indicated above as being decreased in exosomes compared to the corresponding cells in standard culture.

The term "expressed" is used to describe the presence of a marker within a cell or microparticle. In order to be considered as being expressed, a marker must be present at a detectable level. By "detectable level" is meant that the marker can be detected using one of the standard laboratory methodologies such as qRT-PCR, or qPCR, blotting, Mass Spectrometry or FACS analysis. A gene is considered to be expressed by a cell or microparticle of the population of the invention if expression can be reasonably detected at a crossing point (cp) values below or equal 35. The terms "express" and "expression" have corresponding meanings. At an expression level below this cp value, a marker is considered not to be expressed. The comparison between the expression level of a marker in a stem cell or microparticle of the invention, and the expression level of the same marker in another cell or microparticle, such as for example an mesenchymal stem cell, may preferably be conducted by comparing the two cell/microparticle types that have been isolated from the same species. Preferably this species is a mammal, and more preferably this species is human. Such comparison may conveniently be conducted using a reverse transcriptase polymerase chain reaction (RT-PCR) experiment.

As used herein, the term "significant expression" or its equivalent terms "positive" and "+" when used in regard to a marker shall be taken to mean that, in a cell or microparticle population, more than 20%, preferably more than, 30%, 40%, 50%, 60%, 70%, 80%, 90% 95%, 98%, 99% or even all of the cells of the cells/microparticles express said marker.

As used herein, "negative" or "-" as used with respect to markers shall be taken to mean that, in a cell or microparticle population, less than 20%, 10%, preferably less than 9%, 8%, 7%, 6%, 5%, 4%, 3%, 2%, 1 % or none of the cells/microparticles express said marker.

Expression of microparticle surface markers may be determined, for example, by means of flow cytometry and/or FACS for a specific cell surface marker using conventional methods and apparatus (for example a Beckman Coulter Epics XL FACS system used with commercially available antibodies and standard protocols known in the art) to determine whether the signal for a specific microparticle surface marker is greater than a background signal. The background signal is defined as the signal intensity generated by a non-specific antibody of the same isotype as the specific antibody used to detect each surface marker. For a marker to be considered positive the specific signal observed is typically more than 20%, preferably stronger than 30%, 40%, 50%, 60%, 70%, 80%, 90%, 100%, 500%, 1000%, 5000%, 10000% or above, greater relative to the background signal intensity. Alternative methods for analysing expression of microparticle surface markers of interest include visual analysis by electron microscopy using antibodies against cell-surface markers of interest.

"Fluorescence activated cell sorting (FACS)" is a method of cell purification based on the use of fluorescent labelled antibodies. The antibodies are directed to a marker on the cell surface, and therefore bind to the cells of interest. The cells are then separated based upon the fluorescent emission peak of the cells.

Microparticle markers (including surface and intracellular proteins) can also be analysed by various methods known to one skilled in the art to assay protein expression, including but not limited to gel electrophoresis followed by western blotting with suitable antibodies, immunoprecipitation followed by electrophoretic analysis, and/or electron microscopy as described above, with microparticle permeabilisation for intraparticle markers. For example, expression of one or more tetraspanins may be assayed using one or more of the above methods or any other method known to one skilled in the art. RNA levels may also be analysed to assess marker expression, for example qRT-PCR.

*Microparticle Function*

As noted above, a neural stem cell microparticle retains at least one biological function of the stem cell from which it is derived. Biological functions that may be retained include the ability to promote angiogenesis, tissue regeneration, tissue repair, and/or neurogenesis, the ability to effect cognitive improvement in the brain of a patient that has suffered a stroke, or the ability to accelerate blood flow recovery in peripheral arterial disease.

For example, CTX0E03 cells are known to inhibit T cell activation in a PBMC assay and, in one embodiment, the microparticles of the invention retain this ability to inhibit T cell activation in a PBMC assay. PBMC assays are well-known to the skilled person and kits for performing the assay are commercially available.

Example 8, Table 2 and Figure 6 demonstrate that CTX0E03 stem cell exosomes retain the ability to close a wound in a "scratch" model of wound healing. The results show that the migration activity of normal human dermal fibroblasts (NHDF) cultured in CTX0E03 conditioned media is almost the same as the migration activity observed on the addition of purified exosomes. Accordingly, one biological function that microparticles of the invention may retain is the ability to stimulate migration activity of normal human dermal fibroblasts (NHDF). NHDF migration assays are known in the art. Stimulation of NHDF migration may be determined using an *in vitro* scratch (wound closure) assay, for example the assay of Example 8(A). Wound closure is calculated as the area covered by NHDF cells in relation to the initial wound area as determined at 0 hours. Stimulation of NHDF migration in this assay is typically defined as an increase in wound closure, typically a wound closure at least 1.2x greater, more typically at least 1.5x greater, than the wound closure under basal conditions (without the microparticles) after 24 hours. After 48 hours, the wound closure is typically at least 1.2x greater or 1.5x greater, more typically at least 2x greater, than the wound closure under basal conditions (without the microparticles). Stimulation of NHDF migration may also be defined as causing a wound closure of 100%, as determined by the scratch assay, at least 24 hours before 100% wound closure is observed under basal conditions.

Example 8 also shows that microvesicles of the invention are able to stimulate angiogenesis of primary HUVECs and to stimulate neurite outgrowth of PC-12 cells. Accordingly, a biological function that microparticles of the invention may retain is the ability to stimulate angiogenesis of primary HUVECs and/or to stimulate neurite outgrowth of PC-12 cells. Angiogenesis and neurite outgrowth assays are known in the art. Stimulation of angiogenesis of primary HUVECs may be determined using a 24 hour angiogenesis assay using an ibidi $\mu$-slide and Wimtube detection and analysis of tube length and bifurcation points, for example the assay of Example 8(B). Stimulation of angiogenesis in this assay is typically defined as an increase compared to basal angiogenesis, e.g. >100% basal angiogenesis, typically at least 110%, at least 120% or at least 140% basal angiogenesis (i.e. at least 1.1x, at least 1.2x or at least 1.4x the basal level of angiogenesis). Stimulation of neurite outgrowth may be determined by detecting outgrowth of PC-12 cells through a 1$\mu$m insert, for example the assay of Example 8(C). Stimulation of neurite outgrowth in this assay is typically defined as an increase in neurite outgrowth compared to basal conditions (without microparticles), or an increase in neurite outgrowth when the microparticle is combined with NGF compared to the addition of NGF alone, as quantified by a spectrophotometer.

The proteomic analysis in Example 13 indicates that neural stem cell exosomes comprise biological functions associated with the production, packaging, function and degradation of genetic material. Accordingly, in one embodiment, exosomes of the invention retain these functions, typically one or more of RNA polymerase function, RNA degradation function, ribosome function and spliceosome function.

*Immunogenicity*

The (allogeneic) neural stem cell microparticles of the invention typically either do not trigger an immune response *in vitro* or *in vivo* or trigger an immune response which is substantially weaker than that which would be expected to be triggered upon injection of an allogeneic stem cell population into a patient. In certain aspects of the invention, the neural stem cell microparticles are considered not to trigger an immune response if at least about 70% of the microparticles do not trigger an immune response. In some embodiments, at least about 80%, at least about 90% or at least about 95%, 99% or more of the microparticles do not trigger an immune response. Preferably the microparticles of the invention do not trigger an antibody mediated immune response or do not trigger a humoral immune response. More preferably the microparticles of the invention do not trigger either an antibody mediated response or a humoral immune response *in vitro.* More preferably still, the microparticles of the invention do not trigger a mixed lymphocyte immune response. It will be understood by one skilled in the art that the ability of the cells of the invention to trigger an immune response can be tested in a variety of ways.

CTX0E03 cells transplanted in a rodent model of limb ischemia have been previously demonstrated a faster and transient up-regulation of host genes involved in angiogenesis, such as CCL11, CCL2, CXCL1, CXCL5, IGF1, IL1$\beta$, IL6, HGF, HIF1$\alpha$, bFGF, VEGFA, and VEGFC, compared to vehicle treated controls. hNSC treatment transiently elevates host innate immune and angiogenic responses and accelerates tissue regeneration.

The CTX0E03 cell line has been previously demonstrated, using a human PBMC assay, not to be immunogenic. Accordingly, microparticles produced by CTX0E03 cells are also expected to be non-immunogenic. The lack of immunogenicity allows the microparticles to avoid clearance by the host/patient immune system and thereby exert their therapeutic effect without a deleterious immune and inflammatory response.

*Neural Stem Cells*

The neural stem cell that produces the microparticle may be a stem cell line, i.e. a culture of stably dividing stem cells. A stem cell line can to be grown in large quantities using a single, defined source. Immortalisation may arise from a spontaneous event or may be achieved by introducing exogenous genetic information into the stem cell which encodes immortalisation factors, resulting in unlimited cell growth of the stem cell under suitable culture conditions. Such exogenous genetic factors may include the gene "myc", which encodes the transcription factor Myc. The exogenous genetic information may be introduced into the stem cell through a variety of suitable means, such as transfection or transduction. For transduction, a genetically engineered viral vehicle may be used, such as one derived from retroviruses, for example lentivirus.

Additional advantages can be gained by using a conditionally immortalised stem cell line, in which the expression of the immortalisation factor can be regulated without adversely affecting the production of therapeutically effective microparticles. This may be achieved by introducing an immortalisation factor which is inactive unless the cell is supplied with an activating agent. Such an immortalisation factor may be a gene such as c-mycER. The c-MycER gene product is a fusion protein comprising a c-Myc variant fused to the ligand-binding domain of a mutant estrogen receptor. C-MycER only drives cell proliferation in the presence of the synthetic steroid 4-hydroxytamoxifen (4-OHT) (Littlewood et al.1995). This approach allows for controlled expansion of neural stem cells *in vitro,* while avoiding undesired *in vivo* effects on host cell proliferation (e.g. tumour formation) due to the presence of c-Myc or the gene encoding it in microparticles derived from the neural stem cell line. A suitable c-mycER conditionally immortalized neural stem cell is described in United States Patent 7416888. The use of a conditionally immortalised neural stem cell line therefore provides an improvement over existing stem cell microparticle isolation and production.

Preferred conditionally-immortalised cell lines include the CTX0E03, STR0C05 and HPC0A07 neural stem cell lines, which have been deposited at the European Collection of Animal Cultures (ECACC), Vaccine Research and Production laboratories, Public Health Laboratory Services, Porton Down, Salisbury, Wiltshire, SP4 0JG, with Accession No. 04091601 (CTX0E03); Accession No.04110301 (STR0C05); and Accession No.04092302 (HPC0A07).

The derivation and provenance of these cells is described in EP1645626 B1. The advantages of these cells are retained by microparticles produced by these cells.

The cells of the CTX0E03 cell line may be cultured in the following culture conditions:

- Human Serum Albumin 0.03%
- Transferrin, Human 5μg/ml
- Putrescine Dihydrochloride 16.2 μg/ml
- Insulin Human recombinant 5 μ/ml
- Progesterone 60 ng/ml
- L-Glutamine 2 mM
- Sodium Selenite (selenium) 40 ng/ml

Plus basic Fibroblast Growth Factor (10 ng/ml), epidermal growth factor (20 ng/ml) and 4-hydroxytamoxifen 100nM for cell expansion. The cells can be differentiated by removal of the 4-hydroxytamoxifen. Typically, the cells can either be cultured at 5% $CO_2$/37°C or under hypoxic conditions of 5%, 4%, 3%, 2% or 1% $O_2$. These cell lines do not require serum to be cultured successfully. Serum is required for the successful culture of many cell lines, but contains many contaminants including its own exosomes. A further advantage of the CTX0E03, STR0C05 or HPC0A07 neural stem cell lines, or any other cell line that does not require serum, is that the contamination by serum is avoided.

The cells of the CTX0E03 cell line (and microparticles derived from these cells) are multipotent cells originally derived from 12 week human fetal cortex. The isolation, manufacture and protocols for the CTX0E03 cell line is described in detail by Sinden, et al. (U.S. Pat. 7,416,888 and EP1645626 B1). The CTX0E03 cells are not "embryonic stem cells", i.e. they are not pluripotent cells derived from the inner cell mass of a blastocyst; isolation of the original cells did not result in the destruction of an embryo.

The CTX0E03 cells (and microparticles derived from these cells) are angiogenic and so are useful in treating diseases requiring angiogenesis, such as Peripheral Arterial Disease. The cells (and microparticles derived from these cells) are also neurogenic and are therefore useful in treating diseases requiring neurogenesis, such as the ischaemia (stroke) damaged brain. CTX0E03 is a clonal cell line that contains a single copy of the c-mycER transgene that was delivered by retroviral infection and is conditionally regulated by 4-OHT (4-hydroxytamoxifen). The C-mycER transgene expresses a fusion protein that stimulates cell proliferation in the presence of 4-OHT and therefore allows controlled expansion when cultured in the presence of 4-OHT. This cell line is clonal, expands rapidly in culture (doubling time 50-60 hours) and has a normal human karyotype (46 XY). It is genetically stable and can be grown in large numbers.

The cells are safe and non-tumorigenic. In the absence of growth factors and 4-OHT, the cells undergo growth arrest and differentiate into neurons and astrocytes. Once implanted into an ischemia-damaged brain, these cells migrate only to areas of tissue damage.

The development of the CTX0E03 cell line has allowed the scale-up of a consistent product for clinical use. Production of cells from banked materials allows for the generation of cells in quantities for commercial application (Hodges et al, 2007).

Pollock *et al* 2006 describes that transplantation of CTX0E03 in a rat model of stroke (MCAo) caused statistically significant improvements in both sensorimotor function and gross motor asymmetry at 6-12 weeks post-grafting. These data indicate that CTX0E03has the appropriate biological and manufacturing characteristics necessary for development as a therapeutic cell line.

Stevanato et al 2009 confirms that CTX0E03 cells downregulated c-mycERTAM transgene expression both *in vitro* following EGF, bFGF and 4-OHT withdrawal and *in vivo* following implantation in MCAo rat brain. The silencing of the c-mycERTAM transgene in vivo provides an additional safety feature of CTX0E03 cells for potential clinical application.

Smith *et al* 2012 describe preclinical efficacy testing of CTX0E03 in a rat model of stroke (transient middle cerebral artery occlusion). The results indicate that CTX0E03 implants robustly recover behavioral dysfunction over a 3 month time frame and that this effect is specific to their site of implantation. Lesion topology is potentially an important factor in the recovery, with a stroke confined to the striatum showing a better outcome compared to a larger area of damage.

Neural retinal stem cell lines (for example as described in US 7514259) may also be used according to the invention.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase to which cells growing on a petri dish or other solid or semisolid support are exposed. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise of four basic chemical groups: amino acids, carbohydrates, inorganic salts, and vitamins. A basal medium generally serves as the basis for a more complex medium, to which supplements such as serum, buffers, growth factors, lipids, and the like are added. In one aspect, the growth medium may be a complex medium with the necessary growth factors to support the growth and expansion of the cells of the invention while maintaining their self-renewal capability. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

## Pharmaceutical Compositions

The neural stem cell microparticle of the invention is useful in therapy and can therefore be formulated as a pharmaceutical composition. A pharmaceutically acceptable composition typically includes at least one pharmaceutically acceptable carrier, diluent, vehicle and/or excipient in addition to the microparticles of the invention. An example of a suitable carrier is Ringer's Lactate solution. A thorough discussion of such components is provided in Gennaro (2000) Remington: The Science and Practice of Pharmacy. 20th edition, ISBN: 0683306472.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The composition, if desired, can also contain minor amounts of pH buffering agents. The carrier may comprise storage media such as Hypothermosol®, commercially available from BioLife Solutions Inc., USA. Examples of suitable pharmaceutical carriers are described in "Remington's Pharmaceutical Sciences" by E W Martin. Such compositions will contain a prophylactically or therapeutically effective amount of a prophylactic or therapeutic microparticle preferably in purified form, together with a suitable amount of carrier so as to provide the form for proper administration to the subject. The formulation should suit the mode of administration. In a preferred embodiment, the pharmaceutical compositions are sterile and in suitable form for administration to a subject, preferably an animal subject, more preferably a mammalian subject, and most preferably a human subject.

The pharmaceutical composition of the invention may be in a variety of forms. These include, for example, semi-solid, and liquid dosage forms, such as lyophilized preparations, liquid solutions or suspensions, injectable and infusible solutions. The pharmaceutical composition is preferably injectable. A particular advantage of the microparticles of the invention is their improved robustness compared to the stem cells from which they are obtained; the microparticles can therefore be subjected to formulation, such as lyophilisation, that would not be suitable for stem cells.

It is preferred that the methods, medicaments and compositions of the invention are used for treating or repairing damaged tissue, and/or for the treatment, modulation, prophylaxis, and/or amelioration of one or more symptoms associated with tissue disorders. Particularly preferred is the use of the methods, medicaments, compositions and microparticles of the invention in regenerative therapy, typically the treatment of stroke, peripheral arterial disease or blindness-causing diseases of the retina.

Pharmaceutical compositions will generally be in aqueous form. Compositions may include a preservative and/or an antioxidant.

To control tonicity, the pharmaceutical composition can comprise a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride and calcium chloride.

Compositions may include one or more buffers. Typical buffers include: a phosphate buffer; a Tris buffer; a borate buffer; a succinate buffer; a histidine buffer; or a citrate buffer. Buffers will typically be included at a concentration in the 5-20mM range. The pH of a composition will generally be between 5 and 8, and more typically between 6 and 8 e.g. between 6.5 and 7.5, or between 7.0 and 7.8.

The composition is preferably sterile. The composition is preferably gluten free. The composition is preferably non-pyrogenic.

In a typical embodiment, the microparticles are suspended in a composition comprising 6-hydroxy-2,5,7,8-tetramethylchroman-2-carboxylic acid (Trolox®), $Na^+$, $K^+$, $Ca^{2+}$, $Mg^{2+}$, $Cl^-$, $H_2P0_4^-$, HEPES, lactobionate, sucrose, mannitol, glucose, dextron-40, adenosine and glutathione. Typically, the composition will not include a dipolar aprotic solvent, e.g. DMSO. Suitable compositions are available commercially, e.g. HypoThermasol®-FRS. Such compositions are advantageous as they allow the microparticles to be stored at 4°C to 25°C for extended periods (hours to days) or preserved at cryothermic temperatures, i.e. temperatures below -20°C. The microparticles may then be administered in this composition after thawing.

The pharmaceutical composition can be administered by any appropriate route, which will be apparent to the skilled person depending on the disease or condition to be treated. Typical routes of administration include intravenous, intra-arterial, intramuscular, subcutaneous, intracranial, intranasal or intraperitoneal. For treatment of a disorder of the brain, one option is to administer the microparticles intra-cerebrally, typically to the site of damage or disease.

The microparticles will be administered at a therapeutically or prophylactically-effective dose, which will be apparent to the skilled person. Due to the low or non-existent immunogenicity of the microparticles, it is possible to administer repeat doses without inducing a deleterious immune response.

Therapeutic uses

The microparticles of the invention are useful in the treatment or prophylaxis of disease. Accordingly, the invention includes a method of treating or preventing a disease or disorder in a patient using a microparticle of the invention. The term "patient" includes human and other mammalian subjects that receive either prophylactic or therapeutic treatment.

As noted above, the compositions comprising miRNAs of the invention are also useful in these therapies, and references to therapeutic uses of microparticles herein therefore applies equally to the compositions comprising miRNAs.

Therapeutically useful microparticles of the invention have regenerative activity. A microparticle having regenerative activity is a microparticle that is capable of activating or enhancing regenerative processes, or inhibiting or reducing degenerative processes. Regenerative processes lead to renewal, restoration, repair and/or growth of cells and tissues. Degenerative processes lead to a loss of cell or tissue integrity and/or function. This may be particularly useful in treating damaged or disturbed cells or tissues, such as those resulting from Stroke, psychiatric disorders, myocardial infarction, Amyotrophic lateral sclerosis and Peripheral arterial disease.

The microparticles of the invention are useful in tissue regeneration. "Tissue regeneration" is the process of increasing the number of cells in a tissue following a trauma. The trauma can be anything which causes the cell number to diminish. For example, an accident, an autoimmune disorder or a disease state could constitute trauma. Tissue regeneration increases the cell number within the tissue and enables connections between cells of the tissue to be reestablished, and the functionality of the tissue to be regained.

The therapy may be regenerative therapy requiring tissue replacement, regeneration or repair. The therapy may be for a neurological disease, disorder or deficit. The therapy may improve functional and/or cognitive recovery. The therapy may be of stroke, peripheral arterial disease, neuropathy or any other disease or disorder that requires tissue regeneration, revascularisation or local anti-inflammatory action, including:

(i) Neurological disorder, disease or deficit, such as Parkinson's disease, Alzheimer's disease, Stroke, or ALS;
(ii) Lysosomal storage disorders;
(iii) Cardiovascular disorders, such as Myocardial Infarction, congestive heart failure, Peripheral Arterial Disease, diabetic ulcers, wound healing;
(iv) Diseases of the lung, including Idiopathic Pulmonary Fibrosis, Respiratory Distress Syndrome, Chronic Obstructive Pulmonary Disease, Idiopathic Pulmonary Hypertension, Cystic Fibrosis and Asthma;
(v) Metabolic or inflammatory disorders, such as Diabetes (I or II), rheumatoid arthritis, osteoarthritis, lupus, Crohn's disease, Inflammatory Bowel Disease, or Graft versus Host Disease;
(vi) Psychiatric disorders, such as Depression, Bipolar disorder, Schizophrenia or an Autistic syndrome disorder such as Autism, Asperger's syndrome or Rett Syndrome;
(vii) Blindness-causing diseases of the retina, such as Age-related macular degeneration, Stargardt disease, diabetic retinopathy, retinitis pigmentosa; and
(viii) Demyelinating diseases, such as multiple sclerosis, cerebral palsy, central pontine myelinolysis, tabes dorsalis, transverse myelitis, Devic's disease, progressive multifocal leukoencephalopathy, optic neuritis, leukodystrophies, Guillain-Barre syndrome, Anti-MAG peripheral neuropathy and Charcot-Marie-Tooth disease.

In one embodiment, the microparticle and compositions containing them are not used for immune modulation.

In one embodiment, the therapy is not related to immunomodulation.

The invention also provides a method for treating or preventing a disease or condition comprising administering an effective amount of the microparticle of the invention, thereby treating or preventing the disease. Typically, the disease or condition is as identified above.

The microparticles of the invention can be used to treat the same diseases as the stem cells from which they are obtained. Neural stem cells are known to be useful in the treatment of diseases including: Stroke, brain damage such as motor, sensory and/or cognitive deficit, psychiatric disorders, myocardial infarction, Amyotrophic lateral sclerosis, limb ischaemia, peripheral arterial disease. Accordingly, the microparticles of the invention are also useful in the treatment of Stroke, brain damage such as motor, sensory and/or cognitive deficit, psychiatric disorders, myocardial infarction, Amyotrophic lateral sclerosis, limb ischaemia, peripheral arterial disease.

Figure 6 and Example 8 demonstrate that exosomes obtained from neural stem cells stimulate wound healing. Accordingly, in one embodiment, exosomes of the invention are used to treat a disease or condition requiring tissue replacement, regeneration or repair. Such conditions include diabetic ulcers and wound healing. Figure 6C shows that exosomes isolated from NSCs cultured for 6 weeks are more efficacious than exosomes isolated from NSCs cultured for 2 weeks. Accordingly, in one embodiment, exosomes isolated from NSCs (typically CTX0E03 cells) that have been cultured (typically in a multi-compartment bioreactor) for at least 2 weeks, more typically at least 4 weeks or at least 6 weeks, are used to treat a disease or condition requiring tissue replacement, regeneration or repair. Optionally, the NSCs have been cultured for no more than ten weeks, e.g. between 2 and 10 weeks, between 3 and 10 weeks, between 4 and 10 weeks, between 5 and 10 weeks or between 6 and 10 weeks.

The observed increased efficacy of exosomes isolated from NSCs (CTX0E03 cells) that have been cultured (in a multi-compartment bioreactor) for 6 weeks correlates with the observed reduction in size of the exosomes to around 70nm diameter, which also occurred after culturing the cells for 6 weeks. Accordingly, in one embodiment, exosomes isolated from NSCs (typically CTX0E03 cells) that have been cultured (typically in a multi-compartment bioreactor) for at least 6 weeks are used to treat a disease or condition requiring tissue replacement, regeneration or repair. As noted above, optionally the NSCs have been cultured for no more than ten weeks, e.g. between 6 and 10 weeks. In another embodiment, exosomes isolated from NSCs (typically CTX0E03 cells) having a diameter less than 100nm, typically less than 80nm, for example around 70nm diameter, are used to treat a disease or condition requiring tissue replacement, regeneration or repair.

As shown in Figure 12 and discussed in Example 8, microvesicles obtained from neural stem cells stimulate angiogenesis. Accordingly, in one embodiment, microvesicles of the invention are used to treat a disease or condition requiring angiogenesis, typically a disease or disorder that is treated by tissue regeneration and/or revascularisation. Microvesicles of the invention can be used in the treatment of cardiovascular disorders, such as Myocardial Infarction, congestive heart failure, Peripheral Arterial Disease, diabetic ulcers and wound healing. The stimulation of angiogenesis is also therapeutically useful in the treatment of ischaemia, in particular cardiac ischaemia and limb ischaemia. Figure 12 shows that microvesicles harvested from NSCs cultured for at least 3 weeks are more efficacious than microvesicles isolated from NSCs cultured for 1 or 2 weeks. Accordingly, in one embodiment, microvesicles isolated from NSCs (typically CTX0E03 cells) that have been cultured (typically in a multi-compartment bioreactor) for at least 3 weeks, more typically at least 4 weeks or at least 6 weeks, are used to treat a disease or condition requiring angiogenesis. Optionally, the NSCs have been cultured for no more than ten weeks, e.g. between 3 and 10 weeks, between 4 and 10 weeks, between 5 and 10 weeks or between 6 and 10 weeks.

As shown in Figure 13 and discussed in Example 8, microvesicles obtained from neural stem cells stimulate neurite outgrowth. Accordingly, in one embodiment, microvesicles of the invention are used to treat a neurological disease, disorder or deficit, such as Parkinson's disease, Alzheimer's disease, Stroke, neuropathy or ALS.

In prophylactic applications, pharmaceutical compositions or medicaments are administered to a patient susceptible to, or otherwise at risk of, a particular disease in an amount sufficient to eliminate or reduce the risk or delay the outset of the disease. In therapeutic applications, compositions or medicaments are administered to a patient suspected of, or already suffering from such a disease in an amount sufficient to cure, or at least partially arrest, the symptoms of the disease and its complications. An amount adequate to accomplish this is defined as a therapeutically-or pharmaceutically-effective dose. In both prophylactic and therapeutic regimes, agents are typically administered in several dosages until a sufficient response has been achieved. Typically, the response is monitored and repeated dosages are given if the response starts to fade.

The microparticles of the invention may optionally be combined with a stem cell to provide a combination therapy. The stem cell is optionally the stem cell from which the microparticle is derived, e.g. if the microparticle is an exosome from a CTX0E03 cell, then the stem cell for use in combination therapy may be a CTX0E03 cell. A stem cell and microparticle can optionally be (i) administered together in a single pharmaceutical composition, (ii) administered contemporaneously or simultaneously but separately, or (iii) administered separately and sequentially, e.g. stem cell followed by microparticle, or microparticle followed by stem cell. When the stem cell and microparticle are administered separately and sequentially, the duration between the administration of the cell and microparticle may be one hour, one day, one week, two weeks or more.

In one embodiment, a prophylactic therapy induces tolerance, typically immunotolerance, in a host that is to receive the stem cells from which the microparticle is derived. In one embodiment, the administration of one or more doses of microparticles of the invention to a patient, prior to administration of a stem cell therapy, can be used to reduce the risk of an adverse immune response, i.e. "rejection", of the stem cell therapy. In another embodiment, tolerance to the stem cells can be increased by administering stem cells together with microparticles of the invention, as discussed above.

Effective doses of the compositions of the present invention, for the treatment of the above described conditions vary depending upon many different factors, including means of administration, target site, physiological state of the patient, whether the patient is human or an animal, other medications administered, and whether treatment is prophylactic or therapeutic. Usually, the patient is a human.

The CTX0E03 cell line has been shown to be effective in treating stroke, peripheral arterial disease, brain damage such as motor, sensory and/or cognitive deficit, and psychiatric disorders. The cells are currently being tested in a clinical trial for treatment of disabled stroke patients (Clinicaltrials.gov Identifier: NCT01151124). WO-A-2012/004611 describes the use of the CTX0E03 cells in treating psychiatric disorders including unipolar and bipolar depression, schizophrenia, obsessive compulsive disorder, autism and autistic syndrome disorders. Accordingly, microparticles produced by CTX0E03 cells are also able to treat stroke, peripheral arterial disease, blindness-causing diseases of the retina (such as retinitis pigmentosa), brain damage such as motor, sensory and/or cognitive deficit, and psychiatric disorders.

As used herein, the terms "treat", "treatment", "treating" and "therapy" when used directly in reference to a patient or subject shall be taken to mean the amelioration of one or more symptoms associated with a disorder, or the prevention or prophylaxis of a disorder or one or more symptoms associated with a disorder. The disorders to be treated include, but are not limited to, a degenerative disorder, a disorder involving tissue destruction, a neoplastic disorder, an inflammatory disorder, an autoimmune disease or an immunologically mediated disease including rejection of transplanted organs and tissues. Amelioration or prevention of symptoms results from the administration of the microparticles of the invention, or of a pharmaceutical composition comprising these microparticles, to a subject in need of said treatment.

Tracing administered cells and microparticles *in vivo*

The present invention provides a distinct marker profile for microparticles produced by neural stem cells. It is therefore possible to detect the presence of these microparticles *in vivo,* by testing a sample obtained from a patient and determining whether the marker profile in the sample matches that of the microparticles. If the sample profile matches the profile of the microparticles described herein, then this confirms the presence of the microparticles. This can be used to detect not only the presence and/or biodistribution of the microparticles themselves, but also the presence of stem cells producing the microparticles. This is particularly useful when detecting whether a stem cell administered *in vivo* has engrafted into the host tissue, and/or has migrated, for example in ADME(T) studies.

Detection of the microparticles *in vivo* can be used to monitor the course of a treatment wherein microparticles or stem cells are administered to a patient. Determining the presence, absence or amount of microparticles or cells producing microparticles of the invention in a patient allows the dosage regime to be altered accordingly, e.g. to increase or decrease the dose as required to provide an effective amount of microparticles or stem cells *in vivo.*

Methods of producing microparticles

Microparticles are isolated from stem cell conditioned media. The "conditioned medium" (CM) may be a growth medium for stem cells, which has been used to culture a mass culture of stem cells for at least about 12 hours, at least about 24 hours, at least about 48 hours or least about 72 hours, typically up to 168 hours (7 days), removed and sterilized by any suitable means, preferably by filtration, prior to use, if required.

Alternatively, microparticles may be harvested from a two-compartment bioreactor which allows the cell culture, and hence the conditioned media, to be maintained for longer periods of time, for example at least 2 weeks, at least 3 weeks, at least 4 weeks, at least 5 weeks, at least 6 weeks or more. The system maintains the cells and secreted microparticles within a small cell compartment (approximately 15ml) which is separated from a larger reservoir of medium by a 10kDa semi-permeable membrane. This allows the efficient removal of metabolic waste products while effectively maintaining an extremely high cell density to maximize microparticle production. Example 9, and Figures 7 and 8, demonstrate that use of a two-compartment bioreactor results in a much higher yield of microparticles than is obtained when a standard cell culture flask (T175 flask) is used.

The microparticles may be separated from other media components based on molecular weight, size, shape, hydrodynamic radius, composition, charge, substrate-ligand interaction, absorbance or scattering of electromagnetic waves, or biological activity. In one embodiment, the conditioned media is filtered using a filter of appropriate size to separate the desired microparticle, for example a 100K MWCO filter. Optionally, the stem cell-conditioned medium is concentrated prior to the isolation of the microparticles by subjecting the concentrated NSC-conditioned medium to size exclusion chromatography. The UV absorbant fractions can then be selected for isolation of the microparticles of interest.

Different microparticles can be isolated from the media by using different isolation techniques and parameters. For example, exosomes have a vesicle density of 1.13-1.19 g/mL and can be isolated by differential centrifugation and sucrose gradient ultracentrifugation at 100,000-200,000g. Microvesicles can be isolated by filtration (100K MWCO) and differential centrifugation at 18,000-20,000g. Membrane particles have a density of 1.04-01.07 g/ml and Exosome-like vesicles have a density of 1.1 g/ml.

A typical production method comprises: culturing stem cells to produce conditioned media; removing cell debris by centrifugation at 1500 rpm; isolating microvesicles (<1000kDa) by ultrafiltration through a 100K MWCO filter or isolating exosomes (30-100nm) by ultracentrifugation at 120,000g; followed by quantification using a BCA protein assay.

<u>Conditionally immortalised stem cells as producer cells for microparticles</u>

In one aspect of the invention, conditionally immortalised stem cells are used to produce microparticles such as microvesicles and/or exosomes. These conditionally immortalised stem cells are typically neural stem cells, but may be a stem cell of any type, for example a haematopoietic stem cell or a mesenchymal stem cell. A method of producing stem cell microparticles is therefore provided, comprising the steps of culturing conditionally-immortalised stem cells and harvesting the microparticles that are produced by the cells. Conditional immortalisation of stem cells is known in the art, as described above. For the avoidance of doubt, this method is not limited to the use of neural stem cells.

When the stem cell used to produce microparticles is a neural stem cell, it may be any of the neural stem cells described herein, for example the CTX0E03 conditionally-immortalised cell line which is clonal, standardised, shows clear safety *in vitro* and *in vivo* and can be manufactured to scale thereby providing a unique resource for stable exosome production. Alternatively, the neural stem cells may be neural retinal stem cell lines, optionally as described in US 7514259.

When the stem cell used to produce microparticles is a mesenchymal stem cell, it may optionally be a conditionally-immortalised adipose-derived stem cell ("ADSC") or a conditionally-immortalised version of the mesenchymal stem cells described in WO-A-2009/105044; these cells are CD29+, CD44+, CD49a+/e+, CD105+, CD166+, CD34-, CD45-.

<u>Methods of inducing microparticle secretion</u>

The inventors have found that it is possible to increase the production of microparticles by stem cells. This finding, which is not limited to neural stem cells and can be used for the production of microparticles from any stem cell, allows for an improved yield of microparticles to be obtained from a stem cell culture.

A first technique to increase the production of microparticles by the stem cells is to treat the stem cells with one or more of TGF-$\beta$, IFN-$\gamma$ or TNF-$\alpha$, typically at between 1 and 25ng/ml e.g. 10ng/ml, for between 12 to 96 hours prior to the removal of conditioned media.

As explained in Example 2 below, the frequency of the occurrence of multivesicular bodies (MVBs) was observed to be altered by the presence of TGF-$\beta$, IFN-$\gamma$ or TNF-$\alpha$ (10ng/ml). The frequency was highest in the presence of TGF-$\beta$, followed by IFN-$\gamma$, followed by TNF-$\alpha$. Therefore, adding one or more of TGF-$\beta$, IFN-$\gamma$ or TNF-$\alpha$ to the stem cell culture medium will stimulate the production of microparticles by the cells. The microparticles can then be harvested, by separating the microparticles from other components as described above.

A second technique to increase the production of microparticles by the stem cells is to culture the cells under hypoxic conditions. Culturing cells under hypoxic conditions is well-known to the skilled person, and involves culturing the cells in an atmosphere that has less than atmospheric level of $O_2$, i.e. less than 21% $O_2$ This is typically achieved by placing the cells in an incubator that allows oxygen levels to be changed. Hypoxic culture typically involves culturing in an atmosphere containing less than 10% $O_2$, more typically 5% or less $O_2$, for example 4% or less, 3% or less, 2% or less, or 1% or less $O_2$.

The inventors have also realised that co-culturing a stem cell with a different cell type can alter the production of microparticles by the stem cell. The different cell type may be a non-stem cell, i.e. a terminally differentiated cell type. Typically, the different cell type is one with which the stem cell would interact *in vivo.* In one embodiment, neural stem cells are co-cultured with epithelial cells such as endothelial cells, typically Human Umbilical Vein Endothelial Cells (HUVEC). It has been observed that *in vivo,* NSCs and the vasculature interact, with proliferating NSCs being localized in close proximity or adjacent to blood vessels. Receptor tyrosine kinase activation and signal protein secretion has also been observed to be upregulated when NSCs are co-cultured with endothelial cells, again indicating that the vasculature modulates the proliferation capacity of NSCs. Without wishing to be bound by theory, the inventors believe that *in vivo,* there is a pivotal interplay between NSCs and microvessels (i.e. endothelial cells) in the process of tissue regeneration, through amplification of cytokine expression. Microparticles, e.g. exosomes, derived from NSCs (for example CTX0E03 cells) co-cultured with endothelial cells (for example HUVEC) are therefore primed for therapeutic use, because they have been produced in an environment that mimics the *in vivo* environment in which the stem cells and microparticles are active.

Therefore, culturing a stem cell with a different cell type may improve the amount of microparticles produced and/or may refine the content of the microparticles, typically so that the microparticles produced by the stem cells are biased towards an activated state of tissue repair. Accordingly, microparticles produced by stem cells that have been co-cultured with other cells, e.g. NSCs co-cultured with endothelial cells, are advantageous. These microparticles may be obtained by isolation from the co-cultured stem-cell conditioned media, as described herein.

Surprisingly, the present inventors have realised that the amount of microparticles produced by stem cells can be increased greatly simply by culturing stem cells in a multi-compartment bioreactor. This finding is not limited to neural stem cells and applies generally to the culture of all stem cells. Accordingly, one aspect of the invention provides a method of producing microparticles from stem cells that have been cultured in a multi-compartment bioreactor. The cells from which the microparticles are harvested have typically been cultured for at least one week, typically at least 8, 9, 10, 11, 12, 13 or 14 days, for example 15 days, 16 days, 17 days, 18 days, 19 days, 20 days, 21 days or more, for example at least three weeks, four weeks, five weeks, six weeks or more. It can be seen from Figure 8 that the increase in microparticle production, week on week, is not merely additive but is exponential. The prolonged culture typically has been observed in the Integra Celline system two-compartment bioreactor (commercially available from Integra Biosciences AG, Zizers, Switzerland) but the findings are not limited to this specific multi-compartment bioreactor; any multi-compartment bioreactor can be used. This culture method can be used to produce microparticles from any stem cell type, including but not limited to neural stem cells and mesenchymal stem cells.

Method of screening for an agent that alters microparticle production

The invention provides a method of screening for an agent that alters the production of a microparticle by a stem cell. This method comprises contacting a stem cell with a candidate agent, typically under conditions suitable for microparticle production, and observing whether (i) the rate of production of microparticles by the contacted stem cell increases or decreases, or (ii) the characteristics (e.g. size, protein, mRNA or miRNA content) of the microparticles changes, compared to a control stem cell that is not contacted with the agent.

Method for screening total RNA composition of conditioned medium

Following centrifugation (5 min at 1500 rpm), microparticles are collected from conditioned medium through filtration (0.02-0.2$\mu$m, or 100K MWCO). Total RNA is obtained using trizol based extraction followed by purification using Qiagen RNaesy mini kit. The extract in water has a 260:280 nm absorbance suggesting that it may be RNA. Total RNA is retro-transcribed with either a protocol suitable for mRNA (Superscript II RT, Invitrogen) or miRNA (mScript RT kit, Qiagen). Validation of mRNA and miRNA presence is proven by qRT-PCR using primers for ATP5B and YWHAZ for mRNA, and U6B and 15a for miRNA housekeeping genes respectively. The RNA may be assessed by a generic gene expression analysis assay such as an array (micro array or PCR based array), and sequencing.

Kits

The invention provides a kit for use in a method for producing the microparticle of the invention. The kit comprises a neural stem cell culture medium, a neural stem cell and instructions for producing the microparticle of any of claims 1-16 or 23 using the kit. Optionally, the kit comprises one or more components of claims 19 or 21. The kit may also comprise a microparticle according to the invention, for use as a control. The control microparticle is optionally lypohilised. The kit may also contain optionally a detection agent suitable for detection of the produced microparticles, for example an antibody that binds specifically to a marker protein that can be used to identify the microparticle.

The invention is further described with reference to the following non-limiting examples.

## Examples

**Example 1:** Preparation of neural stem cells and neural stem cell microparticles for visualisation by electron microscopy.

Method

**Embedding CTX0E03 cells for electron microscopy**

- 5 x 70% CTX0E03 cultures
- Treat with +/-4OHT, IFNγ, TNFα and TGFβ (all at 10ng for 24hrs)
- Detach cells and fix overnight in 2.5% Gluteraldehyde in 0.1M Cacodylate pH7.4
- Cells spun down 300g
- Buffered osmium 2%, 1.5hrs
- Spin, wash water, overnight
- Uranium acetate 2%, 2hrs
- Spin, wash water, 30mins
- Ethanol gradient 20, 35, 50, 70, 80, 90, 100%, over weekend.
- 100% propylene oxide (PO), 1hr
- Spin, 50% Agar LV resin in PO, 1hr
- 75% LV resin/PO 5hrs
- 100% resin overnight at 60°C
- Cool to RT before cutting (60-80nm), Imaged TEM at 200Kv.

Results

Figure 1A-E shows the electron micrographs of the multivesicular bodies (MVBs) containing exosomes of approximately 30nm - 50nm in diameter. Figure 1F shows microvesicles >100nm in diameter.

**Example 2:** Production of neural stem cell microparticles from a neural stem cell line.

Method

5 Sub-confluent flasks containing the same culture of CTX0E03 cells were individually treated with either 10ng/ml TGF-β, 10ng/ml IFNγ, or 10ng/ml TNFα alongside full growth media controls with or without the addition of 4OHT. 72 hours after treatment, the cells were collected using trypzean/EDTA, washed and fixed overnight in 2.5% Gluteraldehyde in 0.1M Cacodylate pH7.4 ready for electron microscopy evaluation.

*Results*

The frequency of the occurrence of multivesicular bodies (MVBs) was observed to be altered by the presence of TGF-β, IFN-γ or TNF-α. The frequency was highest in the presence of TGF-β, followed by IFN-γ, followed by TNF-α.

Conclusion

The production of microparticles from neural stem cells can be stimulated by the addition of the factors TGF-β, IFN-γ or TNF-α. This has the potential for more efficient production of microparticles.

**Example 3:** Purification, quantification and characterisation of neural stem cell microparticles.

Method

An outline protocol for producing large quantities of microparticles is provided in Figure 2. The main steps are purification, quantification, characterisation, efficacy testing and manufacture.

(1) Purification
Microparticles can be purified from stem cell-conditioned medium by ultracentrifugation, e.g. at 100000 x g for 1-2 hours. Alternative or additional methods for purification of may be used, such as antibody-based methods, e.g. immunoprecipitation, magnetic bead purification, resin-based purification, using specific antibodies.
(2) Quantification
Purified microparticles can be quantified by quantification of total nucleic acid or protein levels, e.g. various PCR or colorimetric protein quantification methods such as such as the BCA assay. Other quantification techniques may alternatively be used, including an electron microscopy grid or an immune-assay using antibodies or antibody fragments that specifically bind to microparticle-specific markers (e.g. ELISA, immunoblotting).
(3) Characterisation
The microparticles can be functionally or structurally characterised. RNA/mRNA/miRNA and protein profiling can be used using methods well known in the art (SDS-PAGE, mass spectrometry, PCR). Constitutively secreted microparticles can be tested and compared to microparticles that have been induced by addition of an inducing agent such as transforming growth factor-beta (TGF-β), interferon-gamma (INF-γ) and/or tumour necrosis factor-alpha (TNF-α).

(4) Therapeutic Efficacy

The efficacy of the microparticles can be tested by *in vitro* and *in vivo* assays. For *in vitro* evaluation, neural stem cell microparticles can be added to cultures of monocytes, PBMCs, endothelial cells and/or fibroblasts and the effect of the microparticles on these cells evaluated. Administration of neural stem cell microparticles to suitable animal models can be used to evaluate the *in vivo* efficacy. Clinical trials can be performed to evaluate safety and outcome of neural stem cell microparticles in human subjects.

(5) Manufacture/Scale-Up

Bioreactors, such as the Integra disposable T1000, can be used for the large-scale manufacture of neural stem cell microparticles. The purified microparticles are then formulated as a therapeutic product.

**Example 4:** miRNA characterization in CTX0E03 microparticles

Methods

- 3 conditions: CTX0E03 cells in standard culture; microparticles obtained from CTX0E03 cells in standard culture; and purified exosomes derived from CTX0E03 cells in Integra CELLine system (see Examples 7 to 11, below)
- Investigation of miRNA array using qRT-PCR panel (Qiagen) according to manufacturer's instruction. This assay provides high precision and high sensitivity, with data normalization sensitive to method/choice of reference genes. It does not provide genome wide sequencing.

Results:

A) List of miRNAs with a cp ≤ 35 found in (i) standard CTX0E03 cells, (ii) filtered conditioned medium (0.02-0.2μm filter) i.e. microparticles and (iii) exosomes derived from Integra CELLine system (preliminary miRNA qRT-PCR miscript array (Qiagen) results).

B) Arithmetic and geometric mean of the reference (housekeeping) genes

A

| Mature miRNA | CTX0E03 std culture | CM microparticles | CM exosome Integra |
|---|---|---|---|
| hsa-miR-21-5p | 19.52 | 20.9 | 20.72 |
| hsa-let-7a-5p | 22.64 | 23.11 | 22.36 |
| hsa-miR-125b-5p | 21.64 | 23.25 | 21.74 |
| hsa-miR-9-5p | 22.58 | 23.64 | 22.94 |
| hsa-miR-92a-3p | 23.2 | 23.94 | 24.01 |
| hsa-miR-24-3p | 23.73 | 24.24 | 23.83 |
| hsa-miR-20a-5p | 23.45 | 24.43 | 25.06 |
| hsa-miR-16-5p | 23.14 | 24.72 | 24.32 |
| hsa-miR-100-5p | 23.28 | 24.74 | 23.04 |
| hsa-let-7b-5p | 24.67 | 24.75 | 23.7 |
| hsa-let-7f-5p | 23.93 | 25.09 | 23.86 |
| hsa-miR-17-5p | 24.56 | 25.24 | 26.13 |
| hsa-miR-23b-3p | 24.3 | 25.3 | 24.13 |
| hsa-miR-106b-5p | 24.4 | 25.41 | 26.16 |
| hsa-miR-222-3p | 23.25 | 25.49 | 23.17 |
| hsa-let-7e-5p | 24.57 | 25.58 | 24.16 |
| hsa-miR-26a-5p | 23.4 | 25.63 | 24.2 |

(continued)

| Mature miRNA | CTX0E03 std culture | CM microparticles | CM exosome Integra |
|---|---|---|---|
| hsa-miR-181a-5p | 25.16 | 25.7 | 24.32 |
| hsa-miR-125a-5p | 23.56 | 25.75 | 24.88 |
| hsa-miR-103a-3p | 24.65 | 25.8 | 25.77 |
| hsa-let-7i-5p | 24.37 | 25.98 | 24.23 |
| hsa-miR-99a-5p | 24.44 | 26.05 | 23.44 |
| hsa-let-7c | 25.76 | 26.12 | 24.07 |
| hsa-let-7g | 25.2 | 26.15 | 25.17 |
| hsa-miR-195-5p | 24.72 | 26.34 | 25.67 |
| hsa-miR-93-5p | 25.15 | 26.48 | 26.06 |
| hsa-miR-22-3p | 25.03 | 26.49 | 25.66 |
| hsa-miR-20b-5p | 26.03 | 26.86 | 27.42 |
| hsa-miR-18a-5p | 26.71 | 26.87 | 29.06 |
| hsa-miR-15b-5p | 25.1 | 26.92 | 26.43 |
| hsa-let-7d-5p | 26.84 | 26.96 | 26.52 |
| hsa-miR-424-5p | 25.56 | 27.72 | 26.66 |
| hsa-miR-15a-5p | 26.88 | 27.89 | 29.3 |
| hsa-miR-130a-3p | 27.23 | 28.26 | 28.49 |
| hsa-miR-33a-5p | 30.34 | 28.54 | 34.18 |
| hsa-miR-128- | 26.94 | 28.64 | 27.66 |
| hsa-miR-218-5p | 27.79 | 28.68 | 28.03 |
| hsa-miR-301a-3p | 29.53 | 28.69 | 31.57 |
| hsa-miR-134 | 28.3 | 28.76 | 28.76 |
| hsa-miR-101-3p | 28.44 | 28.82 | 31.64 |
| hsa-miR-7-5p | 29.71 | 28.82 | 30.22 |
| hsa-miR-18b-5p | 28.83 | 28.85 | 35.47 |
| hsa-miR-185-5p | 28.34 | 28.99 | 28.13 |
| hsa-miR-378-3p | 29.76 | 29.25 | 28.97 |
| hsa-miR-132-3p | 28.65 | 29.32 | 27.72 |
| hsa-miR-345-5p | 28.49 | 29.52 | 29.66 |
| hsa-miR-219-5p | 30.58 | 29.52 | 32.7 |
| hsa-miR-127-5p | 30.05 | 29.95 | 31.11 |
| hsa-miR-146b-5p | 30.53 | 30.54 | 28.07 |
| hsa-miR-10a-5p | 27.1 | 30.69 | 28.32 |
| hsa-miR-210 | 29.85 | 30.83 | 30.65 |
| hsa-miR-129-5p | 32.51 | 30.98 | 31.69 |
| hsa-miR-137 | 31.46 | 31.13 | 30.95 |
| hsa-miR-182-5p | 28.34 | 31.64 | 31.27 |
| hsa-miR-124-3p | 33.38 | 31.71 | 33.07 |

(continued)

| Mature miRNA | CTX0E03 std culture | CM microparticles | CM exosome Integra |
|---|---|---|---|
| hsa-miR-96-5p | 29.77 | 32.27 | 34.67 |
| hsa-miR-192-5p | 31.42 | 32.42 | 32.52 |
| hsa-miR-126-3p | 31.73 | 32.44 | 32.05 |
| hsa-miR-194-5p | 31.11 | 32.49 | 31.72 |
| hsa-miR-375 | 33.77 | 32.94 | 30.94 |
| hsa-miR-205-5p | 35 | 33.01 | 32.72 |
| hsa-miR-183-5p | 29.88 | 33.21 | 31.74 |
| hsa-miR-10b-5p | 29.6 | 33.22 | 30.79 |
| hsa-miR-302a-3p | 29.67 | 33.6 | 31.69 |
| hsa-miR-214-3p | 34.19 | 33.76 | 32.11 |
| hsa-miR-141-3p | 35 | 33.96 | 34.51 |
| hsa-miR-302c-3p | 31.6 | 34.29 | 33.93 |
| hsa-miR-196a-5p | 35 | 34.65 | 35.75 |
| hsa-miR-150-5p | 34.59 | 34.76 | 34.59 |
| hsa-miR-155-p | 32.04 | 35.75 | 32.76 |

B

| | CTX0E03 std culture | CM microparticles | CM exosome Integra |
|---|---|---|---|
| Avg. of Arithmetic Mean | 23.54 | 23.82 | 24.79 |
| Avg. of Geometric Mean | 23.48 | 23.8 | 24.62 |

**Example 5:** CTX0E03 conditioned medium analysis using a protein dot blot

Methods

- Conditioned 24hr and 72 hrs conditioned medium (RMM and ITS medium)
- The collected media has been 'concentrated' by dialysis and the proteins biotinylated (typical total protein concentration appears to be 0.5 mg/ml). The media is then incubated with the Raybiotech L507 human protein arrays (total protein concentration 0.1 mg/ml). Following washing and incubation of the array with HRP-conjugated streptavidin, the presence of proteins is detected by chemiluminescence. The array provides qualitative data (i.e. the protein is present, but no indication of its level of expression compared to other proteins).

Results

| Cytokine Name | Cytokine Full Name | Function |
|---|---|---|
| EDA-A2 | ectodysplasin-A2 | May be involved in proper formation of skin appendages |
| Galectin-3* | Galectin-3 | Galactose-specific lectin which binds IgE. May mediate with the alpha-3, beta-1 integrin the stimulation by CSPG4 of endothelial cells migration. |

(continued)

| Cytokine Name | Cytokine Full Name | Function |
|---|---|---|
| IGFBP-2 | Insulin-like growth factor binding proteins 2 | IGF-binding proteins prolong the half-life of the IGFs and have been shown to either inhibit or stimulate the growth promoting effects of the IGFs on cell culture. |
| IGFBP-rp1/IGFBP-7 | Insulin-like Growth Factor Binding Protein Related Protein-1 Insulin-like Growth Factor Binding Protein-7 | soluble proteins that bind IGFs with high affinity. |
| IL-1a† | Interleukin 1 alpha | potent mediator of inflammation |
| | | and immunity |
| LECT2† | Leukocyte cell-derived chemotaxin-2 | Has a neutrophil chemotactic activity. Also a positive regulator of chondrocyte proliferation. |
| MCP-1† | Monocyte chemoattractant protein 1 | plays a role in the recruitment of monocytes to sites of injury and infection. |
| SPARC* | Secreted Protein, Acidic Cysteine-rich-related modular calcium-binding protein 1 [Precursor] | matricellular protein that modulates cell adhesion and proliferation and is thought to function in tissue remodeling and angiogenesis |
| TIMP-1* | Tissue inhibitor of metalloproteinasess-2 | Complexes with metalloproteinases (such as collagenases) and irreversibly inactivates them. Also mediates erythropoiesis in vitro; but, unlike IL-3, it is species-specific, stimulating the growth and differentiation of only human and murine erythroid progenitors. |
| Thrombospondin-1* | Thrombospondin-1 | multimodular secreted protein that associates with the extracellular matrix and possesses a variety of biologic functions, including a potent angiogenic activity. |
| VEGF* | Vascular endothelial growth factor | Growth factor active in angiogenesis, vasculogenesis and endothelial cell growth. |
| These proteins show expression in some instances -though may also be present in media. | | |
| EGF R/ErbB1 | Epidermal growth factor receptor | Receptor for EGF, but also for other members of the EGF family, as TGF-alpha, amphiregulin, betacellulin, heparin-binding EGF-like growth factor |
| MDC* | A disintegrin and metalloproteinase domain 11 Metalloproteinase-like, disintegrin-like, and cysteine-rich protein MDC | Probable ligand for integrin in the brain. This is a non catalytic metalloprotease-like protein. |
| Endostatin* | Endostatin | Angiogenesis inhibitor; inhibits endothelial cell migration but may not effect proliferation. May work in balance with VEGF to maintain level of angiogenesis. |
| Follistatin | Follistatin | Regulates stem cell renewal versus differentiation by inhibiting pro-differentiation proteins |
| Csk† | cytoplasmic tyrosine kinase | Activity is required for interleukin 6 (IL-6) induced differentiation. May play a role in the growth and differentiation of hematopoietic cells. May be involved in signal transduction in endocardial and arterial endothelial cells. |
| * = angiogenesis<br>† = inflammation | | |

**Example 6:** Conditioned medium analysis using Human angiogenesis ELISA strips (Signosis)

Method

Human angiogenesis ELISA strips (Signosis) were utilized according to manufacturer's instruction. Fresh RMM medium and 24 hour conditioned CTX0E03 RMM medium were analyzed for 8 angiogenesis cytokines; tumor necrosis factor $\alpha$ (TNF$\alpha$), insulin-like growth factor 1 (IGF-1), VEGFA, interleukin-6 (IL-6), bFGF, transforming growth factor $\beta$ 1 (TGF$\beta$1), EGF, and leptin. Individual wells of the strip, coated with each of the primary antibodies directed against the specific angiogenesis cytokines were loaded with test samples. Absorbance was measured by a spectrophotometer at 450 nm. The concentrations of the angiogenesis cytokines were directly proportional to the color intensity of the test sample.

The results are shown in Figure 3.

**Example 7:** Integra CELLINE - Disposable Bioreactor for the production of Micro particles from CTX0E03 cells.

Efficient micro particle production and harvest from a cell line relies upon maintaining optimal culture conditions for the greatest density of cells. Any restriction in the oxygen or nutrients supplied to the cells or an accumulation of waste metabolic products will limit the life span of the culture, and hence the micro particle production.

The two-compartment CELLine AD 1000 is designed to accommodate adherent cells attached to a matrix inlay within a small cell compartment, separated from a larger media reservoir by means of a 10kDa semi-permeable membrane. This membrane allows a continuous diffusion of nutrients and removal of waste products, while concentrating any micro particles produced by the cell within the smaller cell compartment. Due to the large volume capacity (1 litre) of the media compartment, the system has the potential to maintain high density cultures for longer periods of time without the need for a media change. The production of exosomes from mesothelioma tumour cell cultures is described in Mitchell *et al,* 2008.

Method

In order to obtain optimal performance of the CELLine AD1000, place 25ml of complete growth medium (RMM with growth factors and 4OHT) into the medium compartment of the flask to pre-wet the semi-permeable membrane. Allow the flask to sit for 5 minutes at room temperature before coating the matrix inlay with mouse Laminin by adding 15ml of laminin solution (20$\mu$g/ml in DMEM/F12) to the cell compartment for a minimum of 1 hour at 37°C. Remove the laminin solution and add 15ml of warm DMEM/F12 to the cell compartment to remove any excess laminin. Avoiding the matrix inlay drying, slowly introduce approximately 15x$10^6$ CTX0E03 cells in a total of 15ml of complete growth medium. Take care to remove any air bubbles from the cell compartment. Carefully add a further 460ml of complete growth medium to the cell compartment before incubating the flask overnight in 5% $CO_2$ at 37°C. The next day remove the medium from the cell compartment and replace with 15ml of pre warmed growth medium.

Every 7 days harvest the microparticles/medium from the cell compartment. Centrifuge the medium at 1500rpm for 5 minutes to remove any cell debris and store at -80°C. Carefully add another 15ml of pre-warmed complete growth medium in to the cell compartment and 485ml of complete growth medium to the medium compartment and incubate for another 7 days. Microparticles were isolated by 100K MWCO filtration. Repeat as necessary.

Figure 4A shows the amount of protein extracted from 15ml of media containing microparticles purified using the Integra system compared to normal culture conditions (3 days T175). Milligrams of protein measured by BCA assay. Figure 5 shows the corresponding quantity of isolated total RNA measured at 260/280nm.

Marker characterisations indicated that both purified populations (microvesicles and exosomes) express CD63 and CD81 (determined by FACS - Figure 4B). Only the exosomes express the endosomal marker Alix (determined by Western blot, data not shown).

**Example 8: Efficacy Assays**

*(A) Comparison of the function of CTX0E03 conditioned media with the function of purified exosomes from CTX0E03 cells in a wound healing assay.*

Method - Wound closure/ scratch assay

- Seed 0.25x$10^6$ NHDF (normal human dermal fibroblasts) per well of a 12 well plate and allow to become confluent (24 hours)

- Remove growth factors for 24hrs
- Remove cells (scratch) and incubate with exosomes/conditioned media
- Image effected area over 48hrs
- Estimate area using Image J

*Results*

**Table 2** - Wound closure/scratch assay representing the migration activity of normal human dermal fibroblasts (NHDF) cultured in CTX0E03 conditioned media or upon the addition of purified exosomes.

| | Wound closure (%) | | |
|---|---|---|---|
| | 0h | 24h | 48h |
| CTX0E03 conditioned media | 0% | 100% | |
| 2ug/ml exosomes | 0% | 95.4% | 100% |
| Contral | 0% | 48.1% | 49,7% |

Wound closure was calculated as the area covered by cells in relation to the initial wound area, as determined at 0h. Wound closure is expressed as the percentage of the initial wound area at time 0h. These data are also shown, photographically, in Figure 6A.

Figure 6B shows that 10μg CTX0E03 exosomes significantly increase wound closure (as determined in the HDNF scratch/migration assay) after 72 hours, compared to basal conditions (without exosomes).

Further experiments confirmed that exosomes purified (by ultracentrifugation; quantified by BCA protein assay; characterised as >99% positive for CD63 and CD81 and having a greater expression level of Alix compared to the corresponding microparticle fraction) from all time points (weeks 2-6) during continuous culture (using Integra CELLine bioreactors in the presence of growth factors and 4OHT) significantly enhanced fibroblast migration and wound healing, with a peak response between 5-10μg/ml compared to basal conditions. Figure 6C shows the % healed areas for basal conditions, 2μg/ml exosomes, 6 μg/ml exosomes, 20 μg/ml exosomes and an LSGS (low serum growth supplement) positive control. The top panel of Figure 6C shows exosomes isolated from CTX0E03 cells cultured for 2 weeks in the Integra Celline system and the bottom panel of Figure 6C shows exosomes isolated from CTX0E03 cells cultured for 6 weeks in the Integra Celline system. These data show that all doses of all tested NSC exosomes provide increased healing compared to basal conditions, with % healing approaching the positive control (LSGS) after 72 hours.

The data in Figure 6C also show that the exosomes isolated from NSCs cultured for 6 weeks cause faster healing (than 2 week exosomes), with the % healed approaching 100% after only 48 hours, for all doses.

Figure 6D shows the results of an *in vivo* injection wound assay in a mouse, confirming that CTX0E03 cells stimulated wound healing to a statistically-significant degree *in vivo.* This is a simple *in vivo* bioassay which can be used to confirm the efficacy of microparticles *in vivo.*

**Conclusion** Exosomes released from the human neural stem cell line CTX0E03 enhance fibroblast migration in an *in vitro* model of wound healing, suggesting that exosomes may contribute to the mechanisms by which hNSCs promote repair. Exosomes isolated from cells cultured for 6 weeks show improved wound healing efficacy *in vitro,* compared to exosomes isolated from cells cultured for 2 weeks.

*(B) Stimulation of Angiogenesis*

A 24 hour assay to detect angiogenesis on primary HUVECs was carried out using an Ibidi μ-slide and automated Wimtube detection and analysis (of tube length and bifurcation points). Microvesicles harvested from Integra flasks at 1, 2, 3, 4 and 6 weeks were added to HUVECs and angiogenesis compared to basal HUVECs (without addition). LSGS (low serum growth supplement) was used as a positive control. The results, depicted in Figure 12, show that neural stem cell microvesicles increase angiogenesis. Further, these data show that a larger increase in angiogenesis is provided by microvesicles harvested after at least 3 weeks of culture (i.e. after 3 weeks, 4 weeks and 6 weeks culture in an Integra celline bioreactor), than is provided by microvesicles cultured for 1 or 2 weeks. Microvesicles cultured for at least 3 weeks stimulated angiogenesis to a statistically significant level, and a level that approaches that of the positive control. The largest increase in angiogenesis is shown to be provided by microvesicles harvested after 4 weeks; these microvesicles stimulated angiogenesis by the same amount as the positive control.

These data indicate that hNSC microvesicles stimulate angiogenesis.

*(C) Stimulation of Neurite Outgrowth*

Neurite outgrowth was determined using PC-12 cells though a 1μm insert. After 72 hours, the PC-12 cell bodies were removed and the neurites stained on the underside of the insert. The stain was then extracted and quantified on a spectrophotometer. Microvesicles harvested from Integra flasks at 2 weeks were added to the cells at 0.03μg, 0.3μg and 3μg, each with 100ng/ml NGF (nerve growth factor). Neurite outgrowth was compared to basal cells (without addition). 100ng/ml NGF was used as a control. As shown in Figure 13, the addition of 3μg hNSC microvesicles caused a noticeable increase in neurite outgrowth, compared to the addition of NGF alone.

These data indicate that hNSC microvesicles stimulate neurite outgrowth.

**Example 9:** Production of exosomes using the Integra CELLine system.

CTX0E03 cells were cultured using the Integra CELLine system and exosomes were purified as described in Example 7. The concentration of exosomes purified from the medium using the CELLine system at the 3 week time point, and as a control a standard T175 system as routinely used in the art, was quantified (using a BCA assay to estimate protein content). Figure 7 shows that the production of exosomes using the Integra CELLine system is increased several fold, compared to using conventional culture (T175 flasks).

Using the Integra CELLine system, CTX0E03 cells were cultured over a 3-week period and medium was harvested at week 1, 2 and 3 for purification and quantification of exosomes, as described in Example 7. Figure 8A shows that the production of microparticles increases exponentially over the 3-week culture period, enabling efficient and large-scale production of microparticles. The concentration of exosomes harvested from a single Integra CELLine flask was then monitored over 1-6 weeks of continuous CTX0E03 culture, with the results shown below and depicted in Figure 8B:

| Integra time point | Total quantity of exosomes (ug) | Exosomes ug/ml |
|---|---|---|
| | | |
| Week 1 | 12 | 0.80 |
| Week 2 | 112 | 7.47 |
| Week 3 | 88 | 5.87 |
| Week 4 | 148 | 9.87 |
| Week 5 | 240 | 16.00 |
| Week 6 | 440 | 29.33 |

These results show that exosome production is surprisingly enhanced when stem cells are cultured in a multi-compartment bioreactor for weeks, typically at least three weeks.

**Example 10:** Characterisation of phenotype of cells obtained from the Integra CELLine and the standard (T175) culture system.

CTX0E03 cells were cultured using the Integra CELLine bioreactor and standard culture, as described in Example 7. Expression of DCX and GFAP protein markers was confirmed using marker-specific antibodies and fluorescence microscopy.

Expression of DCX, GALC, GFAP, TUBB3, GDNF and IDO markers was detected by qRT-PCR in samples obtained from the cells. Marker expression was compared between microparticles obtained from standard (T175) culture and exosomes obtained from the 3 week cultured Integra CELLine system, assessed against a baseline of the expression level in CTX0E03 cells in standard (T175) culture.

The inventors observed a striking difference in marker expression of cells obtained from the Integra CELLine system as compared to control cells obtained from standard. Markers of partially-differentiated cells were increased several fold in cells cultured in the Integra CELLine system, compared to control cells obtained from standard cultures (Figure 9). Particularly striking changes are increased expression of the markers DCX1 (doublecortin - a marker for entry into the neural lineage), GFAP (glial fibrillary acidic protein - a marker for entry into the astrocytic lineage), GDNF (glial cell-derived neurotrophic factor) and IDO (indoleamine 2,3-dioxygenase). This indicates that in neural stem cells cultured in a two-compartment bioreactor partially differentiate into cells of neural (DCX+) or astrocytic (GFAP+) lineage. The expression of DCX and GFAP in the Integra-cultured cells was confirmed by fluorescence microscopy, demonstrating that CTX0E03 cells cultured using the Integra CELLine bioreactor have a more differentiated neuronal phenotype than standard CTX0E03 cells.

**Example 11:** Characterisation of miRNA expression profiles of exosomes obtained from Integra CELLine cultures and microparticles obtained from standard (T175) cultures.

CTX0E03 cells were cultured for three weeks using the Integra CELLine culture and in the standard culture in single-compartment T-175 flasks. Exosomes were purified from the Integra culture and microparticles were purified from the standard T-175 culture as described in Example 7. The relative expression levels of various miRNAs expressed in the exosomes and microparticles obtained from either the standard culture or the Integra CELLine system were determined with an miRNA array using qRT-PCR panel (Qiagen) according to manufacturer's instruction, and converted into fold up and down regulation levels as compared to a standard CTX0E03 cell line control group (see Table 3 and Figure 10). These data show a differential miRNA expression profile between exosomes obtained from the Integra CELLine culture system for 3 weeks, microparticles, and cells obtained from the standard single-flask culture.

**Table 3:** Fold-regulation of miRNAs in microparticles obtained from standard culture or exosomes from the Integra CELLine system, relative to control (CTX0E03 cells).

| | **Standard Culture (microparticles)** | **Integra (exosomes)** |
|---|---|---|
| **miRNA** | **Fold regulation relative to control (CTX0E03 cells)** | |
| hsa-miR-146b-5p | -1.0222 | 10.5805 |
| hsa-let-7c | -1.6954 | 4.7678 |
| hsa-miR-99a-5p | -3.5349 | 3.3714 |
| hsa-miR-132-3p | -1.9163 | 3.088 |
| hsa-miR-378-3p | 1.2731 | 3.0175 |
| hsa-miR-181a-5p | -1.7431 | 2.9147 |
| hsa-let-7b-5p | -1.4658 | 2.7574 |
| hsa-miR-100-5p | -3.208 | 1.977 |
| hsa-let-7e-5p | -2.7101 | 1.9274 |
| hsa-miR-23b-3p | -2.3322 | 1.8834 |
| hsa-miR-185-5p | -1.9119 | 1.8532 |
| hsa-let-7i-5p | -3.5677 | 1.8404 |
| hsa-let-7a-5p | -1.851 | 1.7736 |
| hsa-let-7d-5p | -1.5 | 1.7654 |
| hsa-let-7g-5p | -2.2527 | 1.7092 |
| hsa-miR-222-3p | -5.8092 | 1.6779 |
| hsa-let-7f-5p | -2.8712 | 1.5948 |
| hsa-miR-218-5p | -1.9611 | 1.5619 |
| hsa-miR-24-3p | -1.6721 | 1.5511 |
| hsa-miR-9-5p | -2.2475 | 1.4109 |
| hsa-miR-126-3p | -2.1263 | 1.203 |
| hsa-miR-134 | -1.6567 | 1.1783 |
| hsa-miR-128 | -3.5842 | 1.0743 |
| hsa-miR-155-5p | -8.8458 | 1.0425 |
| hsa-miR-22-3p | -3.4782 | -1.0023 |

(continued)

| | Standard Culture (microparticles) | Integra (exosomes) |
|---|---|---|
| miRNA | Fold regulation relative to control (CTX0E03 cells) | |
| hsa-miR-26a-5p | -5.3579 | -1.0187 |
| hsa-miR-210 | -2.3107 | -1.0449 |
| hsa-miR-92a-3p | -1.9885 | -1.0693 |
| hsa-miR-93-5p | -3.056 | -1.1701 |
| hsa-miR-424-5p | -4.9189 | -1.2086 |
| hsa-miR-195-5p | -3.8951 | -1.2541 |
| hsa-miR-127-5p | -1.1316 | -1.2953 |
| hsa-miR-21-5p | -2.8845 | -1.3044 |
| hsa-miR-103a-3p | -2.6482 | -1.3287 |
| hsa-miR-16-5p | -3.5267 | -1.3692 |
| hsa-miR-125a-5p | -5.1159 | -1.434 |
| hsa-miR-10a-5p | -14.4701 | -1.434 |
| hsa-miR-10b-5p | -15.1194 | -1.4373 |
| hsa-miR-345-5p | -2.5521 | -1.4406 |
| hsa-miR-130a-3p | -2.6178 | -1.5728 |
| hsa-miR-15b-5p | -4.4025 | -1.6058 |
| hsa-miR-20b | -2.1312 | -1.6096 |
| hsa-miR-20a-5p | -2.3107 | -1.8319 |
| hsa-miR-17-5p | -1.9296 | -1.8319 |
| hsa-miR-7-5p | -1.5105 | -2.042 |
| hsa-miR-106b-5p | -2.4708 | -2.1287 |
| hsa-miR-101-3p | 1.4794 | -2.4453 |
| hsa-miR-302a-3p | -18.0634 | -2.4623 |
| hsa-miR-301a-3p | 1.4931 | -2.5257 |
| hsa-miR-183-5p | -13.9772 | -2.5847 |
| hsa-miR-219-5p | 1.6994 | -2.7321 |
| hsa-miR-18a-5p | -1.4028 | -3.2792 |
| hsa-miR-15a-5p | -2.4766 | -3.3714 |
| hsa-miR-182-5p | -12.5099 | -4.9588 |
| hsa-miR-33a-5p | 2.7927 | -9.1472 |
| hsa-miR-96-5p | -7.0047 | -18.9396 |
| hsa-miR-18b-5p | -1.3519 | -49.18 |

Values were calculated from raw data using the following equations:

$$\Delta CT\ (\text{sample/control}) = \text{Average CT (GOI)} - \text{Average CT (HKG)}$$

$$\text{Fold expression (sample/control)} = 2^{-(\text{Average } \Delta\text{CT})}$$

$$\text{Fold change} = \frac{\text{Fold expression (sample)}}{\text{Fold expression (control)}}$$

$$\text{If (fold change)} > 1 \text{ then (fold regulation)} = \text{(fold change)}$$

$$\text{If (fold change)} < 1 \text{ then (fold regulation)} = -\left(\frac{1}{\text{fold change}}\right)$$

Wherein:

CT = cycle threshold
GOI = gene of interest (investigated miRNA)
HKG = housekeeping genes (reference miRNAs used to normalize the data)

**Example 12: Total miRNA analysis**

Cells can shuttle RNA into microparticles determined for release into the extracellular space. This allows the conveyance of genetically encoded messages between cells. We here collectively refer to extracellular RNA as 'shuttle RNA'. We aimed to analyze comprehensively non coding RNA species released by CTX0E03 neural stem cells (NSCs) using Next Generation Sequencing.

Non coding RNAs are divided in two categories (small and long). Small non coding RNA biotypes include ribosomal RNA (rRNA), small nucleolar (snoRNA), small nuclear RNA (snRNA), microRNA (miRNA), miscellaneous other RNA (misc_RNA, e.g. RMRP, vault RNA, metazoa SRP, and RNY), and long non coding RNA biotypes includes long non-coding RNAs (IncRNAs) and large intergenic non-coding RNAs (lincRNAs).

Here, we characterized shuttle RNAs, including small and long non coding RNAs, released from NSC derived exosomes and microvesicles (MV) and compared with the RNA contents of the producer NSCs.

***A) Total RNA contents in cells, exosomes and microvesicles identified by Agilent RNA bioanalyser***

The RNA in both exosomes and microvesicles mainly consists of small RNA species as shown in Fig. 14. The majority of the nucleotides (nt) was ≤200 as shown against the molecular ladder.

***B) RNA composition***

Small RNA sequencing libraries were generated to investigate the composition of shuttle and cellular RNA by deep sequencing (Next Generation Sequencing). The results are shown in Figure 15.

***C) Deep sequencing of CTX0E03 cell, microvesicle and exosome miRNA expression from standard (T175) cultures.***

Deep sequencing is based on the preparation of a cDNA library following by sequencing and provides information regarding the total sequence read out of different miRNAs in the microvesicles and exosomes. These deep sequence data complement the qRT-PCR array data shown above and provide a comprehensive analysis of the miRNA profile of the cells and microparticles. Unlike the qRT-PCR array analysis, deep sequencing is not restricted to identification of sequences present in the probe array and so the sequences to be identified do not need to be known in advance. Deep sequencing also provides direct read-out and the ability to sequence very short sequences. However, deep sequencing is not suitable for detection of transcripts with low expression.

Method

The presence of a variety of miRNAs in parental cells and their exosomes (30-100μm) and microvesicles (100-1000 μm), purified by differential centrifugation, was identified by deep sequencing, following construction of 1 tagged miRNA library for each sample.
Additionally, specific primers for highly shuttled miRNAs (e.g. hsa-miR-1246) were designed and used in real-time reverse transcription PCR (qRT-PCR) to trace exosomes/microvesicles following *in vivo* implantation.
Deep sequencing was performed by GATC Biotech (Germany) and required the preparation of a tagged miRNA library for each samples followed by sequencing, and miRBase scanning:

- Construction of tagged miRNA libraries (22 to 30 nt)

    ◦ Sequencing libraries were generated by ligation of specific RNA adapter to both 3' and 5' ends for each sample followed by reverse transcription, amplification, and purification of smallRNA libraries (size range of contained smallRNA fraction 22 - 30 nt).

- Sequencing on an Illumina HiSeq 2000 (single read)

    ◦ Sequencing was performed using Illumina HiSeq 2000 (single read). Analysis of one pool could include up to 45,000,000 single read, and each read length is up to 50 bases. Sequencing was quality controlled by using FastQ Files (sequences and quality scores).

- Identification of known miRNAs was performed as followed:

    ◦ RNA adapters were trimmed from resulting sequences and raw data cleaned. Raw data were clustered and for each cluster a number of reads was provided. MiRNAs were identified by miRBase scanning (Ssearch).

Results

Many microvesicle and exosome miRNAs were enriched relative to the cells, indicating that cells specially sort miRNAs for extracellular release. Furthermore, miRNA contents were similar in both exosomes and microvesicles, indicating a common apparatus of selective miRNA uptake in excreted microvesicles. Without wishing to be bound by theory, this may indicate that miRNA content in secreted microvesicles and exosomes can be used as a fingerprint to identify hNSC subtypes.
The deep sequencing analysis therefore identified a unique set of miRNAs in both hNSC exosomes and microvesicles not previously reported. MiRNA content in excreted vesicles is similar, but showed a preferential miRNA uptake compared with hNSC. These findings could support biological effects mediated by shuttle miRNA not previously described for hNSC.
The results are detailed in Tables 4 to 9, below. The data are also depicted in Figure 11, which clearly shows the significantly different miRNA profiles present in the microvesicles and exosomes, compared to the cells. In summary, these data show a massive increase in the amount (read counts) of hsa-miR-1246, hsa-miR-4492, hsa-miR-4488 and hsa-miR-4532 in microvesicles and exosomes compared to the cells. Large increases are also seen in hsa-miR-4508, hsa-miR-4516, has-miR-3676-5p and hsa-miR-4485. Massive decreases are seen in the amounts (read counts) of certain miRNAs, including hsa-let-7a-5p, has-miR-92b-3p, has-miR-21-5p. hsa-miR-92a-3p, hsa-miR-10a-5p, hsa-100-5p and hsa-99b-5p.
The presence of each of hsa-miR-1246, hsa-miR-4488, hsa-miR-4492, hsa-miR-4508, hsa-miR-4516 and hsa-miR-4532 in the exosomes was validated by qRT-PCR (data not shown).
Plotting the deep sequencing results in the exosomes and microvesicles as relative fold change compared to the cells confirms that hsa-miR-1246, hsa-miR-4492, hsa-miR-4488 and hsa-miR-4532 are significantly upregulated in the exosomes and microvesicles compared to the cells. This comparison also shows that miRNA hsa-miR-3195 is the miRNA that is most upregulated, in both exosomes and microvesicles. Although the absolute reads of hsa-miR-3195 are in the range of ~40 for exosomes and microvesicles, there is no hsa-miR-3195 present in the cells.
As noted in Example 11 above, miRNA contents in exosomes, microparticles, and parental cells were also tested and validated using *PCR array* analysis. The following miRNAs were found present by qRT-PCR: hsa-let-7g-5p, hsa-miR-101-3p, hsa-miR-10a-5p, hsa-miR-10b-5p, hsa-miR-125b-5p, hsa-miR-128, hsa-miR-130a-3p, hsa-miR-134, hsa-miR-137, hsa-miR-146b-5p, hsa-miR-15a-5p, hsa-miR-15b-5p, hsa-miR-16-5p, hsa-miR-17-5p, hsa-miR-181a-5p,hsa-miR-182-5p, hsa-miR-185-5p, hsa-miR-18b-5p, hsa-miR-192-5p, hsa-miR-194-5p, hsa-miR-195-5p, hsa-miR-20a-5p, hsa-miR-20b-5p, hsa-miR-210, hsa-miR-21-5p, hsa-miR-218-5p,hsa-miR-219-5p,hsa-miR-222-3p, hsa-miR-22-3p, hsa-miR-23b-3p, hsa-miR-24-3p,hsa-miR-26a-5p, hsa-miR-301a-3p, hsa-miR-302a-3p,hsa-miR-302c-3p,hsa-miR-345-5p, hsa-miR-378a-3p, hsa-miR-7-5p, hsa-miR-92a-3p,hsa-miR-93-5p,hsa-miR-9-5p,hsa-miR-96-5p, and hsa-miR-99a-5p.

**Table 4: Cells EH**

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-let-7a-5p | UGAGGUAGUAGGUUGUAUAGUU | 1 | 22 | 75110 |
| hsa-miR-10a-5p | UACCCUGUAGAUCCGAAUUUGUG | 2 | 23 | 52927 |
| hsa-miR-100-5p | AACCCGUAGAUCCGAACUUGUG | 3 | 22 | 52451 |
| hsa-miR-99b-5p | CACCCGUAGAACCGACCUUGCG | 4 | 22 | 39457 |
| hsa-miR-486-5p | UCCUGUACUGAGCUGCCCCGAG | 5 | 22 | 20310 |
| hsa-miR-27b-3p | UUCACAGUGGCUAAGUUCUGC | 6 | 21 | 16900 |
| hsa-miR-92a-3p | UAUUGCACUUGUCCCGGCCUGU | 7 | 22 | 14359 |
| hsa-miR-191-5p | CAACGGAAUCCCAAAAGCAGCUG | 8 | 23 | 12591 |
| hsa-miR-21-5p | UAGCUUAUCAGACUGAUGUUGA | 9 | 22 | 11943 |
| hsa-miR-98 | UGAGGUAGUAAGUUGUAUUGUU | 10 | 22 | 11760 |
| hsa-let-7f-5p | UGAGGUAGUAGAUUGUAUAGUU | 11 | 22 | 10349 |
| hsa-miR-26a-5p | UUCAAGUAAUCCAGGAUAGGCU | 12 | 22 | 9900 |
| hsa-miR-92b-3p | UAUUGCACUCGUCCCGGCCUCC | 13 | 22 | 9794 |
| hsa-miR-127-3p | UCGGAUCCGUCUGAGCUUGGCU | 14 | 22 | 7064 |
| hsa-miR-181a-5p | AACAUUCAACGCUGUCGGUGAGU | 15 | 23 | 6956 |
| hsa-miR-182-5p | UUUGGCAAUGGUAGAACUCACACU | 16 | 24 | 5531 |
| hsa-let-7c | UGAGGUAGUAGGUUGUAUGGUU | 17 | 22 | 5103 |
| hsa-miR-379-5p | UGGUAGACUAUGGAACGUAGG | 18 | 21 | 4746 |
| hsa-miR-146b-5p | UGAGAACUGAAUUCCAUAGGCU | 19 | 22 | 4552 |
| hsa-miR-21-3p | CAACACCAGUCGAUGGGCUGU | 20 | 21 | 4089 |
| hsa-miR-1246 | AAUGGAUUUUUGGAGCAGG | 21 | 19 | 3973 |
| hsa-let-7i-5p | UGAGGUAGUAGUUUGUGCUGUU | 22 | 22 | 3015 |
| hsa-miR-4532 | CCCCGGGGAGCCCGGCG | 23 | 17 | 2847 |
| hsa-miR-183-5p | UAUGGCACUGGUAGAAUUCACU | 24 | 22 | 2695 |
| hsa-miR-151a-3p | CUAGACUGAAGCUCCUUGAGG | 25 | 21 | 2681 |
| hsa-miR-501-3p | AAUGCACCCGGGCAAGGAUUCU | 26 | 22 | 2649 |
| hsa-let-7e-5p | UGAGGUAGGAGGUUGUAUAGUU | 27 | 22 | 2449 |
| hsa-let-7b-5p | UGAGGUAGUAGGUUGUGUGGUU | 28 | 22 | 2435 |
| hsa-miR-16-5p | UAGCAGCACGUAAAUAUUGGCG | 29 | 22 | 2173 |
| hsa-miR-30a-5p | UGUAAACAUCCUCGACUGGAAG | 30 | 22 | 2001 |
| hsa-miR-30d-5p | UGUAAACAUCCCCGACUGGAAG | 31 | 22 | 1977 |
| hsa-miR-409-5p | AGGUUACCCGAGCAACUUUGCAU | 32 | 23 | 1871 |
| hsa-miR-22-3p | AAGCUGCCAGUUGAAGAACUGU | 33 | 22 | 1826 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-4492 | GGGGCUGGGCGCGCGCC | 34 | 17 | 1754 |
| hsa-miR-125a-5p | UCCCUGAGACCCUUUAACCUGUGA | 35 | 24 | 1451 |
| hsa-miR-222-3p | AGCUACAUCUGGCUACUGGGU | 36 | 21 | 1422 |
| hsa-miR-151a-5p | UCGAGGAGCUCACAGUCUAGU | 37 | 21 | 1386 |
| hsa-miR-181b-5p | AACAUUCAUUGCUGUCGGUGGGU | 38 | 23 | 1382 |
| hsa-miR-221-5p | ACCUGGCAUACAAUGUAGAUUU | 39 | 22 | 1363 |
| hsa-miR-186-5p | CAAAGAAUUCUCCUUUUGGGCU | 40 | 22 | 1225 |
| hsa-miR-423-5p | UGAGGGGCAGAGAGCGAGACUUU | 41 | 23 | 1080 |
| hsa-miR-125b-5p | UCCCUGAGACCCUAACUUGUGA | 42 | 22 | 1002 |
| hsa-let-7g-5p | UGAGGUAGUAGUUUGUACAGUU | 43 | 22 | 959 |
| hsa-miR-500a-3p | AUGCACCUGGGCAAGGAUUCUG | 44 | 22 | 923 |
| hsa-miR-30e-5p | UGUAAACAUCCUUGACUGGAAG | 45 | 22 | 911 |
| hsa-miR-27a-3p | UUCACAGUGGCUAAGUUCCGC | 46 | 21 | 867 |
| hsa-miR-409-3p | GAAUGUUGCUCGGUGAACCCCU | 47 | 22 | 865 |
| hsa-miR-148b-3p | UCAGUGCAUCACAGAACUUUGU | 48 | 22 | 856 |
| hsa-miR-125b-1-3p | ACGGGUUAGGCUCUUGGGAGCU | 49 | 22 | 851 |
| hsa-miR-410 | AAUAUAACACAGAUGGCCUGU | 50 | 21 | 848 |
| hsa-miR-381 | UAUACAAGGGCAAGCUCUCUGU | 51 | 22 | 842 |
| hsa-miR-99a-5p | AACCCGUAGAUCCGAUCUUGUG | 52 | 22 | 773 |
| hsa-let-7d-5p | AGAGGUAGUAGGUUGCAUAGUU | 53 | 22 | 765 |
| hsa-miR-148a-3p | UCAGUGCACUACAGAACUUUGU | 54 | 22 | 702 |
| hsa-miR-23a-3p | AUCACAUUGCCAGGGAUUUCC | 55 | 21 | 654 |
| hsa-miR-28-3p | CACUAGAUUGUGAGCUCCUGGA | 56 | 22 | 593 |
| hsa-miR-423-3p | AGCUCGGUCUGAGGCCCCUCAGU | 57 | 23 | 557 |
| hsa-miR-9-5p | UCUUUGGUUAUCUAGCUGUAUGA | 58 | 23 | 518 |
| hsa-miR-23b-3p | AUCACAUUGCCAGGGAUUACC | 59 | 21 | 508 |
| hsa-miR-941 | CACCCGGCUGUGUGCACAUGUGC | 60 | 23 | 492 |
| hsa-miR-4488 | AGGGGGCGGGCUCCGGCG | 61 | 18 | 485 |
| hsa-miR-103a-3p | AGCAGCAUUGUACAGGGCUAUGA | 62 | 23 | 459 |
| hsa-miR-25-3p | CAUUGCACUUGUCUCGGUCUGA | 63 | 22 | 436 |
| hsa-miR-889 | UUAAUAUCGGACAACCAUUGU | 64 | 21 | 411 |
| hsa-miR-378a-3p | ACUGGACUUGGAGUCAGAAGG | 65 | 21 | 410 |
| hsa-miR-30c-5p | UGUAAACAUCCUACACUCUCAGC | 66 | 23 | 378 |
| hsa-miR-4485 | UAACGGCCGCGGUACCCUAA | 67 | 20 | 358 |
| hsa-miR-125b-2-3p | UCACAAGUCAGGCUCUUGGGAC | 68 | 22 | 352 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-671-3p | UCCGGUUCUCAGGGCUCCACC | 69 | 21 | 350 |
| hsa-miR-361-5p | UUAUCAGAAUCUCCAGGGGUAC | 70 | 22 | 337 |
| hsa-miR-30e-3p | CUUUCAGUCGGAUGUUUACAGC | 71 | 22 | 294 |
| hsa-miR-1271-5p | CUUGGCACCUAGCAAGCACUCA | 72 | 22 | 288 |
| hsa-miR-589-5p | UGAGAACCACGUCUGCUCUGAG | 73 | 22 | 282 |
| hsa-miR-374a-5p | UUAUAAUACAACCUGAUAAGUG | 74 | 22 | 275 |
| hsa-miR-769-5p | UGAGACCUCUGGGUUCUGAGCU | 75 | 22 | 263 |
| hsa-miR-345-5p | GCUGACUCCUAGUCCAGGGCUC | 76 | 22 | 249 |
| hsa-miR-30a-3p | CUUUCAGUCGGAUGUUUGCAGC | 77 | 22 | 236 |
| hsa-miR-15b-5p | UAGCAGCACAUCAUGGUUUACA | 78 | 22 | 229 |
| hsa-miR-221-3p | AGCUACAUUGUCUGCUGGGUUUC | 79 | 23 | 225 |
| hsa-miR-31-5p | AGGCAAGAUGCUGGCAUAGCU | 80 | 21 | 213 |
| hsa-miR-342-3p | UCUCACACAGAAAUCGCACCCGU | 81 | 23 | 205 |
| hsa-miR-136-3p | CAUCAUCGUCUCAAAUGAGUCU | 82 | 22 | 203 |
| hsa-miR-493-3p | UGAAGGUCUACUGUGUGCCAGG | 83 | 22 | 192 |
| hsa-miR-720 | UCUCGCUGGGGCCUCCA | 84 | 17 | 154 |
| hsa-miR-7-5p | UGGAAGACUAGUGAUUUUGUUGU | 85 | 23 | 154 |
| hsa-miR-130b-3p | CAGUGCAAUGAUGAAAGGGCAU | 86 | 22 | 150 |
| hsa-miR-192-5p | CUGACCUAUGAAUUGACAGCC | 87 | 21 | 138 |
| hsa-miR-493-5p | UUGUACAUGGUAGGCUUUCAUU | 88 | 22 | 115 |
| hsa-miR-204-5p | UUCCCUUUGUCAUCCUAUGCCU | 89 | 22 | 113 |
| hsa-miR-26b-5p | UUCAAGUAAUUCAGGAUAGGU | 90 | 21 | 107 |
| hsa-miR-1307-5p | UCGACCGGACCUCGACCGGCU | 91 | 21 | 105 |
| hsa-let-7d-3p | CUAUACGACCUGCUGCCUUUCU | 92 | 22 | 103 |
| hsa-miR-340-5p | UUAUAAAGCAAUGAGACUGAUU | 93 | 22 | 100 |
| hsa-miR-134 | UGUGACUGGUUGACCAGAGGGG | 94 | 22 | 99 |
| hsa-miR-432-5p | UCUUGGAGUAGGUCAUUGGGUGG | 95 | 23 | 97 |
| hsa-miR-30b-5p | UGUAAACAUCCUACACUCAGCU | 96 | 22 | 96 |
| hsa-miR-320a | AAAAGCUGGGUUGAGAGGGCGA | 97 | 22 | 95 |
| hsa-miR-100-3p | CAAGCUUGUAUCUAUAGGUAUG | 98 | 22 | 94 |
| hsa-miR-744-5p | UGCGGGGCUAGGGCUAACAGCA | 99 | 22 | 89 |
| hsa-miR-181a-3p | ACCAUCGACCGUUGAUUGUACC | 100 | 22 | 86 |
| hsa-miR-34a-5p | UGGCAGUGUCUUAGCUGGUUGU | 101 | 22 | 85 |
| hsa-miR-181a-2-3p | ACCACUGACCGUUGACUGUACC | 102 | 22 | 81 |
| hsa-miR-190a | UGAUAUGUUUGAUAUAUUAGGU | 103 | 22 | 79 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-132-3p | UAACAGUCUACAGCCAUGGUCG | 104 | 22 | 78 |
| hsa-miR-181c-5p | AACAUUCAACCUGUCGGUGAGU | 105 | 22 | 76 |
| hsa-miR-29a-3p | UAGCACCAUCUGAAAUCGGUUA | 106 | 22 | 75 |
| hsa-miR-301a-3p | CAGUGCAAUAGUAUUGUCAAAGC | 107 | 23 | 75 |
| hsa-miR-411-5p | UAGUAGACCGUAUAGCGUACG | 108 | 21 | 75 |
| hsa-miR-128 | UCACAGUGAACCGGUCUCUUU | 109 | 21 | 74 |
| hsa-miR-4516 | GGGAGAAGGGUCGGGGC | 110 | 17 | 74 |
| hsa-miR-425-5p | AAUGACACGAUCACUCCCGUUGA | 111 | 23 | 72 |
| hsa-miR-130b-5p | ACUCUUUCCCUGUUGCACUAC | 112 | 21 | 71 |
| hsa-miR-130a-3p | CAGUGCAAUGUUAAAAGGGCAU | 113 | 22 | 67 |
| hsa-miR-30d-3p | CUUUCAGUCAGAUGUUUGCUGC | 114 | 22 | 65 |
| hsa-miR-654-5p | UGGUGGGCCGCAGAACAUGUGC | 115 | 22 | 65 |
| hsa-miR-93-5p | CAAAGUGCUGUUCGUGCAGGUAG | 116 | 23 | 65 |
| hsa-miR-487b | AAUCGUACAGGGUCAUCCACUU | 117 | 22 | 63 |
| hsa-miR-484 | UCAGGCUCAGUCCCCUCCCGAU | 118 | 22 | 62 |
| hsa-miR-24-3p | UGGCUCAGUUCAGCAGGAACAG | 119 | 22 | 61 |
| hsa-miR-4677-3p | UCUGUGAGACCAAAGAACUACU | 120 | 22 | 61 |
| hsa-miR-149-5p | UCUGGCUCCGUGUCUUCACUCCC | 121 | 23 | 56 |
| hsa-miR-197-3p | UUCACCACCUUCUCCACCCAGC | 122 | 22 | 56 |
| hsa-miR-96-5p | UUUGGCACUAGCACAUUUUUGCU | 123 | 23 | 56 |
| hsa-miR-1307-3p | ACUCGGCGUGGCGUCGGUCGUG | 124 | 22 | 55 |
| hsa-miR-34c-5p | AGGCAGUGUAGUUAGCUGAUUGC | 125 | 23 | 53 |
| hsa-miR-370 | GCCUGCUGGGGUGGAACCUGGU | 126 | 22 | 52 |
| hsa-miR-148b-5p | AAGUUCUGUUAUACACUCAGGC | 127 | 22 | 51 |
| hsa-miR-335-5p | UCAAGAGCAAUAACGAAAAAUGU | 128 | 23 | 51 |
| hsa-miR-4461 | GAUUGAGACUAGUAGGGCUAGGC | 129 | 23 | 50 |
| hsa-miR-27a-5p | AGGGCUUAGCUGCUUGUGAGCA | 130 | 22 | 49 |
| hsa-miR-363-3p | AAUUGCACGGUAUCCAUCUGUA | 131 | 22 | 47 |
| hsa-miR-431-5p | UGUCUUGCAGGCCGUCAUGCA | 132 | 21 | 47 |
| hsa-miR-877-5p | GUAGAGGAGAUGGCGCAGGG | 133 | 20 | 46 |
| hsa-miR-550a-5p | AGUGCCUGAGGGAGUAAGAGCCC | 134 | 23 | 45 |
| hsa-miR-4508 | GCGGGGCUGGGCGCGCG | 135 | 17 | 44 |
| hsa-miR-541-3p | UGGUGGGCACAGAAUCUGGACU | 136 | 22 | 42 |
| hsa-miR-135b-5p | UAUGGCUUUUCAUUCCUAUGUGA | 137 | 23 | 40 |
| hsa-miR-140-3p | UACCACAGGGUAGAACCACGG | 138 | 21 | 39 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-362-5p | AAUCCUUGGAACCUAGGUGUGAGU | 139 | 24 | 37 |
| hsa-miR-455-3p | GCAGUCCAUGGGCAUAUACAC | 140 | 21 | 37 |
| hsa-miR-758 | UUUGUGACCUGGUCCACUAACC | 141 | 22 | 37 |
| hsa-miR-101-3p | UACAGUACUGUGAUAACUGAA | 142 | 21 | 36 |
| hsa-miR-374b-5p | AUAUAAUACAACCUGCUAAGUG | 143 | 22 | 36 |
| hsa-miR-148a-5p | AAAGUUCUGAGACACUCCGACU | 144 | 22 | 35 |
| hsa-miR-17-5p | CAAAGUGCUUACAGUGCAGGUAG | 145 | 23 | 35 |
| hsa-miR-20a-5p | UAAAGUGCUUAUAGUGCAGGUAG | 146 | 23 | 35 |
| hsa-miR-874 | CUGCCCUGGCCCGAGGGACCGA | 147 | 22 | 35 |
| hsa-miR-193b-3p | AACUGGCCCUCAAAGUCCCGCU | 148 | 22 | 34 |
| hsa-miR-548ah-3p | CAAAAACUGCAGUUACUUUUGC | 149 | 22 | 34 |
| hsa-miR-539-3p | AUCAUACAAGGACAAUUUCUUU | 150 | 22 | 33 |
| hsa-miR-421 | AUCAACAGACAUUAAUUGGGCGC | 151 | 23 | 31 |
| hsa-miR-28-5p | AAGGAGCUCACAGUCUAUUGAG | 152 | 22 | 30 |
| hsa-miR-485-3p | GUCAUACACGGCUCUCCUCUCU | 153 | 22 | 29 |
| hsa-miR-2467-5p | UGAGGCUCUGUUAGCCUUGGCUC | 154 | 23 | 26 |
| hsa-miR-4449 | CGUCCCGGGGCUGCGCGAGGCA | 155 | 22 | 26 |
| hsa-miR-24-2-5p | UGCCUACUGAGCUGAAACACAG | 156 | 22 | 25 |
| hsa-miR-181d | AACAUUCAUUGUUGUCGGUGGGU | 157 | 23 | 24 |
| hsa-miR-323a-3p | CACAUUACACGGUCGACCUCU | 158 | 21 | 24 |
| hsa-miR-106b-3p | CCGCACUGUGGGUACUUGCUGC | 159 | 22 | 23 |
| hsa-miR-125a-3p | ACAGGUGAGGUUCUUGGGAGCC | 160 | 22 | 23 |
| hsa-miR-330-5p | UCUCUGGGCCUGUGUCUUAGGC | 161 | 22 | 23 |
| hsa-miR-1275 | GUGGGGGAGAGGCUGUC | 162 | 17 | 22 |
| hsa-miR-19b-3p | UGUGCAAAUCCAUGCAAAACUGA | 163 | 23 | 22 |
| hsa-miR-301b | CAGUGCAAUGAUAUUGUCAAAGC | 164 | 23 | 21 |
| hsa-miR-485-5p | AGAGGCUGGCCGUGAUGAAUUC | 165 | 22 | 21 |
| hsa-miR-29b-3p | UAGCACCAUUUGAAAUCAGUGUU | 166 | 23 | 20 |
| hsa-miR-3158-3p | AAGGGCUUCCUCUCUGCAGGAC | 167 | 22 | 20 |
| hsa-miR-431-3p | CAGGUCGUCUUGCAGGGCUUCU | 168 | 22 | 20 |
| hsa-miR-454-3p | UAGUGCAAUAUUGCUUAUAGGGU | 169 | 23 | 20 |
| hsa-miR-106b-5p | UAAAGUGCUGACAGUGCAGAU | 170 | 21 | 19 |
| hsa-miR-1973 | ACCGUGCAAAGGUAGCAUA | 171 | 19 | 19 |
| hsa-miR-31-3p | UGCUAUGCCAACAUAUUGCCAU | 172 | 22 | 19 |
| hsa-miR-374a-3p | CUUAUCAGAUUGUAUUGUAAUU | 173 | 22 | 19 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-433 | AUCAUGAUGGGCUCCUCGGUGU | 174 | 22 | 19 |
| hsa-miR-4417 | GGUGGGCUUCCCGGAGGG | 175 | 18 | 19 |
| hsa-miR-143-3p | UGAGAUGAAGCACUGUAGCUC | 176 | 21 | 18 |
| hsa-miR-19a-3p | UGUGCAAAUCUAUGCAAAACUGA | 177 | 23 | 18 |
| hsa-miR-532-5p | CAUGCCUUGAGUGUAGGACCGU | 178 | 22 | 18 |
| hsa-miR-561-5p | AUCAAGGAUCUUAAACUUUGCC | 179 | 22 | 18 |
| hsa-miR-663b | GGUGGCCCGGCCGUGCCUGAGG | 180 | 22 | 18 |
| hsa-miR-1301 | UUGCAGCUGCCUGGGAGUGACUUC | 181 | 24 | 17 |
| hsa-miR-299-3p | UAUGUGGGAUGGUAAACCGCUU | 182 | 22 | 17 |
| hsa-miR-9-3p | AUAAAGCUAGAUAACCGAAAGU | 183 | 22 | 17 |
| hsa-miR-17-3p | ACUGCAGUGAAGGCACUUGUAG | 184 | 22 | 15 |
| hsa-miR-376c | AACAUAGAGGAAAUUCCACGU | 185 | 21 | 15 |
| hsa-miR-424-5p | CAGCAGCAAUUCAUGUUUUGAA | 186 | 22 | 15 |
| hsa-miR-660-5p | UACCCAUUGCAUAUCGGAGUUG | 187 | 22 | 15 |
| hsa-miR-153 | UUGCAUAGUCACAAAAGUGAUC | 188 | 22 | 14 |
| hsa-miR-3605-5p | UGAGGAUGGAUAGCAAGGAAGCC | 189 | 23 | 14 |
| hsa-miR-3687 | CCCGGACAGGCGUUCGUGCGACGU | 190 | 24 | 14 |
| hsa-miR-4284 | GGGCUCACAUCACCCCAU | 191 | 18 | 14 |
| hsa-miR-455-5p | UAUGUGCCUUUGGACUACAUCG | 192 | 22 | 14 |
| hsa-miR-543 | AAACAUUCGCGGUGCACUUCUU | 193 | 22 | 14 |
| hsa-miR-1276 | UAAAGAGCCCUGUGGAGACA | 194 | 20 | 13 |
| hsa-miR-330-3p | GCAAAGCACACGGCCUGCAGAGA | 195 | 23 | 13 |
| hsa-miR-369-3p | AAUAAUACAUGGUUGAUCUUU | 196 | 21 | 13 |
| hsa-miR-4786-5p | UGAGACCAGGACUGGAUGCACC | 197 | 22 | 13 |
| hsa-miR-548k | AAAAGUACUUGCGGAUUUUGCU | 198 | 22 | 13 |
| hsa-miR-1226-3p | UCACCAGCCCUGUGUUCCCUAG | 199 | 22 | 12 |
| hsa-miR-188-3p | CUCCCACAUGCAGGGUUUGCA | 200 | 21 | 12 |
| hsa-miR-27b-5p | AGAGCUUAGCUGAUUGGUGAAC | 201 | 22 | 12 |
| hsa-miR-377-5p | AGAGGUUGCCCUUGGUGAAUUC | 202 | 22 | 12 |
| hsa-miR-487a | AAUCAUACAGGGACAUCCAGUU | 203 | 22 | 12 |
| hsa-miR-92a-1-5p | AGGUUGGGAUCGGUUGCAAUGCU | 204 | 23 | 12 |
| hsa-miR-135b-3p | AUGUAGGGCUAAAAGCCAUGGG | 205 | 22 | 11 |
| hsa-miR-218-5p | UUGUGCUUGAUCUAACCAUGU | 206 | 21 | 11 |
| hsa-miR-3943 | UAGCCCCCAGGCUUCACUUGGCG | 207 | 23 | 11 |
| hsa-miR-92b-5p | AGGGACGGGACGCGGUGCAGUG | 208 | 22 | 11 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-1185-1-3p | AUAUACAGGGGGAGACUCUUAU | 209 | 22 | 10 |
| hsa-miR-1273g-3p | ACCACUGCACUCCAGCCUGAG | 210 | 21 | 10 |
| hsa-miR-2355-5p | AUCCCCAGAUACAAUGGACAA | 211 | 21 | 10 |
| hsa-miR-23a-5p | GGGGUUCCUGGGGAUGGGAUUU | 212 | 22 | 10 |
| hsa-miR-30c-1-3p | CUGGGAGAGGGUUGUUUACUCC | 213 | 22 | 10 |
| hsa-miR-329 | AACACACCUGGUUAACCUCUUU | 214 | 22 | 10 |
| hsa-miR-337-3p | CUCCUAUAUGAUGCCUUUCUUC | 215 | 22 | 10 |
| hsa-miR-3609 | CAAAGUGAUGAGUAAUACUGGCUG | 216 | 24 | 10 |
| hsa-miR-378a-5p | CUCCUGACUCCAGGUCCUGUGU | 217 | 22 | 10 |
| hsa-miR-3929 | GAGGCUGAUGUGAGUAGACCACU | 218 | 23 | 10 |
| hsa-miR-4745-5p | UGAGUGGGGCUCCCGGGACGGCG | 219 | 23 | 10 |
| hsa-miR-5096 | GUUUCACCAUGUUGGUCAGGC | 220 | 21 | 10 |
| hsa-miR-656 | AAUAUUAUACAGUCAACCUCU | 221 | 21 | 10 |
| hsa-let-7a-3p | CUAUACAAUCUACUGUCUUUC | 222 | 21 | 9 |
| hsa-miR-15a-5p | UAGCAGCACAUAAUGGUUUGUG | 223 | 22 | 9 |
| hsa-miR-185-5p | UGGAGAGAAAGGCAGUUCCUGA | 224 | 22 | 9 |
| hsa-miR-25-5p | AGGCGGAGACUUGGGCAAUUG | 225 | 21 | 9 |
| hsa-miR-3065-5p | UCAACAAAAUCACUGAUGCUGGA | 226 | 23 | 9 |
| hsa-miR-3176 | ACUGGCCUGGGACUACCGG | 227 | 19 | 9 |
| hsa-miR-339-3p | UGAGCGCCUCGACGACAGAGCCG | 228 | 23 | 9 |
| hsa-miR-374b-3p | CUUAGCAGGUUGUAUUAUCAUU | 229 | 22 | 9 |
| hsa-miR-4435 | AUGGCCAGAGCUCACACAGAGG | 230 | 22 | 9 |
| hsa-miR-4448 | GGCUCCUUGGUCUAGGGGUA | 231 | 20 | 9 |
| hsa-miR-4497 | CUCCGGGACGGCUGGGC | 232 | 17 | 9 |
| hsa-miR-4521 | GCUAAGGAAGUCCUGUGCUCAG | 233 | 22 | 9 |
| hsa-miR-539-5p | GGAGAAAUUAUCCUUGGUGUGU | 234 | 22 | 9 |
| hsa-miR-548ah-5p | AAAAGUGAUUGCAGUGUUUG | 235 | 20 | 9 |
| hsa-miR-1910 | CCAGUCCUGUGCCUGCCGCCU | 236 | 21 | 8 |
| hsa-miR-376a-3p | AUCAUAGAGGAAAAUCCACGU | 237 | 21 | 8 |
| hsa-miR-382-5p | GAAGUUGUUCGUGGUGGAUUCG | 238 | 22 | 8 |
| hsa-miR-3940-3p | CAGCCCGGAUCCCAGCCCACUU | 239 | 22 | 8 |
| hsa-miR-494 | UGAAACAUACACGGGAAACCUC | 240 | 22 | 8 |
| hsa-miR-495 | AAACAAACAUGGUGCACUUCUU | 241 | 22 | 8 |
| hsa-miR-545-3p | UCAGCAAACAUUUAUUGUGUGC | 242 | 22 | 8 |
| hsa-miR-99b-3p | CAAGCUCGUGUCUGUGGGUCCG | 243 | 22 | 8 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-1197 | UAGGACACAUGGUCUACUUCU | 244 | 21 | 7 |
| hsa-miR-181b-3p | CUCACUGAACAAUGAAUGCAA | 245 | 21 | 7 |
| hsa-miR-212-5p | ACCUUGGCUCUAGACUGCUUACU | 246 | 23 | 7 |
| hsa-miR-3200-3p | CACCUUGCGCUACUCAGGUCUG | 247 | 22 | 7 |
| hsa-miR-340-3p | UCCGUCUCAGUUACUUUAUAGC | 248 | 22 | 7 |
| hsa-miR-3607-5p | GCAUGUGAUGAAGCAAAUCAGU | 249 | 22 | 7 |
| hsa-miR-361-3p | UCCCCCAGGUGUGAUUCUGAUUU | 250 | 23 | 7 |
| hsa-miR-3656 | GGCGGGUGCGGGGGUGG | 251 | 17 | 7 |
| hsa-miR-532-3p | CCUCCCACACCCAAGGCUUGCA | 252 | 22 | 7 |
| hsa-miR-574-3p | CACGCUCAUGCACACACCCACA | 253 | 22 | 7 |
| hsa-miR-107 | AGCAGCAUUGUACAGGGCUAUCA | 254 | 23 | 6 |
| hsa-miR-127-5p | CUGAAGCUCAGAGGGCUCUGAU | 255 | 22 | 6 |
| hsa-miR-18a-5p | UAAGGUGCAUCUAGUGCAGAUAG | 256 | 23 | 6 |
| hsa-miR-26a-2-3p | CCUAUUCUUGAUUACUUGUUUC | 257 | 22 | 6 |
| hsa-miR-296-5p | AGGGCCCCCCCUCAAUCCUGU | 258 | 21 | 6 |
| hsa-miR-3648 | AGCCGCGGGGAUCGCCGAGGG | 259 | 21 | 6 |
| hsa-miR-382-3p | AAUCAUUCACGGACAACACUU | 260 | 21 | 6 |
| hsa-miR-3939 | UACGCGCAGACCACAGGAUGUC | 261 | 22 | 6 |
| hsa-miR-432-3p | CUGGAUGGCUCCUCCAUGUCU | 262 | 21 | 6 |
| hsa-miR-4423-5p | AGUUGCCUUUUUGUUCCCAUGC | 263 | 22 | 6 |
| hsa-miR-4466 | GGGUGCGGGCCGGCGGGG | 264 | 18 | 6 |
| hsa-miR-454-5p | ACCCUAUCAAUAUUGUCUCUGC | 265 | 22 | 6 |
| hsa-miR-4746-5p | CCGGUCCCAGGAGAACCUGCAGA | 266 | 23 | 6 |
| hsa-miR-496 | UGAGUAUUACAUGGCCAAUCUC | 267 | 22 | 6 |
| hsa-miR-548o-3p | CCAAAACUGCAGUUACUUUUGC | 268 | 22 | 6 |
| hsa-miR-1248 | ACCUUCUUGUAUAAGCACUGUGCUAAA | 269 | 27 | 5 |
| hsa-miR-1254 | AGCCUGGAAGCUGGAGCCUGCAGU | 270 | 24 | 5 |
| hsa-miR-1296 | UUAGGGCCCUGGCUCCAUCUCC | 271 | 22 | 5 |
| hsa-miR-136-5p | ACUCCAUUUGUUUUGAUGAUGGA | 272 | 23 | 5 |
| hsa-miR-199a-5p | CCCAGUGUUCAGACUACCUGUUC | 273 | 23 | 5 |
| hsa-miR-296-3p | GAGGGUUGGGUGGAGGCUCUCC | 274 | 22 | 5 |
| hsa-miR-3177-3p | UGCACGGCACUGGGGACACGU | 275 | 21 | 5 |
| hsa-miR-324-3p | ACUGCCCCAGGUGCUGCUGG | 276 | 20 | 5 |
| hsa-miR-337-5p | GAACGGCUUCAUACAGGAGUU | 277 | 21 | 5 |
| hsa-miR-342-5p | AGGGGUGCUAUCUGUGAUUGA | 278 | 21 | 5 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-365b-3p | UAAUGCCCCUAAAAAUCCUUAU | 279 | 22 | 5 |
| hsa-miR-3676-5p | AGGAGAUCCUGGGUU | 280 | 15 | 5 |
| hsa-miR-502-3p | AAUGCACCUGGGCAAGGAUUCA | 281 | 22 | 5 |
| hsa-miR-505-3p | CGUCAACACUUGCUGGUUUCCU | 282 | 22 | 5 |
| hsa-miR-550a-3p | UGUCUUACUCCCUCAGGCACAU | 283 | 22 | 5 |
| hsa-miR-5587-3p | GCCCCGGGCAGUGUGAUCAUC | 284 | 21 | 5 |
| hsa-miR-641 | AAAGACAUAGGAUAGAGUCACCUC | 285 | 24 | 5 |
| hsa-miR-655 | AUAAUACAUGGUUAACCUCUUU | 286 | 22 | 5 |
| hsa-miR-664-3p | UAUUCAUUUAUCCCCAGCCUACA | 287 | 23 | 5 |
| hsa-miR-671-5p | AGGAAGCCCUGGAGGGGCUGGAG | 288 | 23 | 5 |
| hsa-miR-760 | CGGCUCUGGGUCUGUGGGGA | 289 | 20 | 5 |
| hsa-let-7e-3p | CUAUACGGCCUCCUAGCUUUCC | 290 | 22 | 4 |
| hsa-miR-1268a | CGGGCGUGGUGGUGGGGG | 291 | 18 | 4 |
| hsa-miR-1273f | GGAGAUGGAGGUUGCAGUG | 292 | 19 | 4 |
| hsa-miR-1286 | UGCAGGACCAAGAUGAGCCCU | 293 | 21 | 4 |
| hsa-miR-1291 | UGGCCCUGACUGAAGACCAGCAGU | 294 | 24 | 4 |
| hsa-miR-141-3p | UAACACUGUCUGGUAAAGAUGG | 295 | 22 | 4 |
| hsa-miR-1468 | CUCCGUUUGCCUGUUUCGCUG | 296 | 21 | 4 |
| hsa-miR-328 | CUGGCCCUCUCUGCCCUUCCGU | 297 | 22 | 4 |
| hsa-miR-424-3p | CAAAACGUGAGGCGCUGCUAU | 298 | 21 | 4 |
| hsa-miR-4454 | GGAUCCGAGUCACGGCACCA | 299 | 20 | 4 |
| hsa-miR-4463 | GAGACUGGGGUGGGGCC | 300 | 17 | 4 |
| hsa-miR-4671-3p | UUAGUGCAUAGUCUUUGGUCU | 301 | 21 | 4 |
| hsa-miR-4775 | UUAAUUUUUUGUUUCGGUCACU | 302 | 22 | 4 |
| hsa-miR-500a-5p | UAAUCCUUGCUACCUGGGUGAGA | 303 | 23 | 4 |
| hsa-miR-548b-5p | AAAAGUAAUUGUGGUUUUGGCC | 304 | 22 | 4 |
| hsa-miR-573 | CUGAAGUGAUGUGUAACUGAUCAG | 305 | 24 | 4 |
| hsa-miR-576-5p | AUUCUAAUUUCUCCACGUCUUU | 306 | 22 | 4 |
| hsa-miR-625-3p | GACUAUAGAACUUUCCCCCUCA | 307 | 22 | 4 |
| hsa-miR-652-3p | AAUGGCGCCACUAGGGUUGUG | 308 | 21 | 4 |
| hsa-miR-665 | ACCAGGAGGCUGAGGCCCCU | 309 | 20 | 4 |
| hsa-miR-766-3p | ACUCCAGCCCCACAGCCUCAGC | 310 | 22 | 4 |
| hsa-miR-935 | CCAGUUACCGCUUCCGCUACCGC | 311 | 23 | 4 |
| hsa-miR-937 | AUCCGCGCUCUGACUCUCUGCC | 312 | 22 | 4 |
| hsa-miR-1180 | UUUCCGGCUCGCGUGGGUGUGU | 313 | 22 | 3 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-1185-2-3p | AUAUACAGGGGGAGACUCUCAU | 314 | 22 | 3 |
| hsa-miR-132-5p | ACCGUGGCUUUCGAUUGUUACU | 315 | 22 | 3 |
| hsa-miR-16-2-3p | CCAAUAUUACUGUGCUGCUUUA | 316 | 22 | 3 |
| hsa-miR-20b-5p | CAAAGUGCUCAUAGUGCAGGUAG | 317 | 23 | 3 |
| hsa-miR-2116-3p | CCUCCCAUGCCAAGAACUCCC | 318 | 21 | 3 |
| hsa-miR-299-5p | UGGUUUACCGUCCCACAUACAU | 319 | 22 | 3 |
| hsa-miR-30b-3p | CUGGGAGGUGGAUGUUUACUUC | 320 | 22 | 3 |
| hsa-miR-30c-2-3p | CUGGGAGAAGGCUGUUUACUCU | 321 | 22 | 3 |
| hsa-miR-3187-3p | UUGGCCAUGGGGCUGCGCGG | 322 | 20 | 3 |
| hsa-miR-3615 | UCUCUCGGCUCCUCGCGGCUC | 323 | 21 | 3 |
| hsa-miR-3620 | UCACCCUGCAUCCCGCACCCAG | 324 | 22 | 3 |
| hsa-miR-3654 | GACUGGACAAGCUGAGGAA | 325 | 19 | 3 |
| hsa-miR-3662 | GAAAAUGAUGAGUAGUGACUGAUG | 326 | 24 | 3 |
| hsa-miR-3681-5p | UAGUGGAUGAUGCACUCUGUGC | 327 | 22 | 3 |
| hsa-miR-4286 | ACCCCACUCCUGGUACC | 328 | 17 | 3 |
| hsa-miR-4640-3p | CACCCCCUGUUUCCUGGCCCAC | 329 | 22 | 3 |
| hsa-miR-4717-3p | ACACAUGGGUGGCUGUGGCCU | 330 | 21 | 3 |
| hsa-miR-542-3p | UGUGACAGAUUGAUAACUGAAA | 331 | 22 | 3 |
| hsa-miR-5584-5p | CAGGGAAAUGGGAAGAACUAGA | 332 | 22 | 3 |
| hsa-miR-570-3p | CGAAAACAGCAAUUACCUUUGC | 333 | 22 | 3 |
| hsa-miR-574-5p | UGAGUGUGUGUGUGUGAGUGUGU | 334 | 23 | 3 |
| hsa-miR-628-3p | UCUAGUAAGAGUGGCAGUCGA | 335 | 21 | 3 |
| hsa-miR-654-3p | UAUGUCUGCUGACCAUCACCUU | 336 | 22 | 3 |
| hsa-miR-769-3p | CUGGGAUCUCCGGGGUCUUGGUU | 337 | 23 | 3 |
| hsa-miR-943 | CUGACUGUUGCCGUCCUCCAG | 338 | 21 | 3 |
| hsa-let-7b-3p | CUAUACAACCUACUGCCUUCCC | 339 | 22 | 2 |
| hsa-miR-1244 | AAGUAGUUGGUUUGUAUGAGAUGGUU | 340 | 26 | 2 |
| hsa-miR-1255a | AGGAUGAGCAAAGAAAGUAGAUU | 341 | 23 | 2 |
| hsa-miR-1273e | UUGCUUGAACCCAGGAAGUGGA | 342 | 22 | 2 |
| hsa-miR-1289 | UGGAGUCCAGGAAUCUGCAUUUU | 343 | 23 | 2 |
| hsa-miR-152 | UCAGUGCAUGACAGAACUUGG | 344 | 21 | 2 |
| hsa-miR-194-5p | UGUAACAGCAACUCCAUGUGGA | 345 | 22 | 2 |
| hsa-miR-195-5p | UAGCAGCACAGAAAUAUUGGC | 346 | 21 | 2 |
| hsa-miR-200c-3p | UAAUACUGCCGGGUAAUGAUGGA | 347 | 23 | 2 |
| hsa-miR-212-3p | UAACAGUCUCCAGUCACGGCC | 348 | 21 | 2 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-222-5p | CUCAGUAGCCAGUGUAGAUCCU | 349 | 22 | 2 |
| hsa-miR-3065-3p | UCAGCACCAGGAUAUUGUUGGAG | 350 | 23 | 2 |
| hsa-miR-3115 | AUAUGGGUUUACUAGUUGGU | 351 | 20 | 2 |
| hsa-miR-3126-5p | UGAGGGACAGAUGCCAGAAGCA | 352 | 22 | 2 |
| hsa-miR-3174 | UAGUGAGUUAGAGAUGCAGAGCC | 353 | 23 | 2 |
| hsa-miR-324-5p | CGCAUCCCCUAGGGCAUUGGUGU | 354 | 23 | 2 |
| hsa-miR-33a-5p | GUGCAUUGUAGUUGCAUUGCA | 355 | 21 | 2 |
| hsa-miR-3677-3p | CUCGUGGGCUCUGGCCACGGCC | 356 | 22 | 2 |
| hsa-miR-369-5p | AGAUCGACCGUGUUAUAUUCGC | 357 | 22 | 2 |
| hsa-miR-425-3p | AUCGGGAAUGUCGUGUCCGCCC | 358 | 22 | 2 |
| hsa-miR-4426 | GAAGAUGGACGUACUUU | 359 | 17 | 2 |
| hsa-miR-4467 | UGGCGGCGGUAGUUAUGGGCUU | 360 | 22 | 2 |
| hsa-miR-4482-3p | UUUCUAUUUCUCAGUGGGGCUC | 361 | 22 | 2 |
| hsa-miR-4515 | AGGACUGGACUCCCGGCAGCCC | 362 | 22 | 2 |
| hsa-miR-4792 | CGGUGAGCGCUCGCUGGC | 363 | 18 | 2 |
| hsa-miR-659-5p | AGGACCUUCCCUGAACCAAGGA | 364 | 22 | 2 |
| hsa-miR-663a | AGGCGGGGCGCCGCGGGACCGC | 365 | 22 | 2 |
| hsa-miR-940 | AAGGCAGGGCCCCCGCUCCCC | 366 | 21 | 2 |
| hsa-miR-99a-3p | CAAGCUCGCUUCUAUGGGUCUG | 367 | 22 | 2 |
| hsa-miR-1185-5p | AGAGGAUACCCUUUGUAUGUU | 368 | 21 | 1 |
| hsa-miR-1225-3p | UGAGCCCCUGUGCCGCCCCCAG | 369 | 22 | 1 |
| hsa-miR-1237 | UCCUUCUGCUCCGUCCCCCAG | 370 | 21 | 1 |
| hsa-miR-1252 | AGAAGGAAAUUGAAUUCAUUUA | 371 | 22 | 1 |
| hsa-miR-1257 | AGUGAAUGAUGGGUUCUGACC | 372 | 21 | 1 |
| hsa-miR-1260b | AUCCCACCACUGCCACCAU | 373 | 19 | 1 |
| hsa-miR-1273d | GAACCCAUGAGGUUGAGGCUGCAGU | 374 | 25 | 1 |
| hsa-miR-1290 | UGGAUUUUUGGAUCAGGGA | 375 | 19 | 1 |
| hsa-miR-1306-3p | ACGUUGGCUCUGGUGGUG | 376 | 18 | 1 |
| hsa-miR-1321 | CAGGGAGGUGAAUGUGAU | 377 | 18 | 1 |
| hsa-miR-1343 | CUCCUGGGGCCCGCACUCUCGC | 378 | 22 | 1 |
| hsa-miR-138-5p | AGCUGGUGUUGUGAAUCAGGCCG | 379 | 23 | 1 |
| hsa-miR-140-5p | CAGUGGUUUUACCCUAUGGUAG | 380 | 22 | 1 |
| hsa-miR-146b-3p | UGCCCUGUGGACUCAGUUCUGG | 381 | 22 | 1 |
| hsa-miR-186-3p | GCCCAAAGGUGAAUUUUUUGGG | 382 | 22 | 1 |
| hsa-miR-1908 | CGGCGGGGACGGCGAUUGGUC | 383 | 21 | 1 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-1915-3p | CCCCAGGGCGACGCGGCGGG | 384 | 20 | 1 |
| hsa-miR-1915-5p | ACCUUGCCUUGCUGCCCGGGCC | 385 | 22 | 1 |
| hsa-miR-193a-3p | AACUGGCCUACAAAGUCCCAGU | 386 | 22 | 1 |
| hsa-miR-19b-1-5p | AGUUUUGCAGGUUUGCAUCCAGC | 387 | 23 | 1 |
| hsa-miR-208b | AUAAGACGAACAAAAGGUUUGU | 388 | 22 | 1 |
| hsa-miR-2110 | UUGGGGAAACGGCCGCUGAGUG | 389 | 22 | 1 |
| hsa-miR-219-1-3p | AGAGUUGAGUCUGGACGUCCCG | 390 | 22 | 1 |
| hsa-miR-26b-3p | CCUGUUCUCCAUUACUUGGCUC | 391 | 22 | 1 |
| hsa-miR-2964a-3p | AGAAUUGCGUUUGGACAAUCAGU | 392 | 23 | 1 |
| hsa-miR-29a-5p | ACUGAUUUCUUUUGGUGUUCAG | 393 | 22 | 1 |
| hsa-miR-3126-3p | CAUCUGGCAUCCGUCACACAGA | 394 | 22 | 1 |
| hsa-miR-3130-3p | GCUGCACCGGAGACUGGGUAA | 395 | 21 | 1 |
| hsa-miR-3130-5p | UACCCAGUCUCCGGUGCAGCC | 396 | 21 | 1 |
| hsa-miR-3140-5p | ACCUGAAUUACCAAAAGCUUU | 397 | 21 | 1 |
| hsa-miR-3155a | CCAGGCUCUGCAGUGGGAACU | 398 | 21 | 1 |
| hsa-miR-3157-3p | CUGCCCUAGUCUAGCUGAAGCU | 399 | 22 | 1 |
| hsa-miR-3180-3p | UGGGGCGGAGCUUCCGGAGGCC | 400 | 22 | 1 |
| hsa-miR-323b-5p | AGGUUGUCCGUGGUGAGUUCGCA | 401 | 23 | 1 |
| hsa-miR-339-5p | UCCCUGUCCUCCAGGAGCUCACG | 402 | 23 | 1 |
| hsa-miR-34a-3p | CAAUCAGCAAGUAUACUGCCCU | 403 | 22 | 1 |
| hsa-miR-34b-3p | CAAUCACUAACUCCACUGCCAU | 404 | 22 | 1 |
| hsa-miR-34c-3p | AAUCACUAACCACACGGCCAGG | 405 | 22 | 1 |
| hsa-miR-3658 | UUUAAGAAAACACCAUGGAGAU | 406 | 22 | 1 |
| hsa-miR-365a-5p | AGGGACUUUUGGGGGCAGAUGUG | 407 | 23 | 1 |
| hsa-miR-3676-3p | CCGUGUUUCCCCCACGCUUU | 408 | 20 | 1 |
| hsa-miR-3691-5p | AGUGGAUGAUGGAGACUCGGUAC | 409 | 23 | 1 |
| hsa-miR-376a-5p | GUAGAUUCUCCUUCUAUGAGUA | 410 | 22 | 1 |
| hsa-miR-378g | ACUGGGCUUGGAGUCAGAAG | 411 | 20 | 1 |
| hsa-miR-3909 | UGUCCUCUAGGGCCUGCAGUCU | 412 | 22 | 1 |
| hsa-miR-3928 | GGAGGAACCUUGGAGCUUCGGC | 413 | 22 | 1 |
| hsa-miR-3942-3p | UUUCAGAUAACAGUAUUACAU | 414 | 21 | 1 |
| hsa-miR-3944-5p | UGUGCAGCAGGCCAACCGAGA | 415 | 21 | 1 |
| hsa-miR-3960 | GGCGGCGGCGGAGGCGGGGG | 416 | 20 | 1 |
| hsa-miR-4326 | UGUUCCUCUGUCUCCCAGAC | 417 | 20 | 1 |
| hsa-miR-4444 | CUCGAGUUGGAAGAGGCG | 418 | 18 | 1 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-4450 | UGGGGAUUUGGAGAAGUGGUGA | 419 | 22 | 1 |
| hsa-miR-4642 | AUGGCAUCGUCCCCUGGUGGCU | 420 | 22 | 1 |
| hsa-miR-4668-5p | AGGGAAAAAAAAAAGGAUUUGUC | 421 | 23 | 1 |
| hsa-miR-4673 | UCCAGGCAGGAGCCGGACUGGA | 422 | 22 | 1 |
| hsa-miR-4688 | UAGGGGCAGCAGAGGACCUGGG | 423 | 22 | 1 |
| hsa-miR-4700-3p | CACAGGACUGACUCCUCACCCCAGUG | 424 | 26 | 1 |
| hsa-miR-4731-3p | CACACAAGUGGCCCCCAACACU | 425 | 22 | 1 |
| hsa-miR-4749-3p | CGCCCCUCCUGCCCCCACAG | 426 | 20 | 1 |
| hsa-miR-4769-5p | GGUGGGAUGGAGAGAAGGUAUGAG | 427 | 24 | 1 |
| hsa-miR-4800-5p | AGUGGACCGAGGAAGGAAGGA | 428 | 21 | 1 |
| hsa-miR-491-5p | AGUGGGGAACCCUUCCAUGAGG | 429 | 22 | 1 |
| hsa-miR-501-5p | AAUCCUUUGUCCCUGGGUGAGA | 430 | 22 | 1 |
| hsa-miR-5092 | AAUCCACGCUGAGCUUGGCAUC | 431 | 22 | 1 |
| hsa-miR-541-5p | AAAGGAUUCUGCUGUCGGUCCCACU | 432 | 25 | 1 |
| hsa-miR-542-5p | UCGGGGAUCAUCAUGUCACGAGA | 433 | 23 | 1 |
| hsa-miR-551b-3p | GCGACCCAUACUUGGUUUCAG | 434 | 21 | 1 |
| hsa-miR-5690 | UCAGCUACUACCUCUAUUAGG | 435 | 21 | 1 |
| hsa-miR-577 | UAGAUAAAAUAUUGGUACCUG | 436 | 21 | 1 |
| hsa-miR-584-3p | UCAGUUCCAGGCCAACCAGGCU | 437 | 22 | 1 |
| hsa-miR-589-3p | UCAGAACAAAUGCCGGUUCCCAGA | 438 | 24 | 1 |
| hsa-miR-616-5p | ACUCAAAACCCUUCAGUGACUU | 439 | 22 | 1 |
| hsa-miR-628-5p | AUGCUGACAUAUUUACUAGAGG | 440 | 22 | 1 |
| hsa-miR-629-5p | UGGGUUUACGUUGGGAGAACU | 441 | 21 | 1 |
| hsa-miR-644b-3p | UUCAUUUGCCUCCCAGCCUACA | 442 | 22 | 1 |
| hsa-miR-664-5p | ACUGGCUAGGGAAAAUGAUUGGAU | 443 | 24 | 1 |
| hsa-miR-922 | GCAGCAGAGAAUAGGACUACGUC | 444 | 23 | 1 |

**Table 5: Cells EI**

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-let-7a-5p | UGAGGUAGUAGGUUGUAUAGUU | 1 | 22 | 305060 |
| hsa-miR-92b-3p | UAUUGCACUCGUCCCGGCCUCC | 13 | 22 | 242715 |
| hsa-miR-21-5p | UAGCUUAUCAGACUGAUGUUGA | 9 | 22 | 154626 |
| hsa-miR-92a-3p | UAUUGCACUUGUCCCGGCCUGU | 7 | 22 | 137412 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-127-3p | UCGGAUCCGUCUGAGCUUGGCU | 14 | 22 | 110806 |
| hsa-miR-100-5p | AACCCGUAGAUCCGAACUUGUG | 3 | 22 | 109290 |
| hsa-miR-27b-3p | UUCACAGUGGCUAAGUUCUGC | 6 | 21 | 91902 |
| hsa-miR-191-5p | CAACGGAAUCCCAAAAGCAGCUG | 8 | 23 | 89150 |
| hsa-miR-26a-5p | UUCAAGUAAUCCAGGAUAGGCU | 12 | 22 | 88724 |
| hsa-miR-99b-5p | CACCCGUAGAACCGACCUUGCG | 4 | 22 | 87399 |
| hsa-let-7f-5p | UGAGGUAGUAGAUUGUAUAGUU | 11 | 22 | 78395 |
| hsa-miR-181a-5p | AACAUUCAACGCUGUCGGUGAGU | 15 | 23 | 47686 |
| hsa-miR-486-5p | UCCUGUACUGAGCUGCCCCGAG | 5 | 22 | 41639 |
| hsa-miR-30a-5p | UGUAAACAUCCUCGACUGGAAG | 30 | 22 | 35465 |
| hsa-miR-98 | UGAGGUAGUAAGUUGUAUUGUU | 10 | 22 | 30440 |
| hsa-miR-151a-3p | CUAGACUGAAGCUCCUUGAGG | 25 | 21 | 29047 |
| hsa-miR-21-3p | CAACACCAGUCGAUGGGCUGU | 20 | 21 | 27733 |
| hsa-miR-30d-5p | UGUAAACAUCCCCGACUGGAAG | 31 | 22 | 27307 |
| hsa-let-7c | UGAGGUAGUAGGUUGUAUGGUU | 17 | 22 | 27224 |
| hsa-miR-10a-5p | UACCCUGUAGAUCCGAAUUUGUG | 2 | 23 | 26908 |
| hsa-miR-22-3p | AAGCUGCCAGUUGAAGAACUGU | 33 | 22 | 26456 |
| hsa-miR-182-5p | UUUGGCAAUGGUAGAACUCACACU | 16 | 24 | 25885 |
| hsa-miR-222-3p | AGCUACAUCUGGCUACUGGGU | 36 | 21 | 22187 |
| hsa-miR-125a-5p | UCCCUGAGACCCUUUAACCUGUGA | 35 | 24 | 20960 |
| hsa-miR-16-5p | UAGCAGCACGUAAAUAUUGGCG | 29 | 22 | 19856 |
| hsa-let-7b-5p | UGAGGUAGUAGGUUGUGUGGUU | 28 | 22 | 19774 |
| hsa-miR-151a-5p | UCGAGGAGCUCACAGUCUAGU | 37 | 21 | 19773 |
| hsa-let-7e-5p | UGAGGUAGGAGGUUGUAUAGUU | 27 | 22 | 19035 |
| hsa-miR-125b-5p | UCCCUGAGACCCUAACUUGUGA | 42 | 22 | 17965 |
| hsa-let-7i-5p | UGAGGUAGUAGUUUGUGCUGUU | 22 | 22 | 17802 |
| hsa-let-7g-5p | UGAGGUAGUAGUUUGUACAGUU | 43 | 22 | 15467 |
| hsa-miR-409-3p | GAAUGUUGCUCGGUGAACCCCU | 47 | 22 | 14133 |
| hsa-miR-30e-5p | UGUAAACAUCCUUGACUGGAAG | 45 | 22 | 13889 |
| hsa-miR-181b-5p | AACAUUCAUUGCUGUCGGUGGGU | 38 | 23 | 12606 |
| hsa-miR-186-5p | CAAAGAAUUCUCCUUUUGGGCU | 40 | 22 | 12441 |
| hsa-miR-381 | UAUACAAGGGCAAGCUCUCUGU | 51 | 22 | 9851 |
| hsa-miR-423-5p | UGAGGGGCAGAGAGCGAGACUUU | 41 | 23 | 8893 |
| hsa-miR-30c-5p | UGUAAACAUCCUACACUCUCAGC | 66 | 23 | 8737 |
| hsa-miR-410 | AAUAUAACACAGAUGGCCUGU | 50 | 21 | 8509 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-146b-5p | UGAGAACUGAAUUCCAUAGGCU | 19 | 22 | 8434 |
| hsa-miR-654-3p | UAUGUCUGCUGACCAUCACCUU | 336 | 22 | 8392 |
| hsa-miR-9-5p | UCUUUGGUUAUCUAGCUGUAUGA | 58 | 23 | 7957 |
| hsa-miR-28-3p | CACUAGAUUGUGAGCUCCUGGA | 56 | 22 | 7767 |
| hsa-miR-148a-3p | UCAGUGCACUACAGAACUUUGU | 54 | 22 | 6599 |
| hsa-miR-379-5p | UGGUAGACUAUGGAACGUAGG | 18 | 21 | 6135 |
| hsa-let-7d-5p | AGAGGUAGUAGGUUGCAUAGUU | 53 | 22 | 5972 |
| hsa-miR-183-5p | UAUGGCACUGGUAGAAUUCACU | 24 | 22 | 5477 |
| hsa-miR-25-3p | CAUUGCACUUGUCUCGGUCUGA | 63 | 22 | 5303 |
| hsa-miR-423-3p | AGCUCGGUCUGAGGCCCCUCAGU | 57 | 23 | 5225 |
| hsa-miR-889 | UUAAUAUCGGACAACCAUUGU | 64 | 21 | 4597 |
| hsa-miR-221-5p | ACCUGGCAUACAAUGUAGAUUU | 39 | 22 | 4379 |
| hsa-miR-125b-1-3p | ACGGGUUAGGCUCUUGGGAGCU | 49 | 22 | 4192 |
| hsa-miR-409-5p | AGGUUACCCGAGCAACUUUGCAU | 32 | 23 | 3970 |
| hsa-miR-4492 | GGGGCUGGGCGCGCGCC | 34 | 17 | 3864 |
| hsa-miR-148b-3p | UCAGUGCAUCACAGAACUUUGU | 48 | 22 | 3593 |
| hsa-miR-103a-3p | AGCAGCAUUGUACAGGGCUAUGA | 62 | 23 | 3518 |
| hsa-miR-1271-5p | CUUGGCACCUAGCAAGCACUCA | 72 | 22 | 3477 |
| hsa-miR-136-3p | CAUCAUCGUCUCAAAUGAGUCU | 82 | 22 | 3373 |
| hsa-miR-769-5p | UGAGACCUCUGGGUUCUGAGCU | 75 | 22 | 2957 |
| hsa-miR-4532 | CCCCGGGGAGCCCGGCG | 23 | 17 | 2915 |
| hsa-miR-378a-3p | ACUGGACUUGGAGUCAGAAGG | 65 | 21 | 2895 |
| hsa-miR-99a-5p | AACCCGUAGAUCCGAUCUUGUG | 52 | 22 | 2767 |
| hsa-miR-221-3p | AGCUACAUUGUCUGCUGGGUUUC | 79 | 23 | 2764 |
| hsa-miR-30e-3p | CUUUCAGUCGGAUGUUUACAGC | 71 | 22 | 2441 |
| hsa-miR-26b-5p | UUCAAGUAAUUCAGGAUAGGU | 90 | 21 | 2432 |
| hsa-miR-4488 | AGGGGGCGGGCUCCGGCG | 61 | 18 | 2391 |
| hsa-miR-27a-3p | UUCACAGUGGCUAAGUUCCGC | 46 | 21 | 2385 |
| hsa-miR-23b-3p | AUCACAUUGCCAGGGAUUACC | 59 | 21 | 2316 |
| hsa-miR-500a-3p | AUGCACCUGGGCAAGGAUUCUG | 44 | 22 | 2144 |
| hsa-miR-941 | CACCCGGCUGUGUGCACAUGUGC | 60 | 23 | 2114 |
| hsa-miR-23a-3p | AUCACAUUGCCAGGGAUUUCC | 55 | 21 | 2086 |
| hsa-miR-30a-3p | CUUUCAGUCGGAUGUUUGCAGC | 77 | 22 | 2045 |
| hsa-miR-30b-5p | UGUAAACAUCCUACACUCAGCU | 96 | 22 | 1936 |
| hsa-miR-501-3p | AAUGCACCCGGGCAAGGAUUCU | 26 | 22 | 1895 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-130b-3p | CAGUGCAAUGAUGAAAGGGCAU | 86 | 22 | 1862 |
| hsa-miR-1246 | AAUGGAUUUUUGGAGCAGG | 21 | 19 | 1783 |
| hsa-miR-140-3p | UACCACAGGGUAGAACCACGG | 138 | 21 | 1735 |
| hsa-miR-31-5p | AGGCAAGAUGCUGGCAUAGCU | 80 | 21 | 1705 |
| hsa-miR-493-3p | UGAAGGUCUACUGUGUGCCAGG | 83 | 22 | 1698 |
| hsa-miR-181c-5p | AACAUUCAACCUGUCGGUGAGU | 105 | 22 | 1554 |
| hsa-miR-93-5p | CAAAGUGCUGUUCGUGCAGGUAG | 116 | 23 | 1492 |
| hsa-miR-181a-2-3p | ACCACUGACCGUUGACUGUACC | 102 | 22 | 1491 |
| hsa-miR-15b-5p | UAGCAGCACAUCAUGGUUUACA | 78 | 22 | 1465 |
| hsa-miR-7-5p | UGGAAGACUAGUGAUUUUGUUGU | 85 | 23 | 1460 |
| hsa-miR-192-5p | CUGACCUAUGAAUUGACAGCC | 87 | 21 | 1453 |
| hsa-miR-425-5p | AAUGACACGAUCACUCCCGUUGA | 111 | 23 | 1432 |
| hsa-miR-204-5p | UUCCCUUUGUCAUCCUAUGCCU | 89 | 22 | 1378 |
| hsa-miR-340-5p | UUAUAAAGCAAUGAGACUGAUU | 93 | 22 | 1360 |
| hsa-miR-190a | UGAUAUGUUUGAUAUAUUAGGU | 103 | 22 | 1305 |
| hsa-miR-34a-5p | UGGCAGUGUCUUAGCUGGUUGU | 101 | 22 | 1283 |
| hsa-miR-20a-5p | UAAAGUGCUUAUAGUGCAGGUAG | 146 | 23 | 1257 |
| hsa-miR-29a-3p | UAGCACCAUCUGAAAUCGGUUA | 106 | 22 | 1206 |
| hsa-miR-361-5p | UUAUCAGAAUCUCCAGGGGUAC | 70 | 22 | 1173 |
| hsa-miR-671-3p | UCCGGUUCUCAGGGCUCCACC | 69 | 21 | 1166 |
| hsa-miR-411-5p | UAGUAGACCGUAUAGCGUACG | 108 | 21 | 1130 |
| hsa-miR-589-5p | UGAGAACCACGUCUGCUCUGAG | 73 | 22 | 1067 |
| hsa-miR-130a-3p | CAGUGCAAUGUUAAAAGGGCAU | 113 | 22 | 1020 |
| hsa-miR-320a | AAAAGCUGGGUUGAGAGGGCGA | 97 | 22 | 994 |
| hsa-miR-149-5p | UCUGGCUCCGUGUCUUCACUCCC | 121 | 23 | 948 |
| hsa-miR-335-5p | UCAAGAGCAAUAACGAAAAAUGU | 128 | 23 | 945 |
| hsa-miR-134 | UGUGACUGGUUGACCAGAGGGG | 94 | 22 | 941 |
| hsa-miR-17-5p | CAAAGUGCUUACAGUGCAGGUAG | 145 | 23 | 939 |
| hsa-miR-493-5p | UUGUACAUGGUAGGCUUUCAUU | 88 | 22 | 876 |
| hsa-miR-34c-5p | AGGCAGUGUAGUUAGCUGAUUGC | 125 | 23 | 846 |
| hsa-miR-484 | UCAGGCUCAGUCCCCUCCCGAU | 118 | 22 | 835 |
| hsa-miR-181a-3p | ACCAUCGACCGUUGAUUGUACC | 100 | 22 | 803 |
| hsa-miR-24-3p | UGGCUCAGUUCAGCAGGAACAG | 119 | 22 | 740 |
| hsa-miR-128 | UCACAGUGAACCGGUCUCUUU | 109 | 21 | 707 |
| hsa-miR-342-3p | UCUCACACAGAAAUCGCACCCGU | 81 | 23 | 698 |

(continued)

| **CELLS - CTX0E03 07EI** | | | | |
|---|---|---|---|---|
| MIRNA | MIRNA.SEQUENCE | SEQ ID NO: | MIRNA LENGTH | READ COUNTS |
| hsa-miR-454-3p | UAGUGCAAUAUUGCUUAUAGGGU | 169 | 23 | 690 |
| hsa-miR-1307-5p | UCGACCGGACCUCGACCGGCU | 91 | 21 | 616 |
| hsa-miR-487b | AAUCGUACAGGGUCAUCCACUU | 117 | 22 | 590 |
| hsa-miR-130b-5p | ACUCUUUCCCUGUUGCACUAC | 112 | 21 | 568 |
| hsa-miR-197-3p | UUCACCACCUUCUCCACCCAGC | 122 | 22 | 544 |
| hsa-miR-432-5p | UCUUGGAGUAGGUCAUUGGGUGG | 95 | 23 | 542 |
| hsa-miR-374a-5p | UUAUAAUACAACCUGAUAAGUG | 74 | 22 | 537 |
| hsa-miR-345-5p | GCUGACUCCUAGUCCAGGGCUC | 76 | 22 | 527 |
| hsa-miR-744-5p | UGCGGGGCUAGGGCUAACAGCA | 99 | 22 | 515 |
| hsa-miR-376c | AACAUAGAGGAAAUUCCACGU | 185 | 21 | 506 |
| hsa-miR-181d | AACAUUCAUUGUUGUCGGUGGGU | 157 | 23 | 497 |
| hsa-miR-363-3p | AAUUGCACGGUAUCCAUCUGUA | 131 | 22 | 493 |
| hsa-miR-539-3p | AUCAUACAAGGACAAUUUCUUU | 150 | 22 | 493 |
| hsa-miR-758 | UUUGUGACCUGGUCCACUAACC | 141 | 22 | 477 |
| hsa-miR-323a-3p | CACAUUACACGGUCGACCUCU | 158 | 21 | 443 |
| hsa-miR-107 | AGCAGCAUUGUACAGGGCUAUCA | 254 | 23 | 431 |
| hsa-miR-720 | UCUCGCUGGGGCCUCCA | 84 | 17 | 427 |
| hsa-miR-654-5p | UGGUGGGCCGCAGAACAUGUGC | 115 | 22 | 409 |
| hsa-miR-370 | GCCUGCUGGGGUGGAACCUGGU | 126 | 22 | 406 |
| hsa-miR-421 | AUCAACAGACAUUAAUUGGGCGC | 151 | 23 | 399 |
| hsa-miR-30d-3p | CUUUCAGUCAGAUGUUUGCUGC | 114 | 22 | 358 |
| hsa-miR-148b-5p | AAGUUCUGUUAUACACUCAGGC | 127 | 22 | 354 |
| hsa-miR-1301 | UUGCAGCUGCCUGGGAGUGACUUC | 181 | 24 | 346 |
| hsa-miR-374b-5p | AUAUAAUACAACCUGCUAAGUG | 143 | 22 | 339 |
| hsa-miR-125b-2-3p | UCACAAGUCAGGCUCUUGGGAC | 68 | 22 | 333 |
| hsa-miR-28-5p | AAGGAGCUCACAGUCUAUUGAG | 152 | 22 | 332 |
| hsa-miR-495 | AAACAAACAUGGUGCACUUCUU | 241 | 22 | 321 |
| hsa-miR-15a-5p | UAGCAGCACAUAAUGGUUUGUG | 223 | 22 | 320 |
| hsa-miR-100-3p | CAAGCUUGUAUCUAUAGGUAUG | 98 | 22 | 314 |
| hsa-miR-193b-3p | AACUGGCCCUCAAAGUCCCGCU | 148 | 22 | 305 |
| hsa-miR-330-5p | UCUCUGGGCCUGUGUCUUAGGC | 161 | 22 | 303 |
| hsa-miR-376a-3p | AUCAUAGAGGAAAAUCCACGU | 237 | 21 | 298 |
| hsa-miR-135b-5p | UAUGGCUUUUCAUUCCUAUGUGA | 137 | 23 | 289 |
| hsa-miR-301a-3p | CAGUGCAAUAGUAUUGUCAAAGC | 107 | 23 | 280 |
| hsa-miR-218-5p | UUGUGCUUGAUCUAACCAUGU | 206 | 21 | 276 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-143-3p | UGAGAUGAAGCACUGUAGCUC | 176 | 21 | 256 |
| hsa-miR-27b-5p | AGAGCUUAGCUGAUUGGUGAAC | 201 | 22 | 255 |
| hsa-miR-369-3p | AAUAAUACAUGGUUGAUCUUU | 196 | 21 | 255 |
| hsa-miR-877-5p | GUAGAGGAGAUGGCGCAGGG | 133 | 20 | 249 |
| hsa-miR-19b-3p | UGUGCAAAUCCAUGCAAAACUGA | 163 | 23 | 246 |
| hsa-miR-424-5p | CAGCAGCAAUUCAUGUUUUGAA | 186 | 22 | 245 |
| hsa-miR-660-5p | UACCCAUUGCAUAUCGGAGUUG | 187 | 22 | 244 |
| hsa-miR-532-5p | CAUGCCUUGAGUGUAGGACCGU | 178 | 22 | 238 |
| hsa-miR-299-3p | UAUGUGGGAUGGUAAACCGCUU | 182 | 22 | 235 |
| hsa-miR-431-3p | CAGGUCGUCUUGCAGGGCUUCU | 168 | 22 | 231 |
| hsa-miR-374a-3p | CUUAUCAGAUUGUAUUGUAAUU | 173 | 22 | 220 |
| hsa-miR-148a-5p | AAAGUUCUGAGACACUCCGACU | 144 | 22 | 214 |
| hsa-miR-4516 | GGGAGAAGGGUCGGGGC | 110 | 17 | 207 |
| hsa-miR-92b-5p | AGGGACGGGACGCGGUGCAGUG | 208 | 22 | 206 |
| hsa-miR-16-2-3p | CCAAUAUUACUGUGCUGCUUUA | 316 | 22 | 202 |
| hsa-miR-101-3p | UACAGUACUGUGAUAACUGAA | 142 | 21 | 201 |
| hsa-let-7a-3p | CUAUACAAUCUACUGUCUUUC | 222 | 21 | 199 |
| hsa-miR-4485 | UAACGGCCGCGGUACCCUAA | 67 | 20 | 195 |
| hsa-miR-455-3p | GCAGUCCAUGGGCAUAUACAC | 140 | 21 | 192 |
| hsa-miR-185-5p | UGGAGAGAAAGGCAGUUCCUGA | 224 | 22 | 188 |
| hsa-miR-1185-1-3p | AUAUACAGGGGGAGACUCUUAU | 209 | 22 | 187 |
| hsa-miR-1197 | UAGGACACAUGGUCUACUUCU | 244 | 21 | 185 |
| hsa-miR-106b-3p | CCGCACUGUGGGUACUUGCUGC | 159 | 22 | 178 |
| hsa-miR-24-2-5p | UGCCUACUGAGCUGAAACACAG | 156 | 22 | 178 |
| hsa-miR-4677-3p | UCUGUGAGACCAAAGAACUACU | 120 | 22 | 177 |
| hsa-miR-380-3p | UAUGUAAUAUGGUCCACAUCUU | 445 | 22 | 174 |
| hsa-miR-548k | AAAAGUACUUGCGGAUUUUGCU | 198 | 22 | 171 |
| hsa-miR-1307-3p | ACUCGGCGUGGCGUCGGUCGUG | 124 | 22 | 169 |
| hsa-miR-485-3p | GUCAUACACGGCUCUCCUCUCU | 153 | 22 | 168 |
| hsa-miR-494 | UGAAACAUACACGGGAAACCUC | 240 | 22 | 165 |
| hsa-miR-17-3p | ACUGCAGUGAAGGCACUUGUAG | 184 | 22 | 163 |
| hsa-miR-561-5p | AUCAAGGAUCUUAAACUUUGCC | 179 | 22 | 160 |
| hsa-miR-27a-5p | AGGGCUUAGCUGCUUGUGAGCA | 130 | 22 | 158 |
| hsa-miR-874 | CUGCCCUGGCCCGAGGGACCGA | 147 | 22 | 151 |
| hsa-miR-9-3p | AUAAAGCUAGAUAACCGAAAGU | 183 | 22 | 151 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-96-5p | UUUGGCACUAGCACAUUUUUGCU | 123 | 23 | 151 |
| hsa-miR-656 | AAUAUUAUACAGUCAACCUCU | 221 | 21 | 147 |
| hsa-miR-379-3p | UAUGUAACAUGGUCCACUAACU | 446 | 22 | 145 |
| hsa-miR-382-5p | GAAGUUGUUCGUGGUGGAUUCG | 238 | 22 | 144 |
| hsa-miR-541-3p | UGGUGGGCACAGAAUCUGGACU | 136 | 22 | 141 |
| hsa-miR-337-3p | CUCCUAUAUGAUGCCUUUCUUC | 215 | 22 | 139 |
| hsa-miR-15b-3p | CGAAUCAUUAUUUGCUGCUCUA | 447 | 22 | 137 |
| hsa-miR-20b-5p | CAAAGUGCUCAUAGUGCAGGUAG | 317 | 23 | 136 |
| hsa-miR-329 | AACACACCUGGUUAACCUCUUU | 214 | 22 | 136 |
| hsa-miR-3676-5p | AGGAGAUCCUGGGUU | 280 | 15 | 134 |
| hsa-miR-543 | AAACAUUCGCGGUGCACUUCUU | 193 | 22 | 134 |
| hsa-miR-365b-3p | UAAUGCCCCUAAAAAUCCUUAU | 279 | 22 | 133 |
| hsa-miR-125a-3p | ACAGGUGAGGUUCUUGGGAGCC | 160 | 22 | 131 |
| hsa-miR-3065-5p | UCAACAAAAUCACUGAUGCUGGA | 226 | 23 | 130 |
| hsa-miR-1296 | UUAGGGCCCUGGCUCCAUCUCC | 271 | 22 | 126 |
| hsa-miR-935 | CCAGUUACCGCUUCCGCUACCGC | 311 | 23 | 118 |
| hsa-miR-132-3p | UAACAGUCUACAGCCAUGGUCG | 104 | 22 | 116 |
| hsa-miR-4284 | GGGCUCACAUCACCCCAU | 191 | 18 | 116 |
| hsa-miR-487a | AAUCAUACAGGGACAUCCAGUU | 203 | 22 | 113 |
| hsa-miR-574-5p | UGAGUGUGUGUGUGUGAGUGUGU | 334 | 23 | 113 |
| hsa-miR-301b | CAGUGCAAUGAUAUUGUCAAAGC | 164 | 23 | 111 |
| hsa-miR-548o-3p | CCAAAACUGCAGUUACUUUUGC | 268 | 22 | 105 |
| hsa-miR-18a-5p | UAAGGUGCAUCUAGUGCAGAUAG | 256 | 23 | 104 |
| hsa-miR-485-5p | AGAGGCUGGCCGUGAUGAAUUC | 165 | 22 | 104 |
| hsa-miR-548ah-5p | AAAAGUGAUUGCAGUGUUUG | 235 | 20 | 103 |
| hsa-miR-361-3p | UCCCCCAGGUGUGAUUCUGAUUU | 250 | 23 | 101 |
| hsa-miR-433 | AUCAUGAUGGGCUCCUCGGUGU | 174 | 22 | 101 |
| hsa-miR-337-5p | GAACGGCUUCAUACAGGAGUU | 277 | 21 | 100 |
| hsa-miR-1276 | UAAAGAGCCCUGUGGAGACA | 194 | 20 | 99 |
| hsa-miR-30c-1-3p | CUGGGAGAGGGUUGUUUACUCC | 213 | 22 | 99 |
| hsa-miR-31-3p | UGCUAUGCCAACAUAUUGCCAU | 172 | 22 | 96 |
| hsa-miR-424-3p | CAAAACGUGAGGCGCUGCUAU | 298 | 21 | 96 |
| hsa-miR-550a-5p | AGUGCCUGAGGGAGUAAGAGCCC | 134 | 23 | 95 |
| hsa-miR-4454 | GGAUCCGAGUCACGGCACCA | 299 | 20 | 94 |
| hsa-miR-541-5p | AAAGGAUUCUGCUGUCGGUCCCACU | 432 | 25 | 92 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-106b-5p | UAAAGUGCUGACAGUGCAGAU | 170 | 21 | 89 |
| hsa-miR-153 | UUGCAUAGUCACAAAAGUGAUC | 188 | 22 | 88 |
| hsa-miR-135b-3p | AUGUAGGGCUAAAAGCCAUGGG | 205 | 22 | 87 |
| hsa-miR-574-3p | CACGCUCAUGCACACACCCACA | 253 | 22 | 87 |
| hsa-miR-1226-3p | UCACCAGCCCUGUGUUCCCUAG | 199 | 22 | 85 |
| hsa-miR-576-5p | AUUCUAAUUUCUCCACGUCUUU | 306 | 22 | 84 |
| hsa-miR-127-5p | CUGAAGCUCAGAGGGCUCUGAU | 255 | 22 | 83 |
| hsa-miR-155-5p | UUAAUGCUAAUCGUGAUAGGGGU | 448 | 23 | 83 |
| hsa-miR-3176 | ACUGGCCUGGGACUACCGG | 227 | 19 | 83 |
| hsa-miR-382-3p | AAUCAUUCACGGACAACACUU | 260 | 21 | 83 |
| hsa-miR-1275 | GUGGGGGAGAGGCUGUC | 162 | 17 | 82 |
| hsa-miR-671-5p | AGGAAGCCCUGGAGGGGCUGGAG | 288 | 23 | 82 |
| hsa-miR-23a-5p | GGGGUUCCUGGGGAUGGGAUUU | 212 | 22 | 81 |
| hsa-miR-25-5p | AGGCGGAGACUUGGGCAAUUG | 225 | 21 | 80 |
| hsa-miR-641 | AAAGACAUAGGAUAGAGUCACCUC | 285 | 24 | 80 |
| hsa-miR-19a-3p | UGUGCAAAUCUAUGCAAAACUGA | 177 | 23 | 79 |
| hsa-miR-377-3p | AUCACACAAAGGCAACUUUUGU | 449 | 22 | 78 |
| hsa-miR-454-5p | ACCCUAUCAAUAUUGUCUCUGC | 265 | 22 | 78 |
| hsa-miR-496 | UGAGUAUUACAUGGCCAAUCUC | 267 | 22 | 78 |
| hsa-miR-29b-3p | UAGCACCAUUUGAAAUCAGUGUU | 166 | 23 | 77 |
| hsa-miR-26a-2-3p | CCUAUUCUUGAUUACUUGUUUC | 257 | 22 | 76 |
| hsa-miR-1260b | AUCCCACCACUGCCACCAU | 373 | 19 | 74 |
| hsa-miR-2467-5p | UGAGGCUCUGUUAGCCUUGGCUC | 154 | 23 | 74 |
| hsa-miR-377-5p | AGAGGUUGCCCUUGGUGAAUUC | 202 | 22 | 74 |
| hsa-miR-330-3p | GCAAAGCACACGGCCUGCAGAGA | 195 | 23 | 73 |
| hsa-miR-1180 | UUUCCGGCUCGCGUGGGUGUGU | 313 | 22 | 71 |
| hsa-miR-99b-3p | CAAGCUCGUGUCUGUGGGUCCG | 243 | 22 | 71 |
| hsa-miR-299-5p | UGGUUUACCGUCCCACAUACAU | 319 | 22 | 69 |
| hsa-miR-374b-3p | CUUAGCAGGUUGUAUUAUCAUU | 229 | 22 | 69 |
| hsa-miR-4746-5p | CCGGUCCCAGGAGAACCUGCAGA | 266 | 23 | 69 |
| hsa-miR-331-3p | GCCCCUGGGCCUAUCCUAGAA | 450 | 21 | 68 |
| hsa-miR-340-3p | UCCGUCUCAGUUACUUUAUAGC | 248 | 22 | 68 |
| hsa-miR-92a-1-5p | AGGUUGGGAUCGGUUGCAAUGCU | 204 | 23 | 68 |
| hsa-miR-542-3p | UGUGACAGAUUGAUAACUGAAA | 331 | 22 | 66 |
| hsa-miR-431-5p | UGUCUUGCAGGCCGUCAUGCA | 132 | 21 | 65 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-1254 | AGCCUGGAAGCUGGAGCCUGCAGU | 270 | 24 | 61 |
| hsa-miR-3158-3p | AAGGGCUUCCUCUCUGCAGGAC | 167 | 22 | 61 |
| hsa-miR-362-5p | AAUCCUUGGAACCUAGGUGUGAGU | 139 | 24 | 61 |
| hsa-miR-30c-2-3p | CUGGGAGAAGGCUGUUUACUCU | 321 | 22 | 59 |
| hsa-miR-4461 | GAUUGAGACUAGUAGGGCUAGGC | 129 | 23 | 59 |
| hsa-miR-3200-3p | CACCUUGCGCUACUCAGGUCUG | 247 | 22 | 57 |
| hsa-miR-215 | AUGACCUAUGAAUUGACAGAC | 451 | 21 | 56 |
| hsa-miR-1185-5p | AGAGGAUACCCUUUGUAUGUU | 368 | 21 | 55 |
| hsa-miR-328 | CUGGCCCUCUCUGCCCUUCCGU | 297 | 22 | 55 |
| hsa-miR-655 | AUAAUACAUGGUUAACCUCUUU | 286 | 22 | 55 |
| hsa-miR-181b-3p | CUCACUGAACAAUGAAUGCAA | 245 | 21 | 54 |
| hsa-miR-376b | AUCAUAGAGGAAAAUCCAUGUU | 452 | 22 | 54 |
| hsa-miR-486-3p | CGGGGCAGCUCAGUACAGGAU | 453 | 21 | 54 |
| hsa-miR-760 | CGGCUCUGGGUCUGUGGGGA | 289 | 20 | 54 |
| hsa-miR-3909 | UGUCCUCUAGGGCCUGCAGUCU | 412 | 22 | 53 |
| hsa-miR-4508 | GCGGGGCUGGGCGCGCG | 135 | 17 | 53 |
| hsa-miR-4521 | GCUAAGGAAGUCCUGUGCUCAG | 233 | 22 | 53 |
| hsa-let-7e-3p | CUAUACGGCCUCCUAGCUUUCC | 290 | 22 | 52 |
| hsa-miR-455-5p | UAUGUGCCUUUGGACUACAUCG | 192 | 22 | 52 |
| hsa-miR-93-3p | ACUGCUGAGCUAGCACUUCCCG | 454 | 22 | 51 |
| hsa-miR-151b | UCGAGGAGCUCACAGUCU | 455 | 18 | 49 |
| hsa-miR-887 | GUGAACGGGCGCCAUCCCGAGG | 456 | 22 | 49 |
| hsa-miR-152 | UCAGUGCAUGACAGAACUUGG | 344 | 21 | 48 |
| hsa-miR-324-3p | ACUGCCCCAGGUGCUGCUGG | 276 | 20 | 48 |
| hsa-miR-1266 | CCUCAGGGCUGUAGAACAGGGCU | 457 | 23 | 47 |
| hsa-miR-302b-3p | UAAGUGCUUCCAUGUUUUAGUAG | 458 | 23 | 47 |
| hsa-miR-548e | AAAAACUGAGACUACUUUUGCA | 459 | 22 | 47 |
| hsa-miR-502-3p | AAUGCACCUGGGCAAGGAUUCA | 281 | 22 | 46 |
| hsa-miR-302d-3p | UAAGUGCUUCCAUGUUUGAGUGU | 460 | 23 | 45 |
| hsa-miR-3943 | UAGCCCCCAGGCUUCACUUGGCG | 207 | 23 | 45 |
| hsa-miR-1286 | UGCAGGACCAAGAUGAGCCCU | 293 | 21 | 44 |
| hsa-miR-3605-5p | UGAGGAUGGAUAGCAAGGAAGCC | 189 | 23 | 44 |
| hsa-miR-505-3p | CGUCAACACUUGCUGGUUUCCU | 282 | 22 | 44 |
| hsa-miR-3615 | UCUCUCGGCUCCUCGCGGCUC | 323 | 21 | 43 |
| hsa-miR-4435 | AUGGCCAGAGCUCACACAGAGG | 230 | 22 | 43 |

(continued)

| **CELLS - CTX0E03 07EI** | | | | |
|---|---|---|---|---|
| MIRNA | MIRNA.SEQUENCE | SEQ ID NO: | MIRNA LENGTH | READ COUNTS |
| hsa-miR-598 | UACGUCAUCGUUGUCAUCGUCA | 461 | 22 | 43 |
| hsa-miR-126-5p | CAUUAUUACUUUUGGUACGCG | 462 | 21 | 42 |
| hsa-miR-4671-3p | UUAGUGCAUAGUCUUUGGUCU | 301 | 21 | 41 |
| hsa-miR-652-3p | AAUGGCGCCACUAGGGUUGUG | 442 | 21 | 41 |
| hsa-miR-3687 | CCCGGACAGGCGUUCGUGCGACGU | 190 | 24 | 40 |
| hsa-miR-4286 | ACCCCACUCCUGGUACC | 328 | 17 | 40 |
| hsa-miR-590-3p | UAAUUUUAUGUAUAAGCUAGU | 463 | 21 | 40 |
| hsa-miR-1285-3p | UCUGGGCAACAAAGUGAGACCU | 464 | 22 | 39 |
| hsa-miR-2355-5p | AUCCCCAGAUACAAUGGACAA | 593 | 21 | 38 |
| hsa-miR-550a-3p | UGUCUUACUCCCUCAGGCACAU | 283 | 22 | 38 |
| hsa-let-7d-3p | CUAUACGACCUGCUGCCUUUCU | 92 | 22 | 37 |
| hsa-miR-136-5p | ACUCCAUUUGUUUUGAUGAUGGA | 272 | 23 | 37 |
| hsa-miR-1468 | CUCCGUUUGCCUGUUUCGCUG | 296 | 21 | 37 |
| hsa-miR-3609 | CAAAGUGAUGAGUAAUACUGGCUG | 216 | 24 | 37 |
| hsa-miR-548b-5p | AAAAGUAAUUGUGGUUUUGGCC | 304 | 22 | 37 |
| hsa-miR-664-3p | UAUUCAUUUAUCCCCAGCCUACA | 287 | 23 | 37 |
| hsa-miR-99a-3p | CAAGCUCGCUUCUAUGGGUCUG | 367 | 22 | 37 |
| hsa-miR-532-3p | CCUCCCACACCCAAGGCUUGCA | 252 | 22 | 36 |
| hsa-miR-10b-5p | UACCCUGUAGAACCGAAUUUGUG | 465 | 23 | 33 |
| hsa-miR-369-5p | AGAUCGACCGUGUUAUAUUCGC | 357 | 22 | 33 |
| hsa-miR-3161 | CUGAUAAGAACAGAGGCCCAGAU | 466 | 23 | 32 |
| hsa-miR-3940-3p | CAGCCCGGAUCCCAGCCCACUU | 239 | 22 | 32 |
| hsa-miR-663b | GGUGGCCCGGCCGUGCCUGAGG | 180 | 22 | 32 |
| hsa-miR-219-2-3p | AGAAUUGUGGCUGGACAUCUGU | 467 | 22 | 31 |
| hsa-miR-2277-5p | AGCGCGGGCUGAGCGCUGCCAGUC | 735 | 24 | 31 |
| hsa-miR-4448 | GGCUCCUUGGUCUAGGGGUA | 231 | 20 | 31 |
| hsa-miR-339-5p | UCCCUGUCCUCCAGGAGCUCACG | 402 | 23 | 30 |
| hsa-miR-3613-5p | UGUUGUACUUUUUUUUUUGUUC | 469 | 22 | 30 |
| hsa-miR-4775 | UUAAUUUUUUGUUUCGGUCACU | 302 | 22 | 30 |
| hsa-miR-212-5p | ACCUUGGCUCUAGACUGCUUACU | 246 | 23 | 29 |
| hsa-miR-324-5p | CGCAUCCCCUAGGGCAUUGGUGU | 354 | 23 | 27 |
| hsa-miR-4326 | UGUUCCUCUGUCUCCCAGAC | 417 | 20 | 27 |
| hsa-miR-582-3p | UAACUGGUUGAACAACUGAACC | 470 | 22 | 27 |
| hsa-miR-34a-3p | CAAUCAGCAAGUAUACUGCCCU | 403 | 22 | 26 |
| hsa-miR-106a-5p | AAAAGUGCUUACAGUGCAGGUAG | 471 | 23 | 25 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-4745-5p | UGAGUGGGGCUCCCGGGACGGCG | 219 | 23 | 25 |
| hsa-miR-769-3p | CUGGGAUCUCCGGGGUCUUGGUU | 337 | 23 | 25 |
| hsa-miR-1268a | CGGGCGUGGUGGUGGGGG | 291 | 18 | 24 |
| hsa-miR-154-3p | AAUCAUACACGGUUGACCUAUU | 472 | 22 | 24 |
| hsa-miR-188-3p | CUCCCACAUGCAGGGUUUGCA | 200 | 21 | 24 |
| hsa-miR-29c-3p | UAGCACCAUUUGAAAUCGGUUA | 473 | 22 | 24 |
| hsa-miR-539-5p | GGAGAAAUUAUCCUUGGUGUGU | 234 | 22 | 24 |
| hsa-miR-766-3p | ACUCCAGCCCCACAGCCUCAGC | 310 | 22 | 24 |
| hsa-miR-30b-3p | CUGGGAGGUGGAUGUUUACUUC | 320 | 22 | 23 |
| hsa-miR-3177-3p | UGCACGGCACUGGGGACACGU | 275 | 21 | 23 |
| hsa-miR-191-3p | GCUGCGCUUGGAUUUCGUCCCC | 474 | 22 | 22 |
| hsa-miR-296-3p | GAGGGUUGGGUGGAGGCUCUCC | 274 | 22 | 22 |
| hsa-miR-296-5p | AGGGCCCCCCCUCAAUCCUGU | 258 | 21 | 22 |
| hsa-miR-339-3p | UGAGCGCCUCGACGACAGAGCCG | 228 | 23 | 22 |
| hsa-miR-501-5p | AAUCCUUUGUCCCUGGGUGAGA | 430 | 22 | 22 |
| hsa-miR-200b-3p | UAAUACUGCCUGGUAAUGAUGA | 475 | 22 | 21 |
| hsa-miR-212-3p | UAACAGUCUCCAGUCACGGCC | 348 | 21 | 21 |
| hsa-miR-26b-3p | CCUGUUCUCCAUUACUUGGCUC | 391 | 22 | 21 |
| hsa-miR-665 | ACCAGGAGGCUGAGGCCCCU | 309 | 20 | 21 |
| hsa-miR-668 | UGUCACUCGGCUCGGCCCACUAC | 476 | 23 | 21 |
| hsa-miR-146a-5p | UGAGAACUGAAUUCCAUGGGUU | 477 | 22 | 20 |
| hsa-miR-1973 | ACCGUGCAAAGGUAGCAUA | 171 | 19 | 20 |
| hsa-miR-210 | CUGUGCGUGUGACAGCGGCUGA | 478 | 22 | 20 |
| hsa-miR-3607-5p | GCAUGUGAUGAAGCAAAUCAGU | 249 | 22 | 20 |
| hsa-miR-378a-5p | CUCCUGACUCCAGGUCCUGUGU | 217 | 22 | 20 |
| hsa-miR-4449 | CGUCCCGGGGCUGCGCGAGGCA | 155 | 22 | 20 |
| hsa-miR-138-5p | AGCUGGUGUUGUGAAUCAGGCCG | 379 | 23 | 19 |
| hsa-miR-146b-3p | UGCCCUGUGGACUCAGUUCUGG | 381 | 22 | 18 |
| hsa-miR-3065-3p | UCAGCACCAGGAUAUUGUUGGAG | 350 | 23 | 18 |
| hsa-miR-4417 | GGUGGGCUUCCCGGAGGG | 175 | 18 | 18 |
| hsa-miR-497-5p | CAGCAGCACACUGUGGUUUGU | 479 | 21 | 18 |
| hsa-miR-500a-5p | UAAUCCUUGCUACCUGGGUGAGA | 303 | 23 | 18 |
| hsa-miR-625-3p | GACUAUAGAACUUUCCCCCUCA | 307 | 22 | 18 |
| hsa-miR-628-3p | UCUAGUAAGAGUGGCAGUCGA | 335 | 21 | 18 |
| hsa-miR-1343 | CUCCUGGGGCCCGCACUCUCGC | 378 | 22 | 17 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-3648 | AGCCGCGGGGAUCGCCGAGGG | 259 | 21 | 17 |
| hsa-miR-432-3p | CUGGAUGGCUCCUCCAUGUCU | 262 | 21 | 17 |
| hsa-miR-4482-3p | UUUCUAUUUCUCAGUGGGGCUC | 361 | 22 | 17 |
| hsa-miR-542-5p | UCGGGGAUCAUCAUGUCACGAGA | 433 | 23 | 17 |
| hsa-miR-551b-3p | GCGACCCAUACUUGGUUUCAG | 434 | 21 | 17 |
| hsa-miR-7-1-3p | CAACAAAUCACAGUCUGCCAUA | 480 | 22 | 17 |
| hsa-miR-219-1-3p | AGAGUUGAGUCUGGACGUCCCG | 390 | 22 | 16 |
| hsa-miR-3656 | GGCGGGUGCGGGGGUGG | 251 | 17 | 16 |
| hsa-miR-3661 | UGACCUGGGACUCGGACAGCUG | 481 | 22 | 16 |
| hsa-miR-411-3p | UAUGUAACACGGUCCACUAACC | 482 | 22 | 16 |
| hsa-miR-5096 | GUUUCACCAUGUUGGUCAGGC | 220 | 21 | 16 |
| hsa-miR-577 | UAGAUAAAAUAUUGGUACCUG | 436 | 21 | 16 |
| hsa-let-7i-3p | CUGCGCAAGCUACUGCCUUGCU | 483 | 22 | 15 |
| hsa-miR-132-5p | ACCGUGGCUUUCGAUUGUUACU | 315 | 22 | 15 |
| hsa-miR-140-5p | CAGUGGUUUUACCCUAUGGUAG | 380 | 22 | 15 |
| hsa-miR-195-5p | UAGCAGCACAGAAAUAUUGGC | 346 | 21 | 15 |
| hsa-miR-3187-3p | UUGGCCAUGGGGCUGCGCGG | 322 | 20 | 15 |
| hsa-miR-342-5p | AGGGGUGCUAUCUGUGAUUGA | 278 | 21 | 15 |
| hsa-miR-34b-3p | CAAUCACUAACUCCACUGCCAU | 404 | 22 | 15 |
| hsa-miR-4661-5p | AACUAGCUCUGUGGAUCCUGAC | 484 | 22 | 15 |
| hsa-miR-584-5p | UUAUGGUUUGCCUGGGACUGAG | 485 | 22 | 15 |
| hsa-miR-744-3p | CUGUUGCCACUAACCUCAACCU | 486 | 22 | 15 |
| hsa-miR-770-5p | UCCAGUACCACGUGUCAGGGCCA | 487 | 23 | 15 |
| hsa-miR-3677-3p | CUCGUGGGCUCUGGCCACGGCC | 356 | 22 | 14 |
| hsa-miR-425-3p | AUCGGGAAUGUCGUGUCCGCCC | 358 | 22 | 14 |
| hsa-miR-548ah-3p | CAAAAACUGCAGUUACUUUUGC | 149 | 22 | 14 |
| hsa-miR-5699 | UCCUGUCUUUCCUUGUUGGAGC | 488 | 22 | 14 |
| hsa-miR-582-5p | UUACAGUUGUUCAACCAGUUACU | 489 | 23 | 14 |
| hsa-miR-1185-2-3p | AUAUACAGGGGGAGACUCUCAU | 314 | 22 | 13 |
| hsa-miR-1249 | ACGCCCUUCCCCCCCUUCUUCA | 490 | 22 | 13 |
| hsa-miR-1255a | AGGAUGAGCAAAGAAAGUAGAUU | 341 | 23 | 13 |
| hsa-miR-1910 | CCAGUCCUGUGCCUGCCGCCU | 236 | 21 | 13 |
| hsa-miR-301a-5p | GCUCUGACUUUAUUGCACUACU | 491 | 22 | 13 |
| hsa-miR-5001-3p | UUCUGCCUCUGUCCAGGUCCUU | 492 | 22 | 13 |
| hsa-miR-5094 | AAUCAGUGAAUGCCUUGAACCU | 493 | 22 | 13 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-628-5p | AUGCUGACAUAUUUACUAGAGG | 440 | 22 | 13 |
| hsa-miR-629-5p | UGGGUUUACGUUGGGAGAACU | 441 | 21 | 13 |
| hsa-miR-937 | AUCCGCGCUCUGACUCUCUGCC | 312 | 22 | 13 |
| hsa-miR-940 | AAGGCAGGGCCCCCGCUCCCC | 366 | 21 | 13 |
| hsa-miR-1248 | ACCUUCUUGUAUAAGCACUGUGCUAAA | 269 | 27 | 12 |
| hsa-miR-194-5p | UGUAACAGCAACUCCAUGUGGA | 345 | 22 | 12 |
| hsa-miR-199b-3p | ACAGUAGUCUGCACAUUGGUUA | 494 | 22 | 12 |
| hsa-miR-22-5p | AGUUCUUCAGUGGCAAGCUUUA | 495 | 22 | 12 |
| hsa-miR-3605-3p | CCUCCGUGUUACCUGUCCUCUAG | 496 | 23 | 12 |
| hsa-miR-3654 | GACUGGACAAGCUGAGGAA | 325 | 19 | 12 |
| hsa-miR-504 | AGACCCUGGUCUGCACUCUAUC | 497 | 22 | 12 |
| hsa-miR-1291 | UGGCCCUGACUGAAGACCAGCAGU | 294 | 24 | 11 |
| hsa-miR-1299 | UUCUGGAAUUCUGUGUGAGGGA | 498 | 22 | 11 |
| hsa-miR-188-5p | CAUCCCUUGCAUGGUGGAGGG | 499 | 21 | 11 |
| hsa-miR-222-5p | CUCAGUAGCCAGUGUAGAUCCU | 349 | 22 | 11 |
| hsa-miR-331-5p | CUAGGUAUGGUCCCAGGGAUCC | 500 | 22 | 11 |
| hsa-miR-3939 | UACGCGCAGACCACAGGAUGUC | 261 | 22 | 11 |
| hsa-miR-154-5p | UAGGUUAUCCGUGUUGCCUUCG | 501 | 22 | 10 |
| hsa-miR-18a-3p | ACUGCCCUAAGUGCUCCUUCUGG | 502 | 23 | 10 |
| hsa-miR-1908 | CGGCGGGGACGGCGAUUGGUC | 383 | 21 | 10 |
| hsa-miR-200c-3p | UAAUACUGCCGGGUAAUGAUGGA | 347 | 23 | 10 |
| hsa-miR-2116-3p | CCUCCCAUGCCAAGAACUCCC | 318 | 21 | 10 |
| hsa-miR-302a-3p | UAAGUGCUUCCAUGUUUUGGUGA | 503 | 23 | 10 |
| hsa-miR-3174 | UAGUGAGUUAGAGAUGCAGAGCC | 353 | 23 | 10 |
| hsa-miR-326 | CCUCUGGGCCCUUCCUCCAG | 504 | 20 | 10 |
| hsa-let-7g-3p | CUGUACAGGCCACUGCCUUGC | 505 | 21 | 9 |
| hsa-miR-141-3p | UAACACUGUCUGGUAAAGAUGG | 295 | 22 | 9 |
| hsa-miR-24-1-5p | UGCCUACUGAGCUGAUAUCAGU | 506 | 22 | 9 |
| hsa-miR-3115 | AUAUGGGUUUACUAGUUGGU | 351 | 20 | 9 |
| hsa-miR-3180-3p | UGGGGCGGAGCUUCCGGAGGCC | 400 | 22 | 9 |
| hsa-miR-33a-5p | GUGCAUUGUAGUUGCAUUGCA | 355 | 21 | 9 |
| hsa-miR-34c-3p | AAUCACUAACCACACGGCCAGG | 405 | 22 | 9 |
| hsa-miR-3929 | GAGGCUGAUGUGAGUAGACCACU | 218 | 23 | 9 |
| hsa-miR-4517 | AAAUAUGAUGAAACUCACAGCUGAG | 507 | 25 | 9 |
| hsa-miR-576-3p | AAGAUGUGGAAAAAUUGGAAUC | 508 | 22 | 9 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-1229 | CUCUCACCACUGCCCUCCCACAG | 509 | 23 | 8 |
| hsa-miR-1289 | UGGAGUCCAGGAAUCUGCAUUUU | 343 | 23 | 8 |
| hsa-miR-1915-5p | ACCUUGCCUUGCUGCCCGGGCC | 385 | 22 | 8 |
| hsa-miR-23b-5p | UGGGUUCCUGGCAUGCUGAUUU | 510 | 22 | 8 |
| hsa-miR-302a-5p | ACUUAAACGUGGAUGUACUUGCU | 511 | 23 | 8 |
| hsa-miR-3938 | AAUUCCCUUGUAGAUAACCCGG | 512 | 22 | 8 |
| hsa-miR-4466 | GGGUGCGGGCCGGCGGGG | 264 | 18 | 8 |
| hsa-miR-4786-5p | UGAGACCAGGACUGGAUGCACC | 197 | 22 | 8 |
| hsa-miR-589-3p | UCAGAACAAAUGCCGGUUCCCAGA | 438 | 24 | 8 |
| hsa-miR-616-5p | ACUCAAAACCCUUCAGUGACUU | 439 | 22 | 8 |
| hsa-miR-943 | CUGACUGUUGCCGUCCUCCAG | 338 | 21 | 8 |
| hsa-miR-1237 | UCCUUCUGCUCCGUCCCCCAG | 370 | 21 | 7 |
| hsa-miR-1915-3p | CCCCAGGGCGACGCGGCGGG | 384 | 20 | 7 |
| hsa-miR-3620 | UCACCCUGCAUCCCGCACCCAG | 324 | 22 | 7 |
| hsa-miR-3691-5p | AGUGGAUGAUGGAGACUCGGUAC | 409 | 23 | 7 |
| hsa-miR-4426 | GAAGAUGGACGUACUUU | 359 | 17 | 7 |
| hsa-let-7a-2-3p | CUGUACAGCCUCCUAGCUUUCC | 513 | 22 | 6 |
| hsa-miR-10a-3p | CAAAUUCGUAUCUAGGGGAAUA | 514 | 22 | 6 |
| hsa-miR-1287 | UGCUGGAUCAGUGGUUCGAGUC | 515 | 22 | 6 |
| hsa-miR-145-5p | GUCCAGUUUUCCCAGGAAUCCCU | 516 | 23 | 6 |
| hsa-miR-29b-1-5p | GCUGGUUUCAUAUGGUGGUUUAGA | 517 | 24 | 6 |
| hsa-miR-3128 | UCUGGCAAGUAAAAAACUCUCAU | 518 | 23 | 6 |
| hsa-miR-33b-5p | GUGCAUUGCUGUUGCAUUGC | 519 | 20 | 6 |
| hsa-miR-3681-5p | UAGUGGAUGAUGCACUCUGUGC | 327 | 22 | 6 |
| hsa-miR-3685 | UUUCCUACCCUACCUGAAGACU | 520 | 22 | 6 |
| hsa-miR-3918 | ACAGGGCCGCAGAUGGAGACU | 521 | 21 | 6 |
| hsa-miR-551b-5p | GAAAUCAAGCGUGGGUGAGACC | 522 | 22 | 6 |
| hsa-miR-1273f | GGAGAUGGAGGUUGCAGUG | 292 | 19 | 5 |
| hsa-miR-1273g-3p | ACCACUGCACUCCAGCCUGAG | 210 | 21 | 5 |
| hsa-miR-1304-5p | UUUGAGGCUACAGUGAGAUGUG | 523 | 22 | 5 |
| hsa-miR-1538 | CGGCCCGGGCUGCUGCUGUUCCU | 524 | 23 | 5 |
| hsa-miR-181c-3p | AACCAUCGACCGUUGAGUGGAC | 525 | 22 | 5 |
| hsa-miR-193a-5p | UGGGUCUUUGCGGGCGAGAUGA | 526 | 22 | 5 |
| hsa-miR-208b | AUAAGACGAACAAAAGGUUUGU | 388 | 22 | 5 |
| hsa-miR-219-5p | UGAUUGUCCAAACGCAAUUCU | 527 | 21 | 5 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-3159 | UAGGAUUACAAGUGUCGGCCAC | 528 | 22 | 5 |
| hsa-miR-3173-5p | UGCCCUGCCUGUUUUCUCCUUU | 529 | 22 | 5 |
| hsa-miR-3175 | CGGGGAGAGAACGCAGUGACGU | 530 | 22 | 5 |
| hsa-miR-3200-5p | AAUCUGAGAAGGCGCACAAGGU | 531 | 22 | 5 |
| hsa-miR-3662 | GAAAAUGAUGAGUAGUGACUGAUG | 326 | 24 | 5 |
| hsa-miR-3928 | GGAGGAACCUUGGAGCUUCGGC | 413 | 22 | 5 |
| hsa-miR-4709-3p | UUGAAGAGGAGGUGCUCUGUAGC | 532 | 23 | 5 |
| hsa-miR-4787-3p | GAUGCGCCGCCCACUGCCCCGCGC | 533 | 24 | 5 |
| hsa-miR-499a-5p | UUAAGACUUGCAGUGAUGUUU | 534 | 21 | 5 |
| hsa-miR-545-3p | UCAGCAAACAUUUAUUGUGUGC | 242 | 22 | 5 |
| hsa-miR-548u | CAAAGACUGCAAUUACUUUUGCG | 535 | 23 | 5 |
| hsa-miR-659-5p | AGGACCUUCCCUGAACCAAGGA | 364 | 22 | 5 |
| hsa-miR-1257 | AGUGAAUGAUGGGUUCUGACC | 372 | 21 | 4 |
| hsa-miR-1292 | UGGGAACGGGUUCCGGCAGACGCUG | 536 | 25 | 4 |
| hsa-miR-1914-5p | CCCUGUGCCCGGCCCACUUCUG | 537 | 22 | 4 |
| hsa-miR-195-3p | CCAAUAUUGGCUGUGCUGCUCC | 538 | 22 | 4 |
| hsa-miR-2110 | UUGGGGAAACGGCCGCUGAGUG | 389 | 22 | 4 |
| hsa-miR-302c-5p | UUUAACAUGGGGGUACCUGCUG | 539 | 22 | 4 |
| hsa-miR-3126-3p | CAUCUGGCAUCCGUCACACAGA | 394 | 22 | 4 |
| hsa-miR-3126-5p | UGAGGGACAGAUGCCAGAAGCA | 352 | 22 | 4 |
| hsa-miR-3150a-5p | CAACCUCGACGAUCUCCUCAGC | 540 | 22 | 4 |
| hsa-miR-3157-3p | CUGCCCUAGUCUAGCUGAAGCU | 399 | 22 | 4 |
| hsa-miR-323b-3p | CCCAAUACACGGUCGACCUCUU | 541 | 22 | 4 |
| hsa-miR-335-3p | UUUUUCAUUAUUGCUCCUGACC | 542 | 22 | 4 |
| hsa-miR-3607-3p | ACUGUAAACGCUUUCUGAUG | 543 | 20 | 4 |
| hsa-miR-3653 | CUAAGAAGUUGACUGAAG | 544 | 18 | 4 |
| hsa-miR-3663-3p | UGAGCACCACACAGGCCGGGCGC | 545 | 23 | 4 |
| hsa-miR-376a-5p | GUAGAUUCUCCUUCUAUGAGUA | 410 | 22 | 4 |
| hsa-miR-4423-3p | AUAGGCACCAAAAAGCAACAA | 662 | 21 | 4 |
| hsa-miR-4423-5p | AGUUGCCUUUUUGUUCCCAUGC | 263 | 22 | 4 |
| hsa-miR-4463 | GAGACUGGGGUGGGGCC | 300 | 17 | 4 |
| hsa-miR-449a | UGGCAGUGUAUUGUUAGCUGGU | 547 | 22 | 4 |
| hsa-miR-4511 | GAAGAACUGUUGCAUUUGCCCU | 548 | 22 | 4 |
| hsa-miR-4640-3p | CACCCCCUGUUUCCUGGCCCAC | 329 | 22 | 4 |
| hsa-miR-4800-3p | CAUCCGUCCGUCUGUCCAC | 549 | 19 | 4 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-505-5p | GGGAGCCAGGAAGUAUUGAUGU | 550 | 22 | 4 |
| hsa-miR-548a-3p | CAAAACUGGCAAUUACUUUUGC | 551 | 22 | 4 |
| hsa-miR-570-3p | CGAAAACAGCAAUUACCUUUGC | 333 | 22 | 4 |
| hsa-miR-663a | AGGCGGGGCGCCGCGGGACCGC | 365 | 22 | 4 |
| hsa-miR-877-3p | UCCUCUUCUCCCUCCUCCCAG | 552 | 21 | 4 |
| hsa-miR-103a-2-5p | AGCUUCUUUACAGUGCUGCCUUG | 553 | 23 | 3 |
| hsa-miR-1268b | CGGGCGUGGUGGUGGGGGUG | 554 | 20 | 3 |
| hsa-miR-1270 | CUGGAGAUAUGGAAGAGCUGUGU | 555 | 23 | 3 |
| hsa-miR-1293 | UGGGUGGUCUGGAGAUUUGUGC | 556 | 22 | 3 |
| hsa-miR-1322 | GAUGAUGCUGCUGAUGCUG | 557 | 19 | 3 |
| hsa-miR-150-5p | UCUCCCAACCCUUGUACCAGUG | 558 | 22 | 3 |
| hsa-miR-190b | UGAUAUGUUUGAUAUUGGGUU | 559 | 21 | 3 |
| hsa-miR-193a-3p | AACUGGCCUACAAAGUCCCAGU | 386 | 22 | 3 |
| hsa-miR-193b-5p | CGGGGUUUUGAGGGCGAGAUGA | 560 | 22 | 3 |
| hsa-miR-199a-5p | CCCAGUGUUCAGACUACCUGUUC | 273 | 23 | 3 |
| hsa-miR-20a-3p | ACUGCAUUAUGAGCACUUAAAG | 561 | 22 | 3 |
| hsa-miR-216a | UAAUCUCAGCUGGCAACUGUGA | 562 | 22 | 3 |
| hsa-miR-2682-5p | CAGGCAGUGACUGUUCAGACGUC | 563 | 23 | 3 |
| hsa-miR-2964a-5p | AGAUGUCCAGCCACAAUUCUCG | 564 | 22 | 3 |
| hsa-miR-3177-5p | UGUGUACACACGUGCCAGGCGCU | 565 | 23 | 3 |
| hsa-miR-320c | AAAAGCUGGGUUGAGAGGGU | 566 | 20 | 3 |
| hsa-miR-323a-5p | AGGUGGUCCGUGGCGCGUUCGC | 567 | 22 | 3 |
| hsa-miR-3622a-5p | CAGGCACGGGAGCUCAGGUGAG | 568 | 22 | 3 |
| hsa-miR-3912 | UAACGCAUAAUAUGGACAUGU | 569 | 21 | 3 |
| hsa-miR-3934 | UCAGGUGUGGAAACUGAGGCAG | 570 | 22 | 3 |
| hsa-miR-3942-3p | UUUCAGAUAACAGUAUUACAU | 414 | 21 | 3 |
| hsa-miR-3942-5p | AAGCAAUACUGUUACCUGAAAU | 571 | 22 | 3 |
| hsa-miR-4523 | GACCGAGAGGGCCUCGGCUGU | 572 | 21 | 3 |
| hsa-miR-4640-5p | UGGGCCAGGGAGCAGCUGGUGGG | 573 | 23 | 3 |
| hsa-miR-4671-5p | ACCGAAGACUGUGCGCUAAUCU | 574 | 22 | 3 |
| hsa-miR-4709-5p | ACAACAGUGACUUGCUCUCCAA | 575 | 22 | 3 |
| hsa-miR-4731-3p | CACACAAGUGGCCCCCAACACU | 425 | 22 | 3 |
| hsa-miR-4731-5p | UGCUGGGGGCCACAUGAGUGUG | 576 | 22 | 3 |
| hsa-miR-4762-5p | CCAAAUCUUGAUCAGAAGCCU | 577 | 21 | 3 |
| hsa-miR-5010-5p | AGGGGGAUGGCAGAGCAAAAUU | 578 | 22 | 3 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-502-5p | AUCCUUGCUAUCUGGGUGCUA | 579 | 21 | 3 |
| hsa-miR-548d-5p | AAAAGUAAUUGUGGUUUUUGCC | 580 | 22 | 3 |
| hsa-miR-548i | AAAAGUAAUUGCGGAUUUUGCC | 581 | 22 | 3 |
| hsa-miR-548j | AAAAGUAAUUGCGGUCUUUGGU | 582 | 22 | 3 |
| hsa-miR-5587-3p | GCCCCGGGCAGUGUGAUCAUC | 284 | 21 | 3 |
| hsa-miR-1225-3p | UGAGCCCCUGUGCCGCCCCCAG | 369 | 22 | 2 |
| hsa-miR-1227 | CGUGCCACCCUUUUCCCCAG | 583 | 20 | 2 |
| hsa-miR-1252 | AGAAGGAAAUUGAAUUCAUUUA | 371 | 22 | 2 |
| hsa-miR-1280 | UCCCACCGCUGCCACCC | 584 | 17 | 2 |
| hsa-miR-1288 | UGGACUGCCCUGAUCUGGAGA | 585 | 21 | 2 |
| hsa-miR-1303 | UUUAGAGACGGGGUCUUGCUCU | 586 | 22 | 2 |
| hsa-miR-1306-3p | ACGUUGGCUCUGGUGGUG | 376 | 18 | 2 |
| hsa-miR-139-5p | UCUACAGUGCACGUGUCUCCAG | 587 | 22 | 2 |
| hsa-miR-149-3p | AGGGAGGGACGGGGGCUGUGC | 588 | 21 | 2 |
| hsa-miR-16-1-3p | CCAGUAUUAACUGUGCUGCUGA | 589 | 22 | 2 |
| hsa-miR-1909-5p | UGAGUGCCGGUGCCUGCCCUG | 590 | 21 | 2 |
| hsa-miR-224-5p | CAAGUCACUAGUGGUUCCGUU | 591 | 21 | 2 |
| hsa-miR-2276 | UCUGCAAGUGUCAGAGGCGAGG | 592 | 22 | 2 |
| hsa-miR-2355-3p | AUUGUCCUUGCUGUUUGGAGAU | 468 | 22 | 2 |
| hsa-miR-2964a-3p | AGAAUUGCGUUUGGACAAUCAGU | 392 | 23 | 2 |
| hsa-miR-29c-5p | UGACCGAUUUCUCCUGGUGUUC | 594 | 22 | 2 |
| hsa-miR-3074-3p | GAUAUCAGCUCAGUAGGCACCG | 595 | 22 | 2 |
| hsa-miR-3120-3p | CACAGCAAGUGUAGACAGGCA | 596 | 21 | 2 |
| hsa-miR-3130-5p | UACCCAGUCUCCGGUGCAGCC | 396 | 21 | 2 |
| hsa-miR-3140-3p | AGCUUUUGGGAAUUCAGGUAGU | 597 | 22 | 2 |
| hsa-miR-3155a | CCAGGCUCUGCAGUGGGAACU | 398 | 21 | 2 |
| hsa-miR-3163 | UAUAAAAUGAGGGCAGUAAGAC | 598 | 22 | 2 |
| hsa-miR-3167 | AGGAUUUCAGAAAUACUGGUGU | 599 | 22 | 2 |
| hsa-miR-363-5p | CGGGUGGAUCACGAUGCAAUUU | 600 | 22 | 2 |
| hsa-miR-3676-3p | CCGUGUUUCCCCCACGCUUU | 408 | 20 | 2 |
| hsa-miR-378g | ACUGGGCUUGGAGUCAGAAG | 411 | 20 | 2 |
| hsa-miR-4467 | UGGCGGCGGUAGUUAUGGGCUU | 360 | 22 | 2 |
| hsa-miR-4498 | UGGGCUGGCAGGGCAAGUGCUG | 601 | 22 | 2 |
| hsa-miR-4654 | UGUGGGAUCUGGAGGCAUCUGG | 420 | 22 | 2 |
| hsa-miR-4659a-3p | UUUCUUCUUAGACAUGGCAACG | 603 | 22 | 2 |

(continued)

| **CELLS - CTX0E03 07EI** | | | | |
|---|---|---|---|---|
| MIRNA | MIRNA.SEQUENCE | SEQ ID NO: | MIRNA LENGTH | READ COUNTS |
| hsa-miR-4662a-5p | UUAGCCAAUUGUCCAUCUUUAG | 604 | 22 | 2 |
| hsa-miR-4683 | UGGAGAUCCAGUGCUCGCCCGAU | 605 | 23 | 2 |
| hsa-miR-4738-3p | UGAAACUGGAGCGCCUGGAGGA | 606 | 22 | 2 |
| hsa-miR-4746-3p | AGCGGUGCUCCUGCGGGCCGA | 607 | 21 | 2 |
| hsa-miR-4748 | GAGGUUUGGGGAGGAUUUGCU | 608 | 21 | 2 |
| hsa-miR-4792 | CGGUGAGCGCUCGCUGGC | 363 | 18 | 2 |
| hsa-miR-491-5p | AGUGGGGAACCCUUCCAUGAGG | 429 | 22 | 2 |
| hsa-miR-5000-3p | UCAGGACACUUCUGAACUUGGA | 609 | 22 | 2 |
| hsa-miR-503 | UAGCAGCGGGAACAGUUCUGCAG | 610 | 23 | 2 |
| hsa-miR-5189 | UCUGGGCACAGGCGGAUGGACAGG | 611 | 24 | 2 |
| hsa-miR-548aq-3p | CAAAAACUGCAAUUACUUUUGC | 612 | 22 | 2 |
| hsa-miR-548av-3p | AAAACUGCAGUUACUUUUGC | 613 | 20 | 2 |
| hsa-miR-5584-5p | CAGGGAAAUGGGAAGAACUAGA | 332 | 22 | 2 |
| hsa-miR-5690 | UCAGCUACUACCUCUAUUAGG | 435 | 21 | 2 |
| hsa-miR-573 | CUGAAGUGAUGUGUAACUGAUCAG | 305 | 24 | 2 |
| hsa-miR-597 | UGUGUCACUCGAUGACCACUGU | 614 | 22 | 2 |
| hsa-miR-622 | ACAGUCUGCUGAGGUUGGAGC | 615 | 21 | 2 |
| hsa-miR-636 | UGUGCUUGCUCGUCCCGCCCGCA | 616 | 23 | 2 |
| hsa-miR-1193 | GGGAUGGUAGACCGGUGACGUGC | 617 | 23 | 1 |
| hsa-miR-1224-3p | CCCCACCUCCUCUCUCCUCAG | 618 | 21 | 1 |
| hsa-miR-122-5p | UGGAGUGUGACAAUGGUGUUUG | 720 | 22 | 1 |
| hsa-miR-1228-5p | GUGGGCGGGGGCAGGUGUGUG | 620 | 21 | 1 |
| hsa-miR-1244 | AAGUAGUUGGUUUGUAUGAGAUGGUU | 340 | 26 | 1 |
| hsa-miR-1247-5p | ACCCGUCCCGUUCGUCCCCGGA | 621 | 22 | 1 |
| hsa-miR-1255b-5p | CGGAUGAGCAAAGAAAGUGGUU | 622 | 22 | 1 |
| hsa-miR-1269b | CUGGACUGAGCCAUGCUACUGG | 623 | 22 | 1 |
| hsa-miR-1272 | GAUGAUGAUGGCAGCAAAUUCUGAAA | 624 | 26 | 1 |
| hsa-miR-1273c | GGCGACAAAACGAGACCCUGUC | 625 | 22 | 1 |
| hsa-miR-1273e | UUGCUUGAACCCAGGAAGUGGA | 342 | 22 | 1 |
| hsa-miR-1282 | UCGUUUGCCUUUUUCUGCUU | 626 | 20 | 1 |
| hsa-miR-1290 | UGGAUUUUUGGAUCAGGGA | 375 | 19 | 1 |
| hsa-miR-1294 | UGUGAGGUUGGCAUUGUUGUCU | 627 | 22 | 1 |
| hsa-miR-1306-5p | CCACCUCCCCUGCAAACGUCCA | 628 | 22 | 1 |
| hsa-miR-1321 | CAGGGAGGUGAAUGUGAU | 377 | 18 | 1 |
| hsa-miR-135a-5p | UAUGGCUUUUUAUUCCUAUGUGA | 629 | 23 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-137 | UUAUUGCUUAAGAAUACGCGUAG | 630 | 23 | 1 |
| hsa-miR-142-5p | CAUAAAGUAGAAAGCACUACU | 631 | 21 | 1 |
| hsa-miR-143-5p | GGUGCAGUGCUGCAUCUCUGGU | 632 | 22 | 1 |
| hsa-miR-15a-3p | CAGGCCAUAUUGUGCUGCCUCA | 633 | 22 | 1 |
| hsa-miR-186-3p | GCCCAAAGGUGAAUUUUUUGGG | 382 | 22 | 1 |
| hsa-miR-192-3p | CUGCCAAUUCCAUAGGUCACAG | 634 | 22 | 1 |
| hsa-miR-19b-1-5p | AGUUUUGCAGGUUUGCAUCCAGC | 387 | 23 | 1 |
| hsa-miR-200a-3p | UAACACUGUCUGGUAACGAUGU | 635 | 22 | 1 |
| hsa-miR-204-3p | GCUGGGAAGGCAAAGGGACGU | 636 | 21 | 1 |
| hsa-miR-214-3p | ACAGCAGGCACAGACAGGCAGU | 637 | 22 | 1 |
| hsa-miR-29a-5p | ACUGAUUUCUUUUGGUGUUCAG | 393 | 22 | 1 |
| hsa-miR-3064-5p | UCUGGCUGUUGUGGUGUGCAA | 638 | 21 | 1 |
| hsa-miR-3116 | UGCCUGGAACAUAGUAGGGACU | 639 | 22 | 1 |
| hsa-miR-3125 | UAGAGGAAGCUGUGGAGAGA | 640 | 20 | 1 |
| hsa-miR-3127-3p | UCCCCUUCUGCAGGCCUGCUGG | 641 | 22 | 1 |
| hsa-miR-3130-3p | GCUGCACCGGAGACUGGGUAA | 395 | 21 | 1 |
| hsa-miR-3140-5p | ACCUGAAUUACCAAAAGCUUU | 397 | 21 | 1 |
| hsa-miR-3157-5p | UUCAGCCAGGCUAGUGCAGUCU | 642 | 22 | 1 |
| hsa-miR-3179 | AGAAGGGGUGAAAUUUAAACGU | 643 | 22 | 1 |
| hsa-miR-3181 | AUCGGGCCCUCGGCGCCGG | 644 | 19 | 1 |
| hsa-miR-3187-5p | CCUGGGCAGCGUGUGGCUGAAGG | 645 | 23 | 1 |
| hsa-miR-3190-5p | UCUGGCCAGCUACGUCCCCA | 646 | 20 | 1 |
| hsa-miR-3198 | GUGGAGUCCUGGGGAAUGGAGA | 647 | 22 | 1 |
| hsa-miR-320b | AAAAGCUGGGUUGAGAGGGCAA | 648 | 22 | 1 |
| hsa-miR-323b-5p | AGGUUGUCCGUGGUGAGUUCGCA | 401 | 23 | 1 |
| hsa-miR-3591-5p | UUUAGUGUGAUAAUGGCGUUUGA | 649 | 23 | 1 |
| hsa-miR-3619-5p | UCAGCAGGCAGGCUGGUGCAGC | 650 | 22 | 1 |
| hsa-miR-3659 | UGAGUGUUGUCUACGAGGGCA | 651 | 21 | 1 |
| hsa-miR-3674 | AUUGUAGAACCUAAGAUUGGCC | 652 | 22 | 1 |
| hsa-miR-3679-3p | CUUCCCCCCAGUAAUCUUCAUC | 653 | 22 | 1 |
| hsa-miR-375 | UUUGUUCGUUCGGCUCGCGUGA | 654 | 22 | 1 |
| hsa-miR-378b | ACUGGACUUGGAGGCAGAA | 655 | 19 | 1 |
| hsa-miR-3908 | GAGCAAUGUAGGUAGACUGUUU | 656 | 22 | 1 |
| hsa-miR-3911 | UGUGUGGAUCCUGGAGGAGGCA | 657 | 22 | 1 |
| hsa-miR-3913-5p | UUUGGGACUGAUCUUGAUGUCU | 658 | 22 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-3917 | GCUCGGACUGAGCAGGUGGG | 659 | 20 | 1 |
| hsa-miR-3944-3p | UUCGGGCUGGCCUGCUGCUCCGG | 660 | 23 | 1 |
| hsa-miR-429 | UAAUACUGUCUGGUAAAACCGU | 661 | 22 | 1 |
| hsa-miR-4421 | ACCUGUCUGUGGAAAGGAGCUA | 718 | 22 | 1 |
| hsa-miR-4443 | UUGGAGGCGUGGGUUUU | 663 | 17 | 1 |
| hsa-miR-4459 | CCAGGAGGCGGAGGAGGUGGAG | 664 | 22 | 1 |
| hsa-miR-4473 | CUAGUGCUCUCCGUUACAAGUA | 665 | 22 | 1 |
| hsa-miR-4479 | CGCGCGGCCGUGCUCGGAGCAG | 666 | 22 | 1 |
| hsa-miR-4497 | CUCCGGGACGGCUGGGC | 232 | 17 | 1 |
| hsa-miR-4504 | UGUGACAAUAGAGAUGAACAUG | 667 | 22 | 1 |
| hsa-miR-4520b-3p | UUUGGACAGAAAACACGCAGGU | 668 | 22 | 1 |
| hsa-miR-452-5p | AACUGUUUGCAGAGGAAACUGA | 669 | 22 | 1 |
| hsa-miR-4636 | AACUCGUGUUCAAAGCCUUUAG | 670 | 22 | 1 |
| hsa-miR-4659b-3p | UUUCUUCUUAGACAUGGCAGCU | 671 | 22 | 1 |
| hsa-miR-4664-3p | CUUCCGGUCUGUGAGCCCCGUC | 672 | 22 | 1 |
| hsa-miR-4665-5p | CUGGGGGACGCGUGAGCGCGAGC | 673 | 23 | 1 |
| hsa-miR-4666a-5p | AUACAUGUCAGAUUGUAUGCC | 674 | 21 | 1 |
| hsa-miR-4673 | UCCAGGCAGGAGCCGGACUGGA | 422 | 22 | 1 |
| hsa-miR-4681 | AACGGGAAUGCAGGCUGUAUCU | 675 | 22 | 1 |
| hsa-miR-4682 | UCUGAGUUCCUGGAGCCUGGUCU | 676 | 23 | 1 |
| hsa-miR-4690-5p | GAGCAGGCGAGGCUGGGCUGAA | 677 | 22 | 1 |
| hsa-miR-4699-5p | AGAAGAUUGCAGAGUAAGUUCC | 678 | 22 | 1 |
| hsa-miR-4700-3p | CACAGGACUGACUCCUCACCCCAGUG | 424 | 26 | 1 |
| hsa-miR-4706 | AGCGGGGAGGAAGUGGGCGCUGCUU | 679 | 25 | 1 |
| hsa-miR-4721 | UGAGGGCUCCAGGUGACGGUGG | 680 | 22 | 1 |
| hsa-miR-4728-3p | CAUGCUGACCUCCCUCCUGCCCCAG | 681 | 25 | 1 |
| hsa-miR-4742-5p | UCAGGCAAAGGGAUAUUUACAGA | 682 | 23 | 1 |
| hsa-miR-4747-3p | AAGGCCCGGGCUUUCCUCCCAG | 683 | 22 | 1 |
| hsa-miR-4749-5p | UGCGGGGACAGGCCAGGGCAUC | 684 | 22 | 1 |
| hsa-miR-4755-3p | AGCCAGGCUCUGAAGGGAAAGU | 685 | 22 | 1 |
| hsa-miR-4763-5p | CGCCUGCCCAGCCCUCCUGCU | 686 | 21 | 1 |
| hsa-miR-4766-3p | AUAGCAAUUGCUCUUUUGGAA | 687 | 21 | 1 |
| hsa-miR-4781-3p | AAUGUUGGAAUCCUCGCUAGAG | 688 | 22 | 1 |
| hsa-miR-4793-3p | UCUGCACUGUGAGUUGGCUGGCU | 689 | 23 | 1 |
| hsa-miR-488-3p | UUGAAAGGCUAUUUCUUGGUC | 690 | 21 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-4999-5p | UGCUGUAUUGUCAGGUAGUGA | 691 | 21 | 1 |
| hsa-miR-5001-5p | AGGGCUGGACUCAGCGGCGGAGCU | 692 | 24 | 1 |
| hsa-miR-5002-5p | AAUUUGGUUUCUGAGGCACUUAGU | 693 | 24 | 1 |
| hsa-miR-5004-5p | UGAGGACAGGGCAAAUUCACGA | 694 | 22 | 1 |
| hsa-miR-5006-3p | UUUCCCUUUCCAUCCUGGCAG | 695 | 21 | 1 |
| hsa-miR-5088 | CAGGGCUCAGGGAUUGGAUGGAG | 696 | 23 | 1 |
| hsa-miR-544a | AUUCUGCAUUUUUAGCAAGUUC | 697 | 22 | 1 |
| hsa-miR-548al | AACGGCAAUGACUUUUGUACCA | 698 | 22 | 1 |
| hsa-miR-548aq-5p | GAAAGUAAUUGCUGUUUUUGCC | 699 | 22 | 1 |
| hsa-miR-548at-5p | AAAAGUUAUUGCGGUUUUGGCU | 700 | 22 | 1 |
| hsa-miR-548au-5p | AAAAGUAAUUGCGGUUUUUGC | 701 | 21 | 1 |
| hsa-miR-548b-3p | CAAGAACCUCAGUUGCUUUUGU | 702 | 22 | 1 |
| hsa-miR-556-3p | AUAU UACCAU UAGCUCAUCUUU | 703 | 22 | 1 |
| hsa-miR-5582-3p | UAAAACUUUAAGUGUGCCUAGG | 704 | 22 | 1 |
| hsa-miR-5586-3p | CAGAGUGACAAGCUGGUUAAAG | 705 | 22 | 1 |
| hsa-miR-5588-5p | ACUGGCAUUAGUGGGACUUUU | 706 | 21 | 1 |
| hsa-miR-5683 | UACAGAUGCAGAUUCUCUGACUUC | 707 | 24 | 1 |
| hsa-miR-5696 | CUCAUUUAAGUAGUCUGAUGCC | 708 | 22 | 1 |
| hsa-miR-5701 | UUAUUGUCACGUUCUGAUU | 709 | 19 | 1 |
| hsa-miR-5706 | UUCUGGAUAACAUGCUGAAGCU | 710 | 22 | 1 |
| hsa-miR-592 | UUGUGUCAAUAUGCGAUGAUGU | 711 | 22 | 1 |
| hsa-miR-603 | CACACACUGCAAUUACUUUUGC | 712 | 22 | 1 |
| hsa-miR-624-3p | CACAAGGUAUUGGUAUUACCU | 713 | 21 | 1 |
| hsa-miR-885-5p | UCCAUUACACUACCCUGCCUCU | 714 | 22 | 1 |
| hsa-miR-933 | UGUGCGCAGGGAGACCUCUCCC | 715 | 22 | 1 |

**Table 6: Microvesicles EI**

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 32723 |
| hsa-miR-4492 | GGGGCUGGGCGCGCGCC | 34 | 17 | 16225 |
| hsa-miR-4488 | AGGGGGCGGGCUCCGGCG | 61 | 18 | 12878 |
| hsa-miR-4532 | CCCCGGGGAGCCCGGCG | 23 | 17 | 6746 |
| hsa-miR-4508 | GCGGGGCUGGGCGCGCG | 135 | 17 | 531 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-4516 | GGGAGAAGGGUCGGGGC | 110 | 17 | 500 |
| hsa-miR-3676-5p | AGGAGAUCCUGGGUU | 280 | 15 | 357 |
| hsa-miR-4485 | UAACGGCCGCGGUACCCUAA | 67 | 20 | 44 |
| hsa-miR-4497 | CUCCGGGACGGCUGGGC | 232 | 17 | 43 |
| hsa-miR-21-5p | UAGCUUAUCAGACUGAUGUUGA | 9 | 22 | 33 |
| hsa-miR-3195 | CGCGCCGGGCCCGGGUU | 716 | 17 | 28 |
| hsa-miR-3648 | AGCCGCGGGGAUCGCCGAGGG | 259 | 21 | 26 |
| hsa-miR-663b | GGUGGCCCGGCCGUGCCUGAGG | 180 | 22 | 24 |
| hsa-miR-3656 | GGCGGGUGCGGGGGUGG | 251 | 17 | 19 |
| hsa-miR-3687 | CCCGGACAGGCGUUCGUGCGACGU | 190 | 24 | 19 |
| hsa-miR-4466 | GGGUGCGGGCCGGCGGGG | 264 | 18 | 19 |
| hsa-miR-4792 | CGGUGAGCGCUCGCUGGC | 363 | 18 | 19 |
| hsa-miR-99b-5p | CACCCGUAGAACCGACCUUGCG | 4 | 22 | 18 |
| hsa-let-7a-5p | UGAGGUAGUAGGUUGUAUAGUU | 1 | 22 | 15 |
| hsa-miR-1290 | UGGAUUUUUGGAUCAGGGA | 375 | 19 | 7 |
| hsa-miR-1291 | UGGCCCUGACUGAAGACCAGCAGU | 294 | 24 | 7 |
| hsa-miR-182-5p | UUUGGCAAUGGUAGAACUCACACU | 16 | 24 | 7 |
| hsa-miR-5096 | GUUUCACCAUGUUGGUCAGGC | 220 | 21 | 7 |
| hsa-miR-1273f | GGAGAUGGAGGUUGCAGUG | 292 | 19 | 5 |
| hsa-miR-26a-5p | UUCAAGUAAUCCAGGAUAGGCU | 12 | 22 | 5 |
| hsa-miR-4284 | GGGCUCACAUCACCCCAU | 191 | 18 | 5 |
| hsa-miR-92b-3p | UAUUGCACUCGUCCCGGCCUCC | 13 | 22 | 5 |
| hsa-let-7b-5p | UGAGGUAGUAGGUUGUGUGGUU | 28 | 22 | 4 |
| hsa-let-7c | UGAGGUAGUAGGUUGUAUGGUU | 17 | 22 | 4 |
| hsa-let-7f-5p | UGAGGUAGUAGAUUGUAUAGUU | 11 | 22 | 4 |
| hsa-miR-100-5p | AACCCGUAGAUCCGAACUUGUG | 3 | 22 | 4 |
| hsa-miR-1248 | ACCUUCUUGUAUAAGCACUGUGCUAAA | 269 | 27 | 4 |
| hsa-miR-1973 | ACCGUGCAAAGGUAGCAUA | 171 | 19 | 4 |
| hsa-miR-21-3p | CAACACCAGUCGAUGGGCUGU | 20 | 21 | 4 |
| hsa-miR-3654 | GACUGGACAAGCUGAGGAA | 325 | 19 | 4 |
| hsa-miR-92a-3p | UAUUGCACUUGUCCCGGCCUGU | 7 | 22 | 4 |
| hsa-miR-1273g-3p | ACCACUGCACUCCAGCCUGAG | 210 | 21 | 3 |
| hsa-miR-23b-3p | AUCACAUUGCCAGGGAUUACC | 59 | 21 | 3 |
| hsa-miR-3609 | CAAAGUGAUGAGUAAUACUGGCUG | 216 | 24 | 3 |
| hsa-miR-3615 | UCUCUCGGCUCCUCGCGGCUC | 323 | 21 | 3 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-3653 | CUAAGAAGUUGACUGAAG | 544 | 18 | 3 |
| hsa-miR-3960 | GGCGGCGGCGGAGGCGGGGG | 416 | 20 | 3 |
| hsa-miR-4448 | GGCUCCUUGGUCUAGGGGUA | 231 | 20 | 3 |
| hsa-let-7d-5p | AGAGGUAGUAGGUUGCAUAGUU | 92 | 22 | 2 |
| hsa-miR-16-5p | UAGCAGCACGUAAAUAUUGGCG | 29 | 22 | 2 |
| hsa-miR-181a-5p | AACAUUCAACGCUGUCGGUGAGU | 15 | 23 | 2 |
| hsa-miR-181b-5p | AACAUUCAUUGCUGUCGGUGGGU | 38 | 23 | 2 |
| hsa-miR-222-3p | AGCUACAUCUGGCUACUGGGU | 36 | 21 | 2 |
| hsa-miR-24-3p | UGGCUCAGUUCAGCAGGAACAG | 119 | 22 | 2 |
| hsa-miR-3196 | CGGGGCGGCAGGGGCCUC | 717 | 18 | 2 |
| hsa-miR-4419b | GAGGCUGAAGGAAGAUGG | 718 | 18 | 2 |
| hsa-miR-4461 | GAUUGAGACUAGUAGGGCUAGGC | 129 | 23 | 2 |
| hsa-miR-4486 | GCUGGGCGAGGCUGGCA | 719 | 17 | 2 |
| hsa-miR-663a | AGGCGGGGCGCCGCGGGACCGC | 365 | 22 | 2 |
| hsa-miR-9-5p | UCUUUGGUUAUCUAGCUGUAUGA | 58 | 23 | 2 |
| hsa-let-7i-3p | CUGCGCAAGCUACUGCCUUGCU | 483 | 22 | 1 |
| hsa-let-7i-5p | UGAGGUAGUAGUUUGUGCUGUU | 22 | 22 | 1 |
| hsa-miR-1225-5p | GUGGGUACGGCCCAGUGGGGGG | 720 | 22 | 1 |
| hsa-miR-1244 | AAGUAGUUGGUUUGUAUGAGAUGGUU | 340 | 26 | 1 |
| hsa-miR-125b-5p | UCCCUGAGACCCUAACUUGUGA | 42 | 22 | 1 |
| hsa-miR-1275 | GUGGGGGAGAGGCUGUC | 162 | 17 | 1 |
| hsa-miR-1280 | UCCCACCGCUGCCACCC | 584 | 17 | 1 |
| hsa-miR-134 | UGUGACUGGUUGACCAGAGGGG | 94 | 22 | 1 |
| hsa-miR-149-5p | UCUGGCUCCGUGUCUUCACUCCC | 121 | 23 | 1 |
| hsa-miR-191-5p | CAACGGAAUCCCAAAAGCAGCUG | 8 | 23 | 1 |
| hsa-miR-221-3p | AGCUACAUUGUCUGCUGGGUUUC | 79 | 23 | 1 |
| hsa-miR-22-3p | AAGCUGCCAGUUGAAGAACUGU | 33 | 22 | 1 |
| hsa-miR-26b-5p | UUCAAGUAAUUCAGGAUAGGU | 90 | 21 | 1 |
| hsa-miR-30c-5p | UGUAAACAUCCUACACUCUCAGC | 66 | 23 | 1 |
| hsa-miR-30d-5p | UGUAAACAUCCCCGACUGGAAG | 31 | 22 | 1 |
| hsa-miR-3182 | GCUUCUGUAGUGUAGUC | 721 | 17 | 1 |
| hsa-miR-320a | AAAAGCUGGGUUGAGAGGGCGA | 97 | 22 | 1 |
| hsa-miR-34a-5p | UGGCAGUGUCUUAGCUGGUUGU | 101 | 22 | 1 |
| hsa-miR-3607-3p | ACUGUAAACGCUUUCUGAUG | 543 | 20 | 1 |
| hsa-miR-361-5p | UUAUCAGAAUCUCCAGGGGUAC | 70 | 22 | 1 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-3652 | CGGCUGGAGGUGUGAGGA | 722 | 18 | 1 |
| hsa-miR-409-3p | GAAUGUUGCUCGGUGAACCCCU | 47 | 22 | 1 |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-432-5p | UCUUGGAGUAGGUCAUUGGGUGG | 95 | 23 | 1 |
| hsa-miR-4417 | GGUGGGCUUCCCGGAGGG | 175 | 18 | 1 |
| hsa-miR-4426 | GAAGAUGGACGUACUUU | 359 | 17 | 1 |
| hsa-miR-4449 | CGUCCCGGGGCUGCGCGAGGCA | 155 | 22 | 1 |
| hsa-miR-4800-3p | CAUCCGUCCGUCUGUCCAC | 549 | 19 | 1 |
| hsa-miR-484 | UCAGGCUCAGUCCCCUCCCGAU | 118 | 22 | 1 |
| hsa-miR-486-5p | UCCUGUACUGAGCUGCCCCGAG | 5 | 22 | 1 |
| hsa-miR-493-3p | UGAAGGUCUACUGUGUGCCAGG | 83 | 22 | 1 |
| hsa-miR-5095 | UUACAGGCGUGAACCACCGCG | 723 | 21 | 1 |
| hsa-miR-556-3p | AUAUUACCAUUAGCUCAUCUUU | 703 | 22 | 1 |
| hsa-miR-644b-5p | UGGGCUAAGGGAGAUGAUUGGGUA | 724 | 24 | 1 |
| hsa-miR-664-5p | ACUGGCUAGGGAAAAUGAUUGGAU | 443 | 24 | 1 |
| hsa-miR-760 | CGGCUCUGGGUCUGUGGGGA | 289 | 20 | 1 |
| hsa-miR-941 | CACCCGGCUGUGUGCACAUGUGC | 60 | 23 | 1 |
| hsa-miR-98 | UGAGGUAGUAAGUUGUAUUGUU | 10 | 22 | 1 |
| hsa-miR-99a-5p | AACCCGUAGAUCCGAUCUUGUG | 52 | 22 | 1 |

**Table 7: Exosomes EI**

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 83958 |
| hsa-miR-4492 | GGGGCUGGGCGCGCGCC | 34 | 17 | 22482 |
| hsa-miR-4488 | AGGGGGCGGGCUCCGGCG | 61 | 18 | 20618 |
| hsa-miR-4532 | CCCCGGGGAGCCCGGCG | 23 | 17 | 6419 |
| hsa-miR-4516 | GGGAGAAGGGUCGGGGC | 110 | 17 | 904 |
| hsa-miR-4508 | GCGGGGCUGGGCGCGCG | 135 | 17 | 723 |
| hsa-miR-3676-5p | AGGAGAUCCUGGGUU | 280 | 15 | 174 |
| hsa-miR-4485 | UAACGGCCGCGGUACCCUAA | 67 | 20 | 43 |
| hsa-miR-21-5p | UAGCUUAUCAGACUGAUGUUGA | 9 | 22 | 41 |
| hsa-miR-4497 | CUCCGGGACGGCUGGGC | 232 | 17 | 28 |

(continued)

| **EXOSOMES CTX0E03 07EI** | | | | |
|---|---|---|---|---|
| MIRNA | MIRNA.SEQUENCE | SEQ ID NO: | MIRNA LENGTH | READ COUNTS |
| hsa-miR-663b | GGUGGCCCGGCCGUGCCUGAGG | 180 | 22 | 26 |
| hsa-miR-4792 | CGGUGAGCGCUCGCUGGC | 363 | 18 | 24 |
| hsa-miR-4454 | GGAUCCGAGUCACGGCACCA | 299 | 20 | 22 |
| hsa-miR-1291 | UGGCCCUGACUGAAGACCAGCAGU | 294 | 24 | 17 |
| hsa-miR-26a-5p | UUCAAGUAAUCCAGGAUAGGCU | 12 | 22 | 17 |
| hsa-miR-3195 | CGCGCCGGGCCCGGGUU | 716 | 17 | 17 |
| hsa-let-7a-5p | UGAGGUAGUAGGUUGUAUAGUU | 1 | 22 | 15 |
| hsa-miR-182-5p | UUUGGCAAUGGUAGAACUCACACU | 16 | 24 | 15 |
| hsa-miR-99b-5p | CACCCGUAGAACCGACCUUGCG | 4 | 22 | 15 |
| hsa-miR-5096 | GUUUCACCAUGUUGGUCAGGC | 220 | 21 | 14 |
| hsa-miR-3648 | AGCCGCGGGGAUCGCCGAGGG | 259 | 21 | 13 |
| hsa-miR-3654 | GACUGGACAAGCUGAGGAA | 325 | 19 | 13 |
| hsa-miR-4466 | GGGUGCGGGCCGGCGGGG | 264 | 18 | 12 |
| hsa-miR-3687 | CCCGGACAGGCGUUCGUGCGACGU | 190 | 24 | 11 |
| hsa-miR-4284 | GGGCUCACAUCACCCCAU | 191 | 18 | 11 |
| hsa-miR-3656 | GGCGGGUGCGGGGGUGG | 251 | 17 | 10 |
| hsa-miR-3609 | CAAAGUGAUGAGUAAUACUGGCUG | 216 | 24 | 8 |
| hsa-miR-644b-5p | UGGGCUAAGGGAGAUGAUUGGGUA | 724 | 24 | 8 |
| hsa-miR-664-5p | ACUGGCUAGGGAAAAUGAUUGGAU | 443 | 24 | 8 |
| hsa-miR-92a-3p | UAUUGCACUUGUCCCGGCCUGU | 7 | 22 | 7 |
| hsa-miR-92b-3p | UAUUGCACUCGUCCCGGCCUCC | 13 | 22 | 7 |
| hsa-let-7b-5p | UGAGGUAGUAGGUUGUGUGGUU | 28 | 22 | 6 |
| hsa-let-7f-5p | UGAGGUAGUAGAUUGUAUAGUU | 11 | 22 | 6 |
| hsa-miR-127-3p | UCGGAUCCGUCUGAGCUUGGCU | 14 | 22 | 6 |
| hsa-miR-1290 | UGGAUUUUUGGAUCAGGGA | 375 | 19 | 6 |
| hsa-miR-4449 | CGUCCCGGGGCUGCGCGAGGCA | 155 | 22 | 6 |
| hsa-miR-4461 | GAUUGAGACUAGUAGGGCUAGGC | 129 | 23 | 6 |
| hsa-miR-100-5p | AACCCGUAGAUCCGAACUUGUG | 3 | 22 | 5 |
| hsa-miR-1248 | ACCUUCUUGUAUAAGCACUGUGCUAAA | 269 | 27 | 5 |
| hsa-miR-1973 | ACCGUGCAAAGGUAGCAUA | 171 | 19 | 5 |
| hsa-miR-3653 | CUAAGAAGUUGACUGAAG | 544 | 18 | 5 |
| hsa-miR-4417 | GGUGGGCUUCCCGGAGGG | 175 | 18 | 5 |
| hsa-miR-125b-5p | UCCCUGAGACCCUAACUUGUGA | 42 | 22 | 4 |
| hsa-miR-151a-3p | CUAGACUGAAGCUCCUUGAGG | 25 | 21 | 4 |
| hsa-miR-16-5p | UAGCAGCACGUAAAUAUUGGCG | 29 | 22 | 4 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-21-3p | CAACACCAGUCGAUGGGCUGU | 20 | 21 | 4 |
| hsa-miR-23a-3p | AUCACAUUGCCAGGGAUUUCC | 55 | 21 | 4 |
| hsa-miR-4419b | GAGGCUGAAGGAAGAUGG | 718 | 18 | 4 |
| hsa-miR-1273f | GGAGAUGGAGGUUGCAGUG | 292 | 19 | 3 |
| hsa-miR-1273g-3p | ACCACUGCACUCCAGCCUGAG | 210 | 21 | 3 |
| hsa-miR-181b-5p | AACAUUCAUUGCUGUCGGUGGGU | 38 | 23 | 3 |
| hsa-miR-221-3p | AGCUACAUUGUCUGCUGGGUUUC | 79 | 23 | 3 |
| hsa-miR-3615 | UCUCUCGGCUCCUCGCGGCUC | 323 | 21 | 3 |
| hsa-miR-9-5p | UCUUUGGUUAUCUAGCUGUAUGA | 58 | 23 | 3 |
| hsa-let-7c | UGAGGUAGUAGGUUGUAUGGUU | 17 | 22 | 2 |
| hsa-let-7e-5p | UGAGGUAGGAGGUUGUAUAGUU | 27 | 22 | 2 |
| hsa-let-7i-5p | UGAGGUAGUAGUUUGUGCUGUU | 22 | 22 | 2 |
| hsa-miR-103a-3p | AGCAGCAUUGUACAGGGCUAUGA | 62 | 23 | 2 |
| hsa-miR-106b-5p | UAAAGUGCUGACAGUGCAGAU | 170 | 21 | 2 |
| hsa-miR-1273e | UUGCUUGAACCCAGGAAGUGGA | 342 | 22 | 2 |
| hsa-miR-221-5p | ACCUGGCAUACAAUGUAGAUUU | 39 | 22 | 2 |
| hsa-miR-222-3p | AGCUACAUCUGGCUACUGGGU | 36 | 21 | 2 |
| hsa-miR-30d-5p | UGUAAACAUCCCCGACUGGAAG | 31 | 22 | 2 |
| hsa-miR-3960 | GGCGGCGGCGGAGGCGGGGG | 416 | 20 | 2 |
| hsa-let-7d-3p | CUAUACGACCUGCUGCCUUUCU | 92 | 22 | 1 |
| hsa-let-7d-5p | AGAGGUAGUAGGUUGCAUAGUU | 53 | 22 | 1 |
| hsa-let-7g-5p | UGAGGUAGUAGUUUGUACAGUU | 43 | 22 | 1 |
| hsa-let-7i-3p | CUGCGCAAGCUACUGCCUUGCU | 483 | 22 | 1 |
| hsa-miR-10a-5p | UACCCUGUAGAUCCGAAUUUGUG | 2 | 23 | 1 |
| hsa-miR-1181 | CCGUCGCCGCCACCCGAGCCG | 725 | 21 | 1 |
| hsa-miR-1225-3p | UGAGCCCCUGUGCCGCCCCCAG | 369 | 22 | 1 |
| hsa-miR-1244 | AAGUAGUUGGUUUGUAUGAGAUGGUU | 340 | 26 | 1 |
| hsa-miR-125a-5p | UCCCUGAGACCCUUUAACCUGUGA | 35 | 24 | 1 |
| hsa-miR-1296 | UUAGGGCCCUGGCUCCAUCUCC | 271 | 22 | 1 |
| hsa-miR-1307-5p | UCGACCGGACCUCGACCGGCU | 91 | 21 | 1 |
| hsa-miR-146b-5p | UGAGAACUGAAUUCCAUAGGCU | 19 | 22 | 1 |
| hsa-miR-149-5p | UCUGGCUCCGUGUCUUCACUCCC | 121 | 23 | 1 |
| hsa-miR-151a-5p | UCGAGGAGCUCACAGUCUAGU | 37 | 21 | 1 |
| hsa-miR-15b-5p | UAGCAGCACAUCAUGGUUUACA | 78 | 22 | 1 |
| hsa-miR-181a-2-3p | ACCACUGACCGUUGACUGUACC | 102 | 22 | 1 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-181a-5p | AACAUUCAACGCUGUCGGUGAGU | 15 | 23 | 1 |
| hsa-miR-191-5p | CAACGGAAUCCCAAAAGCAGCUG | 8 | 23 | 1 |
| hsa-miR-198 | GGUCCAGAGGGGAGAUAGGUUC | 726 | 22 | 1 |
| hsa-miR-204-5p | UUCCCUUUGUCAUCCUAUGCCU | 89 | 22 | 1 |
| hsa-miR-20a-5p | UAAAGUGCUUAUAGUGCAGGUAG | 146 | 23 | 1 |
| hsa-miR-219-5p | UGAUUGUCCAAACGCAAUUCU | 527 | 21 | 1 |
| hsa-miR-22-3p | AAGCUGCCAGUUGAAGAACUGU | 33 | 22 | 1 |
| hsa-miR-23b-3p | AUCACAUUGCCAGGGAUUACC | 59 | 21 | 1 |
| hsa-miR-26b-3p | CCUGUUCUCCAUUACUUGGCUC | 391 | 22 | 1 |
| hsa-miR-299-5p | UGGUUUACCGUCCCACAUACAU | 319 | 22 | 1 |
| hsa-miR-29a-3p | UAGCACCAUCUGAAAUCGGUUA | 106 | 22 | 1 |
| hsa-miR-30e-3p | CUUUCAGUCGGAUGUUUACAGC | 71 | 22 | 1 |
| hsa-miR-31-3p | UGCUAUGCCAACAUAUUGCCAU | 172 | 22 | 1 |
| hsa-miR-3198 | GUGGAGUCCUGGGGAAUGGAGA | 647 | 22 | 1 |
| hsa-miR-323a-3p | CACAUUACACGGUCGACCUCU | 158 | 21 | 1 |
| hsa-miR-342-3p | UCUCACACAGAAAUCGCACCCGU | 81 | 23 | 1 |
| hsa-miR-3607-3p | ACUGUAAACGCUUUCUGAUG | 543 | 20 | 1 |
| hsa-miR-3651 | CAUAGCCCGGUCGCUGGUACAUGA | 727 | 24 | 1 |
| hsa-miR-378a-3p | ACUGGACUUGGAGUCAGAAGG | 65 | 21 | 1 |
| hsa-miR-379-5p | UGGUAGACUAUGGAACGUAGG | 18 | 21 | 1 |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-425-5p | AAUGACACGAUCACUCCCGUUGA | 111 | 23 | 1 |
| hsa-miR-4258 | CCCCGCCACCGCCUUGG | 728 | 17 | 1 |
| hsa-miR-4426 | GAAGAUGGACGUACUUU | 359 | 17 | 1 |
| hsa-miR-4443 | UUGGAGGCGUGGGUUUU | 663 | 17 | 1 |
| hsa-miR-4448 | GGCUCCUUGGUCUAGGGGUA | 231 | 20 | 1 |
| hsa-miR-4697-3p | UGUCAGUGACUCCUGCCCCUUGGU | 729 | 24 | 1 |
| hsa-miR-4700-3p | CACAGGACUGACUCCUCACCCCAGUG | 424 | 26 | 1 |
| hsa-miR-4700-5p | UCUGGGGAUGAGGACAGUGUGU | 730 | 22 | 1 |
| hsa-miR-4797-3p | UCUCAGUAAGUGGCACUCUGU | 731 | 21 | 1 |
| hsa-miR-484 | UCAGGCUCAGUCCCCUCCCGAU | 118 | 22 | 1 |
| hsa-miR-486-5p | UCCUGUACUGAGCUGCCCCGAG | 5 | 22 | 1 |
| hsa-miR-494 | UGAAACAUACACGGGAAACCUC | 240 | 22 | 1 |
| hsa-miR-500a-5p | UAAUCCUUGCUACCUGGGUGAGA | 303 | 23 | 1 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-644b-3p | UUCAUUUGCCUCCCAGCCUACA | 442 | 22 | 1 |
| hsa-miR-663a | AGGCGGGGCGCCGCGGGACCGC | 365 | 22 | 1 |

**Table 8: Microvesicles EH**

| MICROVESICLES CTX0E03 07EH | | | | |
|---|---|---|---|---|
| MIRNA | MIRNA.SEQUENCE | SEQ ID NO: | MIRNA LENGTH | READ COUNTS |
| | | | | |
| hsa-miR-1246 | AAUGGAUUUUUGGAGCAGG | 21 | 19 | 78791 |
| hsa-miR-4492 | GGGGCUGGGCGCGCGCC | 34 | 17 | 6012 |
| hsa-miR-4532 | CCCCGGGGAGCCCGGCG | 23 | 17 | 3410 |
| hsa-miR-4488 | AGGGGGCGGGCUCCGGCG | 61 | 18 | 1737 |
| hsa-miR-4485 | UAACGGCCGCGGUACCCUAA | 67 | 20 | 319 |
| hsa-miR-4508 | GCGGGGCUGGGCGCGCG | 135 | 17 | 221 |
| hsa-miR-4516 | GGGAGAAGGGUCGGGGC | 110 | 17 | 114 |
| hsa-miR-4466 | GGGUGCGGGCCGGCGGGG | 264 | 18 | 61 |
| hsa-miR-4497 | CUCCGGGACGGCUGGGC | 232 | 17 | 51 |
| hsa-miR-3195 | CGCGCCGGGCCCGGGUU | 716 | 17 | 41 |
| hsa-miR-1973 | ACCGUGCAAAGGUAGCAUA | 171 | 19 | 30 |
| hsa-miR-21-5p | UAGCUUAUCAGACUGAUGUUGA | 9 | 22 | 22 |
| hsa-miR-4284 | GGGCUCACAUCACCCCAU | 191 | 18 | 20 |
| hsa-miR-4792 | CGGUGAGCGCUCGCUGGC | 363 | 18 | 12 |
| hsa-miR-92a-3p | UAUUGCACUUGUCCCGGCCUGU | 7 | 22 | 12 |
| hsa-miR-1291 | UGGCCCUGACUGAAGACCAGCAGU | 294 | 24 | 11 |
| hsa-miR-3676-5p | AGGAGAUCCUGGGUU | 280 | 15 | 10 |
| hsa-miR-100-5p | AACCCGUAGAUCCGAACUUGUG | 3 | 22 | 8 |
| hsa-miR-3656 | GGCGGGUGCGGGGGUGG | 251 | 17 | 8 |
| hsa-miR-663b | GGUGGCCCGGCCGUGCCUGAGG | 180 | 22 | 8 |
| hsa-let-7a-5p | UGAGGUAGUAGGUUGUAUAGUU | 1 | 22 | 7 |
| hsa-miR-1290 | UGGAUUUUUGGAUCAGGGA | 375 | 19 | 7 |
| hsa-miR-3687 | CCCGGACAGGCGUUCGUGCGACGU | 190 | 24 | 7 |
| hsa-miR-4461 | GAUUGAGACUAGUAGGGCUAGGC | 52 | 23 | 6 |
| hsa-miR-664-5p | ACUGGCUAGGGAAAAUGAUUGGAU | 91 | 24 | 6 |
| hsa-miR-92b-3p | UAUUGCACUCGUCCCGGCCUCC | 13 | 22 | 6 |
| hsa-miR-125b-5p | UCCCUGAGACCCUAACUUGUGA | 42 | 22 | 5 |

(continued)

| MICROVESICLES CTX0E03 07EH | | | | |
|---|---|---|---|---|
| MIRNA | MIRNA.SEQUENCE | SEQ ID NO: | MIRNA LENGTH | READ COUNTS |
| hsa-miR-3653 | CUAAGAAGUUGACUGAAG | 544 | 18 | 5 |
| hsa-let-7f-5p | UGAGGUAGUAGAUUGUAUAGUU | 11 | 22 | 4 |
| hsa-miR-16-5p | UAGCAGCACGUAAAUAUUGGCG | 29 | 22 | 4 |
| hsa-miR-181a-5p | AACAUUCAACGCUGUCGGUGAGU | 15 | 23 | 4 |
| hsa-miR-3609 | CAAAGUGAUGAGUAAUACUGGCUG | 216 | 24 | 4 |
| hsa-miR-9-5p | UCUUUGGUUAUCUAGCUGUAUGA | 58 | 23 | 4 |
| hsa-let-7c | UGAGGUAGUAGGUUGUAUGGUU | 17 | 22 | 3 |
| hsa-miR-1244 | AAGUAGUUGGUUUGUAUGAGAUGGUU | 59 | 26 | 3 |
| hsa-miR-127-3p | UCGGAUCCGUCUGAGCUUGGCU | 14 | 22 | 3 |
| hsa-miR-181b-5p | AACAUUCAUUGCUGUCGGUGGGU | 38 | 23 | 3 |
| hsa-miR-21-3p | CAACACCAGUCGAUGGGCUGU | 20 | 21 | 3 |
| hsa-miR-26a-5p | UUCAAGUAAUCCAGGAUAGGCU | 12 | 22 | 3 |
| hsa-miR-30c-5p | UGUAAACAUCCUACACUCUCAGC | 66 | 23 | 3 |
| hsa-miR-3960 | GGCGGCGGCGGAGGCGGGGG | 416 | 20 | 3 |
| hsa-miR-485-3p | GUCAUACACGGCUCUCCUCUCU | 153 | 22 | 3 |
| hsa-let-7b-5p | UGAGGUAGUAGGUUGUGUGGUU | 28 | 22 | 2 |
| hsa-let-7g-5p | UGAGGUAGUAGUUUGUACAGUU | 43 | 22 | 2 |
| hsa-miR-1273f | GGAGAUGGAGGUUGCAGUG | 292 | 19 | 2 |
| hsa-miR-151a-3p | CUAGACUGAAGCUCCUUGAGG | 25 | 21 | 2 |
| hsa-miR-182-5p | UUUGGCAAUGGUAGAACUCACACU | 16 | 24 | 2 |
| hsa-miR-191-5p | CAACGGAAUCCCAAAAGCAGCUG | 8 | 23 | 2 |
| hsa-miR-197-3p | UUCACCACCUUCUCCACCCAGC | 122 | 22 | 2 |
| hsa-miR-423-5p | UGAGGGGCAGAGAGCGAGACUUU | 41 | 23 | 2 |
| hsa-miR-4468 | AGAGCAGAAGGAUGAGAU | 732 | 18 | 2 |
| hsa-miR-644b-5p | UGGGCUAAGGGAGAUGAUUGGGUA | 724 | 24 | 2 |
| hsa-miR-93-5p | CAAAGUGCUGUUCGUGCAGGUAG | 116 | 23 | 2 |
| hsa-let-7d-5p | AGAGGUAGUAGGUUGCAUAGUU | 92 | 22 | 1 |
| hsa-miR-1225-3p | UGAGCCCCUGUGCCGCCCCCAG | 369 | 22 | 1 |
| hsa-miR-1254 | AGCCUGGAAGCUGGAGCCUGCAGU | 270 | 24 | 1 |
| hsa-miR-1273g-3p | ACCACUGCACUCCAGCCUGAG | 210 | 21 | 1 |
| hsa-miR-1275 | GUGGGGGAGAGGCUGUC | 162 | 17 | 1 |
| hsa-miR-1296 | UUAGGGCCCUGGCUCCAUCUCC | 271 | 22 | 1 |
| hsa-miR-1307-5p | UCGACCGGACCUCGACCGGCU | 91 | 21 | 1 |
| hsa-miR-134 | UGUGACUGGUUGACCAGAGGGG | 94 | 22 | 1 |
| hsa-miR-15b-5p | UAGCAGCACAUCAUGGUUUACA | 78 | 22 | 1 |

(continued)

| MICROVESICLES CTX0E03 07EH | | | | |
|---|---|---|---|---|
| MIRNA | MIRNA.SEQUENCE | SEQ ID NO: | MIRNA LENGTH | READ COUNTS |
| hsa-miR-17-5p | CAAAGUGCUUACAGUGCAGGUAG | 145 | 23 | 1 |
| hsa-miR-1972 | UCAGGCCAGGCACAGUGGCUCA | 733 | 22 | 1 |
| hsa-miR-22-3p | AAGCUGCCAGUUGAAGAACUGU | 33 | 22 | 1 |
| hsa-miR-25-3p | CAUUGCACUUGUCUCGGUCUGA | 63 | 22 | 1 |
| hsa-miR-27b-3p | UUCACAGUGGCUAAGUUCUGC | 6 | 21 | 1 |
| hsa-miR-3065-5p | UCAACAAAAUCACUGAUGCUGGA | 226 | 23 | 1 |
| hsa-miR-30d-5p | UGUAAACAUCCCCGACUGGAAG | 31 | 22 | 1 |
| hsa-miR-320a | AAAAGCUGGGUUGAGAGGGCGA | 97 | 22 | 1 |
| hsa-miR-342-3p | UCUCACACAGAAAUCGCACCCGU | 81 | 23 | 1 |
| hsa-miR-3648 | AGCCGCGGGGAUCGCCGAGGG | 259 | 21 | 1 |
| hsa-miR-3652 | CGGCUGGAGGUGUGAGGA | 722 | 18 | 1 |
| hsa-miR-376c | AACAUAGAGGAAAUUCCACGU | 185 | 21 | 1 |
| hsa-miR-378a-3p | ACUGGACUUGGAGUCAGAAGG | 65 | 21 | 1 |
| hsa-miR-409-3p | GAAUGUUGCUCGGUGAACCCCU | 47 | 22 | 1 |
| hsa-miR-433 | AUCAUGAUGGGCUCCUCGGUGU | 174 | 22 | 1 |
| hsa-miR-4417 | GGUGGGCUUCCCGGAGGG | 175 | 18 | 1 |
| hsa-miR-4448 | GGCUCCUUGGUCUAGGGGUA | 231 | 20 | 1 |
| hsa-miR-4454 | GGAUCCGAGUCACGGCACCA | 299 | 20 | 1 |
| hsa-miR-454-3p | UAGUGCAAUAUUGCUUAUAGGGU | 169 | 23 | 1 |
| hsa-miR-4800-3p | CAUCCGUCCGUCUGUCCAC | 549 | 19 | 1 |
| hsa-miR-493-3p | UGAAGGUCUACUGUGUGCCAGG | 83 | 22 | 1 |
| hsa-miR-5095 | UUACAGGCGUGAACCACCGCG | 723 | 21 | 1 |
| hsa-miR-574-3p | CACGCUCAUGCACACACCCACA | 253 | 22 | 1 |
| hsa-miR-665 | ACCAGGAGGCUGAGGCCCCU | 309 | 20 | 1 |
| hsa-miR-720 | UCUCGCUGGGGCCUCCA | 84 | 17 | 1 |
| hsa-miR-99a-5p | AACCCGUAGAUCCGAUCUUGUG | 52 | 22 | 1 |
| hsa-miR-99b-5p | CACCCGUAGAACCGACCUUGCG | 4 | 22 | 1 |

**Table 9: Exosomes EH**

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 111092 |
| hsa-miR-4492 | GGGGCUGGGCGCGCGCC | 34 | 17 | 5188 |
| hsa-miR-4532 | CCCCGGGGAGCCCGGCG | 23 | 17 | 3368 |

(continued)

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-4488 | AGGGGGCGGGCUCCGGCG | 61 | 18 | 1389 |
| hsa-miR-4485 | UAACGGCCGCGGUACCCUAA | 67 | 20 | 386 |
| hsa-miR-4508 | GCGGGGCUGGGCGCGCG | 135 | 17 | 188 |
| hsa-miR-4516 | GGGAGAAGGGUCGGGGC | 110 | 17 | 135 |
| hsa-miR-4497 | CUCCGGGACGGCUGGGC | 232 | 17 | 73 |
| hsa-miR-1973 | ACCGUGCAAAGGUAGCAUA | 171 | 19 | 50 |
| hsa-miR-3195 | CGCGCCGGGCCCGGGUU | 716 | 17 | 48 |
| hsa-miR-4466 | GGGUGCGGGCCGGCGGGG | 264 | 18 | 43 |
| hsa-let-7a-5p | UGAGGUAGUAGGUUGUAUAGUU | 1 | 22 | 20 |
| hsa-miR-99b-5p | CACCCGUAGAACCGACCUUGCG | 4 | 22 | 19 |
| hsa-miR-21-5p | UAGCUUAUCAGACUGAUGUUGA | 9 | 22 | 18 |
| hsa-miR-92a-3p | UAUUGCACUUGUCCCGGCCUGU | 7 | 22 | 18 |
| hsa-miR-3676-5p | AGGAGAUCCUGGGUU | 280 | 15 | 17 |
| hsa-miR-4792 | CGGUGAGCGCUCGCUGGC | 363 | 18 | 15 |
| hsa-miR-664-5p | ACUGGCUAGGGAAAAUGAUUGGAU | 443 | 24 | 13 |
| hsa-miR-100-5p | AACCCGUAGAUCCGAACUUGUG | 3 | 22 | 11 |
| hsa-miR-1291 | UGGCCCUGACUGAAGACCAGCAGU | 294 | 24 | 10 |
| hsa-miR-16-5p | UAGCAGCACGUAAAUAUUGGCG | 29 | 22 | 10 |
| hsa-miR-4284 | GGGCUCACAUCACCCCAU | 191 | 18 | 10 |
| hsa-miR-663b | GGUGGCCCGGCCGUGCCUGAGG | 180 | 22 | 9 |
| hsa-miR-25-3p | CAUUGCACUUGUCUCGGUCUGA | 63 | 22 | 8 |
| hsa-miR-3656 | GGCGGGUGCGGGGGUGG | 251 | 17 | 8 |
| hsa-miR-181a-5p | AACAUUCAACGCUGUCGGUGAGU | 15 | 23 | 7 |
| hsa-miR-26a-5p | UUCAAGUAAUCCAGGAUAGGCU | 12 | 22 | 6 |
| hsa-miR-3654 | GACUGGACAAGCUGAGGAA | 325 | 19 | 6 |
| hsa-miR-644b-5p | UGGGCUAAGGGAGAUGAUUGGGUA | 724 | 24 | 6 |
| hsa-let-7b-5p | UGAGGUAGUAGGUUGUGUGGUU | 28 | 22 | 5 |
| hsa-let-7f-5p | UGAGGUAGUAGAUUGUAUAGUU | 11 | 22 | 5 |
| hsa-miR-1290 | UGGAUUUUUGGAUCAGGGA | 375 | 19 | 5 |
| hsa-miR-4426 | GAAGAUGGACGUACUUU | 359 | 17 | 5 |
| hsa-miR-5096 | GUUUCACCAUGUUGGUCAGGC | 220 | 21 | 5 |
| hsa-miR-125b-5p | UCCCUGAGACCCUAACUUGUGA | 42 | 22 | 4 |
| hsa-miR-1273f | GGAGAUGGAGGUUGCAGUG | 292 | 19 | 4 |
| hsa-miR-191-5p | CAACGGAAUCCCAAAAGCAGCUG | 8 | 23 | 4 |
| hsa-miR-22-3p | AAGCUGCCAGUUGAAGAACUGU | 33 | 22 | 4 |

(continued)

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-3609 | CAAAGUGAUGAGUAAUACUGGCUG | 216 | 24 | 4 |
| hsa-miR-3687 | CCCGGACAGGCGUUCGUGCGACGU | 190 | 24 | 4 |
| hsa-miR-93-5p | CAAAGUGCUGUUCGUGCAGGUAG | 116 | 23 | 4 |
| hsa-miR-1248 | ACCUUCUUGUAUAAGCACUGUGCUAAA | 269 | 27 | 3 |
| hsa-miR-1273g-3p | ACCACUGCACUCCAGCCUGAG | 210 | 21 | 3 |
| hsa-miR-151a-3p | CUAGACUGAAGCUCCUUGAGG | 25 | 21 | 3 |
| hsa-miR-182-5p | UUUGGCAAUGGUAGAACUCACACU | 16 | 24 | 3 |
| hsa-miR-221-3p | AGCUACAUUGUCUGCUGGGUUUC | 79 | 23 | 3 |
| hsa-miR-222-3p | AGCUACAUCUGGCUACUGGGU | 36 | 21 | 3 |
| hsa-miR-29a-3p | UAGCACCAUCUGAAAUCGGUUA | 106 | 22 | 3 |
| hsa-miR-4461 | GAUUGAGACUAGUAGGGCUAGGC | 129 | 23 | 3 |
| hsa-miR-486-5p | UCCUGUACUGAGCUGCCCCGAG | 5 | 22 | 3 |
| hsa-miR-92b-3p | UAUUGCACUCGUCCCGGCCUCC | 13 | 22 | 3 |
| hsa-miR-9-5p | UCUUUGGUUAUCUAGCUGUAUGA | 58 | 23 | 3 |
| hsa-miR-98 | UGAGGUAGUAAGUUGUAUUGUU | 10 | 22 | 3 |
| hsa-let-7d-5p | AGAGGUAGUAGGUUGCAUAGUU | 53 | 22 | 2 |
| hsa-miR-134 | UGUGACUGGUUGACCAGAGGGG | 94 | 22 | 2 |
| hsa-miR-151a-5p | UCGAGGAGCUCACAGUCUAGU | 37 | 21 | 2 |
| hsa-miR-15b-5p | UAGCAGCACAUCAUGGUUUACA | 78 | 22 | 2 |
| hsa-miR-30a-5p | UGUAAACAUCCUCGACUGGAAG | 30 | 22 | 2 |
| hsa-miR-3124-3p | ACUUUCCUCACUCCCGUGAAGU | 734 | 22 | 2 |
| hsa-miR-3653 | CUAAGAAGUUGACUGAAG | 544 | 18 | 2 |
| hsa-let-7c | UGAGGUAGUAGGUUGUAUGGUU | 17 | 22 | 1 |
| hsa-let-7d-3p | CUAUACGACCUGCUGCCUUUCU | 92 | 22 | 1 |
| hsa-let-7g-5p | UGAGGUAGUAGUUUGUACAGUU | 43 | 22 | 1 |
| hsa-let-7i-5p | UGAGGUAGUAGUUUGUGCUGUU | 22 | 22 | 1 |
| hsa-miR-103a-3p | AGCAGCAUUGUACAGGGCUAUGA | 62 | 23 | 1 |
| hsa-miR-106b-5p | UAAAGUGCUGACAGUGCAGAU | 170 | 21 | 1 |
| hsa-miR-1244 | AAGUAGUUGGUUUGUAUGAGAUGGUU | 340 | 26 | 1 |
| hsa-miR-128 | UCACAGUGAACCGGUCUCUUU | 109 | 21 | 1 |
| hsa-miR-1285-3p | UCUGGGCAACAAAGUGAGACCU | 464 | 22 | 1 |
| hsa-miR-1307-3p | ACUCGGCGUGGCGUCGGUCGUG | 124 | 22 | 1 |
| hsa-miR-140-3p | UACCACAGGGUAGAACCACGG | 138 | 21 | 1 |
| hsa-miR-148b-3p | UCAGUGCAUCACAGAACUUUGU | 48 | 22 | 1 |
| hsa-miR-181b-5p | AACAUUCAUUGCUGUCGGUGGGU | 38 | 23 | 1 |

(continued)

| **EXOSOMES CTX0E03 07EH** | | | | |
|---|---|---|---|---|
| MIRNA | MIRNA.SEQUENCE | SEQ ID NO: | MIRNA LENGTH | READ COUNTS |
| hsa-miR-193a-3p | AACUGGCCUACAAAGUCCCAGU | 386 | 22 | 1 |
| hsa-miR-1972 | UCAGGCCAGGCACAGUGGCUCA | 733 | 22 | 1 |
| hsa-miR-21-3p | CAACACCAGUCGAUGGGCUGU | 20 | 21 | 1 |
| hsa-miR-2277-3p | UGACAGCGCCCUGCCUGGCUC | 735 | 21 | 1 |
| hsa-miR-23a-3p | AUCACAUUGCCAGGGAUUUCC | 55 | 21 | 1 |
| hsa-miR-23b-3p | AUCACAUUGCCAGGGAUUACC | 59 | 21 | 1 |
| hsa-miR-24-3p | UGGCUCAGUUCAGCAGGAACAG | 119 | 22 | 1 |
| hsa-miR-27a-3p | UUCACAGUGGCUAAGUUCCGC | 46 | 21 | 1 |
| hsa-miR-27b-3p | UUCACAGUGGCUAAGUUCUGC | 6 | 21 | 1 |
| hsa-miR-299-3p | UAUGUGGGAUGGUAAACCGCUU | 182 | 22 | 1 |
| hsa-miR-30b-5p | UGUAAACAUCCUACACUCAGCU | 96 | 22 | 1 |
| hsa-miR-30c-5p | UGUAAACAUCCUACACUCUCAGC | 66 | 23 | 1 |
| hsa-miR-31-3p | UGCUAUGCCAACAUAUUGCCAU | 172 | 22 | 1 |
| hsa-miR-3196 | CGGGGCGGCAGGGGCCUC | 717 | 18 | 1 |
| hsa-miR-3198 | GUGGAGUCCUGGGGAAUGGAGA | 647 | 22 | 1 |
| hsa-miR-320a | AAAAGCUGGGUUGAGAGGGCGA | 97 | 22 | 1 |
| hsa-miR-329 | AACACACCUGGUUAACCUCUUU | 214 | 22 | 1 |
| hsa-miR-339-5p | UCCCUGUCCUCCAGGAGCUCACG | 402 | 23 | 1 |
| hsa-miR-34a-5p | UGGCAGUGUCUUAGCUGGUUGU | 101 | 22 | 1 |
| hsa-miR-3607-5p | GCAUGUGAUGAAGCAAAUCAGU | 249 | 22 | 1 |
| hsa-miR-3648 | AGCCGCGGGGAUCGCCGAGGG | 259 | 21 | 1 |
| hsa-miR-376c | AACAUAGAGGAAAUUCCACGU | 185 | 21 | 1 |
| hsa-miR-3960 | GGCGGCGGCGGAGGCGGGGG | 416 | 20 | 1 |
| hsa-miR-411-3p | UAUGUAACACGGUCCACUAACC | 482 | 22 | 1 |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-4417 | GGUGGGCUUCCCGGAGGG | 175 | 18 | 1 |
| hsa-miR-4444 | CUCGAGUUGGAAGAGGCG | 418 | 18 | 1 |
| hsa-miR-4499 | AAGACUGAGAGGAGGGA | 736 | 17 | 1 |
| hsa-miR-4521 | GCUAAGGAAGUCCUGUGCUCAG | 233 | 22 | 1 |
| hsa-miR-4680-5p | AGAACUCUUGCAGUCUUAGAUGU | 737 | 23 | 1 |
| hsa-miR-4709-5p | ACAACAGUGACUUGCUCUCCAA | 575 | 22 | 1 |
| hsa-miR-501-3p | AAUGCACCCGGGCAAGGAUUCU | 26 | 22 | 1 |
| hsa-miR-644b-3p | UUCAUUUGCCUCCCAGCCUACA | 442 | 22 | 1 |
| hsa-miR-654-3p | UAUGUCUGCUGACCAUCACCUU | 336 | 22 | 1 |

(continued)

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-9-3p | AUAAAGCUAGAUAACCGAAAGU | 183 | 22 | 1 |
| hsa-miR-940 | AAGGCAGGGCCCCCGCUCCCC | 366 | 21 | 1 |
| hsa-miR-99a-5p | AACCCGUAGAUCCGAUCUUGUG | 52 | 22 | 1 |

***D) Identification of top ranking coding and non-coding RNAs by GENCODE analysis performed in exosomes, MV and producer cells***

**Table 10:** Total number of sequence reads identified by using GENCODE in each tested samples

| CTX0E030EH cells | CTX0E0307E EXO | CTX0E0307EH H MV | CTX0E0307EI cells | CTX0E0307EIEI XO | CTX0E0307EI MV |
|---|---|---|---|---|---|
| 18741941 | 12678688 | 10876797 | 22116110 | 16311289 | 835970 |

Using GENCODE database analysis of the sequence results, seven putative novel miRNA sequences were identified in exosomes (EXO), microvesicles (MV) and producer cells, as shown in Table 11. (nb CTX0E03 07EI MV reads are misrepresented due to the lower amount of starting material - see Table 10). These data are shown graphically in Figure 16, which shows that these sequences are preferentially shuttled into exosomes and microvesicles compared to the cells.

| Gene Symbol | Transcript ID | Length | Type of RNA | CTX0E0307E H cells | CTX0E0307E H EXO | CTX0E0307E H MV | CTX0E0307E I cells | CTX0E0307E IEXO | CTX0E0307EI MV |
|---|---|---|---|---|---|---|---|---|---|
| AC079949.1 | AC079949.1-201 | 57 | Novel miRNA | 2629 | 27006 | 14873 | 2425 | 11433 | 848 |
| AP000318.1 | AP000318.1-201 | 64 | Novel miRNA | 1353 | 9379 | 11002 | 7469 | 2963 | 419 |
| AL161626.1 | AL161626.1-201 | 57 | Novel miRNA | 471 | 4450 | 3712 | 291 | 1263 | 129 |
| AC004943.1 | AC004943.1-201 | 81 | Novel miRNA | 24 | 81 | 43 | 23 | 94 | 5 |
| AL121897.1 | AL121897.1-201 | 89 | Novel miRNA | 6 | 22 | 14 | 2 | 30 | 3 |

**Table 11:** Identification of putative novel miRNA sequences using GENCODE in exosomes (EXO), microvesicles (MV) and producer cells. CTX0E03 07EI MV reads are misrepresented due to the lower amount of starting material (table 1). The transcript IDs are taken from the Ensembl database (www.ensembl.org).

***Validation and of novel miRNAs***

**AC079949.1-201 (SEQ ID NO:738)**

Gene: AC079949.1 ENSG00000239776
>12 dna:chromosome chromosome:GRCh37:12:127650616:127650672:1
GGCCGCGCCCCGTTTCCCAGGACAAAGGGCACTCCGCACCGGACCCTGGTCCCAGCG

For AC079949.1-201 putative mature miRNA, **gaccaggguccggugcggagug (SEQ ID NO:745)** was identified as the possible 5' stem mature miRNA using http://mirna.imbb.forth.gr/MatureBayes.html, a tool for finding mature miRNA within a miRNA precursor sequence using a Naive Bays classifier. Its presence validation was performed using AGGGTCCGGTGCGGAGT (SEQ ID NO:746) primer sequence. This sequence was entered in mirbase (http://www.mirbase.org/) and the following miRNA was found with similar sequence: *Bos taurus* miR-2887-1 (Accession No. MIMAT0013845).
bta-miR-2887 : 9-20 (SEQ ID NO:747)

```
AC079949 (5)     2 ggguccggugcg 13
bta-miR-2887       ||||||||||||
                 9 ggguccggugcg 20
```

The presence of this novel miRNA was tested by qRT-PCR on purified exosomes retro transcribed miRNA.

The same analysis was performed using the 3' stem of AC079949, sequence TGCGGAGTGCCCTTTGTCCT (SEQ ID NO:748), but in this case no similar miRNA was identified in mirbase.

**AP000318.1-201 (SEQ ID NO:739)**

Gene: AP000318.1 ENSG00000266007
>21 dna:chromosome chromosome:GRCh37:21:35677430:35677493:1
CCCACTCCCTGGCGCCGCTTGTGGAGGGCCCAAGTCCTTCTGATTGAGGCCCAACCCGTGGAAG

For AP000318.1-201 putative mature miRNA, **ggagggcccaaguccuucugau** (SEQ ID NO:744) was identified as the possible 5' stem mature miRNA. Its presence validation was performed using GGAGGGCCCAAGTCCTTCTGAT (SEQ ID NO:749) primer sequence. *Caenorhabditis remanei* miR-55 stem-loop was identified as similar miRNA. Primer validation was again carried out by qRT-PCR.
crm-miR-55-5p : 4-17 (SEQ ID NO:750)

```
AP000318.1      20 cccaaguccuucug 7
crm-miR-55-5p      ||||||| ||||||
                 4 cccaagugcuucug 17
```

**AL161626.1-201 (SEQ ID NO:740)**

Gene: AL161626.1 ENSG00000241781
>9 dna:chromosome chromosome:GRCh37:9:79186731:79186787:1
CGCCGGGACCGGGGTCCGGGGCGGAGTGCCCTTCCTCCTGGGAAACGGGGTGCGGC

For AL161626.1-201 putative mature miRNA, **ggcggagugcccuucuuccugg (SEQ ID NO:743)** was identified as the possible 5' stem mature miRNA. Its presence validation was performed using CGGAGTGCCCTTCTTCCT (SEQ ID NO:751) primer sequence. *Zea mays* miR164c stem-loop and *Achypodium distachyon* miR164f stem-loop were identified as similar miRNA. Primer validation was again carried out by qRT-PCR.
zma-miR164c-3p : 4-15 (SEQ ID NO:752)

```
AL161626.1       5 gugcccuucuuc 16
zma-miR164c-3p     ||||||||||||
                 4 gugcccuucuuc 15
```

**AC004943.1 (SEQ ID NO:741)**

Gene: AC004943.1 ENSG00000265573
>16 dna:chromosome chromosome:GRCh37:16:72821592:72821672:-1

GCTTCACGTCCCCACCGGCGGCGGCGGCGGTGGCAGTGGCGGCGGCGGCGGCGGTGGCGG
CGGCGGCGGCGGCGGCGGCTC

**AL121897.1 (SEQ ID NO:742)**

Gene: AL121897.1 ENSG00000264308
>20 dna:chromosome chromosome:GRCh37:20:30865503:30865591:1

GCCGCCCCCGCCGCCGCCGCCGCCGCCGCCGCCGCCGCCGCCGCCCGCTTTCGGCTCGGG
CCTCAGGTGAGTCGGAGGGGCCGGGCGCC

### *Miscellaneous RNA (misc_RNA), including novel putative*

Misc_RNA is short for miscellaneous RNA, a general term for a series of miscellaneous small RNA. Miscellaneous transcript feature are not defined by other RNA keys.

List of top ranking previously known and novel misc_RNAs identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| RPPH1 | RPPH1-201 | 333 | misc RNA | 76 | 2229 | 1785 | 0 | 1077 | 197 |
| RMRP | RMRP-201 | 264 | misc RNA | 139 | 1803 | 1443 | 191 | 659 | 87 |
| RPPH1 | RPPH1-001 | 638 | misc RNA | 182 | 931 | 1372 | 795 | 2017 | 157 |
| VTRNA1-1 | VTRNA1-1-201 | 99 | misc RNA | 43 | 720 | 52 | 247 | 210 | 9 |
| Y_RNA | Y_RNA.321-201 | 93 | Novel misc RN | 159 | 196 | 661 | 960 | 903 | 217 |
| Y_RNA | Y_RNA.725-201 | 95 | Novel misc RN | 1092 | 18 | 74 | 1005 | 39 | 11 |
| Y_RNA | Y_RNA.125-201 | 96 | Novel misc RN | 1079 | 15 | 58 | 906 | 27 | 12 |
| Y_RNA | Y_RNA.118-201 | 99 | Novel misc RN | 134 | 12 | 9 | 156 | 45 | 7 |
| Y_RNA | Y_RNA.394-201 | 109 | Novel misc RN | 9 | 9 | 7 | 33 | 13 | 1 |
| Y_RNA | Y_RNA.687-201 | 111 | Novel misc RN | 36 | 6 | 15 | 103 | 41 | 10 |
| Y_RNA | Y_RNA.144-201 | 102 | Novel misc RN | 129 | 5 | 21 | 187 | 84 | 5 |
| Y_RNA | Y_RNA.337-201 | 105 | Novel misc RN | 7 | 4 | 0 | 15 | 4 | 0 |
| Y_RNA | Y_RNA.413-201 | 97 | Novel misc RN | 136 | 4 | 8 | 125 | 46 | 3 |
| Y_RNA | Y_RNA.30-201 | 103 | Novel misc RN | 74 | 3 | 3 | 62 | 21 | 2 |

**Table 12:** Identification of misc_RNA, including putative novel misc_RNA, sequences using GENCODE in exosomes (EXO), microvesicles (MV) and producer cells. (CTX0E03 07EI MV reads are misrepresented due to the lower amount of starting material - Table 10). The transcript IDs are taken from the Ensembl database (www.ensembl.org).

Among the misc_RNA the following sequences were found preferentially down or up shuttled in exosomes and MV: RPHI, RMRP, and VTRNA1-1 up shuttled and Y_RNA.725-201, and Y_RNA. 125-201 down respectively. RPHI is a ribonuclease P RNA component H1. RMRP gene encodes the RNA component of mitochondrial RNA processing endoribonuclease, which cleaves mitochondrial RNA at a priming site of mitochondrial DNA replication. This RNA also interacts with the telomerase reverse transcriptase catalytic subunit to form a distinct ribonucleoprotein complex that has RNA-dependent RNA polymerase activity and produces double-stranded RNAs that can be processed into small interfering RNA. VTRNA1-1 is vault RNA component 1. Vaults are large cytoplasmic ribonucleoproteins and they are composed of a major vault protein, MVP, 2 minor vault proteins, TEP1 and PARP4, and a non-translated RNA component, VTRNA1-1. Y_RNA.725-201, and Y_RNA.125-201 are novel misc_RNAs and their function is not defined.

### *Metazoa miscellaneous RNA*

The signal recognition particle RNA, also known as 7SL, 6S, ffs, or 4.5S RNA, is the RNA component of the signal recognition particle (SRP) ribonucleoprotein complex. SRP is a universally conserved ribonucleoprotein that directs the traffic of proteins within the cell and allows them to be secreted. The SRP RNA, together with one or more SRP proteins contributes to the binding and release of the signal peptide. The RNA and protein components of this complex are highly conserved but do vary between the different kingdoms of life.

List of top ranking Metazoa misc_RNAs identified using GENCODE sequence data set:

| Gene Symbol | Transcript ID | Length | Type of RNA | CTXOE0307EH cells | CTXOE0307EH EXO | CTXOE0307EH MV | CTXOE0307EI cells | CTXOE0307EI EXO | CTXOE0307EI MV |
|---|---|---|---|---|---|---|---|---|---|
| Metazoa_SRP | Metazoa_SRP.791-201 | 288 | Metazoan signal recogn | 679 | 2324 | 2058 | 771 | 2698 | 465 |
| Metazoa_SRP | Metazoa_SRP.561-201 | 294 | Metazoan signal recogn | 634 | 2006 | 1683 | 744 | 2147 | 432 |
| Metazoa_SRP | Metazoa_SRP.864-201 | 297 | Metazoan signal recogn | 252 | 1884 | 1544 | 78 | 170 | 148 |
| Metazoa_SRP | Metazoa_SRP.824-201 | 297 | Metazoan signal recogn | 438 | 881 | 958 | 505 | 1860 | 342 |
| Metazoa_SRP | Metazoa_SRP.72-201 | 278 | Metazoan signal recogn | 441 | 630 | 631 | 494 | 2184 | 349 |
| Metazoa_SRP | Metazoa_SRP.151-201 | 307 | Metazoan signal recogn | 377 | 464 | 470 | 432 | 1431 | 265 |
| Metazoa_SRP | Metazoa_SRP.208-201 | 277 | Metazoan signal recogn | 382 | 410 | 431 | 422 | 1104 | 242 |
| Metazoa_SRP | Metazoa_SRP.501-201 | 280 | Metazoan signal recogn | 265 | 272 | 266 | 236 | 434 | 44 |
| Metazoa_SRP | Metazoa_SRP.682-201 | 298 | Metazoan signal recogn | 12 | 52 | 21 | 10 | 13 | 2 |

**Table 13:** Identification signal recognition particle RNA (misc_RNA) sequences using GENCODE in exosomes (EXO), microvesicles (MV) and producer cells. The transcript IDs are taken from the Ensembl database (www.ensembl.org).

***RRNA (ribosomal RNA)***

Ribosomal RNA (rRNA) forms part of the protein-synthesizing organelle known as a ribosome and that is exported to the cytoplasm to help translate the information in messenger RNA (mRNA) into protein. Eukaryotic ribosome (80S) rRNA components are: large unit (rRNA 5S, 5.8S, and 28S) small unit (rRNA 18S). Both rRNA 28S and 5.8S are selectively up-shuttled in exosomes and MV.

List of top ranking rRNA identified using GENCODE sequence data set:

| Gene Symbol | Transcript ID | Length | Type of RNA | CTXOE0307EH cells | CTXOE0307EH EXO | CTXOE0307EH MV | CTXOE0307EI cells | CTXOE0307EI EXO | CTXOE0307EI MV |
|---|---|---|---|---|---|---|---|---|---|
| RNA5-8SP6 | RNA5-8SP6-201 | 152 | rRNA | 205008 | 1148190 | 706558 | 213187 | 135909 | 14732 |
| RNA28S5 | RNA28S5-001 | 432 | rRNA | 86111 | 458585 | 516754 | 62829 | 390237 | 47483 |
| RNA18S5 | RNA18S5-001 | 599 | rRNA | 74634 | 52055 | 61639 | 116874 | 138484 | 14616 |
| RNA5-8SP2 | RNA5-8SP2-201 | 152 | rRNA | 6488 | 1719 | 1540 | 9231 | 3112 | 149 |
| RNA5-8SP5 | RNA5-8SP5-201 | 152 | rRNA | 2794 | 7393 | 3924 | 7314 | 3579 | 232 |

**Table 14:** Identification rRNA sequences using GENCODE in exosomes (EXO), microvesicles (MV) and producer cells. The transcript IDs are taken from the Ensembl database (www.ensembl.org).

***Small nucleolar RNA: snoRNA***

Small nucleolar RNAs (snoRNAs) are a class of small RNA molecules that primarily guides chemical modifications of other RNAs, mainly ribosomal RNAs, transfer RNAs and small nuclear RNAs. There are two main classes of snoRNA, the C/D box snoRNAs which are associated with methylation, and the H/ACA box snoRNAs which are associated with pseudouridylation.

List of top ranking snoRNA identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| SNORD3A | SNORD3A-201 | 216 | snoRNA | 1433 | 2085 | 1621 | 906 | 1732 | 120 |
| SNORD3C | SNORD3C-201 | 216 | snoRNA | 1169 | 1702 | 1220 | 639 | 1176 | 86 |
| SNORD29 | SNORD29-201 | 65 | snoRNA | 28130 | 1633 | 1070 | 36677 | 1752 | 45 |
| SNORD83B | SNORD83B-201 | 93 | snoRNA | 1835 | 675 | 487 | 638 | 575 | 29 |
| SNORD30 | SNORD30-201 | 70 | snoRNA | 29743 | 254 | 244 | 29071 | 283 | 24 |

**Table 15:** Identification of snoRNA sequences using GENCODE in exosomes (EXO), microvesicles (MV) and producer cells. The transcript IDs are taken from the Ensembl database (www.ensembl.org).

***Small nuclear RNA (snRNA)***

Small nuclear ribonucleic acid (snRNA), also commonly referred to as U-RNA, is a class of small RNA molecules that make up the major spliceosome are named U1, U2, U4, U5, and U6, and participate in several RNA-RNA and RNA-protein interactions. Their primary function is in the processing of pre-mRNA (hnRNA) in the nucleus. They have also been shown to aide in the regulation of transcription factors (7SK RNA) or RNA polymerase II (B2 RNA), and maintaining the telomeres.

List of top ranking snRNA identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| U2 | U2.38-201 | 191 | snRNA | 1354 | 71596 | 49223 | 751 | 35290 | 1919 |
| U2 | U2.6-201 | 192 | snRNA | 834 | 15561 | 13594 | 303 | 8146 | 272 |
| U1 | U1.81-201 | 164 | snRNA | 584 | 10901 | 7307 | 91 | 3197 | 121 |
| U1 | U1.90-201 | 167 | snRNA | 533 | 9927 | 6689 | 48 | 2187 | 84 |
| U2 | U2.7-201 | 191 | snRNA | 201 | 9267 | 3109 | 288 | 6736 | 262 |

**Table 16A:** Identification of snRNA sequences using GENCODE in exosomes (EXO), microvesicles (MV) and producer cells. The transcript IDs are taken from the Ensembl database (www.ensembl.org).

***LincRNA and novel lincRNA***

Large intergenic non-coding RNAs (lincRNAs) are emerging as key regulators of diverse cellular processes. Determining the function of individual lincRNAs remains a challenge. Long non-coding RNAs (long ncRNAs, IncRNA) are non-protein coding transcripts longer than 200 nucleotides.

List of top ranking previously known and novel lincRNAs identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| RP11-108M9.3 | RP11-108M9.3-0( | 1761 | Novel lincRNA | 244 | 159 | 240 | 539 | 324 | 45 |
| RP11-329L6.1 | RP11-329L6.1-001 | 507 | Novel lincRNA | 19 | 70 | 41 | 29 | 84 | 2 |
| RP11-160E2.6 | RP11-160E2.6-00 | 637 | Novel lincRNA | 228 | 67 | 115 | 489 | 74 | 6 |
| AC004528.3 | AC004528.3-001 | 107 | Novel lincRNA | 16 | 58 | 46 | 14 | 55 | 4 |
| MALAT1 | MALAT1-201 | 4585 | lincRNA | 150 | 308 | 234 | 26 | 182 | 12 |
| GAS5 | GAS5-007 | 2743 | lincRNA | 12024 | 215 | 120 | 46501 | 875 | 13 |

**Table 16B:** Identification of lincRNA and putative novel lincRNA sequences using GENCODE in exosomes (EXO), microvesicles (MV) and producer cells. The transcript IDs are taken from the Ensembl database (www.ensembl.org).

GAS5 lincRNA is highly expressed in cell producer compared to in exosomes and microvesicles (down shuttled in both exosomes and MV).

***mRNA***

Coding sequencing mRNA were also identified.

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| EEF2 | EEF2-201 | 9407 | mRNA | 710 | 578 | 449 | 1155 | 471 | 33 |
| MTRNR2L8 | MTRNR2L8-201 | 1290 | mRNA | 1383 | 548 | 642 | 1323 | 258 | 15 |
| NES | NES-001 | 8635 | mRNA | 668 | 406 | 234 | 1448 | 267 | 20 |
| VIM | VIM-001 | 8316 | mRNA | 563 | 911 | 501 | 1500 | 618 | 36 |

**Table 17:** Identification of mRNA sequences using GENCODE in exosomes (EXO), microvesicles (MV) and producer cells. The transcript IDs are taken from the Ensembl database (www.ensembl.org).

***Example 12: Conclusion***

The main scope of the deep sequence analysis was to identify their miRNA components in neural stem cell-derived vesicles (exosomes and microvesicles). This analysis identified a new set of known and novel miRNAs that are preferentially shuttled into both exosomes and MV. Among the identified miRNAs already included in mirbase database were hsa-miR-1246, hsa-miR-4488, hsa-miR-4492, hsa-miR-4508, hsa-miR-4516, hsa-miR-4532, and among the novel miRNAs were AC079949.1, AP000318.1, AL161626.1, AC004943.1, AL121897.1. Top ranking shuttled miRNAs, including novel ones were validated by qRT-PCR in exosomes.

The size distribution of shuttle RNA, as shown here, is mostly in the range of 20 to 200 nt and other RNA species are released by cells into the extracellular space. By deep sequencing and GENCODE sequence set analysis we found a greater complexity and diversity of non-coding RNA transcripts. We extended this analysis with detailed evaluation and this led to the discovery of preferentially up (defined as log2 fold change $\geq 2$) and down (defined as log2 fold change $\leq - 2$) shuttle of other non-coding RNAs in both exosomes and microvesicles. Differentially shuttled non coding RNA were found in almost all the non-coding RNA subtypes, ribosomal RNA (rRNA), small nucleolar (snoRNA), small nuclear RNA (snRNA), microRNA (miRNA), miscellaneous other RNA (misc_RNA, e.g. RMRP, vault RNA, metazoa SRP, and RNY), and large intergenic non-coding RNAs (lincRNAs).

The unequal distribution of the detected RNA species over cellular and shuttle RNA, combined with increasing evidence for their role in gene regulation strongly suggest that cells specifically release these RNAs to modify the function of target cells.

**Example 13: Proteomic analysis**

Methods

Exosomes and microvesicle fractions were prepared from a CTX0E03 cell Integra culture (week 2), using differential ultracentrifugation. Exosomes and microvesicles were disrupted in modified RIPA buffer (50mM Tris HCl, pH 8.0, 150mM NaCl, 1% SDS, 0.1% Triton X100, 10mM DTT, 1x Complete protease inhibitor (Roche) and 1x PhosStop phosphatase inhibitor (Roche)) and subjected to manual shearing using a 1mL tuberculin syringe and 25 gauge needle. Samples were re-quantitated post disruption using the Qubit fluorometer (Invitrogen). 20$\mu$g of each sample was loaded onto a 4-12% SDS-PAGE gel (Novex, Invitrogen). The gel was excised into forty segments per lane and gel slices were processed using a robot (ProGest, DigiLab) with the following protocol:

a) wash with 25mM ammonium bicarbonate followed by acetonitrile;
b) reduce with 10mM dithiothreitol at 60°C followed by alkylation with 50mM iodoacetamide at room temperature;
c) digest with trypsin (Promega) at 37°C for 4h;
d) quench with formic acid;
e) the supernatant was analysed by mass spectrometry directly without further processing.

*Mass Spectrometry*

Each gel digest was analysed by nano LC/MS/MS with a Waters NanoAcquity HPLC system interfaced to a ThermoFisher Q Exactive. Peptides were loaded on a trapping column and eluted over a 75μm analytical column at 350nL/min; both columns were packed with Jupiter Proteo resin (Phenomenex). The mass spectrometer was operated in data-dependent mode, with MS and MS/MS performed in the Orbitrap at 70,000 FWHM and 17,500 FWHM resolution, respectively.

Exosomes

2572 proteins were identified by Mass spectrometry in exosomes purified by ultracentrifugation. The exosomes were isolated from the initial stages of an Integra culture (week 2). The gene names and corresponding SWISSPROT accession numbers (in brackets) of all 2572 proteins are listed in Table 18 (in alphabetical order of gene name) and the 100 most abundant proteins are listed in Table 19, in order of decreasing abundance. The characteristic exosome markers CD9, CD81 and Alix (also known as PDCD6IP) are present in the most abundant 100 proteins.

**Table 18:** Gene names and SWISSPROT accession numbers of all 2572 proteins identified in CTX0E03 exosomes (listed in alphabetical order of gene name).

| |
|---|
| A1BG (P04217), A2M (P01023), AACS (Q86V21), AAMP (Q13685), AARS (P49588), AARSD1 (Q9BTE6), AASDHPPT (Q9NRN7), ABCA3 (Q99758), ABCE1 (P61221), ABCF1 (Q8NE71), ABCF3 (Q9NUQ8), ABHD10 (Q9NUJ1), ABHD14B (Q96IU4), ABI1 (Q8IZP0), ABR (Q12979), ACAA2 (P42765), ACACA (Q13085), ACADVL (P49748), ACAP2 (Q15057), ACAT1 (P24752), ACAT2 (Q9BWD1), ACBD7 (Q8N6N7), ACLY (P53396), ACO1 (P21399), ACO2 (Q99798), ACOT1 (Q86TX2), ACOT13 (Q9NPJ3), ACOT7 (000154), ACP1 (P24666), ACSL1 (P33121), ACSL3 (O95573), ACSL4 (O60488), ACSS2 (Q9NR19), ACTC1 (P68032), ACTG1 (P63261), ACTL6A (096019), ACTN1 (P12814), ACTN4 (O43707), ACTR10 (Q9NZ32), ACTR1A (P61163), ACTR1B (P42025), ACTR2 (P61160), ACTR3 (P61158), ADAM10 (014672), ADAM12 (043184), ADAMTS15 (Q8TE58), ADAMTS16 (Q8TE57), ADAR (P55265), ADAT2 (Q7Z6V5), ADH5 (P11766), ADI1 (Q9BV57), ADK (P55263), ADRBK1 (P25098), ADRM1 (Q16186), ADSL (P30566), ADSS (P30520), AEBP1 (Q8IUX7), AFM (P43652), AGL (P35573), AGRN (O00468), AGT (P01019), AHCY (P23526), AHCYL1 (O43865), AHNAK (Q09666), AHSA1 (O95433), AHSG (P02765), AIDA (Q96BJ3), AIFM1 (095831), AIMP1 (Q12904), |

(continued)

| |
|---|
| AIMP2 (Q13155), AIP (000170), AK1 (P00568), AK3 (Q9UIJ7), AK4 (P27144), AKAP12 (Q02952), AKAP9 (Q99996), AKR1A1 (P14550), AKR1B1 (P15121), AKR1C1 (Q04828), AKR7A2 (O43488), AKR7A3 (095154), AKT1 (P31749), ALCAM (Q13740), ALDH16A1 (Q8IZ83), ALDH3A1 (P30838), ALDH7A1 (P49419), ALDH9A1 (P49189), ALDOA (P04075), ALDOC (P09972), ALKBH2 (Q6NS38), ALKBH4 (Q9NXW9), AMBP (P02760), AMDHD2 (Q9Y303), AMPD2 (Q01433), AMZ2 (Q86W34), ANAPC1 (Q9H1A4), ANAPC4 (Q9UJX5), ANAPC5 (Q9UJX4), ANAPC7 (Q9UJX3), ANKFY1 (Q9P2R3), ANKRD28 (015084), ANP32A (P39687), ANP32B (Q92688), ANP32E (Q9BTT0), ANXA1 (P04083), ANXA2 (P07355), ANXA4 (P09525), ANXA5 (P08758), ANXA6 (P08133), ANXA7 (P20073), AP1B1 (Q10567), AP1G1 (O43747), AP1M1 (Q9BXS5), AP1S1 (P61966), AP1S2 (P56377), AP2A1 (O95782), AP2A2 (O94973), AP2B1 (P63010), AP2M1 (Q96CW1), AP2S1 (P53680), AP3B1 (O00203), AP3D1 (014617), AP3M1 (Q9Y2T2), AP3S1 (Q92572), AP3S2 (P59780), AP4S1 (Q9Y587), APEH (P13798), APEX1 (P27695), API5 (Q9BZZ5), APIP (Q96GX9), APOA1 (P02647), APOA1BP (Q8NCW5), APOA2 (P02652), APOBEC3C (Q9NRW3), APOC2 (P02655), APOD (P05090), APOH (P02749), APOM (O95445), APPL1 (Q9UKG1), APRT (P07741), AQR (O60306), ARCN1 (P48444), ARF1 (P84077), ARF4 (P18085), ARF5 (P84085), ARF6 (P62330), ARFIP1 (P53367), ARFIP2 (P53365), ARHGAP1 (Q07960), ARHGAP12 (Q8IWW6), ARHGDIA (P52565), ARHGEF1 (Q92888), ARHGEF10 (015013), ARHGEF7 (Q14155), ARIH1 (Q9Y4X5), ARIH2 (O95376), ARL1 (P40616), ARL2 (P36404), ARL3 (P36405), ARL6IP1 (Q15041), ARL8B (Q9NVJ2), ARMC10 (Q8N2F6), ARMC6 (Q6NXE6), ARMC8 (Q8IUR7), ARMC9 (Q7Z3E5), ARMCX3 (Q9UH62), ARPC1A (Q92747), ARPC1B (015143), ARPC2 (015144), ARPC3 (015145), ARPC4 (P59998), ARPC5 (015511), ARPC5L (Q9BPX5), ARRDC1 (Q8N5I2), ASB6 (Q9NWX5), ASCC1 (Q8N9N2), ASCC2 (Q9H1I8), ASCC3 (Q8N3C0), ASF1A (Q9Y294), ASH2L (Q9UBL3), ASMTL (095671), ASNA1 (043681), ASNS (P08243), ASS1 (P00966), ATG16L1 (Q676U5), ATG3 (Q9NT62), ATG4B (Q9Y4P1), ATG7 (O95352), ATIC (P31939), ATL3 (Q6DD88), ATM (Q13315), ATOX1 (O00244), ATP1A1 (P05023), ATP1B1 (P05026), ATP1B3 (P54709), ATP2B1 (P20020), ATP2B4 (P23634), ATP5B (P06576), ATP5E (P56381), ATP5I (P56385), ATP6AP2 (O75787), ATP6V0D1 (P61421), ATP6V1A (P38606), ATP6V1B2 (P21281), ATP6V1C1 (P21283), ATP6V1D (Q9Y5K8), ATP6V1E1 (P36543), ATP6V1G1 (O75348), ATP6V1H (Q9UI12), ATR (Q13535), ATRN (O75882), ATXN10 (Q9UBB4), B2M (P61769), B3GAT3 (O94766), B3GNT1 (O43505), B4GALT7 (Q9UBV7), BAG2 (O95816), BAIAP2 (Q9UQB8), BANF1 (075531), BAT1 (Q13838), BAT3 (P46379), BBOX1 (O75936), BCAS2 (O75934), BCAT1 (P54687), BCCIP (Q9P287), BCL2L13 (Q9BXK5), BCLAF1 (Q9NYF8), BDH2 (Q9BUT1), BICD2 (Q8TD16), BLOC1S1 (P78537), BLVRA (P53004), BLVRB (P30043), BMP1 (P13497), BOLA2 (Q9H3K6), BPGM (P07738), BPHL (Q86WA6), BPNT1 (095861), BRCC3 (P46736), BRE (Q9NXR7), BROX (Q5VW32), BRP16L (P0CB43), BSG (P35613), BST1 (Q10588), BTAF1 (014981), BUB3 (O43684), BUD31 (P41223), BYSL (Q13895), BZW1 (Q7L1Q6), BZW2 (Q9Y6E2), C10orf119 (Q9BTE3), C10orf58 |

(continued)

| |
|---|
| (Q9BRX8), C10orf76 (Q5T2E6), C11orf54 (Q9H0W9), C11orf68 (Q9H3H3), C12orf10 (Q9HB07), C14orf149 (Q96EM0), C14orf166 (Q9Y224), C15orf58 (Q6ZNW5), C16orf13 (Q96S19), C16orf80 (Q9Y6A4), C1D (Q13901), C1orf123 (Q9NWV4), C1orf50 (Q9BV19), C1orf57 (Q9BSD7), C1RL (Q9NZP8), C20orf11 (Q9NWU2), C20orf27 (Q9GZN8), C20orf4 (Q9Y312), C21orf59 (P57076), C22orf25 (Q6ICL3), C22orf28 (Q9Y3I0), C2orf29 (Q9UKZ1), C2orf79 (Q6GMV3), C3orf10 (Q8WUW1), C3orf26 (Q9BQ75), C3orf75 (Q0PNE2), C4orf27 (Q9NWY4), C4orf41 (Q7Z392), C5orf32 (Q9H1C7), C6orf130 (Q9Y530), C6orf211 (Q9H993), C7orf25 (Q9BPX7), C7orf28B (P86790), C7orf41 (Q8N3F0), C7orf59 (Q0VGL1), C9orf142 (Q9BUH6), C9orf23 (Q8N5L8), C9orf41 (Q8N4J0), C9orf64 (Q5T6V5), CA11 (O75493), CAB39 (Q9Y376), CACNA2D1 (P54289), CACYBP (Q9HB71), CAD (P27708), CADM1 (Q9BY67), CADM4 (Q8NFZ8), CALB1 (P05937), CALD1 (Q05682), CALM1 (P62158), CAMK2D (Q13557), CAND1 (Q86VP6), CAP1 (Q01518), CAPN1 (P07384), CAPN2 (P17655), CAPN5 (015484), CAPNS1 (P04632), CAPS (Q13938), CAPZA1 (P52907), CAPZA2 (P47755), CAPZB (P47756), CARHSP1 (Q9Y2V2), CARKD (Q8IW45), CARM1 (Q86X55), CARS (P49589), CASK (014936), CASP3 (P42574), CASP6 (P55212), CAT (P04040), CBFB (Q13951), CBR1 (P16152), CBR3 (O75828), CBS (P35520), CBWD2 (Q8IUF1), CBX1 (P83916), CBX3 (Q13185), CBX5 (P45973), CC2D1A (Q6P1 N0), CC2D1 B (Q5T0F9), CCAR1 (Q8IX12), CCBL1 (Q16773), CCBL2 (Q6YP21), CCDC22 (O60826), CCDC25 (Q86WR0), CCDC53 (Q9Y3C0), CCDC56 (Q9Y2R0), CCDC93 (Q567U6), CCNC (P24863), CCND2 (P30279), CCNH (P51946), CCT2 (P78371), CCT3 (P49368), CCT4 (P50991), CCT5 (P48643), CCT6A (P40227), CCT7 (Q99832), CCT8 (P50990), CD109 (Q6YHK3), CD151 (P48509), CD276 (Q5ZPR3), CD44 (P16070), CD47 (Q08722), CD59 (P13987), CD63 (P08962), CD81 (P60033), CD9 (P21926), CD99 (P14209), CDC16 (Q13042), CDC23 (Q9UJX2), CDC27 (P30260), CDC34 (P49427), CDC37 (Q16543), CDC40 (O60508), CDC42 (P60953), CDC5L (Q99459), CDCP1 (Q9H5V8), CDH2 (P19022), CDK1 (P06493), CDK2 (P24941), CDK2AP2 (O75956), CDK4 (P11802), CDK5 (Q00535), CDK5RAP3 (Q96JB5), CDK7 (P50613), CDKN2A (P42771), CDKN2AIP (Q9NXV6), CELSR1 (Q9NYQ6), CELSR2 (Q9HCU4), CEP57 (Q86XR8), CFL1 (P23528), CFL2 (Q9Y281), CHAC2 (Q8WUX2), CHAF1B (Q13112), CHD4 (Q14839), CHEK2 (096017), CHERP (Q8IWX8), CHID1 (Q9BWS9), CHML (P26374), CHMP1B (Q7LBR1), CHMP2A (O43633), CHMP4A (Q9BY43), CHMP4B (Q9H444), CHMP6 (Q96FZ7), CHORDC1 (Q9UHD1), CHP (Q99653), CHRAC1 (Q9NRG0), CHST14 (Q8NCH0), CHST3 (Q7LGC8), CHURC1 (Q8WUH1), CIAO1 (076071), CIAPIN1 (Q6FI81), CIRH1A (Q969X6), CKAP5 (Q14008), CKB (P12277), CLASP1 (Q7Z460), CLDN3 (015551), CLEC18B (Q6UXF7), CLIC1 (O00299), CLIC4 (Q9Y696), CLLD6 (Q5W111), CLNS1A (P54105), CLP1 (Q92989), CLPB (Q9H078), CLTA (P09496), CLTC (Q00610), CLU (P10909), CMAS (Q8NFW8), CMBL (Q96DG6), CMPK1 (P30085), CNBP (P62633), CNDP2 (Q96KP4), CNN2 (Q99439), CNN3 (Q15417), CNOT1 (A5YKK6), CNOT10 (Q9H9A5), CNOT6L (Q96LI5), CNOT7 (Q9UIV1), CNP (P09543), COASY (Q13057), COBRA1 (Q8WX92), COG1 (Q8WTW3), COG2 (Q14746), COG3 (Q96JB2), COG4 |

(continued)

| |
|---|
| (Q9H9E3), COG5 (Q9UP83), COG6 (Q9Y2V7), COG7 (P83436), COG8 (Q96MW5), COL11A1 (P12107), COL14A1 (Q05707), COL6A1 (P12109), COMMD1 (Q8N668), COMMD10 (Q9Y6G5), COMMD2 (Q86X83), COMMD3 (Q9UBI1), COMMD4 (Q9H0A8), COMMD5 (Q9GZQ3), COMMD6 (Q7Z4G1), COMMD7 (Q86VX2), COMMD8 (Q9NX08), COMMD9 (Q9P000), COMT (P21964), COPA (P53621), COPB1 (P53618), COPB2 (P35606), COPE (014579), COPG (Q9Y678), COPG2 (Q9UBF2), COPS2 (P61201), COPS3 (Q9UNS2), COPS4 (Q9BT78), COPS5 (Q92905), COPS6 (Q7L5N1), COPS7A (Q9UBW8), COPS7B (Q9H9Q2), COPS8 (Q99627), COPZ1 (P61923), CORO1A (P31146), CORO1B (Q9BR76), CORO1C (Q9ULV4), CORO2B (Q9UQ03), CORO7 (P57737), COTL1 (Q14019), COX5A (P20674), COX5B (P10606), COX6C (P09669), COX7A2 (P14406), CP (P00450), CPD (O75976), CPN2 (P22792), CPNE1 (Q99829), CPNE3 (075131), CPNE7 (Q9UBL6), CPSF1 (Q10570), CPSF2 (Q9P2I0), CPSF3 (Q9UKF6), CPSF7 (Q8N684), CPXM1 (Q96SM3), CRIP2 (P52943), CRK (P46108), CRLF3 (Q8IUI8), CRTAP (075718), CRYAB (P02511), CRYM (Q14894), CRYZ (Q08257), CRYZL1 (O95825), CS (O75390), CSDE1 (O75534), CSE1L (P55060), CSK (P41240), CSNK1A1 (P48729), CSNK2A1 (P68400), CSNK2B (P67870), CSRP1 (P21291), CSRP2 (Q16527), CSTB (P04080), CSTF1 (Q05048), CSTF2T (Q9H0L4), CSTF3 (Q12996), CTBP1 (Q13363), CTBP2 (P56545), CTNNA1 (P35221), CTNNB1 (P35222), CTNNBL1 (Q8WYA6), CTNND1 (060716), CTPS (P17812), CTPS2 (Q9NRF8), CTR9 (Q6PD62), CTSC (P53634), CTSD (P07339), CTSF (Q9UBX1), CTSL2 (060911), CTU1 (Q7Z7A3), CTU2 (Q2VPK5), CUL1 (Q13616), CUL2 (Q13617), CUL3 (Q13618), CUL4A (Q13619), CUL4B (Q13620), CUL5 (Q93034), CWF19L1 (Q69YN2), CXADR (P78310), CXorf26 (Q9BVG4), CYB5A (P00167), CYCS (P99999), CYFIP1 (Q7L576), CYFIP2 (Q96F07), CYR61 (O00622), DAG1 (Q14118), DAK (Q3LXA3), DARS (P14868), DAZAP1 (Q96EP5), DBI (P07108), DBN1 (Q16643), DBNL (Q9UJU6), DBR1 (Q9UK59), DCAF7 (P61962), DCAF8 (Q5TAQ9), DCD (P81605), DCK (P27707), DCLK1 (015075), DCPS (Q96C86), DCTD (P32321), DCTN1 (Q14203), DCTN2 (Q13561), DCTN3 (O75935), DCTN4 (Q9UJW0), DCTN5 (Q9BTE1), DCTN6 (O00399), DCUN1D1 (Q96GG9), DCUN1D5 (Q9BTE7), DCXR (Q7Z4W1), DDA1 (Q9BW61), DDAH2 (O95865), DDB1 (Q16531), DDB2 (Q92466), DDI2 (Q5TDH0), DDR1 (Q08345), DDT (P30046), DDX1 (Q92499), DDX17 (Q92841), DDX19A (Q9NUU7), DDX21 (Q9NR30), DDX23 (Q9BUQ8), DDX39 (000148), DDX3X (000571), DDX5 (P17844), DDX51 (Q8N8A6), DDX6 (P26196), DECR1 (Q16698), DEF (Q68CQ4), DEFA1 (P59665), DENR (O43583), DERA (Q9Y315), DFFA (O00273), DHFR (P00374), DHPS (P49366), DHRS1 (Q96LJ7), DHRS11 (Q6UWP2), DHRS4 (Q9BTZ2), DHX15 (043143), DHX16 (060231), DHX29 (Q7Z478), DHX36 (Q9H2U1), DHX9 (Q08211), DIAPH1 (060610), DIAPH2 (O60879), DIMT1L (Q9UNQ2), DIP2B (Q9P265), DIP2C (Q9Y2E4), DIS3 (Q9Y2L1), DIS3L2 (Q8IYB7), DKC1 (O60832), DLG1 (Q12959), DNAH17 (Q9UFH2), DNAJA1 (P31689), DNAJA2 (O60884), DNAJB1 (P25685), DNAJB4 (Q9UDY4), DNAJC13 (075165), DNAJC3 (Q13217), DNAJC7 (Q99615), DNASE1 L1 (P49184), DNM1 (Q05193), DNM1L (O00429), DNM2 (P50570), DNPEP (Q9ULA0), DOCK1 |

(continued)

| |
|---|
| (Q14185), DOCK4 (Q8N1I0), DOCK5 (Q9H7D0), DOCK7 (Q96N67), DOHH (Q9BU89), DOM3Z (O77932), DPCD (Q9BVM2), DPH1 (Q9BZG8), DPH2 (Q9BQC3), DPH5 (Q9H2P9), DPM1 (O60762), DPP3 (Q9NY33), DPP9 (Q86TI2), DPY30 (Q9C005), DPYSL2 (Q16555), DPYSL3 (Q14195), DPYSL4 (014531), DPYSL5 (Q9BPU6), DRG1 (Q9Y295), DRG2 (P55039), DSG1 (Q02413), DSP (P15924), DST (Q03001), DSTN (P60981), DTD1 (Q8TEA8), DTYMK (P23919), DUS2L (Q9NX74), DUSP12 (Q9UNI6), DUSP23 (Q9BVJ7), DUSP3 (P51452), DYM (Q7RTS9), DYNC1H1 (Q14204), DYNC1I2 (Q13409), DYNC1LI1 (Q9Y6G9), DYNC1LI2 (O43237), DYNC2H1 (Q8NCM8), DYNLL1 (P63167), DYNLL2 (Q96FJ2), DYNLRB1 (Q9NP97), DYNLT1 (P63172), ECHDC1 (Q9NTX5), ECHDC3 (Q96DC8), ECHS1 (P30084), ECM29 (Q5VYK3), EDC4 (Q6P2E9), EEA1 (Q15075), EEF1A1 (P68104), EEF1B2 (P24534), EEF1D (P29692), EEF1E1 (O43324), EEF1G (P26641), EEF2 (P13639), EEFSEC (P57772), EFEMP2 (O95967), EFHD2 (Q96C19), EFNB2 (P52799), EFTUD1 (Q7Z2Z2), EFTUD2 (Q15029), EGFR (P00533), EHD1 (Q9H4M9), EHD2 (Q9NZN4), EHD4 (Q9H223), EIF1 (P41567), EIF1AX (P47813), EIF2A (Q9BY44), EIF2AK2 (P19525), EIF2B1 (Q14232), EIF2B2 (P49770), EIF2B3 (Q9NR50), EIF2B4 (Q9UI10), EIF2B5 (Q13144), EIF2C2 (Q9UKV8), EIF2S1 (P05198), EIF2S2 (P20042), EIF2S3 (P41091), EIF3A (Q14152), EIF3B (P55884), EIF3C (Q99613), EIF3D (O15371), EIF3E (P60228), EIF3F (O00303), EIF3G (075821), EIF3H (015372), EIF3I (Q13347), EIF3J (O75822), EIF3K (Q9UBQ5), EIF3L (Q9Y262), EIF3M (Q7L2H7), EIF4A1 (P60842), EIF4A2 (Q14240), EIF4A3 (P38919), EIF4E (P06730), EIF4E2 (O60573), EIF4G1 (Q04637), EIF4G2 (P78344), EIF4G3 (O43432), EIF4H (Q15056), EIF5 (P55010), EIF5A (P63241), EIF5B (060841), EIF6 (P56537), ELAC2 (Q9BQ52), ELAVL1 (Q15717), ELMO2 (Q96JJ3), ELP2 (Q6IA86), ELP3 (Q9H9T3), EMG1 (Q92979), EMILIN1 (Q9Y6C2), EML1 (O00423), EML2 (O95834), EML3 (Q32P44), EML4 (Q9HC35), ENAH (Q8N8S7), ENO1 (P06733), ENO2 (P09104), ENOPH1 (Q9UHY7), ENY2 (Q9NPA8), EPB41L2 (043491), EPB41L3 (Q9Y2J2), EPHA2 (P29317), EPHB3 (P54753), EPHX1 (P07099), EPM2AIP1 (Q7L775), EPRS (P07814), ERH (P84090), ERI1 (Q8IV48), ERI3 (043414), ERP44 (Q9BS26), ESD (P10768), ESYT1 (Q9BSJ8), ETF1 (P62495), ETFA (P13804), ETFB (P38117), EXOC1 (Q9NV70), EXOC2 (Q96KP1), EXOC3 (O60645), EXOC4 (Q96A65), EXOC5 (000471), EXOC6 (Q8TAG9), EXOC7 (Q9UPT5), EXOC8 (Q8IYI6), EXOSC1 (Q9Y3B2), EXOSC2 (Q13868), EXOSC3 (Q9NQT5), EXOSC4 (Q9NPD3), EXOSC5 (Q9NQT4), EXOSC6 (Q5RKV6), EXOSC7 (Q15024), EXOSC8 (Q96B26), EXOSC9 (Q06265), EXTL3 (O43909), EYA3 (Q99504), EZR (P15311), F3 (P13726), F8 (P00451), F8A1 (P23610), FABP5 (Q01469), FABP7 (015540), FADD (Q13158), FAF1 (Q9UNN5), FAH (P16930), FAHD2A (Q96GK7), FAM114A2 (Q9NRY5), FAM115A (Q9Y4C2), FAM120A (Q9NZB2), FAM125A (Q96EY5), FAM127A (A6ZKI3), FAM129B (Q96TA1), FAM136A (Q96C01), FAM168A (Q92567), FAM175B (Q15018), FAM188A (Q9H8M7), FAM3A (P98173), FAM3C (Q92520), FAM45B (Q6NSW5), FAM49B (Q9NUQ9), FAM82B (Q96DB5), FAM84B (Q96KN1), FAM98A (Q8NCA5), FAM98B (Q52LJ0), FARP1 (Q9Y4F1), FARP2 (O94887), FARSA (Q9Y285), FARSB |

(continued)

| |
|---|
| (Q9NSD9), FASN (P49327), FAT1 (Q14517), FBL (P22087), FBLN2 (P98095), FBN1 (P35555), FBN2 (P35556), FBXL18 (Q96ME1), FBXO21 (O94952), FBXO22 (Q8NEZ5), FDFT1 (P37268), FDPS (P14324), FEN1 (P39748), FERMT1 (Q9BQL6), FERMT2 (Q96AC1), FGF1 (P05230), FGFRL1 (Q8N441), FGGY (Q96C11), FH (P07954), FHL1 (Q13642), FHL2 (Q14192), FHL3 (Q13643), FIS1 (Q9Y3D6), FKBP1A (P62942), FKBP3 (Q00688), FKBP4 (Q02790), FKBP5 (Q13451), FLII (Q13045), FLNA (P21333), FLNB (O75369), FLNC (Q14315), FLOT1 (O75955), FMNL2 (Q96PY5), FN3K (Q9H479), FN3KRP (Q9HA64), FNTA (P49354), FNTB (P49356), FOLR1 (P15328), FREM2 (Q5SZK8), FRMD8 (Q9BZ67), FSCN1 (Q16658), FSD1 (Q9BTV5), FTH1 (P02794), FTL (P02792), FTO (Q9C0B1), FTSJD2 (Q8N1G2), FUBP1 (Q96AE4), FUCA2 (Q9BTY2), FUK (Q8N0W3), FXR1 (P51114), G3BP1 (Q13283), G3BP2 (Q9UN86), G6PD (P11413), GAA (P10253), GALK1 (P51570), GALK2 (Q01415), GALNT1 (Q10472), GALNT2 (Q10471), GANAB (Q14697), GAP43 (P17677), GAPDH (P04406), GAPVD1 (Q14C86), GAR1 (Q9NY12), GARS (P41250), GART (P22102), GATSL2 (A6NHX0), GBA (P04062), GBE1 (Q04446), GCLM (P48507), GCN1L1 (Q92616), GDI1 (P31150), GDI2 (P50395), GEMIN5 (Q8TEQ6), GEMIN6 (Q8WXD5), GET4 (Q7L5D6), GFAP (P14136), GFPT1 (Q06210), GFPT2 (O94808), GGCT (O75223), GGPS1 (O95749), GINS1 (Q14691), GINS4 (Q9BRT9), GIPC1 (014908), GIT1 (Q9Y2X7), GLA (P06280), GLB1 (P16278), GLB1L2 (Q8IW92), GLG1 (Q92896), GLIPR2 (Q9H4G4), GLMN (Q92990), GLO1 (Q04760), GLOD4 (Q9HC38), GLRX (P35754), GLRX3 (O76003), GLT25D1 (Q8NBJ5), GLTP (Q9NZD2), GLTPD1 (Q5TA50), GLUD1 (P00367), GLUL (P15104), GMDS (O60547), GMFB (P60983), GMPPA (Q96IJ6), GMPPB (Q9Y5P6), GMPR (P36959), GMPR2 (Q9P2T1), GMPS (P49915), GNA11 (P29992), GNA13 (Q14344), GNAI2 (P04899), GNAI3 (P08754), GNAQ (P50148), GNAS (Q5JWF2), GNB1 (P62873), GNB2 (P62879), GNB2L1 (P63244), GNB4 (Q9HAV0), GNE (Q9Y223), GNG12 (Q9UBI6), GNG4 (P50150), GNG5 (P63218), GNPDA1 (P46926), GNPNAT1 (Q96EK6), GOLGA7 (Q7Z5G4), GOLGB1 (Q14789), GOLIM4 (000461), GOLM1 (Q8NBJ4), GOLPH3 (Q9H4A6), GORASP2 (Q9H8Y8), GPC1 (P35052), GPC4 (O75487), GPC6 (Q9Y625), GPD1L (Q8N335), GPI (P06744), GPLD1 (P80108), GPM6A (P51674), GPM6B (Q13491), GPN1 (Q9HCN4), GPR56 (Q9Y653), GPS1 (Q13098), GPX1 (P07203), GPX4 (P36969), GRB2 (P62993), GRHPR (Q9UBQ7), GRP (Q3ZCW2), GRPEL1 (Q9HAV7), GRWD1 (Q9BQ67), GSK3A (P49840), GSK3B (P49841), GSN (P06396), GSPT1 (P15170), GSS (P48637), GSTK1 (Q9Y2Q3), GSTM2 (P28161), GSTM3 (P21266), GSTM4 (Q03013), GSTO1 (P78417), GSTP1 (P09211), GSTT2 (P0CG29), GSTZ1 (O43708), GTF2F2 (P13984), GTF2H2 (Q13888), GTF2I (P78347), GTF3C1 (Q12789), GTF3C2 (Q8WUA4), GTF3C4 (Q9UKN8), GTPBP1 (000178), GUK1 (Q16774), GYG1 (P46976), GYS1 (P13807), H2AFY (O75367), H2AFZ (P0C0S5), HADH (Q16836), HAGH (Q16775), HARS (P12081), HAT1 (014929), HAUS3 (Q68CZ6), HAUS4 (Q9H6D7), HBA1 (P69905), HBB (P68871), HCFC1 (P51610), HDAC1 (Q13547), HDAC2 (Q92769), HDAC3 (015379), HDHD2 (Q9H0R4), HDLBP (Q00341), HEATR1 (Q9H583), HEATR2 (Q86Y56), HEBP1 (Q9NRV9), HECTD3 (Q5T447), HEG1 |

(continued)

| |
|---|
| (Q9ULI3), HELZ (P42694), HERC4 (Q5GLZ8), HEXB (P07686), HGS (014964), HHIP (Q96QV1), HIBCH (Q6NVY1), HIF1AN (Q9NWT6), HINT1 (P49773), HIP1R (075146), HIST1H1B (P16401), HIST1H1C (P16403), HIST1H2BM (Q99879), HIST1H2BO (P23527), HIST1H4A (P62805), HIST2H2AA3 (Q6FI13), HIST2H3A (Q71DI3), HK1 (P19367), HK2 (P52789), HLA-A (P30443), HLA-A (P01892), HLCS (P50747), HMGA1 (P17096), HMGB1 (P09429), HMGCL (P35914), HMGCS1 (Q01581), HMGN2 (P05204), HNRNPA1 (P09651), HNRNPA2B1 (P22626), HNRNPA3 (P51991), HNRNPAB (Q99729), HNRNPC (P07910), HNRNPD (Q14103), HNRNPF (P52597), HNRNPH1 (P31943), HNRNPH2 (P55795), HNRNPH3 (P31942), HNRNPK (P61978), HNRNPL (P14866), HNRNPM (P52272), HNRNPR (O43390), HNRNPU (Q00839), HNRNPUL2 (Q1KMD3), HNRPDL (014979), HNRPLL (Q8WVV9), HOOK3 (Q86VS8), HP (P00738), HP1BP3 (Q5SSJ5), HPCAL1 (P37235), HPRT1 (P00492), HPX (P02790), HRAS (P01112), HS6ST2 (Q96MM7), HSD17B10 (Q99714), HSD17B4 (P51659), HSP90AA1 (P07900), HSP90AB1 (P08238), HSP90B1 (P14625), HSPA12A (043301), HSPA14 (Q0VDF9), HSPA1A (P08107), HSPA2 (P54652), HSPA4 (P34932), HSPA4L (O95757), HSPA5 (P11021), HSPA8 (P11142), HSPA9 (P38646), HSPB1 (P04792), HSPB11 (Q9Y547), HSPBP1 (Q9NZL4), HSPD1 (P10809), HSPE1 (P61604), HSPG2 (P98160), HSPH1 (Q92598), HTATIP2 (Q9BUP3), HTRA1 (Q92743), HTT (P42858), HUWE1 (Q7Z6Z7), HYOU1 (Q9Y4L1), IARS (P41252), ICAM1 (P05362), IDE (P14735), IDH1 (O75874), IDH2 (P48735), IDI1 (Q13907), IDUA (P35475), IFI16 (Q16666), IFI35 (P80217), IFIT5 (Q13325), IFITM3 (Q01628), IGF1R (P08069), IGF2BP2 (Q9Y6M1), IGF2BP3 (O00425), IGF2R (P11717), IGFBP3 (P17936), IGSF3 (O75054), IGSF8 (Q969P0), IKBKAP (095163), IL1RAP (Q9NPH3), ILF2 (Q12905), ILF3 (Q12906), ILK (Q13418), ILKAP (Q9H0C8), IMP4 (Q96G21), IMPA1 (P29218), IMPA2 (014732), IMPAD1 (Q9NX62), IMPDH2 (P12268), INF2 (Q27J81), INPP1 (P49441), INPPL1 (015357), INTS1 (Q8N201), INTS10 (Q9NVR2), INTS3 (Q68E01), INTS5 (Q6P9B9), IPO11 (Q9UI26), IPO13 (O94829), IPO4 (Q8TEX9), IPO5 (000410), IPO7 (O95373), IPO8 (015397), IPO9 (Q96P70), IQGAP1 (P46940), IRF2BP2 (Q7Z5L9), IRF3 (Q14653), IRGQ (Q8WZA9), ISG15 (P05161), ISOC1 (Q96CN7), ISPD (A4D126), ISYNA1 (Q9NPH2), ITFG3 (Q9H0X4), ITGA2 (P17301), ITGA3 (P26006), ITGA4 (P13612), ITGA5 (P08648), ITGA6 (P23229), ITGA7 (Q13683), ITGAV (P06756), ITGB1 (P05556), ITGB4 (P16144), ITGB8 (P26012), ITPA (Q9BY32), JAM3 (Q9BX67), JUP (P14923), KARS (Q15046), KBTBD4 (Q9NVX7), KBTBD6 (Q86V97), KCTD12 (Q96CX2), KDM1A (O60341), KEAP1 (Q14145), KHDRBS1 (Q07666), KHSRP (Q92945), KIAA0174 (P53990), KIAA0196 (Q12768), KIAA0319L (Q8IZA0), KIAA0664 (075153), KIAA0776 (O94874), KIAA1033 (Q2M389), KIAA1279 (Q96EK5), KIAA1468 (Q9P260), KIAA1598 (A0MZ66), KIAA1797 (Q5VW36), KIAA1967 (Q8N163), KIF1A (Q12756), KIF3A (Q9Y496), KIF5B (P33176), KIF5C (O60282), KLC1 (Q07866), KLC2 (Q9H0B6), KLC4 (Q9NSK0), KLHDC3 (Q9BQ90), KLHL13 (Q9P2N7), KNG1 (P01042), KNTC1 (P50748), KPNA1 (P52294), KPNA2 (P52292), KPNA3 (O00505), KPNA4 (O00629), KPNA6 (O60684), KPNB1 (Q14974), KPRP |

(continued)

| |
|---|
| (Q5T749), KRAS (P01116), KRIT1 (O00522), KRT13 (P13646), KRT14 (P02533), KRT71 (Q3SY84), KTN1 (Q86UP2), L1CAM (P32004), LAGE3 (Q14657), LAMA4 (Q16363), LAMA5 (O15230), LAMB1 (P07942), LAMC1 (P11047), LAMP1 (P11279), LAMP2 (P13473), LANCL1 (O43813), LANCL2 (Q9NS86), LAP3 (P28838), LARP1 (Q6PKG0), LARS (Q9P2J5), LASP1 (Q14847), LCAT (P04180), LCMT1 (Q9UIC8), LDHA (P00338), LDHB (P07195), LDLR (P01130), LEFTY2 (O00292), LEPRE1 (Q32P28), LFNG (Q8NES3), LGALS1 (P09382), LGALS3 (P17931), LGALS3BP (Q08380), LHFP (Q9Y693), LIMA1 (Q9UHB6), LIMS1 (P48059), LIN7C (Q9NUP9), LIPG (Q9Y5X9), LLGL1 (Q15334), LMCD1 (Q9NZU5), LMNA (P02545), LMNB1 (P20700), LOXL4 (Q96JB6), LPL (P06858), LRBA (P50851), LRCH3 (Q96II8), LRG1 (P02750), LRP1 (Q07954), LRRC20 (Q8TCA0), LRRC40 (Q9H9A6), LRRC47 (Q8N1G4), LRRC57 (Q8N9N7), LRSAM1 (Q6UWE0), LRWD1 (Q9UFC0), LSM1 (015116), LSM12 (Q3MHD2), LSM2 (Q9Y333), LSM3 (P62310), LSM4 (Q9Y4Z0), LSM6 (P62312), LSM7 (Q9UK45), LSS (P48449), LTA4H (P09960), LTBP2 (Q14767), LTBP3 (Q9NS15), LUM (P51884), LYPLA1 (O75608), LYPLA2 (O95372), LYPLAL1 (Q5VWZ2), M6PR (P20645), MACF1 (Q9UPN3), MAD1L1 (Q9Y6D9), MAD2L1 (Q13257), MAEA (Q7L5Y9), MAGEE1 (Q9HCI5), MAGOHB (Q96A72), MALT1 (Q9UDY8), MAN1B1 (Q9UKM7), MAN2A1 (Q16706), MANBA (O00462), MAP1B (P46821), MAP1S (Q66K74), MAP2K1 (Q02750), MAP2K2 (P36507), MAP2K3 (P46734), MAP3K4 (Q9Y6R4), MAP4 (P27816), MAP4K4 (095819), MAPK1 (P28482), MAPK12 (P53778), MAPK3 (P27361), MAPK9 (P45984), MAPKAPK2 (P49137), MAPKSP1 (Q9UHA4), MAPRE1 (Q15691), MAPRE3 (Q9UPY8), MARCKS (P29966), MARCKSL1 (P49006), MARK2 (Q7KZI7), MARS (P56192), MAT2A (P31153), MAT2B (Q9NZL9), MATR3 (P43243), MBD3 (O95983), MBNL1 (Q9NR56), MCAM (P43121), MCAT (Q8IVS2), MCM2 (P49736), MCM3 (P25205), MCM4 (P33991), MCM5 (P33992), MCM6 (Q14566), MCM7 (P33993), MCTS1 (Q9ULC4), MDH1 (P40925), MDH2 (P40926), MDK (P21741), MDN1 (Q9NU22), ME1 (P48163), ME2 (P23368), MED1 (Q15648), MED16 (Q9Y2X0), MED17 (Q9NVC6), MED18 (Q9BUE0), MED20 (Q9H944), MED22 (Q15528), MED23 (Q9ULK4), MED27 (Q6P2C8), MED30 (Q96HR3), MED31 (Q9Y3C7), MEMO1 (Q9Y316), MERIT40 (Q9NWV8), METAP1 (P53582), METAP2 (P50579), METT10D (Q86W50), METTL1 (Q9UBP6), METTL11A (Q9BV86), METTL13 (Q8N6R0), METTL2B (Q6P1Q9), METTL5 (Q9NRN9), MFAP2 (P55001), MFAP4 (P55083), MFGE8 (Q08431), MFI2 (P08582), MGAT4B (Q9UQ53), MGAT5 (Q09328), MGEA5 (O60502), MICAL1 (Q8TDZ2), MIF (P14174), MIF4GD (A9UHW6), MINA (Q8IUF8), MINK1 (Q8N4C8), MIOS (Q9NXC5), MIS12 (Q9H081), MKLN1 (Q9UL63), MLTK (Q9NYL2), MMP14 (P50281), MMS19 (Q96T76), MOB2 (Q70IA6), MOBKL1B (Q9H8S9), MOBKL2A (Q96BX8), MOBKL3 (Q9Y3A3), MOCS2 (O96033), MON2 (Q7Z3U7), MORC2 (Q9Y6X9), MOV10 (Q9HCE1), MOXD1 (Q6UVY6), MPI (P34949), MPP6 (Q9NZW5), MPRIP (Q6WCQ1), MPST (P25325), MPZL1 (O95297), MRC2 (Q9UBG0), MRI1 (Q9BV20), MRTO4 (Q9UKD2), MSH2 (P43246), MSN (P26038), MSTO1 (Q9BUK6), MTA1 (Q13330), MTA2 (O94776), MTAP (Q13126), MTHFD1 (P11586), MTHFS (P49914), MTM1 |

(continued)

| |
|---|
| (Q13496), MTMR1 (Q13613), MTMR6 (Q9Y217), MTMR9 (Q96QG7), MTOR (P42345), MTPN (P58546), MTR (Q99707), MVD (P53602), MVK (Q03426), MVP (Q14764), MYADM (Q96S97), MYBBP1A (Q9BQG0), MYCBP (Q99417), MYD88 (Q99836), MYH10 (P35580), MYH9 (P35579), MYL12B (014950), MYL6 (P60660), MYO18A (Q92614), MYO1B (O43795), MYO1C (000159), MYO1E (Q12965), MYO6 (Q9UM54), MYOF (Q9NZM1), MZT1 (Q08AG7), NAA10 (P41227), NAA15 (Q9BXJ9), NAA16 (Q6N069), NAA20 (P61599), NAA30 (Q147X3), NAA38 (O95777), NAA50 (Q9GZZ1), NACA (Q13765), NADSYN1 (Q6IA69), NAE1 (Q13564), NAGK (Q9UJ70), NAGLU (P54802), NAMPT (P43490), NANS (Q9NR45), NAP1L1 (P55209), NAP1L4 (Q99733), NAPA (P54920), NAPG (Q99747), NAPRT1 (Q6XQN6), NARS (O43776), NASP (P49321), NCAM1 (P13591), NCAPD2 (Q15021), NCAPG (Q9BPX3), NCBP1 (Q09161), NCBP2 (P52298), NCDN (Q9UBB6), NCKAP1 (Q9Y2A7), NCKIPSD (Q9NZQ3), NCL (P19338), NCS1 (P62166), NCSTN (Q92542), NDRG3 (Q9UGV2), NDRG4 (Q9ULP0), NDUFA2 (O43678), NDUFA3 (095167), NDUFA5 (Q16718), NDUFAB1 (014561), NDUFS6 (O75380), NEDD4L (Q96PU5), NEFL (P07196), NEK9 (Q8TD19), NES (P48681), NF1 (P21359), NFIC (P08651), NFIX (Q14938), NFKB2 (Q00653), NHLRC2 (Q8NBF2), NHP2L1 (P55769), NID1 (P14543), NIP7 (Q9Y221), NIT1 (Q86X76), NIT2 (Q9NQR4), NLE1 (Q9NVX2), NLGN4X (Q8N0W4), NLN (Q9BYT8), NMD3 (Q96D46), NME1 (P15531), NME2 (P22392), NME3 (Q13232), NME7 (Q9Y5B8), NMT1 (P30419), NNMT (P40261), NOB1 (Q9ULX3), NOL11 (Q9H8H0), NOL6 (Q9H6R4), NOMO2 (Q5JPE7), NONO (Q15233), NOP10 (Q9NPE3), NOP2 (P46087), NOTCH1 (P46531), NOTCH3 (Q9UM47), NOVA2 (Q9UNW9), NPEPPS (P55786), NPLOC4 (Q8TAT6), NPM1 (P06748), NPM3 (O75607), NPTN (Q9Y639), NPW (Q8N729), NQO1 (P15559), NQO2 (P16083), NR2C2AP (Q86WQ0), NRAS (P01111), NRBP1 (Q9UHY1), NRBP2 (Q9NSY0), NRD1 (O43847), NRP2 (O60462), NSF (P46459), NSMAF (Q92636), NSMCE1 (Q8WV22), NSUN2 (Q08J23), NT5C (Q8TCD5), NT5DC1 (Q5TFE4), NTN1 (O95631), NUBP1 (P53384), NUBP2 (Q9Y5Y2), NUCB1 (Q02818), NUDC (Q9Y266), NUDCD1 (Q96RS6), NUDCD2 (Q8WVJ2), NUDT1 (P36639), NUDT10 (Q8NFP7), NUDT12 (Q9BQG2), NUDT16 (Q96DE0), NUDT16L1 (Q9BRJ7), NUDT2 (P50583), NUDT21 (O43809), NUDT4 (Q9NZJ9), NUDT5 (Q9UKK9), NUMA1 (Q14980), NUP188 (Q5SRE5), NUP37 (Q8NFH4), NUP43 (Q8NFH3), NUP54 (Q7Z3B4), NUP88 (Q99567), NUP93 (Q8N1F7), NUTF2 (P61970), NXN (Q6DKJ4), OBFC2B (Q9BQ15), OCRL (Q01968), ODZ2 (Q9NT68), ODZ3 (Q9P273), OGFOD1 (Q8N543), OGT (015294), OLA1 (Q9NTK5), OLFML3 (Q9NRN5), OPA1 (060313), OPLAH (014841), OSBP (P22059), OSBPL1A (Q9BXW6), OSGEP (Q9NPF4), OTUB1 (Q96FW1), OVCA2 (Q8WZ82), OXCT1 (P55809), OXSR1 (O95747), P4HB (P07237), PA2G4 (Q9UQ80), PAAF1 (Q9BRP4), PABPC1 (P11940), PABPC4 (Q13310), PABPN1 (Q86U42), PACSIN2 (Q9UNF0), PACSIN3 (Q9UKS6), PAF1 (Q8N7H5), PAFAH1B1 (P43034), PAFAH1B2 (P68402), PAFAH1B3 (Q15102), PAICS (P22234), PAIP1 (Q9H074), PAK2 (Q13177), PALD (Q9ULE6), PALLD (Q8WX93), PANK4 (Q9NVE7), PAPOLA (P51003), PAPSS1 (O43252), PARF (Q3YEC7), PARK7 (Q99497), PARN (O95453), PARP1 (P09874), PARP4 (Q9UKK3), |

(continued)

| |
|---|
| PARVA (Q9NVD7), PBK (Q96KB5), PBLD (P30039), PCBP1 (Q15365), PCBP2 (Q15366), PCDHB2 (Q9Y5E7), PCDHGB4 (Q9UN71), PCDHGC3 (Q9UN70), PCID2 (Q5JVF3), PCMT1 (P22061), PCNA (P12004), PCOLCE2 (Q9UKZ9), PCYT2 (Q99447), PDCD10 (Q9BUL8), PDCD2L (Q9BRP1), PDCD4 (Q53EL6), PDCD5 (014737), PDCD6 (O75340), PDCD6IP (Q8WUM4), PDCL3 (Q9H2J4), PDDC1 (Q8NB37), PDE12 (Q6L8Q7), PDE6D (O43924), PDGFC (Q9NRA1), PDIA3 (P30101), PDIA6 (Q15084), PDLIM1 (000151), PDLIM4 (P50479), PDLIM5 (Q96HC4), PDLIM7 (Q9NR12), PDRG1 (Q9NUG6), PDRO (Q6IAA8), PDS5A (Q29RF7), PDXK (O00764), PDXP (Q96GD0), PEA15 (Q15121), PEBP1 (P30086), PEF1 (Q9UBV8), PELO (Q9BRX2), PELP1 (Q8IZL8), PEPD (P12955), PFAS (015067), PFDN2 (Q9UHV9), PFDN5 (Q99471), PFDN6 (015212), PFKL (P17858), PFKM (P08237), PFKP (Q01813), PFN1 (P07737), PFN2 (P35080), PGAM1 (P18669), PGAM5 (Q96HS1), PGD (P52209), PGGT1B (P53609), PGK1 (P00558), PGLS (O95336), PGLYRP2 (Q96PD5), PGM1 (P36871), PGM2L1 (Q6PCE3), PGM3 (O95394), PGP (A6NDG6), PGRMC1 (O00264), PGRMC2 (015173), PHF5A (Q7RTV0), PHGDH (043175), PHKB (Q93100), PHLDA3 (Q9Y5J5), PHPT1 (Q9NRX4), PIK3CB (P42338), PIK3R4 (Q99570), PIN1 (Q13526), PIP4K2A (P48426), PIPOX (Q9P0Z9), PITPNB (P48739), PKM2 (P14618), PKP1 (Q13835), PLAA (Q9Y263), PLCD3 (Q8N3E9), PLCG1 (P19174), PLD3 (Q8IV08), PLEC (Q15149), PLEKHB2 (Q96CS7), PLIN3 (O60664), PLOD1 (Q02809), PLOD2 (O00469), PLOD3 (O60568), PLRG1 (O43660), PLS1 (Q14651), PLS3 (P13797), PLSCR3 (Q9NRY6), PLTP (P55058), PLXNA1 (Q9UIW2), PLXNB2 (015031), PLXND1 (Q9Y4D7), PM20D2 (Q8IYS1), PML (P29590), PMM2 (O15305), PMPCA (Q10713), PMPCB (O75439), PMVK (Q15126), PNMA2 (Q9UL42), PNO1 (Q9NRX1), PNP (P00491), PODXL (O00592), POLA1 (P09884), POLD1 (P28340), POLD2 (P49005), POLE3 (Q9NRF9), POLR1A (O95602), POLR1B (Q9H9Y6), POLR1C (015160), POLR1D (Q9Y2S0), POLR1E (Q9GZS1), POLR2A (P24928), POLR2B (P30876), POLR2C (P19387), POLR2E (P19388), POLR2G (P62487), POLR2H (P52434), POLR2J (P52435), POLR2L (P62875), POLR3A (014802), POLR3B (Q9NW08), POLR3C (Q9BUI4), POLR3F (Q9H1D9), POP1 (Q99575), POP4 (O95707), POP5 (Q969H6), POP7 (075817), PPA1 (Q15181), PPA2 (Q9H2U2), PPAT (Q06203), PPCS (Q9HAB8), PPIA (P62937), PPIB (P23284), PPID (Q08752), PPIF (P30405), PPIH (O43447), PPIL1 (Q9Y3C6), PPM1A (P35813), PPM1F (P49593), PPM1G (015355), PPME1 (Q9Y570), PPP1CA (P62136), PPP1CB (P62140), PPP1CC (P36873), PPP1R7 (Q15435), PPP1R8 (Q12972), PPP2CA (P67775), PPP2CB (P62714), PPP2R1A (P30153), PPP2R2A (P63151), PPP2R4 (Q15257), PPP2R5C (Q13362), PPP2R5D (Q14738), PPP2R5E (Q16537), PPP3CA (Q08209), PPP4C (P60510), PPP4R1 (Q8TF05), PPP5C (P53041), PPP6C (O00743), PPP6R3 (Q5H9R7), PPPDE2 (Q6ICB0), PPT1 (P50897), PPWD1 (Q96BP3), PRCP (P42785), PRDX1 (Q06830), PRDX2 (P32119), PRDX3 (P30048), PRDX5 (P30044), PRDX6 (P30041), PREP (P48147), PREPL (Q4J6C6), PRIM1 (P49642), PRIM2 (P49643), PRKACA (P17612), PRKACB (P22694), PRKAG1 (P54619), PRKAR1A (P10644), PRKAR2A (P13861), PRKAR2B (P31323), PRKDC |

(continued)

| |
|---|
| (P78527), PRMT1 (Q99873), PRMT3 (O60678), PRMT5 (014744), PROM1 (O43490), PROSC (O94903), PRPF19 (Q9UMS4), PRPF31 (Q8WWY3), PRPF4 (043172), PRPF4B (Q13523), PRPF8 (Q6P2Q9), PRPS1 (P60891), PRPS2 (P11908), PRPSAP1 (Q14558), PRPSAP2 (O60256), PRSS23 (O95084), PRTFDC1 (Q9NRG1), PSAT1 (Q9Y617), PSMA1 (P25786), PSMA2 (P25787), PSMA3 (P25788), PSMA4 (P25789), PSMA5 (P28066), PSMA6 (P60900), PSMA7 (014818), PSMB1 (P20618), PSMB2 (P49721), PSMB3 (P49720), PSMB4 (P28070), PSMB5 (P28074), PSMB6 (P28072), PSMB7 (Q99436), PSMB8 (P28062), PSMC1 (P62191), PSMC2 (P35998), PSMC3 (P17980), PSMC4 (P43686), PSMC5 (P62195), PSMC6 (P62333), PSMD1 (Q99460), PSMD10 (O75832), PSMD11 (000231), PSMD12 (O00232), PSMD13 (Q9UNM6), PSMD14 (O00487), PSMD2 (Q13200), PSMD3 (O43242), PSMD4 (P55036), PSMD5 (Q16401), PSMD6 (Q15008), PSMD7 (P51665), PSMD8 (P48556), PSMD9 (O00233), PSME1 (Q06323), PSME2 (Q9UL46), PSME3 (P61289), PSME4 (Q14997), PSMF1 (Q92530), PSMG1 (O95456), PSMG2 (Q969U7), PSMG3 (Q9BT73), PSPC1 (Q8WXF1), PSPH (P78330), PTBP1 (P26599), PTGES3 (Q15185), PTGFRN (Q9P2B2), PTGR1 (Q14914), PTGR2 (Q8N8N7), PTK2 (Q05397), PTK7 (Q13308), PTN (P21246), PTP4A1 (Q93096), PTPN1 (P18031), PTPN11 (Q06124), PTPN23 (Q9H3S7), PTPRA (P18433), PTPRG (P23470), PTPRZ1 (P23471), PUF60 (Q9UHX1), PUM1 (Q14671), PURB (Q96QR8), PUS7 (Q96PZ0), PVR (P15151), PWP1 (Q13610), PXDN (Q92626), PXK (Q7Z7A4), PYCR1 (P32322), PYCRL (Q53H96), PYGB (P11216), PYGL (P06737), QARS (P47897), QDPR (P09417), QKI (Q96PU8), QRICH1 (Q2TAL8), QSOX2 (Q6ZRP7), QTRT1 (Q9BXR0), RAB10 (P61026), RAB11A (P62491), RAB11FIP1 (Q6WKZ4), RAB12 (Q6IQ22), RAB13 (P51153), RAB14 (P61106), RAB18 (Q9NP72), RAB1A (P62820), RAB1B (Q9H0U4), RAB21 (Q9UL25), RAB22A (Q9UL26), RAB23 (Q9ULC3), RAB27A (P51159), RAB2A (P61019), RAB34 (Q9BZG1), RAB35 (Q15286), RAB3A (P20336), RAB3GAP1 (Q15042), RAB3GAP2 (Q9H2M9), RAB4A (P20338), RAB5A (P20339), RAB5B (P61020), RAB5C (P51148), RAB6A (P20340), RAB6B (Q9NRW1), RAB7A (P51149), RAB8A (P61006), RAB8B (Q92930), RABAC1 (Q9UI14), RABGAP1 (Q9Y3P9), RABGGTA (Q92696), RABGGTB (P53611), RABIF (P47224), RAC1 (P63000), RAD1 (060671), RAD50 (Q92878), RAE1 (P78406), RAI14 (Q9P0K7), RALA (P11233), RALB (P11234), RALY (Q9UKM9), RAN (P62826), RANBP1 (P43487), RANBP2 (P49792), RANBP6 (O60518), RANBP9 (Q96S59), RANGAP1 (P46060), RAP1A (P62834), RAP1B (P61224), RAP1GDS1 (P52306), RAP2B (P61225), RARS (P54136), RASA1 (P20936), RBBP4 (Q09028), RBBP5 (Q15291), RBBP7 (Q16576), RBBP9 (O75884), RBM12 (Q9NTZ6), RBM15 (Q96T37), RBM17 (Q96I25), RBM22 (Q9NW64), RBM4 (Q9BWF3), RBMX (P38159), RBP1 (P09455), RBPJ (Q06330), RBX1 (P62877), RCC1 (P18754), RCC2 (Q9P258), RCL (O43598), RDX (P35241), RECQL (P46063), REEP5 (Q00765), REEP6 (Q96HR9), REPS1 (Q96D71), RFC4 (P35249), RFC5 (P40937), RFTN1 (Q14699), RHEB (Q15382), RHOA (P61586), RHOB (P62745), RHOC (P08134), RHOF (Q9HBH0), RHOG (P84095), RIC8A (Q9NPQ8), RMND5A (Q9H871), RNASEH2A (O75792), RNASEH2C (Q8TDP1), RNF123 (Q5XPI4), RNF20 |

(continued)

| |
|---|
| (Q5VTR2), RNF213 (Q63HN8), RNF7 (Q9UBF6), RNGTT (O60942), RNH1 (P13489), RNMT (O43148), RNPEP (Q9H4A4), ROBLD3 (Q9Y2Q5), ROCK1 (Q13464), ROCK2 (075116), ROR1 (Q01973), RP2 (O75695), RPA1 (P27694), RPA2 (P15927), RPA3 (P35244), RPE (Q96AT9), RPF2 (Q9H7B2), RPL10 (P27635), RPL10A (P62906), RPL11 (P62913), RPL12 (P30050), RPL13 (P26373), RPL13A (P40429), RPL14 (P50914), RPL15 (P61313), RPL17 (P18621), RPL18 (Q07020), RPL18A (Q02543), RPL19 (P84098), RPL21 (P46778), RPL22 (P35268), RPL22L1 (Q6P5R6), RPL23 (P62829), RPL23A (P62750), RPL24 (P83731), RPL26 (P61254), RPL27 (P61353), RPL27A (P46776), RPL28 (P46779), RPL3 (P39023), RPL30 (P62888), RPL31 (P62899), RPL32 (P62910), RPL34 (P49207), RPL35 (P42766), RPL35A (P18077), RPL36 (Q9Y3U8), RPL36A (P83881), RPL36AL (Q969Q0), RPL37A (P61513), RPL38 (P63173), RPL4 (P36578), RPL5 (P46777), RPL6 (Q02878), RPL7 (P18124), RPL7A (P62424), RPL8 (P62917), RPL9 (P32969), RPLPO (P05388), RPLP1 (P05386), RPLP2 (P05387), RPP30 (P78346), RPP40 (075818), RPRD1A (Q96P16), RPS10 (P46783), RPS11 (P62280), RPS12 (P25398), RPS13 (P62277), RPS14 (P62263), RPS15 (P62841), RPS15A (P62244), RPS16 (P62249), RPS17 (P08708), RPS18 (P62269), RPS19 (P39019), RPS2 (P15880), RPS20 (P60866), RPS21 (P63220), RPS23 (P62266), RPS24 (P62847), RPS25 (P62851), RPS26 (P62854), RPS27 (P42677), RPS27A (P62979), RPS27L (Q71UM5), RPS28 (P62857), RPS29 (P62273), RPS3 (P23396), RPS3A (P61247), RPS4X (P62701), RPS4Y1 (P22090), RPS5 (P46782), RPS6 (P62753), RPS6KA3 (P51812), RPS7 (P62081), RPS8 (P62241), RPS9 (P46781), RPSA (P08865), RQCD1 (Q92600), RRAGA (Q7L523), RRAS (P10301), RRAS2 (P62070), RRBP1 (Q9P2E9), RRM1 (P23921), RRM2 (P31350), RRM2B (Q7LG56), RRP12 (Q5JTH9), RRP9 (043818), RSL1D1 (076021), RSU1 (Q15404), RTCD1 (O00442), RTN3 (095197), RTN4 (Q9NQC3), RUVBL1 (Q9Y265), RUVBL2 (Q9Y230), RWDD2B (P57060), S100A10 (P60903), S100A11 (P31949), S100A13 (Q99584), S100A16 (Q96FQ6), S100A4 (P26447), S100A6 (P06703), S100A8 (P05109), SAAL1 (Q96ER3), SACS (Q9NZJ4), SAE1 (Q9UBE0), SAFB2 (Q14151), SAMHD1 (Q9Y3Z3), SAP18 (O00422), SAR1A (Q9NR31), SARM1 (Q6SZW1), SARS (P49591), SART3 (Q15020), SBDS (Q9Y3A5), SBF1 (O95248), SCARB1 (Q8WTV0), SCARB2 (Q14108), SCFD1 (Q8WVM8), SCLY (Q96I15), SCP2 (P22307), SCPEP1 (Q9HB40), SCRG1 (075711), SCRIB (Q14160), SCRN1 (Q12765), SCRN2 (Q96FV2), SCYL1 (Q96KG9), SCYL2 (Q6P3W7), SDC1 (P18827), SDC2 (P34741), SDCBP (O00560), SDF4 (Q9BRK5), SDHA (P31040), SDK1 (Q7Z5N4), SDSL (Q96GA7), SEC11A (P67812), SEC13 (P55735), SEC22B (O75396), SEC23A (Q15436), SEC23B (Q15437), SEC23IP (Q9Y6Y8), SEC24A (O95486), SEC24B (O95487), SEC24C (P53992), SEC24D (O94855), SEC31A (O94979), SEH1L (Q96EE3), SELH (Q8IZQ5), SEMA3A (Q14563), SEPSECS (Q9HD40), 40787 (Q9NVA2), 37500 (Q15019), 38596 (Q99719), 39326 (Q16181), 39692 (Q92599), 40057 (Q9UHD8), SERBP1 (Q8NC51), SERPINA1 (P01009), SERPINA3 (P01011), SERPINA7 (P05543), SERPINB6 (P35237), SERPINB8 (P50452), SERPINE1 (P05121), SERPINE2 (P07093), SERPING1 (P05155), SERPINH1 (P50454), |

(continued)

| |
|---|
| SETD3 (Q86TU7), SETD7 (Q8WTS6), SF3A1 (Q15459), SF3A2 (Q15428), SF3A3 (Q12874), SF3B1 (O75533), SF3B14 (Q9Y3B4), SF3B2 (Q13435), SF3B3 (Q15393), SF3B4 (Q15427), SF3B5 (Q9BWJ5), SFPQ (P23246), SFRP4 (Q6FHJ7), SGTA (O43765), SH3BP4 (Q9P0V3), SH3GL1 (Q99961), SH3GLB1 (Q9Y371), SHBG (P04278), SHC1 (P29353), SHMT1 (P34896), SHMT2 (P34897), SHOC2 (Q9UQ13), SHPK (Q9UHJ6), SKIV2L (Q15477), SKIV2L2 (P42285), SKP1 (P63208), SLC16A1 (P53985), SLC1A3 (P43003), SLC1A5 (Q15758), SLC29A1 (Q99808), SLC2A1 (P11166), SLC31A1 (015431), SLC3A2 (P08195), SLC44A2 (Q8IWA5), SLC5A3 (P53794), SLC7A5 (Q01650), SLC9A3R1 (014745), SLC9A3R2 (Q15599), SLIRP (Q9GZT3), SMAD4 (Q13485), SMARCA4 (P51532), SMARCA5 (O60264), SMARCC1 (Q92922), SMARCC2 (Q8TAQ2), SMARCD1 (Q96GM5), SMARCD2 (Q92925), SMARCE1 (Q969G3), SMC1A (Q14683), SMC2 (O95347), SMC3 (Q9UQE7), SMC4 (Q9NTJ3), SMC5 (Q8IY18), SMC6 (Q96SB8), SMCHD1 (A6NHR9), SMEK1 (Q6IN85), SMS (P52788), SMU1 (Q2TAY7), SMYD5 (Q6GMV2), SNAP23 (000161), SNAPIN (O95295), SND1 (Q7KZF4), SNF8 (Q96H20), SNRNP200 (O75643), SNRNP40 (Q96DI7), SNRPA1 (P09661), SNRPB (P14678), SNRPD1 (P62314), SNRPD2 (P62316), SNRPD3 (P62318), SNRPE (P62304), SNRPF (P62306), SNRPG (P62308), SNTB1 (Q13884), SNUPN (095149), SNX1 (Q13596), SNX12 (Q9UMY4), SNX17 (Q15036), SNX18 (Q96RF0), SNX2 (O60749), SNX27 (Q96L92), SNX3 (O60493), SNX5 (Q9Y5X3), SNX6 (Q9UNH7), SNX8 (Q9Y5X2), SNX9 (Q9Y5X1), SOD1 (P00441), SORD (Q00796), SORT1 (Q99523), SPAG9 (060271), SPC24 (Q8NBT2), SPC25 (Q9HBM1), SPG21 (Q9NZD8), SPR (P35270), SPRYD4 (Q8WW59), SPTAN1 (Q13813), SPTBN1 (Q01082), SPTBN2 (015020), SRGAP2 (O75044), SRI (P30626), SRM (P19623), SRP14 (P37108), SRP19 (P09132), SRP54 (P61011), SRP68 (Q9UHB9), SRP72 (O76094), SRP9 (P49458), SRPX (P78539), SRPX2 (O60687), SRR (Q9GZT4), SRRT (Q9BXP5), SRSF1 (Q07955), SRSF11 (Q05519), SRSF2 (Q01130), SRSF3 (P84103), SRSF6 (Q13247), SRSF7 (Q16629), SRSF9 (Q13242), SRXN1 (Q9BYN0), SSB (P05455), SSBP1 (Q04837), SSRP1 (Q08945), SSSCA1 (O60232), ST13 (P50502), STAG2 (Q8N3U4), STAM (Q92783), STAMBP (O95630), STAT1 (P42224), STAT3 (P40763), STIP1 (P31948), STK24 (Q9Y6E0), STK25 (O00506), STK38L (Q9Y2H1), STOM (P27105), STON2 (Q8WXE9), STRAP (Q9Y3F4), STUB1 (Q9UNE7), STX12 (Q86Y82), STX4 (Q12846), STX5 (Q13190), STX7 (015400), STXBP1 (P61764), STXBP3 (000186), STYX (Q8WUJ0), SUB1 (P53999), SUDS3 (Q9H7L9), SUGT1 (Q9Y2Z0), SUMO1 (P63165), SUPT16H (Q9Y5B9), SUPT4H1 (P63272), SUPT5H (O00267), SUPT6H (Q7KZ85), SVEP1 (Q4LDE5), SWAP70 (Q9UH65), SYMPK (Q92797), SYNCRIP (O60506), SYNE1 (Q8NF91), SYNE2 (Q8WXH0), SYNGR2 (O43760), SYNJ2BP (P57105), TAB1 (Q15750), TAF9 (Q9Y3D8), TAF9 (Q16594), TAGLN (Q01995), TAGLN2 (P37802), TALDO1 (P37837), TARDBP (Q13148), TARS (P26639), TATDN1 (Q6P1N9), TAX1BP3 (O14907), TBC1D13 (Q9NVG8), TBC1D15 (Q8TC07), TBC1D23 (Q9NUY8), TBC1D24 (Q9ULP9), TBC1D4 (O60343), TBC1D9B (Q66K14), TBCA (O75347), TBCB (Q99426), TBCD (Q9BTW9), TBCE (Q15813), TBL1XR1 (Q9BZK7), TCEA1 (P23193), TCEB1 (Q15369), TCEB2 |

(continued)

| |
|---|
| (Q15370), TCERG1 (O14776), TCP1 (P17987), TDP2 (O95551), TEP1 (Q99973), TEX10 (Q9NXF1), TF (P02787), TFCP2 (Q12800), TFG (Q92734), TFRC (P02786), TGFB1 (P01137), TGFB2 (P61812), TGFBI (Q15582), TGM1 (P22735), TH1L (Q8IXH7), THBS1 (P07996), THBS3 (P49746), THG1L (Q9NWX6), THOC2 (Q8NI27), THOC3 (Q96J01), THOC5 (Q13769), THOC6 (Q86W42), THOC7 (Q6I9Y2), THOP1 (P52888), THUMPD1 (Q9NXG2), THY1 (P04216), THYN1 (Q9P016), TIA1 (P31483), TIGAR (Q9NQ88), TIMM13 (Q9Y5L4), TIMM50 (Q3ZCQ8), TIMM8B (Q9Y5J9), TIMM9 (Q9Y5J7), TIMP1 (P01033), TIPRL (O75663), TKT (P29401), TLN1 (Q9Y490), TLN2 (Q9Y4G6), TM9SF2 (Q99805), TM9SF3 (Q9HD45), TMED10 (P49755), TMED2 (Q15363), TMED7 (Q9Y3B3), TMED9 (Q9BVK6), TMEM167A (Q8TBQ9), TMEM2 (Q9UHN6), TMEM50B (P56557), TMEM87A (Q8NBN3), TMOD3 (Q9NYL9), TNC (P24821), TNPO1 (Q92973), TNPO2 (014787), TNPO3 (Q9Y5L0), TOLLIP (Q9H0E2), TOMM20 (Q15388), TOMM22 (Q9NS69), TOMM34 (Q15785), TOMM5 (Q8N4H5), TOMM70A (O94826), TOP1 (P11387), TOP2B (Q02880), TOR1B (014657), TP53BP1 (Q12888), TP53RK (Q96S44), TPI1 (P60174), TPM3 (P06753), TPM3L (A6NL28), TPM4 (P67936), TPMT (P51580), TPP1 (014773), TPP2 (P29144), TPR (P12270), TPRG1L (Q5T0D9), TPRKB (Q9Y3C4), TPT1 (P13693), TRAF2 (Q12933), TRAP1 (Q12931), TRAPPC1 (Q9Y5R8), TRAPPC2L (Q9UL33), TRAPPC3 (043617), TRAPPC4 (Q9Y296), TRAPPC5 (Q8IUR0), TRAPPC6A (O75865), TRAPPC6B (Q86SZ2), TRIM22 (Q8IYM9), TRIM25 (Q14258), TRIM28 (Q13263), TRIP12 (Q14669), TRIP13 (Q15645), TRIP6 (Q15654), TRMT1 (Q9NXH9), TRMT112 (Q9UI30), TRMT5 (Q32P41), TRMT6 (Q9UJA5), TRMT61A (Q96FX7), TRNT1 (Q96Q11), TROVE2 (P10155), TRRAP (Q9Y4A5), TSG101 (Q99816), TSKU (Q8WUA8), TSPAN14 (Q8NG11), TSPAN4 (014817), TSPAN5 (P62079), TSPAN6 (O43657), TSPAN9 (O75954), TSSC1 (Q53HC9), TSTA3 (Q13630), TTC1 (Q99614), TTC37 (Q6PGP7), TTC38 (Q5R3I4), TTC5 (Q8N0Z6), TTC9C (Q8N5M4), TTL (Q8NG68), TTLL12 (Q14166), TTN (Q8WZ42), TTR (P02766), TTYH1 (Q9H313), TTYH2 (Q9BSA4), TTYH3 (Q9C0H2), TUBA1B (P68363), TUBA1C (Q9BQE3), TUBB (P07437), TUBB2A (Q13885), TUBB2B (Q9BVA1), TUBB2C (P68371), TUBB3 (Q13509), TUBB4 (P04350), TUBB6 (Q9BUF5), TUBG1 (P23258), TUBGCP2 (Q9BSJ2), TUBGCP3 (Q96CW5), TWF1 (Q12792), TWF2 (Q6IBS0), TXN (P10599), TXNDC17 (Q9BRA2), TXNDC9 (014530), TXNL1 (O43396), TXNL4B (Q9NX01), TXNRD1 (Q16881), TYMS (P04818), U2AF1 (Q01081), U2AF2 (P26368), UAP1 (Q16222), UBA1 (P22314), UBA2 (Q9UBT2), UBA3 (Q8TBC4), UBA5 (Q9GZZ9), UBA6 (A0AVT1), UBE2D1 (P51668), UBE2D3 (P61077), UBE2E1 (P51965), UBE2G2 (P60604), UBE2I (P63279), UBE2J2 (Q8N2K1), UBE2K (P61086), UBE2L3 (P68036), UBE2M (P61081), UBE2N (P61088), UBE2O (Q9C0C9), UBE2V1 (Q13404), UBE2V2 (Q15819), UBE2Z (Q9H832), UBE3A (Q05086), UBE4A (Q14139), UBE4B (095155), UBL3 (095164), UBL4A (P11441), UBL5 (Q9BZL1), UBR1 (Q8IWV7), UBR4 (Q5T4S7), UBTD1 (Q9HAC8), UBXN1 (Q04323), UCHL1 (P09936), UCHL3 (P15374), UCHL5 (Q9Y5K5), UCK2 (Q9BZX2), UFC1 (Q9Y3C8), UFD1L (Q92890), UFSP2 (Q9NUQ7), UGDH (060701), UGP2 (Q16851), UMPS (P11172), UNC119B |
| (A6NIH7), UNC45A (Q9H3U1), UPF1 (Q92900), UPP1 (Q16831), UROD (P06132), UROS (P10746), USO1 (O60763), USP10 (Q14694), USP11 (P51784), USP14 (P54578), USP15 (Q9Y4E8), USP24 (Q9UPU5), USP39 (Q53GS9), USP5 (P45974), USP7 (Q93009), USP9X (Q93008), UTP15 (Q8TED0), UXS1 (Q8NBZ7), UXT (Q9UBK9), VAC14 (Q08AM6), VAMP3 (Q15836), VAMP5 (095183), VAPA (Q9P0L0), VAPB (O95292), VARS (P26640), VASN (Q6EMK4), VASP (P50552), VAT1 (Q99536), VAV2 (P52735), VBP1 (P61758), VCAN (P13611), VCL (P18206), VCP (P55072), VIM (P08670), VPRBP (Q9Y4B6), VPS11 (Q9H270), VPS13C (Q709C8), VPS16 (Q9H269), VPS18 (Q9P253), VPS24 (Q9Y3E7), VPS25 (Q9BRG1), VPS26A (O75436), VPS26B (Q4G0F5), VPS28 (Q9UK41), VPS29 (Q9UBQ0), VPS33A (Q96AX1), VPS33B (Q9H267), VPS35 (Q96QK1), VPS36 (Q86VN1), VPS37B (Q9H9H4), VPS39 (Q96JC1), VPS45 (Q9NRW7), VPS4A (Q9UN37), VPS4B (075351), VPS53 (Q5VIR6), VRK1 (Q99986), VTA1 (Q9NP79), VWA1 (Q6PCB0), VWA5A (O00534), WARS (P23381), WASF1 (Q92558), WASL (000401), WDFY1 (Q8IWB7), WDR1 (O75083), WDR11 (Q9BZH6), WDR12 (Q9GZL7), WDR18 (Q9BV38), WDR26 (Q9H7D7), WDR33 (Q9C0J8), WDR4 (P57081), WDR43 (Q15061), WDR45L (Q5MNZ6), WDR48 (Q8TAF3), WDR5 (P61964), WDR54 (Q9H977), WDR55 (Q9H6Y2), WDR59 (Q6PJI9), WDR6 (Q9NNW5), WDR61 (Q9GZS3), WDR73 (Q6P4I2), WDR77 (Q9BQA1), WDR82 (Q6UXN9), WDR91 (A4D1P6), WDR92 (Q96MX6), WNK1 (Q9H4A3), XPNPEP1 (Q9NQW7), XPO1 (014980), XPO4 (Q9C0E2), XPO5 (Q9HAV4), XPO6 (Q96QU8), XPO7 (Q9UIA9), XPOT (O43592), XRCC1 (P18887), XRCC5 (P13010), XRCC6 (P12956), XRN2 (Q9H0D6), YARS (P54577), YBX1 (P67809), YEATS4 (095619), YES1 (P07947), YIPF4 (Q9BSR8), YKT6 (015498), YPEL5 (P62699), YRDC (Q86U90), YTHDF2 (Q9Y5A9), YWHAB (P31946), YWHAE (P62258), YWHAG (P61981), YWHAH (Q04917), YWHAQ (P27348), YWHAZ (P63104), ZC3HAV1L (Q96H79), ZCCHC3 (Q9NUD5), ZER1 (Q7Z7L7), ZFPL1 (095159), ZFR (Q96KR1), ZMAT2 (Q96NC0), ZNF259 (075312), ZW10 (O43264), ZWILCH (Q9H900), ZYG11B (Q9C0D3), ZYX (Q15942), ZZEF1 (043149). |

**Table 19:** 100 most abundant proteins (name and SwissProt accession number) observed in CTX0E03 exosomes

| **Identified proteins** | **Accession number** |
|---|---|
| Actin, cytoplasmic 2 | P63261 |
| Glyceraldehyde-3-phosphate dehydrogenase | P04406 |
| Histone H4 | P62805 |
| Pyruvate kinase isozymes M1/M2 | P14618 |
| Alpha-enolase | P06733 |
| Heat shock protein HSP 90-beta | P08238 |
| Ubiquitin-40S ribosomal protein S27a | P62979 |
| Heat shock cognate 71 kDa protein | P11142 |
| Haptoglobin | P00738 |
| Heat shock protein HSP 90-alpha | P07900 |
| Phosphoglycerate kinase 1 | P00558 |
| Actin, alpha cardiac muscle 1 | P68032 |
| 40S ribosomal protein S3 | P23396 |
| Elongation factor 1-alpha 1 | P68104 |
| GTP-binding nuclear protein Ran | P62826 |
| Histone H2B type 1-M | Q99879 |
| Peptidyl-prolyl cis-trans isomerase A | P62937 |
| Profilin-1 | P07737 |
| Elongation factor 2 | P13639 |
| Fatty acid synthase | P49327 |
| Tubulin beta-2C chain | P68371 |
| Tubulin alpha-1B chain | P68363 |
| Tubulin beta chain | P07437 |
| 40S ribosomal protein S11 | P62280 |
| Eukaryotic initiation factor 4A-I | P60842 |
| T-complex protein 1 subunit theta | P50990 |
| 14-3-3 protein theta | P27348 |
| 40S ribosomal protein S18 | P62269 |
| Tubulin beta-3 chain | Q13509 |
| T-complex protein 1 subunit beta | P78371 |
| 40S ribosomal protein S16 | P62249 |
| Heat shock 70 kDa protein 1A/1 B | P08107 |
| Histone H3.2 | Q71DI3 |
| Transketolase | P29401 |
| 40S ribosomal protein SA | P08865 |
| Clusterin | P10909 |
| Fatty acid-binding protein, brain | O15540 |

(continued)

| Identified proteins | Accession number |
|---|---|
| Hemopexin | P02790 |
| T-complex protein 1 subunit gamma | P49368 |
| Tubulin beta-2B chain | Q9BVA1 |
| Adenosylhomocysteinase | P23526 |
| T-complex protein 1 subunit eta | Q99832 |
| 40S ribosomal protein S15a | P62244 |
| T-complex protein 1 subunit delta | P50991 |
| Vimentin | P08670 |
| Guanine nucleotide-binding protein subunit beta-2-like 1 | P63244 |
| Dihydropyrimidinase-related protein 3 | Q14195 |
| Elongation factor 1-gamma | P26641 |
| Fascin | Q16658 |
| Creatine kinase B-type | P12277 |
| X-ray repair cross-complementing protein 5 | P13010 |
| 40S ribosomal protein S2 | P15880 |
| Histone H2A type 2-A | Q6FI13 |
| 40S ribosomal protein S4, X isoform | P62701 |
| 14-3-3 protein zeta/delta | P63104 |
| Heterogeneous nuclear ribonucleoprotein A1 | P09651 |
| CD81 antigen | P60033 |
| Keratin, type I cytoskeletal 14 | P02533 |
| ATP-citrate synthase | P53396 |
| 40S ribosomal protein S9 | P46781 |
| Transgelin-2 | P37802 |
| Fructose-bisphosphate aldolase A | P04075 |
| Ubiquitin-like modifier-activating enzyme 1 | P22314 |
| Peroxiredoxin-1 | Q06830 |
| 40S ribosomal protein S5 | P46782 |
| T-complex protein 1 subunit epsilon | P48643 |
| 60S ribosomal protein L30 | P62888 |
| T-complex protein 1 subunit alpha | P17987 |
| 60S ribosomal protein L12 | P30050 |
| Cofilin-1 | P23528 |
| Heterogeneous nuclear ribonucleoproteins A2/B1 | P22626 |
| Eukaryotic translation initiation factor 5A-1 | P63241 |
| Phosphoglycerate mutase 1 | P18669 |
| Clathrin heavy chain 1 | Q00610 |
| Dihydropyrimidinase-related protein 2 | Q16555 |

(continued)

| Identified proteins | Accession number |
|---|---|
| 60S ribosomal protein L35a | P18077 |
| T-complex protein 1 subunit zeta | P40227 |
| Carbonyl reductase [NADPH] 1 | P16152 |
| 40S ribosomal protein S3a | P61247 |
| Ferritin heavy chain | P02794 |
| Annexin A2 | P07355 |
| Myosin light polypeptide 6 | P60660 |
| Major vault protein | Q14764 |
| Heterogeneous nuclear ribonucleoprotein D0 | Q14103 |
| 60S acidic ribosomal protein P0 | P05388 |
| X-ray repair cross-complementing protein 6 | P12956 |
| 40S ribosomal protein S20 | P60866 |
| Protein arginine N-methyltransferase 1 | Q99873 |
| 40S ribosomal protein S10 | P46783 |
| Transaldolase | P37837 |
| Histone H2B type 1- | P23527 |
| Triosephosphate isomerase | P60174 |
| Protein S100-A6 | P06703 |
| 40S ribosomal protein S17 | P08708 |
| CD9 antigen | P21926 |
| Filamin-A | P21333 |
| Peptidyl-prolyl cis-trans isomerase FKBP4 | Q02790 |
| Programmed cell death 6-interacting protein | Q8WUM4 |
| Glutathione S-transferase P | P09211 |
| 14-3-3 protein epsilon | P62258 |

Microvesicles

2940 proteins were identified by Mass spectrometry in Microvesicles isolated from the initial stages of an Integra culture (week 2) and purified by centrifugation at 10,000 x g. The gene names and corresponding SWISSPROT accession numbers (in brackets) of all 2940 proteins are listed in Table 20 (in alphabetical order of gene name) and the 100 most abundant proteins are listed in Table 21, in order of decreasing abundance.

**Table 20:** Gene names and SWISSPROT accession numbers of all 2940 proteins identified in CTX0E03 microvesicles (listed in alphabetical order of gene name).

| |
|---|
| A1BG (P04217), AACS (Q86V21), AAMP (Q13685), AARS (P49588), AARSD1 (Q9BTE6), AASDHPPT (Q9NRN7), ABCA3 (Q99758), ABCC1 (P33527), ABCC4 (015439), ABCE1 (P61221), ABCF1 (Q8NE71), ABCF2 (Q9UG63), ABCF3 (Q9NUQ8), ABHD14B (Q96IU4), ABI1 (Q8IZP0), ABR (Q12979), ACAA1 (P09110), ACAA2 (P42765), ACACA (Q13085), ACADM (P11310), ACADVL (P49748), ACAT1 (P24752), ACAT2 (Q9BWD1), ACBD6 (Q9BR61), ACBD7 (Q8N6N7), ACLY (P53396), ACO1 (P21399), ACO2 (Q99798), ACOT1 (Q86TX2), ACOT13 (Q9NPJ3), ACOT7 (000154), ACOX1 (Q15067), ACOX3 (015254), ACP1 (P24666), ACSL1 (P33121), ACSL3 (O95573), ACSL4 (O60488), ACSS2 (Q9NR19), ACTC1 (P68032), ACTG1 (P63261), ACTL6A (096019), ACTN1 (P12814), ACTN4 (O43707), ACTR10 (Q9NZ32), ACTR1A (P61163), ACTR1B (P42025), ACTR2 (P61160), ACTR3 (P61158), ACY1 (Q03154), ADAM10 (014672), ADAM9 (Q13443), ADAMTS15 (Q8TE58), ADAMTS16 (Q8TE57), ADAR (P55265), ADD1 (P35611), ADD3 (Q9UEY8), ADH5 (P11766), ADK (P55263), ADO (Q96SZ5), ADPRH (P54922), ADRBK1 (P25098), ADRM1 (Q16186), ADSL (P30566), ADSS (P30520), AEBP1 (Q8IUX7), AFM (P43652), AGL (P35573), AGPS (000116), AGRN (O00468), AHCY (P23526), AHCYL1 (O43865), AHNAK (Q09666), AHNAK2 (Q8IVF2), AHSA1 (O95433), AHSG (P02765), AIDA (Q96BJ3), AIFM1 (095831), AIMP1 (Q12904), AIMP2 (Q13155), AIP (000170), AK1 (P00568), AK2 (P54819), AK3 (Q9UIJ7), AK4 (P27144), AKAP12 (Q02952), AKAP9 (Q99996), AKR1A1 (P14550), AKR1B1 (P15121), AKR1C1 (Q04828), AKR7A2 (O43488), AKR7A3 (095154), AKT1 (P31749), ALCAM (Q13740), ALDH16A1 (Q8IZ83), ALDH18A1 (P54886), ALDH2 (P05091), ALDH3A1 (P30838), ALDH7A1 (P49419), ALDH9A1 (P49189), ALDOA (P04075), ALDOC (P09972), ALKBH2 (Q6NS38), ALOX12B (O75342), AMDHD2 (Q9Y303), AMPD2 (Q01433), ANAPC1 (Q9H1A4), ANAPC4 (Q9UJX5), ANAPC5 (Q9UJX4), ANAPC7 (Q9UJX3), ANKFY1 (Q9P2R3), ANKRD17 (075179), ANKRD28 (015084), ANKRD52 (Q8NB46), ANP32A (P39687), ANP32B (Q92688), ANP32E (Q9BTT0), ANXA1 (P04083), ANXA11 (P50995), ANXA2 (P07355), ANXA3 (P12429), ANXA4 (P09525), |
| ANXA5 (P08758), ANXA6 (P08133), ANXA7 (P20073), AP1B1 (Q10567), AP1G1 (O43747), AP1M1 (Q9BXS5), AP1S2 (P56377), AP2A1 (O95782), AP2A2 (O94973), AP2B1 (P63010), AP2M1 (Q96CW1), AP2S1 (P53680), AP3B1 (O00203), AP3D1 (014617), AP3M1 (Q9Y2T2), AP3S1 (Q92572), AP4S1 (Q9Y587), APEH (P13798), APEX1 (P27695), API5 (Q9BZZ5), APIP (Q96GX9), APMAP (Q9HDC9), APOA2 (P02652), APOBEC3C (Q9NRW3), APOH (P02749), APOL2 (Q9BQE5), APPL1 (Q9UKG1), APRT (P07741), AQR (O60306), ARAF (P10398), ARCN1 (P48444), ARF1 (P84077), ARF4 (P18085), ARF6 (P62330), ARFGAP2 (Q8N6H7), ARFIP1 (P53367), ARFIP2 (P53365), ARG1 (P05089), ARHGAP1 (Q07960), ARHGAP5 (Q13017), ARHGDIA (P52565), ARHGEF1 (Q92888), ARHGEF10 (015013), ARHGEF6 (Q15052), ARHGEF7 (Q14155), ARIH1 (Q9Y4X5), ARIH2 (O95376), ARL1 (P40616), ARL2 (P36404), ARL3 (P36405), ARL6IP1 (Q15041), ARL8A (Q96BM9), ARL8B (Q9NVJ2), ARMC10 (Q8N2F6), ARMC6 (Q6NXE6), ARMC8 (Q8IUR7), ARMC9 (Q7Z3E5), ARPC1A (Q92747), ARPC1 B (015143), ARPC2 (015144), ARPC3 (015145), ARPC4 (P59998), ARPC5 (015511), ARPC5L (Q9BPX5), ASAH1 (Q13510), ASCC1 (Q8N9N2), ASCC3 (Q8N3C0), ASMTL (095671), ASNA1 (043681), ASNS (P08243), ASPSCR1 (Q9BZE9), ASS1 (P00966), ATAD3A (Q9NVI7), ATE1 (O95260), ATG101 (Q9BSB4), ATG16L1 (Q676U5), ATG3 (Q9NT62), ATG4B (Q9Y4P1), ATG7 (O95352), ATIC (P31939), ATL3 (Q6DD88), ATM (Q13315), ATOX1 (O00244), ATP1A1 (P05023), ATP1B1 (P05026), ATP1B3 (P54709), ATP2A2 (P16615), ATP2B1 (P20020), ATP2B4 (P23634), ATP5A1 (P25705), ATP5B (P06576), ATP5C1 (P36542), ATP5E (P56381), ATP5F1 (P24539), ATP5H (O75947), ATP5I (P56385), ATP5L (O75964), ATP5O (P48047), ATP6AP1 (Q15904), ATP6AP2 (O75787), ATP6V0A1 (Q93050), ATP6V0D1 (P61421), ATP6V1A (P38606), ATP6V1B2 (P21281), ATP6V1C1 (P21283), ATP6V1D (Q9Y5K8), ATP6V1E1 (P36543), ATP6V1G1 (O75348), ATP6V1H (Q9UI12), ATR (Q13535), ATRN (O75882), ATXN10 (Q9UBB4), B2M (P61769), B3GAT3 (O94766), B3GNT1 (O43505), BAG2 (095816), BAG5 (Q9UL15), BAIAP2 (Q9UQB8), BANF1 (075531), BAT1 (Q13838), BAT3 (P46379), BCAM (P50895), BCAS2 (O75934), BCAT1 (P54687), BCCIP (Q9P287), BCL2L12 (Q9HB09), BDH2 (Q9BUT1), BICD2 (Q8TD16), BLMH (Q13867), BLVRA (P53004), BLVRB (P30043), BMP1 (P13497), BOLA2 (Q9H3K6), BOP1 (Q14137), BPGM (P07738), BPNT1 (095861), BRCC3 (P46736), BRE (Q9NXR7), BRIX1 (Q8TDN6), BROX (Q5VW32), BRP16L (POCB43), BSG (P35613), BST1 (Q10588), BTAF1 (014981), BUB3 (O43684), BUD31 (P41223), BYSL (Q13895), BZW1 (Q7L1Q6), BZW2 (Q9Y6E2), C10orf119 (Q9BTE3), C10orf58 (Q9BRX8), C10orf76 (Q5T2E6), C11orf54 (Q9H0W9), C11orf68 (Q9H3H3), C12orf10 (Q9HB07), C12orf57 (Q99622), C14orf149 (Q96EM0), C14orf166 (Q9Y224), C14orf21 (Q86U38), C15orf58 (Q6ZNW5), C16orf13 (Q96S19), C16orf61 (Q9NRP2), C16orf80 (Q9Y6A4), C18orf21 (Q32NC0), C18orf8 (Q96DM3), C1orf123 (Q9NWV4), C1orf128 (Q9GZP4), C1orf57 (Q9BSD7), C20orf11 (Q9NWU2), C20orf4 (Q9Y312), C21orf33 (P30042), C21orf59 (P57076), C22orf28 (Q9Y3I0), C3orf10 (Q8WUW1), C3orf26 (Q9BQ75), C3orf75 (Q0PNE2), C4orf27 (Q9NWY4), C4orf41 (Q7Z392), C4orf43 (Q96EY4), |

(continued)

| |
|---|
| C5orf33 (Q4G0N4), C6orf211 (Q9H993), C7orf28B (P86790), C7orf50 (Q9BRJ6), C7orf59 (Q0VGL1), C8orf33 (Q9H7E9), C9orf142 (Q9BUH6), C9orf23 (Q8N5L8), C9orf41 (Q8N4J0), C9orf64 (Q5T6V5), CA11 (O75493), CA12 (O43570), CA2 (P00918), CAB39 (Q9Y376), CACNA2D1 (P54289), CACYBP (Q9HB71), CAD (P27708), CADM1 (Q9BY67), CADM4 (Q8NFZ8), CALB1 (P05937), CALD1 (Q05682), CALM1 (P62158), CALR (P27797), CALU (O43852), CAMK1 (Q14012), CAMK2D (Q13557), CAMKV (Q8NCB2), CAND1 (Q86VP6), CANX (P27824), CAP1 (Q01518), CAPN1 (P07384), CAPN2 (P17655), CAPN5 (015484), CAPN7 (Q9Y6W3), CAPNS1 (P04632), CAPRIN1 (Q14444), CAPS (Q13938), CAPZA1 (P52907), CAPZA2 (P47755), CAPZB (P47756), CARHSP1 (Q9Y2V2), CARKD (Q8IW45), CARM1 (Q86X55), CARS (P49589), CASK (014936), CASP14 (P31944), CASP3 (P42574), CASP7 (P55210), CAT (P04040), CBFB (Q13951), CBR1 (P16152), CBR3 (O75828), CBS (P35520), CBX1 (P83916), CBX3 (Q13185), CBX5 (P45973), CC2D1A (Q6P1N0), CCAR1 (Q8IX12), CCBL2 (Q6YP21), CCDC102B (Q68D86), CCDC22 (O60826), CCDC25 (Q86WR0), CCDC93 (Q567U6), CCND2 (P30279), CCNY (Q8ND76), CCT2 (P78371), CCT3 (P49368), CCT4 (P50991), CCT5 (P48643), CCT6A (P40227), CCT7 (Q99832), CCT8 (P50990), CD109 (Q6YHK3), CD151 (P48509), CD276 (Q5ZPR3), CD44 (P16070), CD46 (P15529), CD47 (Q08722), CD58 (P19256), CD59 (P13987), CD63 (P08962), CD81 (P60033), CD9 (P21926), CD97 (P48960), CD99 (P14209), CDC123 (O75794), CDC16 (Q13042), CDC23 (Q9UJX2), CDC34 (P49427), CDC37 (Q16543), CDC40 (O60508), CDC42 (P60953), CDC42BPB (Q9Y5S2), CDC5L (Q99459), CDCP1 (Q9H5V8), CDH2 (P19022), CDK1 (P06493), CDK2 (P24941), CDK4 (P11802), CDK5 (Q00535), CDK5RAP3 (Q96JB5), CDK7 (P50613), CDKN2A (P42771), CDKN2AIP (Q9NXV6), CECR5 (Q9BXW7), CELF1 (Q92879), CELSR1 (Q9NYQ6), CELSR2 (Q9HCU4), CFL1 (P23528), CFL2 (Q9Y281), CHCHD3 (Q9NX63), CHD4 (Q14839), CHEK2 (096017), CHERP (Q8IWX8), CHID1 (Q9BWS9), CHMP1A (Q9HD42), CHMP1B (Q7LBR1), CHMP2A (O43633), CHMP4A (Q9BY43), CHMP4B (Q9H444), CHMP5 (Q9NZZ3), CHMP6 (Q96FZ7), CHN1 (P15882), CHORDC1 (Q9UHD1), CHP (Q99653), CHRAC1 (Q9NRG0), CHST3 (Q7LGC8), CIAO1 (076071), CIAPIN1 (Q6FI81), CIRBP (Q14011), CIRH1A (Q969X6), CISD2 (Q8N5K1), CKAP4 (Q07065), CKAP5 (Q14008), CKB (P12277), CLASP1 (Q7Z460), CLIC1 (O00299), CLIC4 (Q9Y696), CLLD6 (Q5W111), CLNS1A (P54105), CLPB (Q9H078), CLTA (P09496), CLTC (Q00610), CLTCL1 (P53675), CLU (P10909), CMBL (Q96DG6), CMC1 (Q7Z7K0), CMPK1 (P30085), CMTM6 (Q9NX76), CNBP (P62633), CNDP2 (Q96KP4), CNN2 (Q99439), CNN3 (Q15417), CNNM3 (Q8NE01), CNOT1 (A5YKK6), CNOT10 (Q9H9A5), CNOT6L (Q96LI5), CNP (P09543), COASY (Q13057), COBRA1 (Q8WX92), COG1 (Q8WTW3), COG3 (Q96JB2), COG4 (Q9H9E3), COG5 (Q9UP83), COG6 (Q9Y2V7), COL11A1 (P12107), COL14A1 (Q05707), COL18A1 (P39060), COL6A1 (P12109), COMMD10 (Q9Y6G5), COMMD2 (Q86X83), COMMD3 (Q9UBI1), COMMD5 (Q9GZQ3), COMMD8 (Q9NX08), COMMD9 (Q9P000), COMT (P21964), COPA (P53621), COPB1 (P53618), COPB2 (P35606), COPE (014579), COPG (Q9Y678), COPG2 (Q9UBF2), COPS2 (P61201), COPS3 (Q9UNS2), |

(continued)

| |
|---|
| COPS4 (Q9BT78), COPS5 (Q92905), COPS6 (Q7L5N1), COPS7A (Q9UBW8), COPS7B (Q9H9Q2), COPS8 (Q99627), CORO1B (Q9BR76), CORO1C (Q9ULV4), CORO2B (Q9UQ03), CORO7 (P57737), COTL1 (Q14019), COX4NB (O43402), COX5A (P20674), COX5B (P10606), COX6C (P09669), CP (P00450), CPD (O75976), CPNE1 (Q99829), CPNE2 (Q96FN4), CPNE3 (075131), CPNE4 (Q96A23), CPNE7 (Q9UBL6), CPOX (P36551), CPSF1 (Q10570), CPSF2 (Q9P2I0), CPSF3 (Q9UKF6), CPSF3L (Q5TA45), CPSF6 (Q16630), CPSF7 (Q8N684), CPXM1 (Q96SM3), CRABP2 (P29373), CRIP2 (P52943), CRK (P46108), CRLF3 (Q8IUI8), CRNKL1 (Q9BZJ0), CRTAP (075718), CRYAB (P02511), CRYM (Q14894), CRYZ (Q08257), CRYZL1 (O95825), CS (O75390), CSDE1 (O75534), CSE1L (P55060), CSK (P41240), CSNK1A1 (P48729), CSNK2A1 (P68400), CSNK2A2 (P19784), CSNK2B (P67870), CSRP1 (P21291), CSRP2 (Q16527), CSTB (P04080), CSTF1 (Q05048), CSTF2T (Q9H0L4), CSTF3 (Q12996), CTBP1 (Q13363), CTBP2 (P56545), CTNNA1 (P35221), CTNNAL1 (Q9UBT7), CTNNB1 (P35222), CTNNBL1 (Q8WYA6), CTNND1 (060716), CTPS (P17812), CTPS2 (Q9NRF8), CTR9 (Q6PD62), CTSC (P53634), CTSD (P07339), CTSF (Q9UBX1), CTSL2 (060911), CTTN (Q14247), CTU1 (Q7Z7A3), CUL1 (Q13616), CUL2 (Q13617), CUL3 (Q13618), CUL4A (Q13619), CUL4B (Q13620), CUL5 (Q93034), CUL7 (Q14999), CXADR (P78310), CXCL14 (095715), CXorf26 (Q9BVG4), CXorf38 (Q8TB03), CYB5R3 (P00387), CYC1 (P08574), CYCS (P99999), CYFIP1 (Q7L576), CYFIP2 (Q96F07), CYR61 (O00622), DAB1 (O75553), DAD1 (P61803), DAG1 (Q14118), DAK (Q3LXA3), DAPK3 (O43293), DARS (P14868), DAZAP1 (Q96EP5), DBI (P07108), DBN1 (Q16643), DBNL (Q9UJU6), DCAF7 (P61962), DCAF8 (Q5TAQ9), DCBLD2 (Q96PD2), DCK (P27707), DCLK1 (015075), DCPS (Q96C86), DCTD (P32321), DCTN1 (Q14203), DCTN2 (Q13561), DCTN3 (O75935), DCTN4 (Q9UJW0), DCTN5 (Q9BTE1), DCTN6 (O00399), DCUN1D1 (Q96GG9), DCUN1D3 (Q8IWE4), DCUN1D5 (Q9BTE7), DCXR (Q7Z4W1), DDA1 (Q9BW61), DDAH1 (O94760), DDAH2 (O95865), DDB1 (Q16531), DDB2 (Q92466), DDI2 (Q5TDH0), DDOST (P39656), DDR1 (Q08345), DDT (P30046), DDX1 (Q92499), DDX17 (Q92841), DDX18 (Q9NVP1), DDX19A (Q9NUU7), DDX20 (Q9UHI6), DDX21 (Q9NR30), DDX23 (Q9BUQ8), DDX24 (Q9GZR7), DDX27 (Q96GQ7), DDX39 (000148), DDX3X (000571), DDX46 (Q7L014), DDX47 (Q9HOS4), DDX49 (Q9Y6V7), DDX5 (P17844), DDX50 (Q9BQ39), DDX51 (Q8N8A6), DDX52 (Q9Y2R4), DDX54 (Q8TDD1), DDX55 (Q8NHQ9), DDX56 (Q9NY93), DDX6 (P26196), DECR1 (Q16698), DECR2 (Q9NUI1), DEF (Q68CQ4), DEK (P35659), DENR (O43583), DERA (Q9Y315), DFFA (O00273), DFFB (O76075), DHCR24 (Q15392), DHCR7 (Q9UBM7), DHFR (P00374), DHPS (P49366), DHRS11 (Q6UWP2), DHRS4 (Q9BTZ2), DHX15 (043143), DHX16 (060231), DHX29 (Q7Z478), DHX30 (Q7L2E3), DHX32 (Q7L7V1), DHX36 (Q9H2U1), DHX37 (Q8IY37), DHX38 (Q92620), DHX9 (Q08211), DIAPH1 (060610), DIAPH2 (O60879), DIMT1L (Q9UNQ2), DIP2A (Q14689), DIP2B (Q9P265), DIP2C (Q9Y2E4), DIS3 (Q9Y2L1), DIS3L2 (Q8IYB7), DKC1 (O60832), DLAT (P10515), DLD (P09622), DLG1 (Q12959), DLGAP4 (Q9Y2H0), DLST (P36957), DMD (P11532), DNAJA1 (P31689), DNAJA2 (O60884), DNAJB1 (P25685), DNAJB11 (Q9UBS4), |

(continued)

| |
|---|
| DNAJB4 (Q9UDY4), DNAJB6 (075190), DNAJC13 (075165), DNAJC2 (Q99543), DNAJC3 (Q13217), DNAJC7 (Q99615), DNASE1L1 (P49184), DNM1 (Q05193), DNM1L (O00429), DNM2 (P50570), DNMT1 (P26358), DNPEP (Q9ULA0), DOCK1 (Q14185), DOCK4 (Q8N1I0), DOCK5 (Q9H7D0), DOCK7 (Q96N67), DOCK9 (Q9BZ29), DOHH (Q9BU89), DPCD (Q9BVM2), DPH2 (Q9BQC3), DPH5 (Q9H2P9), DPM1 (O60762), DPM3 (Q9P2X0), DPP3 (Q9NY33), DPP9 (Q86TI2), DPY30 (Q9C005), DPYSL2 (Q16555), DPYSL3 (Q14195), DPYSL4 (014531), DPYSL5 (Q9BPU6), DRG1 (Q9Y295), DRG2 (P55039), DSC1 (Q08554), DSG1 (Q02413), DSP (P15924), DST (Q03001), DSTN (P60981), DTD1 (Q8TEA8), DTNA (Q9Y4J8), DTYMK (P23919), DUS2L (Q9NX74), DUS3L (Q96G46), DUSP12 (Q9UNI6), DUSP3 (P51452), DYM (Q7RTS9), DYNC1H1 (Q14204), DYNC1I2 (Q13409), DYNC1LI1 (Q9Y6G9), DYNC1LI2 (O43237), DYNC2H1 (Q8NCM8), DYNLL1 (P63167), DYNLL2 (Q96FJ2), DYNLRB1 (Q9NP97), DYNLT1 (P63172), EBNA1BP2 (Q99848), ECE1 (P42892), ECHDC1 (Q9NTX5), ECHS1 (P30084), ECM29 (Q5VYK3), EDC3 (Q96F86), EDC4 (Q6P2E9), EEA1 (Q15075), EEF1A1 (P68104), EEF1B2 (P24534), EEF1D (P29692), EEF1E1 (O43324), EEF1G (P26641), EEF2 (P13639), EEF2K (000418), EEFSEC (P57772), EFEMP2 (O95967), EFHD2 (Q96C19), EFTUD1 (Q7Z2Z2), EFTUD2 (Q15029), EGFR (P00533), EHD1 (Q9H4M9), EHD2 (Q9NZN4), EHD3 (Q9NZN3), EHD4 (Q9H223), EIF1AX (P47813), EIF2A (Q9BY44), EIF2AK2 (P19525), EIF2AK4 (Q9P2K8), EIF2B1 (Q14232), EIF2B2 (P49770), EIF2B3 (Q9NR50), EIF2B4 (Q9UI10), EIF2B5 (Q13144), EIF2C1 (Q9UL18), EIF2C2 (Q9UKV8), EIF2S1 (P05198), EIF2S2 (P20042), EIF2S3 (P41091), EIF3A (Q14152), EIF3B (P55884), EIF3C (Q99613), EIF3D (O15371), EIF3E (P60228), EIF3F (O00303), EIF3G (075821), EIF3H (015372), EIF31 (Q13347), EIF3J (O75822), EIF3K (Q9UBQ5), EIF3L (Q9Y262), EIF3M (Q7L2H7), EIF4A1 (P60842), EIF4A2 (Q14240), EIF4A3 (P38919), EIF4E (P06730), EIF4G1 (Q04637), EIF4G2 (P78344), EIF4H (Q15056), EIF5 (P55010), EIF5A (P63241), EIF5B (060841), EIF6 (P56537), ELAC2 (Q9BQ52), ELAVL1 (Q15717), ELMO2 (Q96JJ3), ELP2 (Q6IA86), ELP3 (Q9H9T3), EMD (P50402), EMG1 (Q92979), EML1 (O00423), EML2 (O95834), EML3 (Q32P44), EML4 (Q9HC35), ENAH (Q8N8S7), ENC1 (014682), ENO1 (P06733), ENO2 (P09104), ENOPH1 (Q9UHY7), ENY2 (Q9NPA8), EPB41L2 (043491), EPB41L3 (Q9Y2J2), EPDR1 (Q9UM22), EPHA2 (P29317), EPHB2 (P29323), EPHB3 (P54753), EPHB4 (P54760), EPHX1 (P07099), EPM2AIP1 (Q7L775), EPN1 (Q9Y6I3), EPRS (P07814), ERBB21P (Q96RT1), ERGIC1 (Q969X5), ERH (P84090), ERI1 (Q8IV48), ERI3 (043414), ERLIN2 (O94905), ERO1L (Q96HE7), ERP29 (P30040), ERP44 (Q9BS26), ESD (P10768), ESYT1 (Q9BSJ8), ETF1 (P62495), ETFA (P13804), ETFB (P38117), EXOC1 (Q9NV70), EXOC2 (Q96KP1), EXOC3 (O60645), EXOC4 (Q96A65), EXOC5 (000471), EXOC6 (Q8TAG9), EXOC6B (Q9Y2D4), EXOC7 (Q9UPT5), EXOC8 (Q8IYI6), EXOSC1 (Q9Y3B2), EXOSC10 (Q01780), EXOSC2 (Q13868), EXOSC3 (Q9NQT5), EXOSC4 (Q9NPD3), EXOSC5 (Q9NQT4), EXOSC6 (Q5RKV6), EXOSC7 (Q15024), EXOSC8 (Q96B26), EXOSC9 (Q06265), EZR (P15311), F11R (Q9Y624), F8 (P00451), F8A1 (P23610), FABP5 (Q01469), FABP7 (015540), FADD (Q13158), |

(continued)

| |
|---|
| FAH (P16930), FAHD1 (Q6P587), FAHD2A (Q96GK7), FAM115A (Q9Y4C2), FAM120A (Q9NZB2), FAM125A (Q96EY5), FAM127A (A6ZKI3), FAM129A (Q9BZQ8), FAM129B (Q96TA1), FAM136A (Q96C01), FAM175B (Q15018), FAM3C (Q92520), FAM45B (Q6NSW5), FAM49B (Q9NUQ9), FAM82B (Q96DB5), FAM84B (Q96KN1), FAM96B (Q9Y3D0), FAM98A (Q8NCA5), FAM98B (Q52LJ0), FANCI (Q9NVI1), FAR1 (Q8WVX9), FARP1 (Q9Y4F1), FARP2 (O94887), FARSA (Q9Y285), FARSB (Q9NSD9), FAS (P25445), FASN (P49327), FAT1 (Q14517), FAU (P62861), FBL (P22087), FBLN2 (P98095), FBN1 (P35555), FBN2 (P35556), FBXL18 (Q96ME1), FBXO21 (O94952), FBXO22 (Q8NEZ5), FBXW11 (Q9UKB1), FCF1 (Q9Y324), FDFT1 (P37268), FDPS (P14324), FDXR (P22570), FEN1 (P39748), FERMT1 (Q9BQL6), FERMT2 (Q96AC1), FFR (Q9UID3), FGFBP3 (Q8TAT2), FH (P07954), FHL1 (Q13642), FHL2 (Q14192), FHL3 (Q13643), FIBP (O43427), FKBP10 (Q96AY3), FKBP1A (P62942), FKBP2 (P26885), FKBP3 (Q00688), FKBP4 (Q02790), FKBP5 (Q13451), FLG (P20930), FLG2 (Q5D862), FLII (Q13045), FLNA (P21333), FLNB (O75369), FLNC (Q14315), FLOT1 (O75955), FLOT2 (Q14254), FMNL2 (Q96PY5), FN3K (Q9H479), FN3KRP (Q9HA64), FNTA (P49354), FNTB (P49356), FOLR1 (P15328), FREM2 (Q5SZK8), FRG1 (Q14331), FRMD5 (Q7Z6J6), FRMD8 (Q9BZ67), FRYL (094915), FSCN1 (Q16658), FSD1 (Q9BTV5), FTH1 (P02794), FTL (P02792), FTO (Q9C0B1), FTSJD2 (Q8N1G2), FUBP1 (Q96AE4), FUBP3 (Q96I24), FUCA2 (Q9BTY2), FUK (Q8N0W3), FUS (P35637), FXR1 (P51114), FXR2 (P51116), FYCO1 (Q9BQS8), FYN (P06241), G3BP1 (Q13283), G3BP2 (Q9UN86), G6PD (P11413), GAA (P10253), GALK1 (P51570), GALK2 (Q01415), GALNT1 (Q10472), GALNT2 (Q10471), GALNT7 (Q86SF2), GAN (Q9H2C0), GANAB (Q14697), GAP43 (P17677), GAPDH (P04406), GAPVD1 (Q14C86), GAR1 (Q9NY12), GARS (P41250), GART (P22102), GATSL2 (A6NHX0), GBA (P04062), GBE1 (Q04446), GBF1 (Q92538), GCDH (Q92947), GCLC (P48506), GCLM (P48507), GCN1L1 (Q92616), GDI1 (P31150), GDI2 (P50395), GEMIN4 (P57678), GEMIN5 (Q8TEQ6), GEMIN6 (Q8WXD5), GET4 (Q7L5D6), GFAP (P14136), GFM1 (Q96RP9), GFPT1 (Q06210), GFPT2 (O94808), GGCT (O75223), GGPS1 (O95749), GINS1 (Q14691), GINS2 (Q9Y248), GINS4 (Q9BRT9), GIPC1 (014908), GIT1 (Q9Y2X7), GLA (P06280), GLB1L2 (Q8IW92), GLE1 (Q53GS7), GLG1 (Q92896), GLIPR2 (Q9H4G4), GLMN (Q92990), GLO1 (Q04760), GLOD4 (Q9HC38), GLRX (P35754), GLRX3 (O76003), GLT25D1 (Q8NBJ5), GLT25D2 (Q8IYK4), GLTP (Q9NZD2), GLUD1 (P00367), GLUL (P15104), GMDS (O60547), GMFB (P60983), GMPPA (Q96IJ6), GMPPB (Q9Y5P6), GMPR (P36959), GMPR2 (Q9P2T1), GMPS (P49915), GNA11 (P29992), GNA12 (Q03113), GNA13 (Q14344), GNAI1 (P63096), GNAI2 (P04899), GNAI3 (P08754), GNAQ (P50148), GNAS (Q5JWF2), GNB1 (P62873), GNB1L (Q9BYB4), GNB2 (P62879), GNB2L1 (P63244), GNB4 (Q9HAV0), GNE (Q9Y223), GNG10 (P50151), GNG12 (Q9UBI6), GNG4 (P50150), GNG5 (P63218), GNL3 (Q9BVP2), GNPDA1 (P46926), GNPNAT1 (Q96EK6), GOLGA7 (Q7Z5G4), GOLM1 (Q8NBJ4), GOLPH3 (Q9H4A6), GORASP2 (Q9H8Y8), GOT1 (P17174), GOT2 (P00505), GPC1 (P35052), GPC4 (O75487), GPC6 (Q9Y625), GPD1L (Q8N335), GPHN (Q9NQX3), GPI (P06744), GPM6A |

(continued)

| |
|---|
| (P51674), GPN1 (Q9HCN4), GPR50 (Q13585), GPR56 (Q9Y653), GPS1 (Q13098), GPSM1 (Q86YR5), GPX1 (P07203), GPX4 (P36969), GRB2 (P62993), GRHPR (Q9UBQ7), GRP (Q3ZCW2), GRWD1 (Q9BQ67), GSDMA (Q96QA5), GSK3A (P49840), GSK3B (P49841), GSN (P06396), GSPT1 (P15170), GSR (P00390), GSS (P48637), GSTK1 (Q9Y2Q3), GSTM2 (P28161), GSTM3 (P21266), GSTM4 (Q03013), GSTO1 (P78417), GSTP1 (P09211), GSTT2 (P0CG29), GSTZ1 (O43708), GTF2E2 (P29084), GTF2F2 (P13984), GTF2H3 (Q13889), GTF2I (P78347), GTF3C2 (Q8WUA4), GTF3C3 (Q9Y5Q9), GTF3C4 (Q9UKN8), GTPBP1 (000178), GTPBP4 (Q9BZE4), GUK1 (Q16774), GYG1 (P46976), GYS1 (P13807), H1F0 (P07305), H1FX (Q92522), H2AFX (P16104), H2AFY (O75367), H2AFZ (P0C0S5), HADH (Q16836), HADHA (P40939), HARS (P12081), HAT1 (014929), HAUS3 (Q68CZ6), HAUS4 (Q9H6D7), HBA1 (P69905), HBB (P68871), HBS1L (Q9Y450), HBXIP (O43504), HCFC1 (P51610), HDAC1 (Q13547), HDAC2 (Q92769), HDDC2 (Q7Z4H3), HDGF (P51858), HDGFRP2 (Q7Z4V5), HDHD2 (Q9H0R4), HDLBP (Q00341), HEATR1 (Q9H583), HEATR2 (Q86Y56), HEBP1 (Q9NRV9), HECTD3 (Q5T447), HERC4 (Q5GLZ8), HEXB (P07686), HGS (014964), HHIP (Q96QV1), HINT1 (P49773), HINT2 (Q9BX68), HINT3 (Q9NQE9), HIP1R (075146), HIST1H1B (P16401), HIST1H1C (P16403), HIST1H1D (P16402), HIST1H1E (P10412), HIST1H2AD (P20671), HIST1H2BJ (P06899), HIST1H2BM (Q99879), HIST1H2BO (P23527), HIST1H4A (P62805), HIST2H2AA3 (Q6FI13), HIST2H2AB (Q8IUE6), HIST2H2BE (Q16778), HIST2H3A (Q71DI3), HIST3H2BB (Q8N257), HK1 (P19367), HK2 (P52789), HLA-A (P30443), HLA-A (P01892), HLA-B (P03989), HMGA1 (P17096), HMGB1 (P09429), HMGB2 (P26583), HMGCL (P35914), HMGCS1 (Q01581), HMGN1 (P05114), HMGN2 (P05204), HMGN4 (O00479), HNRNPA0 (Q13151), HNRNPA1 (P09651), HNRNPA2B1 (P22626), HNRNPA3 (P51991), HNRNPAB (Q99729), HNRNPC (P07910), HNRNPD (Q14103), HNRNPF (P52597), HNRNPH1 (P31943), HNRNPH2 (P55795), HNRNPH3 (P31942), HNRNPK (P61978), HNRNPL (P14866), HNRNPM (P52272), HNRNPR (O43390), HNRNPU (Q00839), HNRNPUL1 (Q9BUJ2), HNRNPUL2 (Q1KMD3), HNRPDL (014979), HNRPLL (Q8WVV9), HOOK3 (Q86VS8), HP (P00738), HP1BP3 (Q5SSJ5), HPCAL1 (P37235), HPRT1 (P00492), HPX (P02790), HRAS (P01112), HRNR (Q86YZ3), HSD17B10 (Q99714), HSD17B12 (Q53GQ0), HSD17B4 (P51659), HSDL2 (Q6YN16), HSP90AA1 (P07900), HSP90AB1 (P08238), HSP90B1 (P14625), HSPA12A (O43301), HSPA14 (QOVDF9), HSPA1A (P08107), HSPA4 (P34932), HSPA4L (O95757), HSPA5 (P11021), HSPA8 (P11142), HSPA9 (P38646), HSPB1 (P04792), HSPBP1 (Q9NZL4), HSPD1 (P10809), HSPE1 (P61604), HSPG2 (P98160), HSPH1 (Q92598), HTRA1 (Q92743), HTT (P42858), HUWE1 (Q7Z6Z7), HYOU1 (Q9Y4L1), IARS (P41252), ICAM1 (P05362), IDE (P14735), IDH1 (O75874), IDH2 (P48735), IDH3A (P50213), IDI1 (Q13907), IFI16 (Q16666), IFIT5 (Q13325), IFITM3 (Q01628), IFRD2 (Q12894), IFT172 (Q9UG01), IGF1R (P08069), IGF2BP2 (Q9Y6M1), IGF2BP3 (O00425), IGF2R (P11717), IGFBP3 (P17936), IGFBP5 (P24593), IGHG1 (P01857), IGHG2 (P01859), IGSF3 (O75054), IGSF8 (Q969P0), IKBKAP (095163), IKBKB (014920), IL1RAP (Q9NPH3), ILF2 (Q12905), ILF3 (Q12906), ILK (Q13418), |

(continued)

| |
|---|
| ILKAP (Q9H0C8), IMMT (Q16891), IMP3 (Q9NV31), IMPA1 (P29218), IMPA2 (O14732), IMPAD1 (Q9NX62), IMPDH1 (P20839), IMPDH2 (P12268), INA (Q16352), INF2 (Q27J81), INPP1 (P49441), INPPL1 (015357), INTS10 (Q9NVR2), INTS3 (Q68E01), INTS7 (Q9NVH2), INTS8 (Q75QN2), IPO11 (Q9UI26), IPO4 (Q8TEX9), IPO5 (000410), IPO7 (O95373), IPO8 (015397), IPO9 (Q96P70), IQGAP1 (P46940), IRF2BP2 (Q7Z5L9), IRF3 (Q14653), IRGQ (Q8WZA9), ISOC1 (Q96CN7), ISYNA1 (Q9NPH2), ITFG3 (Q9H0X4), ITGA2 (P17301), ITGA3 (P26006), ITGA4 (P13612), ITGA5 (P08648), ITGA6 (P23229), ITGA7 (Q13683), ITGAV (P06756), ITGB1 (P05556), ITGB1BP1 (014713), ITGB3 (P05106), ITGB4 (P16144), ITGB5 (P18084), ITGB8 (P26012), ITPA (Q9BY32), JAM3 (Q9BX67), JUP (P14923), KARS (Q15046), KATNB1 (Q9BVA0), KBTBD6 (Q86V97), KCTD21 (Q4G0X4), KDM1A (060341), KEAP1 (Q14145), KHDRBS1 (Q07666), KHSRP (Q92945), KIAA0020 (Q15397), KIAA0090 (Q8N766), KIAA0174 (P53990), KIAA0196 (Q12768), KIAA0664 (075153), KIAA0776 (O94874), KIAA1033 (Q2M389), KIAA1279 (Q96EK5), KIAA1598 (A0MZ66), KIAA1797 (Q5VW36), KIAA1949 (Q6NYC8), KIAA1967 (Q8N163), KIDINS220 (Q9ULH0), KIF1A (Q12756), KIF2A (O00139), KIF5B (P33176), KIF5C (O60282), KLC1 (Q07866), KLHDC4 (Q8TBB5), KLHL13 (Q9P2N7), KLHL22 (Q53GT1), KLHL26 (Q53HC5), KNTC1 (P50748), KPNA1 (P52294), KPNA2 (P52292), KPNA3 (O00505), KPNA4 (O00629), KPNA6 (O60684), KPNB1 (Q14974), KPRP (Q5T749), KRAS (P01116), KRIT1 (O00522), KRT13 (P13646), KRT14 (P02533), KRT71 (Q3SY84), KTN1 (Q86UP2), L1CAM (P32004), LACTB2 (Q53H82), LAMA1 (P25391), LAMA4 (Q16363), LAMA5 (015230), LAMB1 (P07942), LAMB2 (P55268), LAMC1 (P11047), LAMP1 (P11279), LAMP2 (P13473), LANCL1 (043813), LANCL2 (Q9NS86), LAP3 (P28838), LARP1 (Q6PKG0), LARS (Q9P2J5), LAS1L (Q9Y4W2), LASP1 (Q14847), LBR (Q14739), LCMT1 (Q9UIC8), LDHA (P00338), LDHB (P07195), LDLR (P01130), LEFTY2 (O00292), LEPRE1 (Q32P28), LGALS1 (P09382), LGALS3 (P17931), LGALS3BP (Q08380), LGALS7 (P47929), LIMA1 (Q9UHB6), LIMS1 (P48059), LIN7C (Q9NUP9), LIPG (Q9Y5X9), LLGL1 (Q15334), LMAN1 (P49257), LMAN2 (Q12907), LMCD1 (Q9NZU5), LMNA (P02545), LMNB1 (P20700), LMNB2 (Q03252), LNPEP (Q9UIQ6), LOH12CR1 (Q969J3), LONP1 (P36776), LOR (P23490), LOXL4 (Q96JB6), LPHN2 (O95490), LPL (P06858), LRBA (P50851), LRG1 (P02750), LRP1 (Q07954), LRPPRC (P42704), LRRC1 (Q9BTT6), LRRC40 (Q9H9A6), LRRC47 (Q8N1G4), LRRC57 (Q8N9N7), LRRC59 (Q96AG4), LRRC8A (Q8IWT6), LRSAM1 (Q6UWE0), LSM1 (015116), LSM12 (Q3MHD2), LSM2 (Q9Y333), LSM4 (Q9Y4Z0), LSM6 (P62312), LSM7 (Q9UK45), LSS (P48449), LTA4H (P09960), LTBP2 (Q14767), LTBP3 (Q9NS15), LTN1 (O94822), LUC7L (Q9NQ29), LUC7L2 (Q9Y383), LUC7L3 (O95232), LYAR (Q9NX58), LYPLA1 (O75608), LYPLA2 (O95372), LYPLAL1 (Q5VWZ2), LZTR1 (Q8N653), M6PR (P20645), MACF1 (Q9UPN3), MACF1 (Q96PK2), MACROD1 (Q9BQ69), MAD1L1 (Q9Y6D9), MAD2L1 (Q13257), MAGEE1 (Q9HCI5), MAK16 (Q9BXY0), MALT1 (Q9UDY8), MAN1A2 (O60476), MAN1B1 (Q9UKM7), MAN2C1 (Q9NTJ4), MAP1B (P46821), MAP1LC3A (Q9H492), MAP1LC3B2 (A6NCE7), MAP2K1 (Q02750), MAP2K2 (P36507), MAP2K3 |

(continued)

| |
|---|
| (P46734), MAP2K4 (P45985), MAP2K7 (014733), MAP4 (P27816), MAP4K4 (095819), MAPK1 (P28482), MAPK14 (Q16539), MAPK3 (P27361), MAPKSP1 (Q9UHA4), MAPRE1 (Q15691), MAPRE3 (Q9UPY8), MARCKS (P29966), MARCKSL1 (P49006), MARK2 (Q7KZI7), MARS (P56192), MAT2A (P31153), MAT2B (Q9NZL9), MATR3 (P43243), MBD3 (O95983), MBLAC2 (Q68D91), MBNL1 (Q9NR56), MBNL2 (Q5VZF2), MCAM (P43121), MCM2 (P49736), MCM3 (P25205), MCM4 (P33991), MCM5 (P33992), MCM6 (Q14566), MCM7 (P33993), MCTS1 (Q9ULC4), MDH1 (P40925), MDH2 (P40926), MDK (P21741), MDN1 (Q9NU22), ME1 (P48163), ME2 (P23368), MED1 (Q15648), MED10 (Q9BTT4), MED11 (Q9P086), MED17 (Q9NVC6), MED18 (Q9BUE0), MED20 (Q9H944), MED23 (Q9ULK4), MED24 (O75448), MED28 (Q9H204), MED31 (Q9Y3C7), MEMO1 (Q9Y316), MEN1 (O00255), MERIT40 (Q9NWV8), METAP1 (P53582), METAP2 (P50579), METT10D (Q86W50), METTL1 (Q9UBP6), METTL11A (Q9BV86), METTL13 (Q8N6R0), METTL2B (Q6P1Q9), METTL5 (Q9NRN9), METTL9 (Q9H1A3), MFAP2 (P55001), MFAP4 (P55083), MFGE8 (Q08431), MFI2 (P08582), MGEA5 (O60502), MICA (Q29983), MICAL1 (Q8TDZ2), MIF (P14174), MINA (Q8IUF8), MIOS (Q9NXC5), MKI67IP (Q9BYG3), MLEC (Q14165), MLLT4 (P55196), MLST8 (Q9BVC4), MLTK (Q9NYL2), MMP14 (P50281), MMP2 (P08253), MMS19 (Q96T76), MOB2 (Q70IA6), MOBKL1B (Q9H8S9), MOBKL2A (Q96BX8), MOBKL3 (Q9Y3A3), MOCS2 (O96033), MOGS (Q13724), MON2 (Q7Z3U7), MORC2 (Q9Y6X9), MOV10 (Q9HCE1), MOXD1 (Q6UVY6), MPG (P29372), MPI (P34949), MPP6 (Q9NZW5), MPRIP (Q6WCQ1), MPST (P25325), MPZL1 (O95297), MRC2 (Q9UBG0), MRE11A (P49959), MRI1 (Q9BV20), MRPS27 (Q92552), MRPS28 (Q9Y2Q9), MRPS33 (Q9Y291), MRPS34 (P82930), MRPS6 (P82932), MRTO4 (Q9UKD2), MSH2 (P43246), MSH3 (P20585), MSH6 (P52701), MSN (P26038), MSTO1 (Q9BUK6), MTA1 (Q13330), MTA2 (O94776), MTAP (Q13126), MTHFD1 (P11586), MTHFS (P49914), MTM1 (Q13496), MTMR1 (Q13613), MTMR2 (Q13614), MTMR6 (Q9Y217), MTMR9 (Q96QG7), MTOR (P42345), MTPN (P58546), MTR (Q99707), MTRR (Q9UBK8), MVD (P53602), MVK (Q03426), MVP (Q14764), MX1 (P20591), MYADM (Q96S97), MYBBP1A (Q9BQG0), MYCBP (Q99417), MYD88 (Q99836), MYH10 (P35580), MYH14 (Q7Z406), MYH9 (P35579), MYL12B (O14950), MYL6 (P60660), MYO18A (Q92614), MYO1B (O43795), MYO1C (000159), MYO1E (Q12965), MYO5A (Q9Y4I1), MYO6 (Q9UM54), MYOF (Q9NZM1), NAA10 (P41227), NAA15 (Q9BXJ9), NAA16 (Q6N069), NAA25 (Q14CX7), NAA38 (O95777), NAA50 (Q9GZZ1), NACA (Q13765), NAE1 (Q13564), NAGK (Q9UJ70), NAGLU (P54802), NAMPT (P43490), NANS (Q9NR45), NAP1L1 (P55209), NAP1L4 (Q99733), NAPA (P54920), NAPG (Q99747), NAPRT1 (Q6XQN6), NARFL (Q9H6Q4), NARS (O43776), NASP (P49321), NAT10 (Q9H0A0), NAT9 (Q9BTE0), NCAM1 (P13591), NCAN (014594), NCAPD2 (Q15021), NCAPG (Q9BPX3), NCBP1 (Q09161), NCCRP1 (Q6ZVX7), NCDN (Q9UBB6), NCKAP1 (Q9Y2A7), NCKIPSD (Q9NZQ3), NCL (P19338), NCLN (Q969V3), NCS1 (P62166), NCSTN (Q92542), NDOR1 (Q9UHB4), NDRG3 (Q9UGV2), NDRG4 (Q9ULP0), NDUFA2 (O43678), NDUFA7 (095182), NDUFAB1 (014561), NDUFB4 (095168), NDUFC2 (O95298), NDUFS5 (O43920), NDUFS6 |

(continued)

| |
|---|
| (O75380), NEDD8 (Q15843), NEFL (P07196), NEFM (P07197), NEK6 (Q9HC98), NEK9 (Q8TD19), NES (P48681), NF1 (P21359), NF2 (P35240), NFIX (Q14938), NHLRC2 (Q8NBF2), NHP2L1 (P55769), NID1 (P14543), NIP7 (Q9Y221), NIPSNAP1 (Q9BPW8), NIT1 (Q86X76), NIT2 (Q9NQR4), NKRF (015226), NLE1 (Q9NVX2), NLGN4X (Q8N0W4), NLN (Q9BYT8), NMD3 (Q96D46), NME2 (P22392), NME3 (Q13232), NME7 (Q9Y5B8), NMT1 (P30419), NNMT (P40261), NOB1 (Q9ULX3), NOC2L (Q9Y3T9), NOC3L (Q8WTT2), NOC4L (Q9BVI4), NOG (Q13253), NOL11 (Q9H8H0), NOL6 (Q9H6R4), NOL9 (Q5SY16), NOMO2 (Q5JPE7), NONO (Q15233), NOP10 (Q9NPE3), NOP16 (Q9Y3C1), NOP2 (P46087), NOP56 (O00567), NOP58 (Q9Y2X3), NOS1AP (O75052), NOSIP (Q9Y314), NOTCH2 (Q04721), NOVA2 (Q9UNW9), NPC1 (015118), NPC2 (P61916), NPEPPS (P55786), NPLOC4 (Q8TAT6), NPM1 (P06748), NPTN (Q9Y639), NPW (Q8N729), NQO1 (P15559), NQO2 (P16083), NRAS (P01111), NRBP1 (Q9UHY1), NRD1 (O43847), NRP1 (014786), NRP2 (O60462), NSDHL (Q15738), NSF (P46459), NSUN2 (Q08J23), NSUN5 (Q96P11), NSUN6 (Q8TEA1), NT5C (Q8TCD5), NT5C2 (P49902), NT5C3L (Q969T7), NT5E (P21589), NTN1 (095631), NUBP1 (P53384), NUBP2 (Q9Y5Y2), NUCB1 (Q02818), NUCKS1 (Q9H1E3), NUDC (Q9Y266), NUDCD1 (Q96RS6), NUDCD2 (Q8WVJ2), NUDT1 (P36639), NUDT10 (Q8NFP7), NUDT16 (Q96DE0), NUDT16L1 (Q9BRJ7), NUDT21 (O43809), NUDT4 (Q9NZJ9), NUDT5 (Q9UKK9), NUMA1 (Q14980), NUP188 (Q5SRE5), NUP210 (Q8TEM1), NUP37 (Q8NFH4), NUP43 (Q8NFH3), NUP54 (Q7Z3B4), NUP62 (P37198), NUP85 (Q9BW27), NUP88 (Q99567), NUP93 (Q8N1F7), NUTF2 (P61970), NXF1 (Q9UBU9), NXN (Q6DKJ4), NXT1 (Q9UKK6), OAT (P04181), OBSL1 (075147), OCRL (Q01968), ODR4 (Q5SWX8), ODZ2 (Q9NT68), ODZ3 (Q9P273), OGFOD1 (Q8N543), OGT (015294), OLA1 (Q9NTK5), OLFML3 (Q9NRN5), OPA1 (060313), ORC3 (Q9UBD5), OSBP (P22059), OSBPL6 (Q9BZF3), OSGEP (Q9NPF4), OTUB1 (Q96FW1), OVCA2 (Q8WZ82), OXCT1 (P55809), OXSR1 (O95747), P4HA1 (P13674), P4HB (P07237), PA2G4 (Q9UQ80), PAAF1 (Q9BRP4), PABPC1 (P11940), PABPC4 (Q13310), PABPN1 (Q86U42), PACSIN2 (Q9UNF0), PACSIN3 (Q9UKS6), PAF1 (Q8N7H5), PAFAH1B1 (P43034), PAFAH1B2 (P68402), PAFAH1B3 (Q15102), PAICS (P22234), PAIP1 (Q9H074), PAK1IP1 (Q9NWT1), PAK2 (Q13177), PALD (Q9ULE6), PALLD (Q8WX93), PANK4 (Q9NVE7), PAPOLA (P51003), PAPSS1 (O43252), PARK7 (Q99497), PARN (O95453), PARP1 (P09874), PARP4 (Q9UKK3), PARVA (Q9NVD7), PBLD (P30039), PCBD1 (P61457), PCBP1 (Q15365), PCBP2 (Q15366), PCDHB2 (Q9Y5E7), PCDHGC3 (Q9UN70), PCID2 (Q5JVF3), PCMT1 (P22061), PCNA (P12004), PCOLCE2 (Q9UKZ9), PCYOX1 (Q9UHG3), PCYOX1L (Q8NBM8), PCYT2 (Q99447), PDCD10 (Q9BUL8), PDCD11 (Q14690), PDCD4 (Q53EL6), PDCD5 (014737), PDCD6 (O75340), PDCD6IP (Q8WUM4), PDCL3 (Q9H2J4), PDDC1 (Q8NB37), PDE12 (Q6L8Q7), PDGFRA (P16234), PDIA3 (P30101), PDIA4 (P13667), PDIA5 (Q14554), PDIA6 (Q15084), PDLIM1 (000151), PDLIM4 (P50479), PDLIM5 (Q96HC4), PDLIM7 (Q9NR12), PDRO (Q6IAA8), PDS5A (Q29RF7), PDS5B (Q9NTI5), PDXK (O00764), PDXP (Q96GD0), PEA15 (Q15121), PEBP1 (P30086), PECI (075521), PEF1 (Q9UBV8), PELO (Q9BRX2), |

(continued)

| |
|---|
| PELP1 (Q8IZL8), PEPD (P12955), PES1 (000541), PFAS (015067), PFDN1 (O60925), PFDN2 (Q9UHV9), PFDN4 (Q9NQP4), PFDN5 (Q99471), PFDN6 (015212), PFKL (P17858), PFKM (P08237), PFKP (Q01813), PFN1 (P07737), PFN2 (P35080), PGAM1 (P18669), PGAM5 (Q96HS1), PGD (P52209), PGGT1B (P53609), PGK1 (P00558), PGLS (O95336), PGLYRP2 (Q96PD5), PGM1 (P36871), PGM2L1 (Q6PCE3), PGM3 (O95394), PGP (A6NDG6), PGRMC1 (O00264), PGRMC2 (015173), PHB (P35232), PHB2 (Q99623), PHF5A (Q7RTV0), PHF6 (Q8IWS0), PHGDH (043175), PHKB (Q93100), PHLDA1 (Q8WV24), PHLDA3 (Q9Y5J5), PHLDB1 (Q86UU1), PHPT1 (Q9NRX4), PI15 (O43692), PI4KA (P42356), PICALM (Q13492), PIGT (Q969N2), PIK3CA (P42336), PIK3R4 (Q99570), PIN1 (Q13526), PIP4K2A (P48426), PIP4K2B (P78356), PIP4K2C (Q8TBX8), PIPOX (Q9P0Z9), PIPSL (A2A3N6), PITPNB (P48739), PKM2 (P14618), PKP1 (Q13835), PLAA (Q9Y263), PLCB3 (Q01970), PLCD1 (P51178), PLCD3 (Q8N3E9), PLCG1 (P19174), PLCG2 (P16885), PLD3 (Q8IV08), PLEC (Q15149), PLIN2 (Q99541), PLIN3 (O60664), PLK1 (P53350), PLOD1 (Q02809), PLOD2 (O00469), PLOD3 (O60568), PLRG1 (O43660), PLS1 (Q14651), PLS3 (P13797), PLSCR3 (Q9NRY6), PLTP (P55058), PLXNA1 (Q9UIW2), PLXNB2 (015031), PLXND1 (Q9Y4D7), PMM2 (015305), PMPCA (Q10713), PMPCB (O75439), PMVK (Q15126), PNMA2 (Q9UL42), PNN (Q9H307), PNO1 (Q9NRX1), PNP (P00491), PNPLA2 (Q96AD5), PODXL (O00592), POLD1 (P28340), POLD2 (P49005), POLE3 (Q9NRF9), POLR1A (O95602), POLR1B (Q9H9Y6), POLR1C (015160), POLR1D (Q9Y2S0), POLR2A (P24928), POLR2B (P30876), POLR2C (P19387), POLR2E (P19388), POLR2G (P62487), POLR2H (P52434), POLR2J (P52435), POLR2K (P53803), POLR3A (014802), POLR3B (Q9NW08), POLR3C (Q9BUI4), POP1 (Q99575), POP4 (O95707), POP7 (075817), POR (P16435), PPA1 (Q15181), PPA2 (Q9H2U2), PPAN (Q9NQ55), PPAP2A (014494), PPAT (Q06203), PPCS (Q9HAB8), PPFIBP1 (Q86W92), PPIA (P62937), PPIB (P23284), PPIC (P45877), PPID (Q08752), PPIF (P30405), PPIH (O43447), PPIL1 (Q9Y3C6), PPM1F (P49593), PPM1G (015355), PPME1 (Q9Y570), PPP1CA (P62136), PPP1CB (P62140), PPP1CC (P36873), PPP1R14B (Q96C90), PPP1R7 (Q15435), PPP1R8 (Q12972), PPP2CA (P67775), PPP2CB (P62714), PPP2R1A (P30153), PPP2R2A (P63151), PPP2R2D (Q66LE6), PPP2R4 (Q15257), PPP2R5D (Q14738), PPP2R5E (Q16537), PPP3CA (Q08209), PPP4C (P60510), PPP4R1 (Q8TF05), PPP5C (P53041), PPP6C (O00743), PPP6R3 (Q5H9R7), PPPDE2 (Q6ICB0), PPT1 (P50897), PPWD1 (Q96BP3), PRCP (P42785), PRDX1 (Q06830), PRDX2 (P32119), PRDX3 (P30048), PRDX4 (Q13162), PRDX6 (P30041), PREP (P48147), PREPL (Q4J6C6), PRIM1 (P49642), PRIM2 (P49643), PRKAA1 (Q13131), PRKACA (P17612), PRKACB (P22694), PRKAG1 (P54619), PRKAR1A (P10644), PRKAR2A (P13861), PRKCA (P17252), PRKCI (P41743), PRKCSH (P14314), PRKDC (P78527), PRKRA (O75569), PRMT1 (Q99873), PRMT10 (Q6P2P2), PRMT3 (O60678), PRMT5 (014744), PRMT7 (Q9NVM4), PROSC (O94903), PRPF19 (Q9UMS4), PRPF3 (O43395), PRPF31 (Q8WWY3), PRPF4 (043172), PRPF40A (O75400), PRPF4B (Q13523), PRPF6 (O94906), PRPF8 (Q6P2Q9), PRPS1 (P60891), PRPS2 (P11908), PRPSAP2 |

(continued)

| |
|---|
| (O60256), PRRC1 (Q96M27), PRSS23 (O95084), PRTFDC1 (Q9NRG1), PSAP (P07602), PSAT1 (Q9Y617), PSD3 (Q9NYI0), PSENEN (Q9NZ42), PSIP1 (O75475), PSMA1 (P25786), PSMA2 (P25787), PSMA3 (P25788), PSMA4 (P25789), PSMA5 (P28066), PSMA6 (P60900), PSMA7 (014818), PSMB1 (P20618), PSMB2 (P49721), PSMB3 (P49720), PSMB4 (P28070), PSMB5 (P28074), PSMB6 (P28072), PSMB7 (Q99436), PSMC1 (P62191), PSMC2 (P35998), PSMC3 (P17980), PSMC4 (P43686), PSMC5 (P62195), PSMC6 (P62333), PSMD1 (Q99460), PSMD10 (O75832), PSMD11 (000231), PSMD12 (O00232), PSMD13 (Q9UNM6), PSMD14 (O00487), PSMD2 (Q13200), PSMD3 (O43242), PSMD4 (P55036), PSMD5 (Q16401), PSMD6 (Q15008), PSMD7 (P51665), PSMD8 (P48556), PSMD9 (O00233), PSME1 (Q06323), PSME2 (Q9UL46), PSME3 (P61289), PSME4 (Q14997), PSMG1 (O95456), PSMG2 (Q969U7), PSPC1 (Q8WXF1), PSPH (P78330), PTBP1 (P26599), PTGES2 (Q9H7Z7), PTGES3 (Q15185), PTGFRN (Q9P2B2), PTGR1 (Q14914), PTHLH (P12272), PTK2 (Q05397), PTK7 (Q13308), PTMA (P06454), PTN (P21246), PTP4A1 (Q93096), PTPN1 (P18031), PTPN11 (Q06124), PTPN23 (Q9H3S7), PTPRA (P18433), PTPRE (P23469), PTPRG (P23470), PTPRJ (Q12913), PTPRZ1 (P23471), PUF60 (Q9UHX1), PURA (Q00577), PURB (Q96QR8), PUS1 (Q9Y606), PUS7 (Q96PZ0), PVR (P15151), PVRL2 (Q92692), PWP1 (Q13610), PWP2 (Q15269), PXDN (Q92626), PXK (Q7Z7A4), PXN (P49023), PYCR1 (P32322), PYCRL (Q53H96), PYGB (P11216), PYGL (P06737), QARS (P47897), QDPR (P09417), QKI (Q96PU8), QTRT1 (Q9BXR0), RAB10 (P61026), RAB11A (P62491), RAB11FIP1 (Q6WKZ4), RAB12 (Q6IQ22), RAB13 (P51153), RAB14 (P61106), RAB18 (Q9NP72), RAB1A (P62820), RAB1B (Q9H0U4), RAB21 (Q9UL25), RAB22A (Q9UL26), RAB23 (Q9ULC3), RAB27A (P51159), RAB2A (P61019), RAB2B (Q8WUD1), RAB32 (Q13637), RAB34 (Q9BZG1), RAB35 (Q15286), RAB3A (P20336), RAB3GAP1 (Q15042), RAB3GAP2 (Q9H2M9), RAB4A (P20338), RAB5A (P20339), RAB5B (P61020), RAB5C (P51148), RAB6A (P20340), RAB7A (P51149), RAB8A (P61006), RAB8B (Q92930), RABAC1 (Q9UI14), RABGAP1 (Q9Y3P9), RABGGTA (Q92696), RABGGTB (P53611), RABL2A (Q9UBK7), RABL3 (Q5HYI8), RAC1 (P63000), RAC3 (P60763), RAD23B (P54727), RAD50 (Q92878), RAE1 (P78406), RAF1 (P04049), RALA (P11233), RALB (P11234), RALY (Q9UKM9), RAN (P62826), RANBP1 (P43487), RANBP2 (P49792), RANGAP1 (P46060), RAP1A (P62834), RAP1B (P61224), RAP1GDS1 (P52306), RAP2B (P61225), RAPH1 (Q70E73), RARS (P54136), RASA1 (P20936), RASA3 (Q14644), RBBP4 (Q09028), RBBP5 (Q15291), RBBP7 (Q16576), RBM12 (Q9NTZ6), RBM14 (Q96PK6), RBM15 (Q96T37), RBM22 (Q9NW64), RBM25 (P49756), RBM26 (Q5T8P6), RBM28 (Q9NW13), RBM39 (Q14498), RBM4 (Q9BWF3), RBM8A (Q9Y5S9), RBMX (P38159), RBP1 (P09455), RBPJ (Q06330), RBX1 (P62877), RCC1 (P18754), RCC2 (Q9P258), RCL (043598), RCL1 (Q9Y2P8), RCN1 (Q15293), RDH11 (Q8TC12), RDH13 (Q8NBN7), RDX (P35241), RECQL (P46063), RELA (Q04206), REPS1 (Q96D71), RETSAT (Q6NUM9), RFC2 (P35250), RFC3 (P40938), RFC4 (P35249), RFC5 (P40937), RFFL (Q8WZ73), RFTN1 (Q14699), RHEB (Q15382), RHOA (P61586), RHOB (P62745), RHOC (P08134), RHOF (Q9HBH0), RHOG |

(continued)

| |
|---|
| (P84095), RHOT2 (Q8IXI1), RIC8A (Q9NPQ8), RNASEH2C (Q8TDP1), RNF114 (Q9Y508), RNF20 (Q5VTR2), RNF213 (Q63HN8), RNF7 (Q9UBF6), RNGTT (O60942), RNH1 (P13489), RNMT (043148), RNPEP (Q9H4A4), ROBLD3 (Q9Y2Q5), ROCK1 (Q13464), ROCK2 (O75116), RP2 (O75695), RPA1 (P27694), RPA2 (P15927), RPA3 (P35244), RPE (Q96AT9), RPF2 (Q9H7B2), RPIA (P49247), RPL10 (P27635), RPL10A (P62906), RPL11 (P62913), RPL12 (P30050), RPL13 (P26373), RPL13A (P40429), RPL14 (P50914), RPL15 (P61313), RPL17 (P18621), RPL18 (Q07020), RPL18A (Q02543), RPL19 (P84098), RPL21 (P46778), RPL22 (P35268), RPL22L1 (Q6P5R6), RPL23 (P62829), RPL23A (P62750), RPL24 (P83731), RPL26 (P61254), RPL26L1 (Q9UNX3), RPL27 (P61353), RPL27A (P46776), RPL28 (P46779), RPL29 (P47914), RPL3 (P39023), RPL30 (P62888), RPL31 (P62899), RPL32 (P62910), RPL34 (P49207), RPL35 (P42766), RPL35A (P18077), RPL36 (Q9Y3U8), RPL36A (P83881), RPL36AL (Q969Q0), RPL37 (P61927), RPL37A (P61513), RPL38 (P63173), RPL4 (P36578), RPL5 (P46777), RPL6 (Q02878), RPL7 (P18124), RPL7A (P62424), RPL7L1 (Q6DKI1), RPL8 (P62917), RPL9 (P32969), RPLPO (P05388), RPLP1 (P05386), RPLP2 (P05387), RPN1 (P04843), RPN2 (P04844), RPP30 (P78346), RPP38 (P78345), RPRD1A (Q96P16), RPRD1B (Q9NQG5), RPS10 (P46783), RPS11 (P62280), RPS12 (P25398), RPS13 (P62277), RPS14 (P62263), RPS15 (P62841), RPS15A (P62244), RPS16 (P62249), RPS17 (P08708), RPS18 (P62269), RPS19 (P39019), RPS2 (P15880), RPS20 (P60866), RPS21 (P63220), RPS23 (P62266), RPS24 (P62847), RPS25 (P62851), RPS26 (P62854), RPS27 (P42677), RPS27A (P62979), RPS27L (Q71UM5), RPS28 (P62857), RPS29 (P62273), RPS3 (P23396), RPS3A (P61247), RPS4X (P62701), RPS4Y1 (P22090), RPS5 (P46782), RPS6 (P62753), RPS6KA1 (Q15418), RPS6KA3 (P51812), RPS7 (P62081), RPS8 (P62241), RPS9 (P46781), RPSA (P08865), RQCD1 (Q92600), RRAGC (Q9HB90), RRAS2 (P62070), RRBP1 (Q9P2E9), RRM1 (P23921), RRM2 (P31350), RRM2B (Q7LG56), RRP1 (P56182), RRP12 (Q5JTH9), RRP1B (Q14684), RRP7A (Q9Y3A4), RRP9 (043818), RRS1 (Q15050), RSL1D1 (076021), RSL24D1 (Q9UHA3), RSPRY1 (Q96DX4), RSU1 (Q15404), RTCD1 (O00442), RTKN (Q9BST9), RTN3 (O95197), RTN4 (Q9NQC3), RUVBL1 (Q9Y265), RUVBL2 (Q9Y230), RWDD2B (P57060), S100A10 (P60903), S100A11 (P31949), S100A13 (Q99584), S100A16 (Q96FQ6), S100A2 (P29034), S100A4 (P26447), S100A6 (P06703), S100A7 (P31151), S100A8 (P05109), S100A9 (P06702), SAAL1 (Q96ER3), SACS (Q9NZJ4), SAE1 (Q9UBE0), SAMHD1 (Q9Y3Z3), SAP18 (000422), SAR1A (Q9NR31), SARM1 (Q6SZW1), SARNP (P82979), SARS (P49591), SARS2 (Q9NP81), SART3 (Q15020), SBDS (Q9Y3A5), SBF1 (O95248), SCARB1 (Q8WTV0), SCARB2 (Q14108), SCCPDH (Q8NBX0), SCFD1 (Q8WVM8), SCFD2 (Q8WU76), SCP2 (P22307), SCPEP1 (Q9HB40), SCRG1 (075711), SCRIB (Q14160), SCRN1 (Q12765), SCRN2 (Q96FV2), SCYL1 (Q96KG9), SDC2 (P34741), SDC4 (P31431), SDCBP (O00560), SDCCAG1 (O60524), SDCCAG3 (Q96C92), SDHA (P31040), SDHB (P21912), SDK1 (Q7Z5N4), SDSL (Q96GA7), SEC13 (P55735), SEC14L2 (O76054), SEC22B (O75396), SEC23A (Q15436), SEC23B (Q15437), SEC23IP (Q9Y6Y8), SEC24A (O95486), SEC24B (O95487), SEC24C |

(continued)

| |
|---|
| (P53992), SEC24D (O94855), SEC31A (O94979), SEC61B (P60468), SEC61G (P60059), SEH1L (Q96EE3), SELH (Q8IZQ5), SELO (Q9BVL4), SEMA3A (Q14563), SENP3 (Q9H4L4), SEPSECS (Q9HD40), 40422 (Q9P0V9), 40787 (Q9NVA2), 37500 (Q15019), 38596 (Q99719), 39326 (Q16181), 40057 (Q9UHD8), SERBP1 (Q8NC51), SERPINB12 (Q96P63), SERPINB3 (P29508), SERPINB6 (P35237), SERPINH1 (P50454), SESN2 (P58004), SET (Q01105), SETD3 (Q86TU7), SF3A1 (Q15459), SF3A2 (Q15428), SF3A3 (Q12874), SF3B1 (O75533), SF3B14 (Q9Y3B4), SF3B2 (Q13435), SF3B3 (Q15393), SF3B4 (Q15427), SF3B5 (Q9BWJ5), SFN (P31947), SFPQ (P23246), SFRP4 (Q6FHJ7), SFXN3 (Q9BWM7), SGTA (O43765), SH3BGRL3 (Q9H299), SH3BP4 (Q9P0V3), SH3GL1 (Q99961), SH3GLB1 (Q9Y371), SHC1 (P29353), SHMT1 (P34896), SHMT2 (P34897), SHOC2 (Q9UQ13), SHPK (Q9UHJ6), SIRT5 (Q9NXA8), SKIV2L (Q15477), SKIV2L2 (P42285), SKP1 (P63208), SLC12A2 (P55011), SLC12A4 (Q9UP95), SLC16A1 (P53985), SLC1A3 (P43003), SLC1A5 (Q15758), SLC25A10 (Q9UBX3), SLC25A11 (Q02978), SLC25A13 (Q9UJS0), SLC25A22 (Q9H936), SLC25A3 (Q00325), SLC25A5 (P05141), SLC25A6 (P12236), SLC26A2 (P50443), SLC29A1 (Q99808), SLC29A2 (Q14542), SLC2A1 (P11166), SLC30A1 (Q9Y6M5), SLC38A1 (Q9H2H9), SLC3A2 (P08195), SLC44A2 (Q8IWA5), SLC4A2 (P04920), SLC4A7 (Q9Y6M7), SLC5A3 (P53794), SLC5A6 (Q9Y289), SLC6A8 (P48029), SLC7A1 (P30825), SLC7A5 (Q01650), SLC9A3R1 (014745), SLC9A3R2 (Q15599), SLIRP (Q9GZT3), SLK (Q9H2G2), SMAD1 (Q15797), SMAD2 (Q15796), SMARCA4 (P51532), SMARCA5 (O60264), SMARCB1 (Q12824), SMARCC1 (Q92922), SMARCC2 (Q8TAQ2), SMARCD2 (Q92925), SMC1A (Q14683), SMC2 (O95347), SMC3 (Q9UQE7), SMC4 (Q9NTJ3), SMC5 (Q8IY18), SMCHD1 (A6NHR9), SMEK1 (Q6IN85), SMG1 (Q96Q15), SMN1 (Q16637), SMS (P52788), SMU1 (Q2TAY7), SMYD3 (Q9H7B4), SMYD5 (Q6GMV2), SNAP23 (000161), SND1 (Q7KZF4), SNF8 (Q96H20), SNRNP200 (O75643), SNRNP40 (Q96DI7), SNRNP70 (P08621), SNRPA1 (P09661), SNRPB (P14678), SNRPB2 (P08579), SNRPD1 (P62314), SNRPD2 (P62316), SNRPD3 (P62318), SNRPE (P62304), SNRPF (P62306), SNRPG (P62308), SNTB1 (Q13884), SNTB2 (Q13425), SNX1 (Q13596), SNX12 (Q9UMY4), SNX17 (Q15036), SNX18 (Q96RFO), SNX2 (O60749), SNX27 (Q96L92), SNX3 (O60493), SNX5 (Q9Y5X3), SNX6 (Q9UNH7), SNX9 (Q9Y5X1), SOD1 (P00441), SOD2 (P04179), SORD (Q00796), SORT1 (Q99523), SPATS2L (Q9NUQ6), SPC24 (Q8NBT2), SPCS2 (Q15005), SPCS3 (P61009), SPG21 (Q9NZD8), SPIN1 (Q9Y657), SPR (P35270), SPRR1B (P22528), SPRR2E (P22531), SPTAN1 (Q13813), SPTBN1 (Q01082), SPTBN2 (015020), SR140 (015042), SRBD1 (Q8N5C6), SRCRL (A1L4H1), SRGAP2 (O75044), SRI (P30626), SRM (P19623), SRP14 (P37108), SRP19 (P09132), SRP54 (P61011), SRP68 (Q9UHB9), SRP72 (O76094), SRP9 (P49458), SRPK1 (Q96SB4), SRPR (P08240), SRPRB (Q9Y5M8), SRPX (P78539), SRPX2 (O60687), SRR (Q9GZT4), SRRM1 (Q8IYB3), SRRM2 (Q9UQ35), SRRT (Q9BXP5), SRSF1 (Q07955), SRSF10 (O75494), SRSF11 (Q05519), SRSF2 (Q01130), SRSF3 (P84103), SRSF5 (Q13243), SRSF6 (Q13247), SRSF7 (Q16629), SRSF9 (Q13242), SRXN1 (Q9BYN0), SSB (P05455), SSBP1 (Q04837), |

(continued)

| |
|---|
| SSR1 (P43307), SSR3 (Q9UNL2), SSRP1 (Q08945), SSSCA1 (O60232), SSU72 (Q9NP77), ST13 (P50502), STAG1 (Q8WVM7), STAM (Q92783), STAMBP (O95630), STAT1 (P42224), STAT2 (P52630), STAT3 (P40763), STAU1 (O95793), STIP1 (P31948), STK10 (O94804), STK24 (Q9Y6EO), STK25 (O00506), STK38 (Q15208), STK38L (Q9Y2H1), STOM (P27105), STOML2 (Q9UJZ1), STON2 (Q8WXE9), STRAP (Q9Y3F4), STT3A (P46977), STUB1 (Q9UNE7), STX12 (Q86Y82), STX4 (Q12846), STX5 (Q13190), STXBP1 (P61764), STXBP3 (000186), STYX (Q8WUJ0), SUB1 (P53999), SUCLA2 (Q9P2R7), SUCLG2 (Q96I99), SUGT1 (Q9Y2Z0), SULF2 (Q8IWU5), SUMO1 (P63165), SUPT16H (Q9Y5B9), SUPT4H1 (P63272), SUPT5H (O00267), SUPT6H (Q7KZ85), SUSD5 (O60279), SVEP1 (Q4LDE5), SVIL (O95425), SWAP70 (Q9UH65), SYMPK (Q92797), SYNCRIP (O60506), SYNGR2 (O43760), SYNJ2BP (P57105), SYNM (015061), SYPL1 (Q16563), TAB1 (Q15750), TAF9 (Q9Y3D8), TAGLN (Q01995), TAGLN2 (P37802), TALDO1 (P37837), TAOK1 (Q7L7X3), TARDBP (Q13148), TARS (P26639), TATDN1 (Q6P1N9), TAX1BP3 (014907), TBC1D13 (Q9NVG8), TBC1D15 (Q8TC07), TBC1D23 (Q9NUY8), TBC1D24 (Q9ULP9), TBC1D4 (O60343), TBC1D9B (Q66K14), TBCA (O75347), TBCB (Q99426), TBCC (Q15814), TBCD (Q9BTW9), TBCE (Q15813), TBK1 (Q9UHD2), TBL1XR1 (Q9BZK7), TBL2 (Q9Y4P3), TBL3 (Q12788), TBPL1 (P62380), TCEA1 (P23193), TCEB1 (Q15369), TCEB2 (Q15370), TCERG1 (014776), TCF25 (Q9BQ70), TCP1 (P17987), TELO2 (Q9Y4R8), TEX10 (Q9NXF1), TEX15 (Q9BXT5), TF (P02787), TFCP2 (Q12800), TFG (Q92734), TFRC (P02786), TGFB1 (P01137), TGFB2 (P61812), TGFBI (Q15582), TGFBRAP1 (Q8WUH2), TGM1 (P22735), TGM3 (Q08188), TH1L (Q8IXH7), THBS1 (P07996), THBS3 (P49746), THG1L (Q9NWX6), THOC2 (Q8NI27), THOC3 (Q96J01), THOC5 (Q13769), THOC6 (Q86W42), THOC7 (Q6I9Y2), THOP1 (P52888), THTPA (Q9BU02), THUMPD1 (Q9NXG2), THUMPD3 (Q9BV44), THY1 (P04216), THYN1 (Q9P016), TIA1 (P31483), TIAL1 (Q01085), TIGAR (Q9NQ88), TIMM13 (Q9Y5L4), TIMM44 (043615), TIMM50 (Q3ZCQ8), TIMM8A (O60220), TIMM8B (Q9Y5J9), TIMM9 (Q9Y5J7), TIMP2 (P16035), TIPRL (O75663), TJP1 (Q07157), TKT (P29401), TLN1 (Q9Y490), TLN2 (Q9Y4G6), TM9SF3 (Q9HD45), TMED10 (P49755), TMED2 (Q15363), TMED5 (Q9Y3A6), TMED7 (Q9Y3B3), TMED9 (Q9BVK6), TMEFF2 (Q9UIK5), TMEM132A (Q24JP5), TMEM2 (Q9UHN6), TMEM30A (Q9NV96), TMEM33 (P57088), TMOD3 (Q9NYL9), TMPO (P42166), TMX1 (Q9H3N1), TNC (P24821), TNKS1BP1 (Q9C0C2), TNPO1 (Q92973), TNPO2 (014787), TNPO3 (Q9Y5LO), TOM1L2 (Q6ZVM7), TOMM20 (Q15388), TOMM34 (Q15785), TOMM5 (Q8N4H5), TOMM70A (O94826), TOP1 (P11387), TOP2A (P11388), TOP2B (Q02880), TP53I3 (Q53FA7), TP53RK (Q96S44), TPBG (Q13641), TPD52 (P55327), TPI1 (P60174), TPM1 (P09493), TPM2 (P07951), TPM3 (P06753), TPM3L (A6NL28), TPM4 (P67936), TPP2 (P29144), TPT1 (P13693), TRA2A (Q13595), TRA2B (P62995), TRAF2 (Q12933), TRAP1 (Q12931), TRAPPC1 (Q9Y5R8), TRAPPC2L (Q9UL33), TRAPPC3 (043617), TRAPPC4 (Q9Y296), TRAPPC5 (Q8IUR0), TRIM16 (095361), TRIM22 (Q8IYM9), TRIM25 (Q14258), TRIM26 (Q12899), TRIM28 (Q13263), TRIM47 (Q96LD4), TRIM5 (Q9C035), TRIO (O75962), |

(continued)

| |
|---|
| TRIP13 (Q15645), TRIP6 (Q15654), TRMT1 (Q9NXH9), TRMT112 (Q9UI30), TRMT5 (Q32P41), TRMT6 (Q9UJA5), TRMT61A (Q96FX7), TRNT1 (Q96Q11), TROVE2 (P10155), TRRAP (Q9Y4A5), TSG101 (Q99816), TSKU (Q8WUA8), TSN (Q15631), TSPAN14 (Q8NG11), TSPAN6 (O43657), TSR1 (Q2NL82), TSSC1 (Q53HC9), TSTA3 (Q13630), TTC1 (Q99614), TTC15 (Q8WVT3), TTC27 (Q6P3X3), TTC37 (Q6PGP7), TTC38 (Q5R3I4), TTC7B (Q86TV6), TTC9C (Q8N5M4), TTL (Q8NG68), TTLL12 (Q14166), TTN (Q8WZ42), TTYH1 (Q9H313), TTYH3 (Q9C0H2), TUBA1B (P68363), TUBA4A (P68366), TUBB (P07437), TUBB2B (Q9BVA1), TUBB2C (P68371), TUBB3 (Q13509), TUBB6 (Q9BUF5), TUBG1 (P23258), TUBGCP2 (Q9BSJ2), TUBGCP3 (Q96CW5), TUFM (P49411), TWF1 (Q12792), TWF2 (Q6IBS0), TXN (P10599), TXNDC17 (Q9BRA2), TXNDC5 (Q8NBS9), TXNDC9 (014530), TXNL1 (O43396), TXNRD1 (Q16881), TYK2 (P29597), TYMS (P04818), U2AF1 (Q01081), U2AF2 (P26368), UAP1 (Q16222), UBA1 (P22314), UBA2 (Q9UBT2), UBA3 (Q8TBC4), UBA52 (P62987), UBA6 (A0AVT1), UBE2D1 (P51668), UBE2D3 (P61077), UBE2E1 (P51965), UBE2G2 (P60604), UBE2I (P63279), UBE2J2 (Q8N2K1), UBE2K (P61086), UBE2L3 (P68036), UBE2M (P61081), UBE2N (P61088), UBE2O (Q9C0C9), UBE2S (Q16763), UBE2V1 (Q13404), UBE2V2 (Q15819), UBE3A (Q05086), UBE3C (Q15386), UBE4A (Q14139), UBE4B (095155), UBFD1 (014562), UBL3 (095164), UBL4A (P11441), UBL5 (Q9BZL1), UBLCP1 (Q8WVY7), UBP1 (Q9NZI7), UBQLN2 (Q9UHD9), UBR1 (Q8IWV7), UBR4 (Q5T4S7), UBTD1 (Q9HAC8), UBXN1 (Q04323), UBXN6 (Q9BZV1), UCHL1 (P09936), UCHL3 (P15374), UCHL5 (Q9Y5K5), UCK2 (Q9BZX2), UFC1 (Q9Y3C8), UFD1L (Q92890), UGDH (060701), UGGT1 (Q9NYU2), UGP2 (Q16851), ULK3 (Q6PHR2), UMPS (P11172), UNC119B (A6NIH7), UNC45A (Q9H3U1), UPF1 (Q92900), UPP1 (Q16831), UQCRC1 (P31930), UQCRC2 (P22695), UQCRFS1 (P47985), URB1 (O60287), URB2 (Q14146), UROD (P06132), UROS (P10746), USO1 (O60763), USP10 (Q14694), USP11 (P51784), USP13 (Q92995), USP14 (P54578), USP15 (Q9Y4E8), USP24 (Q9UPU5), USP39 (Q53GS9), USP5 (P45974), USP7 (Q93009), USP9X (Q93008), UTP15 (Q8TED0), UTP18 (Q9Y5J1), UTP20 (075691), UTP6 (Q9NYH9), UTRN (P46939), UXS1 (Q8NBZ7), UXT (Q9UBK9), VAC14 (Q08AM6), VAMP3 (Q15836), VAMP5 (O95183), VAPA (Q9P0L0), VAPB (O95292), VARS (P26640), VASP (P50552), VAT1 (Q99536), VAV2 (P52735), VBP1 (P61758), VCAN (P13611), VCL (P18206), VCP (P55072), VDAC1 (P21796), VDAC2 (P45880), VDAC3 (Q9Y277), VIM (P08670), VPRBP (Q9Y4B6), VPS11 (Q9H270), VPS13A (Q96RL7), VPS13C (Q709C8), VPS16 (Q9H269), VPS18 (Q9P253), VPS24 (Q9Y3E7), VPS25 (Q9BRG1), VPS26A (O75436), VPS26B (Q4G0F5), VPS28 (Q9UK41), VPS29 (Q9UBQ0), VPS33A (Q96AX1), VPS33B (Q9H267), VPS35 (Q96QK1), VPS36 (Q86VN1), VPS37B (Q9H9H4), VPS39 (Q96JC1), VPS41 (P49754), VPS45 (Q9NRW7), VPS4A (Q9UN37), VPS4B (075351), VPS53 (Q5VIR6), VPS8 (Q8N3P4), VRK1 (Q99986), VTA1 (Q9NP79), VWA1 (Q6PCB0), VWA5A (O00534), WARS (P23381), WASF2 (Q9Y6W5), WASL (000401), WBSCR22 (O43709), WDFY1 (Q8IWB7), WDR1 (O75083), WDR11 (Q9BZH6), WDR12 (Q9GZL7), WDR18 (Q9BV38), WDR26 (Q9H7D7), WDR3 |
| (Q9UNX4), WDR36 (Q8NI36), WDR4 (P57081), WDR43 (Q15061), WDR45L (Q5MNZ6), WDR48 (Q8TAF3), WDR5 (P61964), WDR54 (Q9H977), WDR6 (Q9NNW5), WDR61 (Q9GZS3), WDR73 (Q6P4I2), WDR74 (Q6RFH5), WDR75 (Q8IWA0), WDR77 (Q9BQA1), WDR82 (Q6UXN9), WDR92 (Q96MX6), WHSC2 (Q9H3P2), WRNIP1 (Q96S55), XP32 (Q5T750), XPC (Q01831), XPNPEP1 (Q9NQW7), XPO1 (014980), XPO4 (Q9C0E2), XPO5 (Q9HAV4), XPO6 (Q96QU8), XPO7 (Q9UIA9), XPOT (O43592), XRCC1 (P18887), XRCC5 (P13010), XRCC6 (P12956), XRN2 (Q9H0D6), YARS (P54577), YBX1 (P67809), YES1 (P07947), YKT6 (015498), YRDC (Q86U90), YTHDC1 (Q96MU7), YTHDF2 (Q9Y5A9), YWHAB (P31946), YWHAE (P62258), YWHAG (P61981), YWHAH (Q04917), YWHAQ (P27348), YWHAZ (P63104), ZC3H15 (Q8WU90), ZC3HAV1 (Q7Z2W4), ZC3HAV1L (Q96H79), ZCCHC3 (Q9NUD5), ZFAND1 (Q8TCF1), ZFR (Q96KR1), ZMAT2 (Q96NC0), ZNF259 (075312), ZNF326 (Q5BKZ1), ZNF330 (Q9Y3S2), ZNF622 (Q969S3), ZNF765 (Q7L2R6), ZNFX1 (Q9P2E3), ZW10 (O43264), ZWILCH (Q9H900), ZYG11B (Q9C0D3), ZYX (Q15942). |

**Table 21:** 100 most abundant proteins (name and SwissProt accession number) in CTX0E03 microvesicles

| Identified proteins | Accession number |
|---|---|
| Actin, cytoplasmic 2 | P63261 |
| Histone H4 | P62805 |
| Histone H2B | Q99879 |
| Histone H3.2 | Q71DI3 |
| Histone H2B type 1 | P23527 |

(continued)

| Identified proteins | Accession number |
|---|---|
| Glyceraldehyde-3-phosphate dehydrogenase | P04406 |
| Histone H2A type 2-A | Q6FI13 |
| Ubiquitin-40S ribosomal protein S27a | P62979 |
| Annexin A2 | P07355 |
| Alpha-enolase | P06733 |
| Pyruvate kinase isozymes M1/M2 | P14618 |
| 60S ribosomal protein L6 | Q02878 |
| Histone H2B type 2-E | Q16778 |
| Heat shock cognate 71 kDa protein | P11142 |
| Actin, alpha cardiac muscle 1 | P68032 |
| Heat shock protein HSP 90-beta | P08238 |
| Histone H2B type 1-J | P06899 |
| Elongation factor 1-alpha 1 | P68104 |
| Tubulin beta-2C chain | P68371 |
| 60S ribosomal protein L18 | Q07020 |
| Tubulin beta chain | P07437 |
| 40S ribosomal protein S2 | P15880 |
| 40S ribosomal protein S11 | P62280 |
| Histone H2B type 3-B | Q8N257 |
| Tubulin alpha-1B chain | P68363 |
| 40S ribosomal protein S3 | P23396 |
| 40S ribosomal protein S3a | P61247 |
| Histone H2A type 1-D | P20671 |
| Elongation factor 2 | P13639 |
| Heat shock protein HSP 90-alpha | P07900 |
| GTP-binding nuclear protein Ran | P62826 |
| 60S ribosomal protein L4 | P36578 |
| 40S ribosomal protein S9 | P46781 |
| Profilin-1 | P07737 |
| 60S ribosomal protein L13a | P40429 |
| Phosphoglycerate kinase 1 | P00558 |
| Fatty acid synthase | P49327 |
| Annexin A1 | P04083 |
| Histone H2A.Z | P0C0S5 |
| Vimentin | P08670 |
| 40S ribosomal protein S6 | P62753 |
| Moesin | P26038 |
| Peptidyl-prolyl cis-trans isomerase A | P62937 |

(continued)

| Identified proteins | Accession number |
|---|---|
| 60S ribosomal protein L26 | P61254 |
| 60S ribosomal protein L3 | P39023 |
| 40S ribosomal protein S8 | P62241 |
| 60S ribosomal protein L28 | P46779 |
| Ezrin | P15311 |
| 40S ribosomal protein S4, X isoform | P62701 |
| 60S ribosomal protein L7a | P62424 |
| 60S ribosomal protein L13 | P26373 |
| 60S ribosomal protein L7 | P18124 |
| 40S ribosomal protein S23 | P62266 |
| 60S ribosomal protein L5 | P46777 |
| Eukaryotic initiation factor 4A-I | P60842 |
| 40S ribosomal protein S24 | P62847 |
| Tubulin beta-2B chain | Q9BVA1 |
| 60S ribosomal protein L8 | P62917 |
| 60S ribosomal protein L15 | P61313 |
| 60S ribosomal protein L10 | P27635 |
| Peroxiredoxin-1 | Q06830 |
| Keratin, type I cytoskeletal 14 | P02533 |
| 14-3-3 protein theta | P27348 |
| 40S ribosomal protein S18 | P62269 |
| Transketolase | P29401 |
| 60S ribosomal protein L24 | P83731 |
| Histone H1.5 | P16401 |
| Cofilin-1 | P23528 |
| Dihydropyrimidinase-related protein 3 | Q14195 |
| 60S ribosomal protein L21 | P46778 |
| 60S ribosomal protein L36 | Q9Y3U8 |
| Sodium/potassium-transporting ATPase subunit alpha-1 | P05023 |
| 40S ribosomal protein S16 | P62249 |
| T-complex protein 1 subunit gamma | P49368 |
| Heterogeneous nuclear ribonucleoprotein A1 | P09651 |
| 60S ribosomal protein L14 | P50914 |
| Heat shock 70 kDa protein 1A/1 B | P08107 |
| T-complex protein 1 subunit theta | P50990 |
| 60S ribosomal protein L30 | P62888 |
| Protein S100-A6 | P06703 |
| 40S ribosomal protein SA | P08865 |

(continued)

| Identified proteins | Accession number |
|---|---|
| CD44 antigen | P16070 |
| 60S ribosomal protein L35a | P18077 |
| Tubulin beta-3 chain | Q13509 |
| T-complex protein 1 subunit delta | P50991 |
| 4F2 cell-surface antigen heavy chain | P08195 |
| T-complex protein 1 subunit beta | P78371 |
| Myosin-9 | P35579 |
| Adenosylhomocysteinase | P23526 |
| Filamin-A | P21333 |
| Fatty acid-binding protein, brain | O15540 |
| Myristoylated alanine-rich C-kinase substrate | P29966 |
| T-complex protein 1 subunit eta | Q99832 |
| Fascin | Q16658 |
| Fructose-bisphosphate aldolase A | P04075 |
| 60S ribosomal protein L27 | P61353 |
| 60S ribosomal protein L17 | P18621 |
| Heterogeneous nuclear ribonucleoproteins A2/B1 | P22626 |
| 60S ribosomal protein L10a | P62906 |
| 60S ribosomal protein L35 | P42766 |

*Discussion of proteomic data*

CD63 (also known as MLA1 and TSPAN30), TSG101 (also known as ESCRT-I complex subunit TSG101), CD109 (also known as 150 kDa TGF-beta-1-binding protein) and thy-1 (also known as CD90) were detected in both exosomes and microvesicles.

Other tetraspanins were also detected: Tetraspanin-4, -5, -6, -9 and 14 were detected in the exosome fraction; tetraspanins-6 and -14 were detected in the microvesicles.

CD133 (also known as AC133, Prominin-1, PROM1, PROML1 and MSTP061) was detected in the exosomes but not the microvesicles.

CD53 (also known as MOX44 and TSPAN25), CD82 (also known as KAI1, SAR2, ST6 and TSPAN27), CD37 (also known as TSPAN26) and CD40 ligand (also known as CD40LG, CD40L and TNFSF5) were not detected in the exosomes or the microvesicles.

Nestin, GFAP and tubulin beta-3 chain (also known as TUBB3) were detected in both the exosome and microvesicle fractions, with tubulin beta-3 chain being particularly prominent within the top 100 proteins in both fractions. Sox2, DCX, GALC, GDNF and IDO were not detected.

Selectins and TNFRI (also known as TNF receptor 1, TNFRSF1A, TNFAR and TNFR1) were not detected.

Integrin alpha-2, -3, -4, -5, -6, -7, -V and integrin beta-1, -4 and -8 were detected in both exosome and microvesicle fractions. Integrin beta-3 and -5 were detected in the microvesicles only.

MHC Class I antigens (*e.g.* HLA_A1, HLA-A2 and HLA-B27) were detected in both the exosomes and microvesicles.

Cell-adhesion molecules (*e.g.* CADM1, CADM4, ICAM1, JAM3, L1CAM, NCAM) were detected in both the exosomes and microvesicles.

Cytoskeletal proteins (*e.g.* actin, vimentin, keratins, catenins, dystroglucan, neurofilament polypeptide, microtubule-associated protein, tubulin, desmoplaktin, plectin, plakophilin, septin, spectrin, talin, vinculin and zyxin) were detected in both the exosome and microvesicle fractions.

GTPases, clathrin, chaperones, heat-shock proteins (*e.g.* Hsp90, Hsp70), splicing factors, translation factors, annexins and growth factors (*e.g.* TGF-beta) were detected in both the exosomes and microvesicles.

Galectin-3, TIMP-1, thrombosponding-1, EGF receptor and CSK were detected in both the exosomes and microvesicles.

Figure 18 compares the proteomic data from the exosomes and microvesicles. Figure 18A illustrates the number of unique proteins within each micro particle population, isolated from week 2 Integra culture system. Figure 18B compares the biological processes associated with the identified proteins within each micro particle population, isolated from week 2 Integra system. The proteins identified within exosomes and microvesicles are associated with very similar biological processes.

Proteins associated with biotin metabolism were only found in exosomes and proteins involved in tryptophan biosynthesis and taurine/alpha-linolenic acid metabolism were only identified in microvesicles.

Figure 18C compares the CTX0E03 proteome to the Mesenchymal Stem Cell exosome proteome disclosed in Lai et al 2012, in which a total of 857 proteins were identified in exosomes released from mesenchymal stem cells.

Figure 18D compares the biological processes associated with the identified proteins within the MSC derived exosomes (Lim 2012) with the neural stem cell derived exosomes of the invention. The three biological processes found to be associated with the MSC derived exosomes only are (in decreasing order of significance): Asthma; phenylalanine, tyrosine and tryptophan biosynthesis; and primary immunodeficiency. The thirty biological processes found to be associated only with the neural stem cell derived exosomes are shown in Figure 19; the most significant biological function identified relates to RNA polymerase.

A further comparison of the 197 biological processes shared by both MSC derived exosomes and NSC derived exosomes shows that NSC exosomes contain notably more processes involved in RNA degradation, the Ribosome and spliceosomes, when compared to MSC exosomes.

The above comparison indicates a number of significant differences between NSC derived exosomes and MSC derived exosomes (as characterised by Lim *et al* 2012). The 4 most significant biological differences identified as present in NSC exosomes compared to being very low/absent in those identified by the Lim's group, all involve proteins associated with the production, packaging, function and degradation of genetic material, i.e RNA polymerase, RNA degradation, Ribosome and spliceosomes.

**Example 14:** Size distribution of Microparticles

NanoSight analysis was undertaken to determine the particle size and concentration of microvesicles ("mv1" to "mv6") and exosomes ("exo1" to "exo6") isolated from CTX0E03 cells cultured in the Integra Celline system for 1, 2, 3, 4, 5 and 6 weeks. All results are based on 5 replicate measurements.

Particle size distribution was measured using Nanoparticle Tracking Analysis (NTA). NTA detects the movement of particles in solution and relates it to particle size. Mode and median particle size was calculated for all samples. Exosome samples were analysed using the most sensitive camera settings in order to capture the smallest vesicles. Microvesicle samples were analysed using less sensitive camera settings to prevent over exposure of the larger vesicles. As a result, some smaller vesicles were not detected in the samples. Although smaller vesicles were present in the MV samples, these represent a small percentage of the sample in terms of mass.

A proportion of Exo1 was labelled with a fluorescent membrane-specific dye (CellMask™) and a combination of NTA analysis with the CellMask™ labelling confirmed that the events detected by NTA correspond to membrane vesicles (data not shown).

The results are shown in Table 22 below, and in Figure 17.

The exosomes show a drop in size at week six, from a mode of approximately 110nm to approximately 70nm, or from a median of approximately 130nm to approximately 75nm. The overall size range, from 70nm to 150nm, is consistent with the size of exosomes from other cell types, described in the art. The observed reduction in size of the exosomes to around 70nm diameter after culturing the cells for 6 weeks correlates with the increased efficacy of exosomes isolated from CTX0E03 cells that have been cultured in a multi-compartment bioreactor for 6 weeks correlates, as reported in Example 8 and Figure 6.

It is also noted that the concentration of microvesicles and exosomes decreases over the six week period of Figure 17, broadly mirroring the improved efficacy observed over time.

The microvesicles are, as expected, larger, with a mode diameter of approximately 150nm - 200nm, or a median diameter of approximately 180nm - 350nm.

**Table 22:** Size distribution of CTX0E03 microvesicles and exosomes.

| **Sample** | Count | Dilution | **Concentration x$10^{12}$/ml** | **Mode (nm)** | **Median (nm)** |
|---|---|---|---|---|---|
| **Exo1 (1)** | 5.204 | 10000 | **32.26** | **107** | **151** |

(continued)

| Sample | Count | Dilution | Concentration x10$^{12}$/ml | Mode (nm) | Median (nm) |
|---|---|---|---|---|---|
| **Exo1 (2)** | 1.734 | 10000 | **10.75** | **135** | **164** |
| **Exo1 (3)** | 6.55 | 10000 | **40.61** | **108** | **128** |
| **Exo2** | 14.33 | 10000 | **88.85** | **118** | **153** |
| | | | | | |
| **Exo3 (1)*** | 2.52 | 10000 | **15.62** | **89** | **115** |
| **Exo3 (2)** | 10.06 | 10000 | **62.37** | **115** | **146** |
| **Exo3 (3)** | 8.98 | 10000 | **55.68** | **128** | **147** |
| | | | | | |
| **Exo4 (1)** | 3.04 | 10000 | **18.85** | **111** | **136** |
| **Exo4 (2)** | 2.89 | 10000 | **17.92** | **110** | **120** |
| **Exo4 (3)** | 2.77 | 10000 | **17.17** | **116** | **134** |
| | | | | | |
| **Exo5 (1)** | 2.34 | 100 | **0.15** | **99** | **117** |
| **Exo5 (2)** | 2.02 | 100 | **0.13** | **102** | **124** |
| **Exo 5 (3)** | 2.08 | 100 | **0.13** | **116** | **127** |
| | | | | | |
| **Exo6 (1)** | 1.45 | 100 | **0.09** | **68** | **74** |
| **Exo6 (2)** | 1.19 | 100 | **0.07** | **69** | **75** |
| | | | | | |
| **MV1 (1)** | 9.314 | 200 | **1.15** | **183** | **212** |
| **MV1 (2)** | 10.76 | 200 | **1.33** | **161** | **214** |
| **MV1 (3)** | 10.738 | 200 | **1.33** | **173** | **198** |
| | | | | | |
| **MV2** | 5.89 | 1000 | **3.65** | **177** | **194** |
| | | | | | |
| **MV3 (1)*** | 5.68 | 2000 | **7.04** | **150** | **186** |
| **MV3 (2)** | 11.5 | 2000 | **14.26** | **221** | **351** |
| **MV3 (3)** | 9.57 | 2000 | **11.87** | **214** | **270** |
| | | | | | |
| **MV4 (1)** | 4.894 | 400 | **1.21** | **209** | **240** |
| **MV4 (2)** | 2.934 | 1000 | **1.82** | **195** | **212** |
| **MV4 (3)** | 2.55 | 1000 | **1.58** | **184** | **221** |
| | | | | | |
| **MV5 (1)** | 1.086 | 200 | **0.13** | **164** | **237** |
| **MV5 (2)** | 1.458 | 200 | **0.18** | **205** | **205** |
| **MV 5 (3)** | 1.3 | 200 | **0.16** | **219** | **210** |
| | | | | | |
| **MV6 (1)** | 0.346 | 200 | **0.04** | **171** | **186** |

(continued)

| Sample | Count | Dilution | Concentration x$10^{12}$/ml | Mode (nm) | Median (nm) |
|---|---|---|---|---|---|
| **MV6 (2)** | 0.37 | 200 | **0.05** | **168** | **212** |
| | | | | | |
| **Media** | 0.14 | 10 | **0.00** | **100** | **149** |
| * large aggregates. | | | | | |

## REFERENCES

Ambros et al RNA 2003. 9: 277-279

Banerjee, S., Williamson, D., Habib, N., Gordon, M., Chataway, J. (2011) Age and Ageing 40:7-13

Chung et al., Cell Stem Cell, 2, 113-117, 2008

Ding, D. C., Shyu, W. C., Lin S. Z. (2011) Cell Transplant 20: 5-14

Einstein, O., Ben-Hur, T. (2008) Arch Neurol 65:452-456

Gennaro (2000) Remington: The Science and Practice of Pharmacy. 20th edition, ISBN: 0683306472

Hassani Z, O'Reilly J, Pearse Y, Stroemer P, Tang E, Sinden J, Price J, Thuret S. "Human neural progenitor cell engraftment increases neurogenesis and microglial recruitment in the brain of rats with stroke." PLoS One. 2012;7(11):e50444. doi: 10.1371/journal.pone.0050444. Epub 2012 Nov 21.

Hodges et al. Cell Transplant. 2007;16(2):101-15

Horie, N., Pereira, N.P., Niizuma, K. Sun, G. et al. (2011) Stem Cells 29:274-285.

Katsuda, Kosaka, Takeshita, Ochiya. Proteomics 2013, 00, 1-17

Katsuda, Tsuchiya, Kosaka, Yoshioka, Takagaki, Oki, Takeshita, Sakai, Kuroda, Ochiya. Scientific Reports 2013, 3:1197, p1-11.

Klimanskaya et al., 2006, Nature 444:481-485

Kornblum, Stroke 2007, 38:810-816

Lai et al "Proteolytic Potential of the MSC Exosome Proteome: Implications for an Exosome-Mediated Delivery of Therapeutic Proteasome". International Journal of Proteomics (2012) Article ID 971907, 14 pages.

Littlewood, T. D., Hancock, D. C., Danielian, P. S. et al. (1995) Nucleic Acid Research 23:1686-1690.

Miljan, E.A. & Sinden, J.D. (2009) Current Opinion in Molecular Therapeutics 4:394-403

Miljan EA, Hines SJ, Pande P, Corteling RL, Hicks C, Zbarsky V, Umachandran, M, Sowinski P, Richardson S, Tang E, Wieruszew M, Patel S, Stroemer P, Sinden JD. Implantation of c-mycER TAM immortalized human mesencephalic-derived clonal cell lines ameliorates behavior dysfunction in a rat model of Parkinson's disease. Stem Cells Dev. 2009 Mar;18(2):307-19

Mitchell et al Journal of Immunological Methods 335 (2008) 98-105

Pollock et al, Exp Neurol. 2006 May;199(1):143-55.

Mark F Pittenger; Alastair M Mackay; Stephen C Beck; Rama K Jaiswal; et al Science; Apr 2, 1999; 284, 5411

Smith, E. J., Stroemer, R.P., Gorenkova, N., Nakajima, M. et al. (2012) Stem Cells 30:785-796.

Stevenato, L., Corteling, R., Stroemer, P., Hope, A. et al. (2009) BMC Neuroscience 10:86

Stroemer, P., Patel, S., Hope, A., Oliveira, C., Pollock, K., Sinden, J. (2009) Neurorehabil Neural Repair 23: 895-909.

Théry, C., Ostrowski, M., Segura, E. et al. (2009) Nature Reviews Immunology 9: 581-593

Their et al, "Direct Conversion of Fibroblasts into Stably Expandable Neural Stem Cells". Cell Stem Cell. 2012 Mar 20.

Timmers, L., Lim, S. K., Arslan, F., Armstrong, J. S. et al. (2007) Stem Cell Res 1: 129-137

Yuan, S.J., Martin, J,, Elia, J., Flippin, J. et al. (2011) PLoS ONE 6:e17540


## Embodiments

The invention comprises at least the following numbered embodiments:

1. A neural stem cell microparticle.
2. The neural stem cell microparticle of embodiment 1, wherein the microparticle is an exosome, microvesicle, membrane particle, membrane vesicle, exosome-like vesicle, ectosome-like vesicle, ectosome or exovesicle.
3. The neural stem cell microparticle of embodiments 1 or 2, wherein the microparticle is derived from a neural stem cell line.

4. The neural stem cell microparticle of embodiment 3, wherein the neural stem cell line is conditionally-immortalised and/or grown in serum free medium.
5. The neural stem cell microparticle of embodiment 4, wherein the neural stem cell line is CTX0E03 having ECACC Accession No. 04091601, STR0C05 having ECACC Accession No.04110301 and HPC0A07 having ECACC Accession No.04092302.
6. The neural stem cell microparticle of any preceding embodiment, wherein the microparticle has:

(a) a size of between 30 nm and 1000 nm, or between 30 and 200 nm, or between 30 and 100 nm, as determined by electron microscopy; or
(b) a density in sucrose of 1.1-1.2 g/ml.

7. The neural stem cell microparticle of any preceding embodiment, comprising RNA.
8. The neural stem cell microparticle of embodiment 7, wherein the RNA is mRNA and/or miRNA.
9. The neural stem cell microparticle of embodiment 8, wherein the microparticle comprises one, two, three or four of hsa-miR-1246, hsa-miR-4492, hsa-miR-4488 and hsa-miR-4532.
10. The neural stem cell microparticle of any preceding embodiment, comprising one or more of:

(a) a lipid selected from ceramide, cholesterol, sphingomyelin, phosphatidylserine, phosphatidylinositol, and/or phosphatidylcholine;
(b) miRNA, optionally selected from hsa-let-7g, hsa-miR-101, hsa-miR-10a, hsa-miR-10b, hsa-miR-126, hsa-miR-128, hsa-miR-129-5p, hsa-miR-130a, hsa-miR-134, hsa-miR-137, hsa-miR-155, hsa-miR-15a, hsa-miR-15b, hsa-miR-16, hsa-miR-17, hsa-miR-182, hsa-miR-183, hsa-miR-185, hsa-miR-18b, hsa-miR-192, hsa-miR-194, hsa-miR-195, hsa-miR-20a, hsa-miR-20b, hsa-miR-210, hsa-miR-218, hsa-miR-301a, hsa-miR-302a, hsa-miR-302c, hsa-miR-345, hsa-miR-375, hsa-miR-378, hsa-miR-7, hsa-miR-9, hsa-miR-93, hsa-miR-96, and hsa-miR-99a;
(c) a tetraspanin, optionally selected from CD63, CD81,CD9, CD53, CD82 and/or CD37;
(d) TSG101, Alix, CD109 and/or thy-1; and/or
(e) CD133.

11. The neural stem cell microparticle of any preceding embodiment, comprising at least 10 of the proteins present in Table 19 or Table 21.
12. The neural stem cell microparticle of any preceding embodiment, comprising at least one biological activity of a neural stem cell or a neural stem cell-conditioned medium.
13. The neural stem cell microparticle of embodiment 12, wherein the at least one biological activity is regenerative activity.
14. The neural stem cell microparticle of any preceding embodiment, for use in therapy.
15. The neural stem cell microparticle of embodiment 14, wherein the therapy is regenerative therapy.
16. The neural stem cell microparticle of embodiments 14 or 15, wherein the therapy is for a

(i) Neurological disorder, disease or deficit, such as Parkinson’s, Alzheimer’s, Stroke, or ALS;
(ii) Lysosomal storage disorder;
(iii) Cardiovascular disorder, such as Myocardial Infarction, congestive heart failure, Peripheral Arterial Disease, diabetic ulcers, wound healing;
(iv) Diseases of the lung, including Idiopathic Pulmonary Fibrosis, Respiratory Distress Syndrome, Chronic Obstructive Pulmonary Disease, Idiopathic Pulmonary Hypertension, Cystic Fibrosis and Asthma
(v) Metabolic or inflammatory disorder, such as Diabetes (I or II), rheumatoid arthritis, osteoarthritis, lupus, Crohn’s disease, Irritable Bowel Disease, or Graft versus Host Disease;
(vi) Psychiatric disorder, such as: Depression, Bipolar, Schizophrenia or an Autistic syndrome disorder such as Autism, Asperger’s syndrome or Rett Syndrome;
(vii) Blindness-causing disease of the retina, such as Age-related macular degeneration, Stargardt disease, diabetic retinopathy, or retinitis pigmentosa; and
(viii) Demyelinating disease, such as multiple sclerosis, central pontine myelinolysis, tabes dorsalis, transverse myelitis, Devic’s disease, progressive multifocal leukoencephalopathy, optic neuritis, leukodystrophies, Guillain-Barre syndrome, Anti-MAG peripheral neuropathy and Charcot-Marie-Tooth disease.

17. The neural stem cell microparticle of embodiments 14-16, wherein the therapy improves functional and/or cognitive recovery.
18. The neural stem cell microparticle of embodiments 14-17, wherein the therapy is of stroke, peripheral arterial disease or blindness-causing diseases of the retina.

19. The neural stem cell microparticle of embodiments 14 to 17, wherein:

(i) the microparticle is an exosome and therapy is of a disease or condition requiring tissue replacement, regeneration or repair; or
(ii) the microparticle is a microvesicle and the therapy is of a disease requiring angiogenesis or a neurological disease, disorder or deficit.

20. Use of a neural stem cell microparticle according to any preceding embodiment, in the manufacture of a medicament for the treatment of a disease.

21. A method of producing a neural stem cell microparticle as defined in embodiments 1-13, comprising isolating a microparticle from a neural stem cell-conditioned medium.

22. A method of producing a stem cell microparticle, comprising isolating a microparticle from a stem cell-conditioned medium wherein:

(i) the stem cell-conditioned medium comprises one or more components which induce the release of microparticles by the stem cells into the medium;
(ii) the stem cells were cultured under hypoxic conditions;
(iii) the stem cells were co-cultured with a different cell type;
(iv) the stem cells were cultured in a multi-compartment bioreactor; and/or
(v) the stem cells were partially-differentiated.

23. A method according to embodiment 22, wherein the stem cell is a neural stem cell, optionally as defined in any of embodiments 3 to 5.

24. A method according to embodiment 22(i) or embodiment 23 when dependent upon embodiment 22(i), wherein the one or more components are selected from: transforming growth factor-beta (TGF-$\beta$), interferon-gamma (INF-$\gamma$) and tumour necrosis factor-alpha (TNF-a).

25. A method according to embodiment 22(iii), embodiment 23 or 24 when dependent upon embodiment 22(iii), wherein the different cell type is an endothelial cell.

26. A microparticle obtainable by the method of any of embodiments 22-25.

27. A composition comprising a microparticle according to any of embodiments 1-13 or 26 and a pharmaceutically acceptable excipient, carrier or diluent.

28. A kit for use in a method for producing the microparticle of any of embodiments 1-13 or 26 comprising: (a) a medium; (b) a neural stem cell; (c) optionally the one or more components of embodiments 22 to 24; (d) optionally the microparticle of any of embodiments 1-13 or 26 suitable for use as a control; (e) optionally a detection agent suitable for specific detection of the produced microparticles; and (f) instructions for producing the microparticle of any of embodiments 1-13 or 26 using the kit.

29. A method of screening for an agent that alters the rate of production of a microparticle by a stem cell, comprising contacting a stem cell with a candidate agent and observing whether the rate production of microparticles by the contacted stem cell increases or decreases compared to a control.

30. A composition comprising two, three or all four of hsa-miR-1246, hsa-miR-4492, hsa-miR-4488 and hsa-miR-4532.

31. A composition according to embodiment 30, which is a pharmaceutical composition comprising a pharmaceutically acceptable carrier, diluent, vehicle and/or excipient.

32. A composition according to embodiment 30 or 31, for use in therapy.

33. A composition for use in therapy according to embodiment 32, wherein the therapy is as defined in embodiment 16.

**Claims**

1. A neural stem cell microparticle.

2. The neural stem cell microparticle of claim 1, wherein the microparticle is an exosome, microvesicle, membrane particle, membrane vesicle, exosome-like vesicle, ectosome-like vesicle, ectosome or exovesicle.

3. The neural stem cell microparticle of claim 1 or claim 2, comprising an exogenous protein, nucleic acid, lipid, drug, produg, therapeutic agent or diagnostic agent.

4. The neural stem cell microparticle of any preceding claim, wherein the microparticle is derived from a neural stem cell line, which is optionally conditionally-immortalised and/or grown in serum free medium, and optionally wherein the neural stem cell line is CTX0E03 having ECACC Accession No. 04091601, STR0C05 having ECACC Accession No.04110301 and HPC0A07 having ECACC Accession No.04092302.

5. The neural stem cell microparticle of any preceding claim, wherein the microparticle has:

   (a) a size of between 30 nm and 1000 nm, or between 30 and 200 nm, or between 30 and 100 nm, as determined by electron microscopy; or
   (b) a density in sucrose of 1.1-1.2 g/ml.

6. The neural stem cell microparticle of any preceding claim, comprising RNA, optionally wherein the RNA is mRNA and/or miRNA, optionally wherein the microparticle comprises one, two, three or four of hsa-miR-1246, hsa-miR-4492, hsa-miR-4488 and hsa-miR-4532.

7. The neural stem cell microparticle of any preceding claim, comprising one or more of:

   (a) a lipid selected from ceramide, cholesterol, sphingomyelin, phosphatidylserine, phosphatidylinositol, and/or phosphatidylcholine;
   (b) a tetraspanin, optionally selected from CD63, CD81,CD9, CD53, CD82 and/or CD37;
   (c) TSG101, Alix, CD109 and/or thy-1;
   (d) CD133; and/or
   (e) Actin cytoplasmic 2, GAP-3-DH, Histone H4, Pyruvate kinase isozymes M1/M2, Alpha-enolase, HSP-90-beta, Ubiquitin-40S ribosomal protein S27a, Heat shock Cognate 71kDa protein, Haptoglobin and HSP-90-alpha.

8. The neural stem cell microparticle of any preceding claim, comprising at least one biological activity of a neural stem cell or a neural stem cell-conditioned medium.

**9.** The neural stem cell microparticle of any preceding claim, for use in therapy.

**10.** A composition comprising a neural stem cell microparticle according to any of claims 1-8 or a neural stem cell microparticle for use of claim 9, and a pharmaceutically acceptable excipient, carrier or diluent.

**11.** A method of producing a neural stem cell microparticle as defined in any of claims 1-8, comprising isolating the microparticle from a neural stem cell or neural stem cell-conditioned medium.

**12.** A method according to claim 11, further comprising the step of loading the isolated microparticle with one or more exogenous nucleic acids, lipids, proteins, drugs, prodrugs, therapeutic agents or diagnostic agents.

**13.** A method of preparing neural stem cell microparticles comprising an exogenous protein, nucleic acid, lipid, drug, prodrug, therapeutic agent or diagnostic agent, comprising the step of:
loading the exogenous protein, nucleic acid, lipid, drug, prodrug, therapeutic agent or diagnostic agent into isolated microparticles.

**14.** A method according to claim 12 or claim 13, wherein the exogenous material is directly added to the microparticles.

**15.** A method according to any of claims 12 to 14, wherein exogenous nucleic acid is loaded into the microparticles by electroporation.

Figure 1

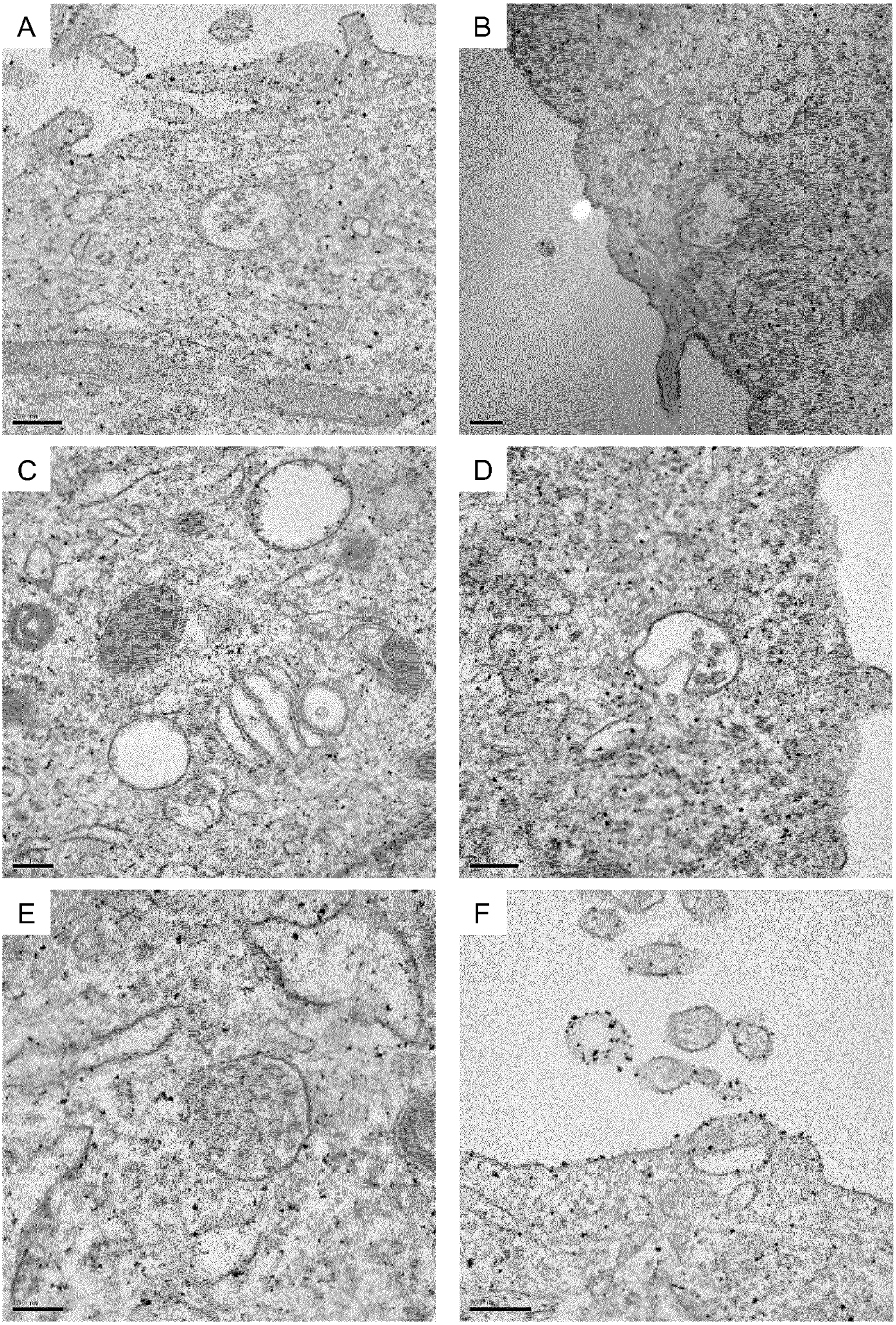

Figure 2

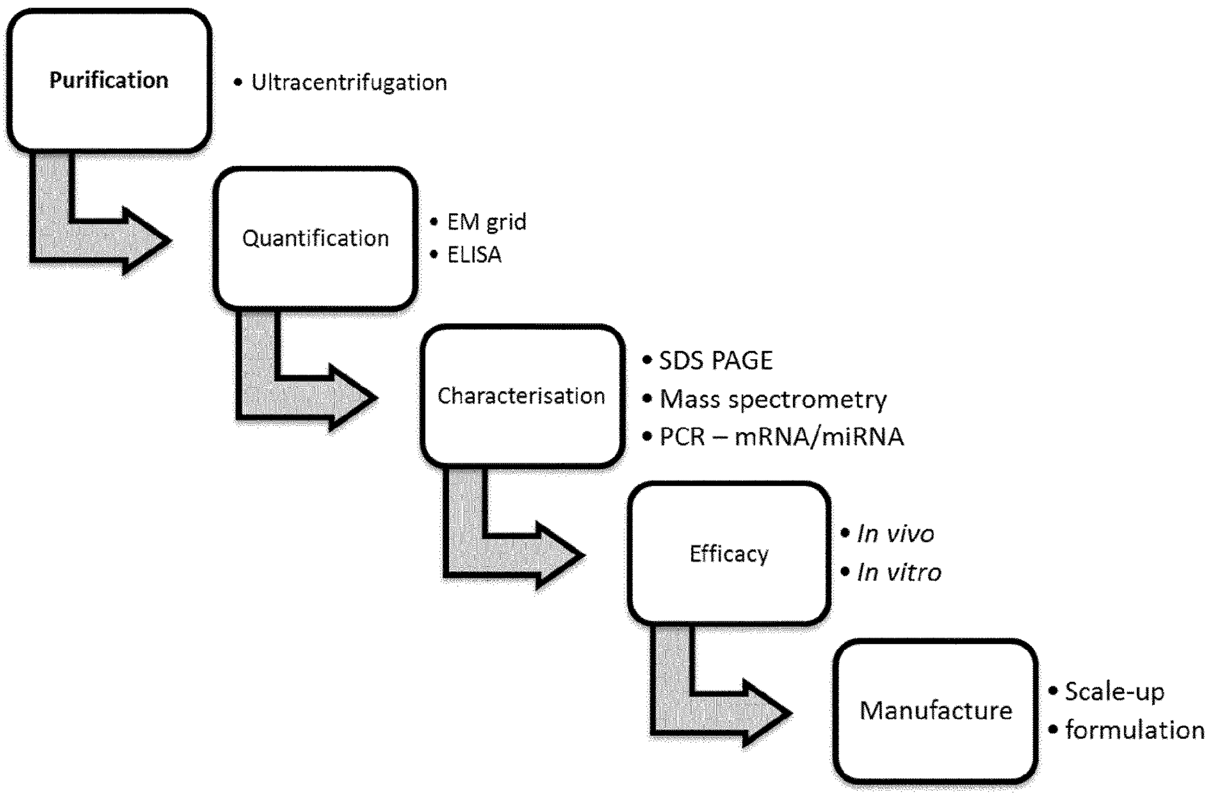
Purification
• Ultracentrifugation
Quantification
• EM grid
• ELISA
Characterisation
• SDS PAGE
• Mass spectrometry
• PCR – mRNA/miRNA
Efficacy
• In vivo
• In vitro
Manufacture
• Scale-up
• formulation

Figure 3

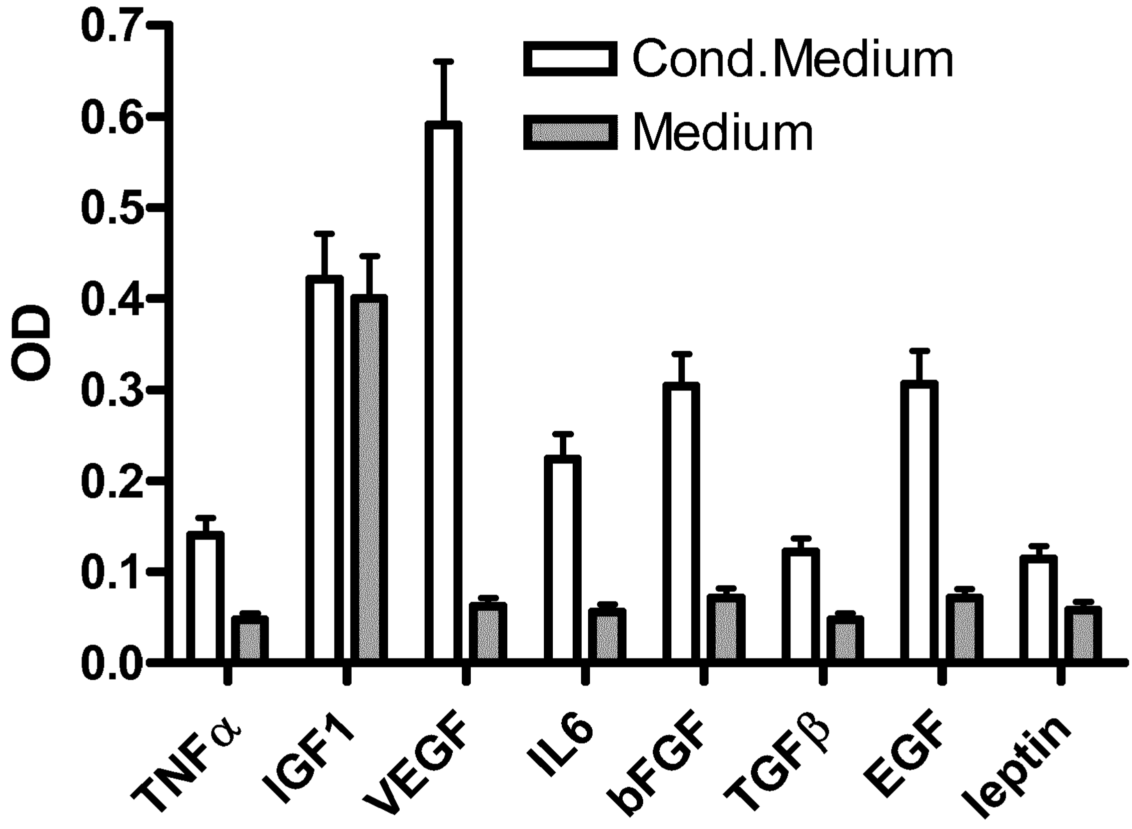
OD
0.7
0.6
0.5
0.4
0.3
0.2
0.1
0.0
Cond.Medium
Medium
TNFα
IGF1
VEGF
IL6
bFGF
TGFβ
EGF
leptin

Figure 4A

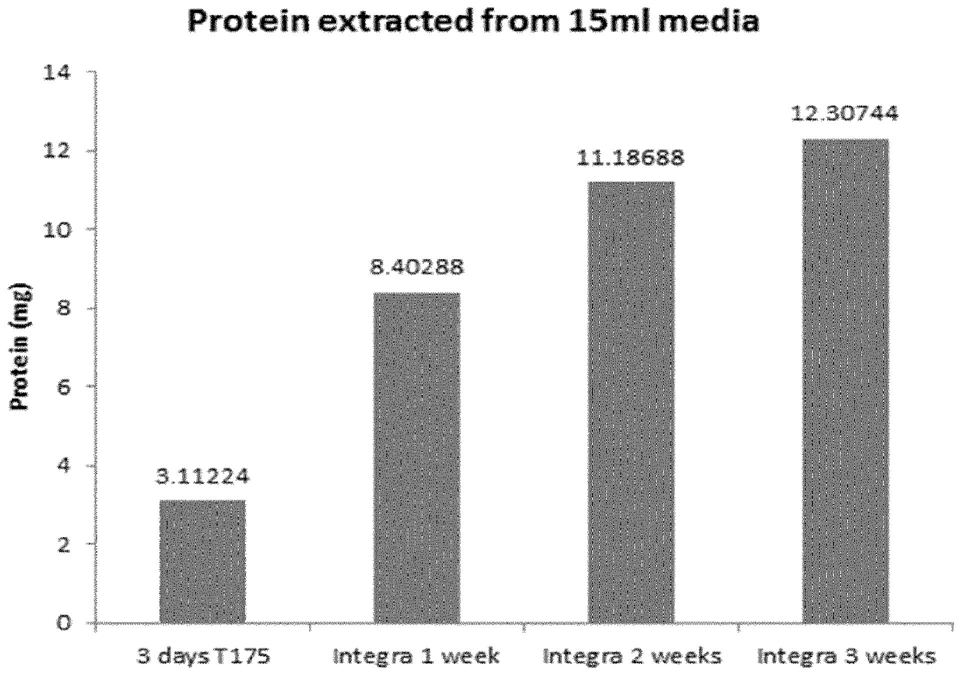


Figure 4B

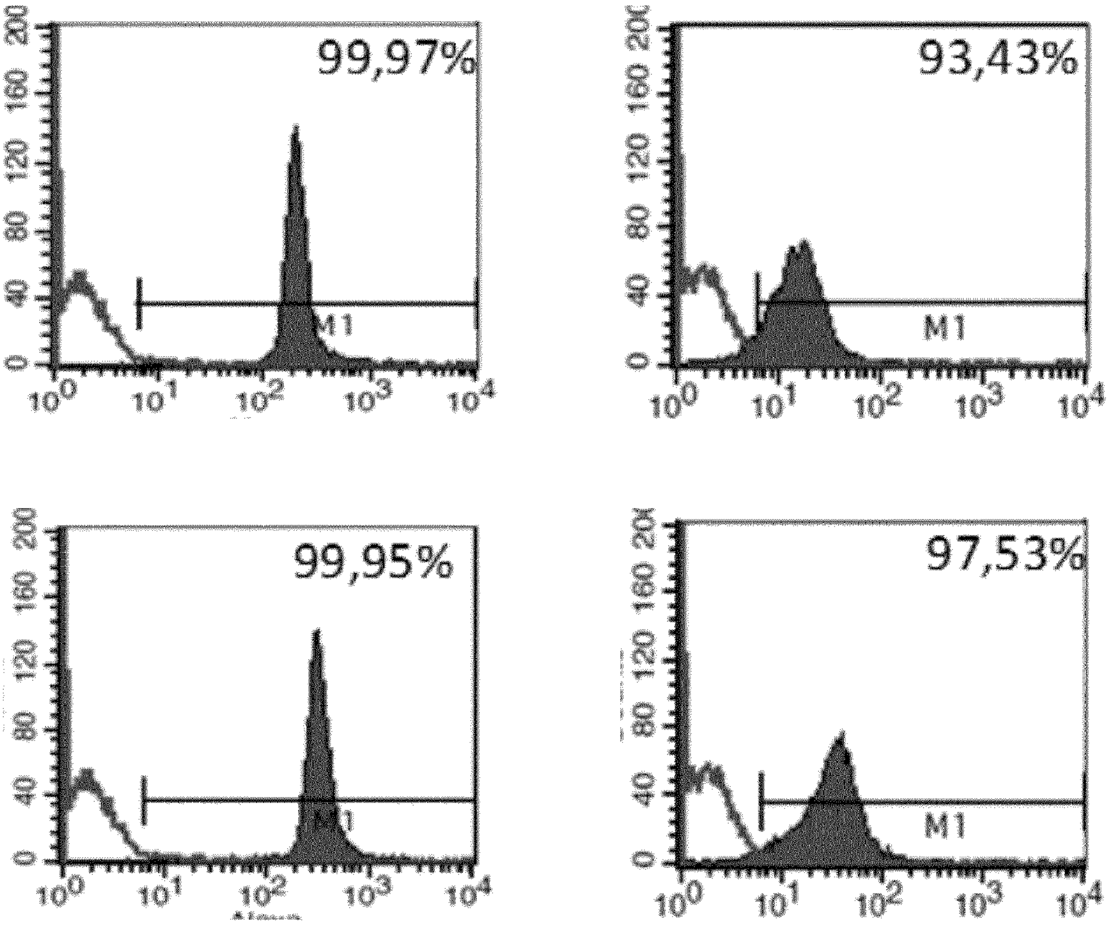

Figure 5

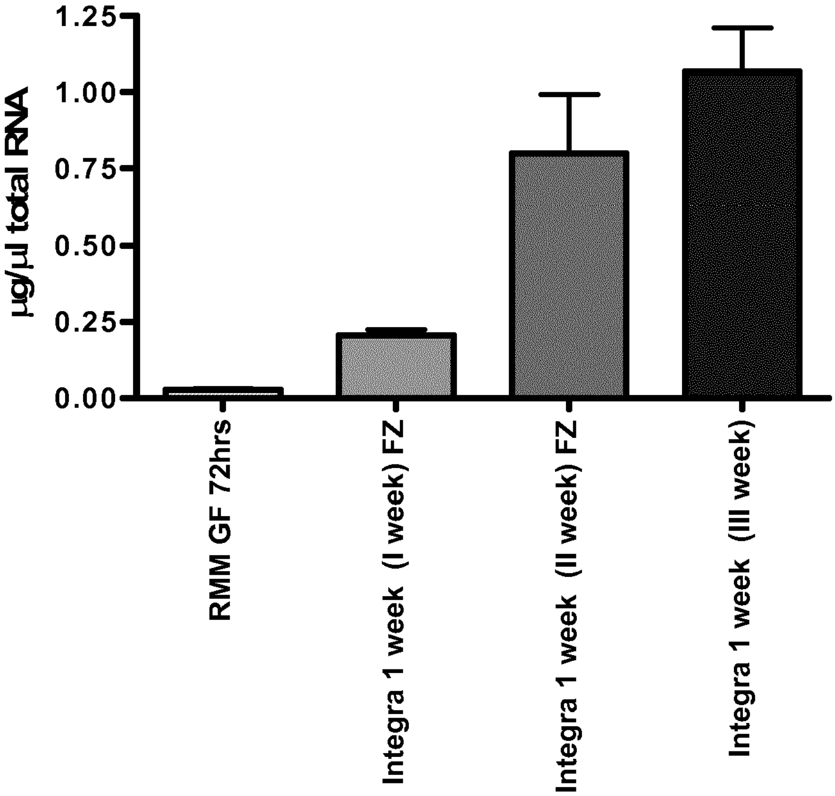
1.25
1.00
0.75
0.50
0.25
0.00
µg/µl total RNA
RMM GF 72hrs
Integra 1 week (I week) FZ
Integra 1 week (II week) FZ
Integra 1 week (III week)

Figure 6A

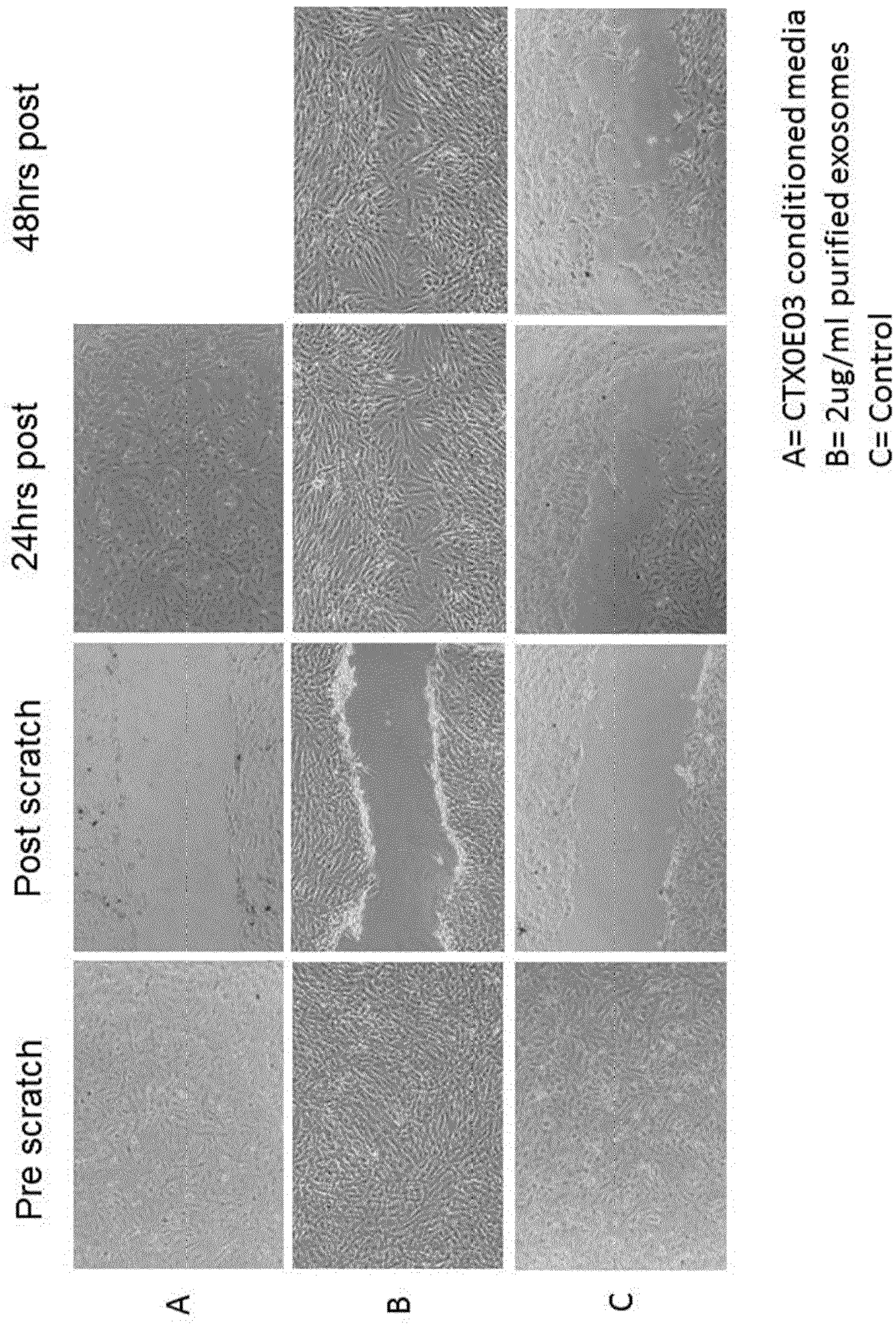
Pre scratch
Post scratch
24hrs post
48hrs post
A
B
C
A= CTX0E03 conditioned media
B= 2ug/ml purified exosomes
C= Control

Figure 6B

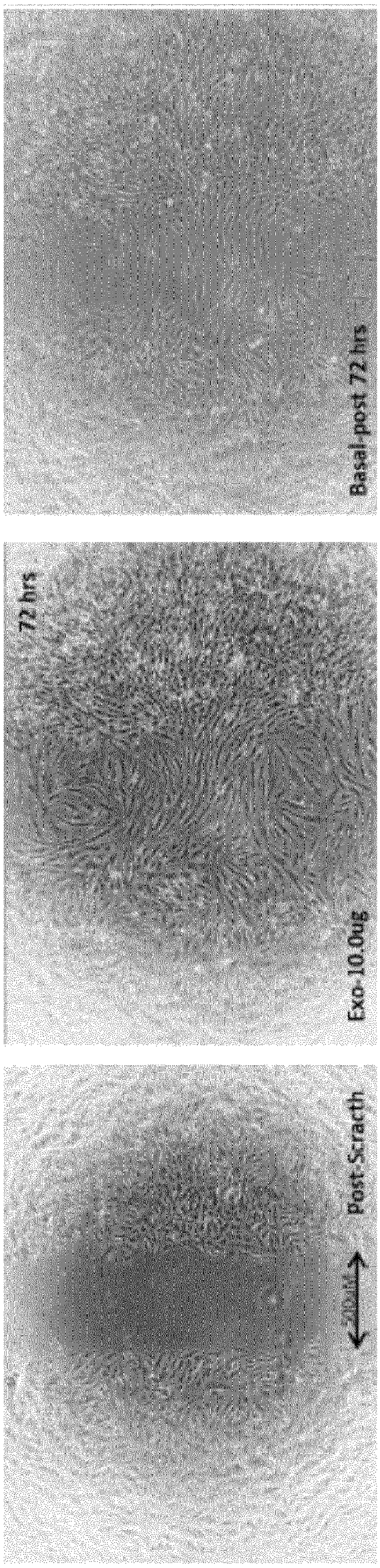

Figure 6C

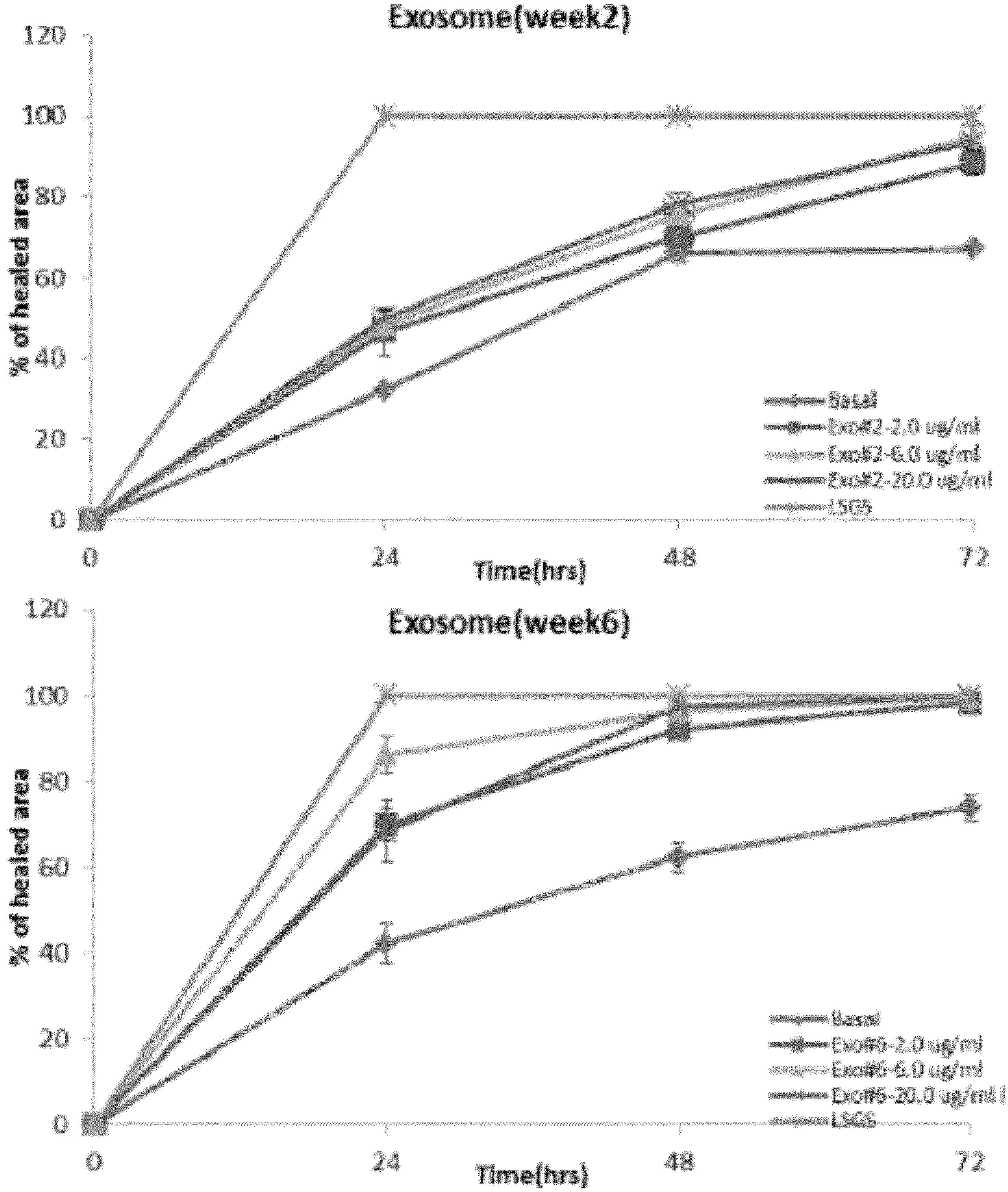
Exosome(week2)
% of healed area
Time(hrs)
Basal
Exo#2-2.0 ug/ml
Exo#2-6.0 ug/ml
Exo#2-20.0 ug/ml
LSGS
Exosome(week6)
% of healed area
Time(hrs)
Basal
Exo#6-2.0 ug/ml
Exo#6-6.0 ug/ml
Exo#6-20.0 ug/ml
LSGS

Figure 6D

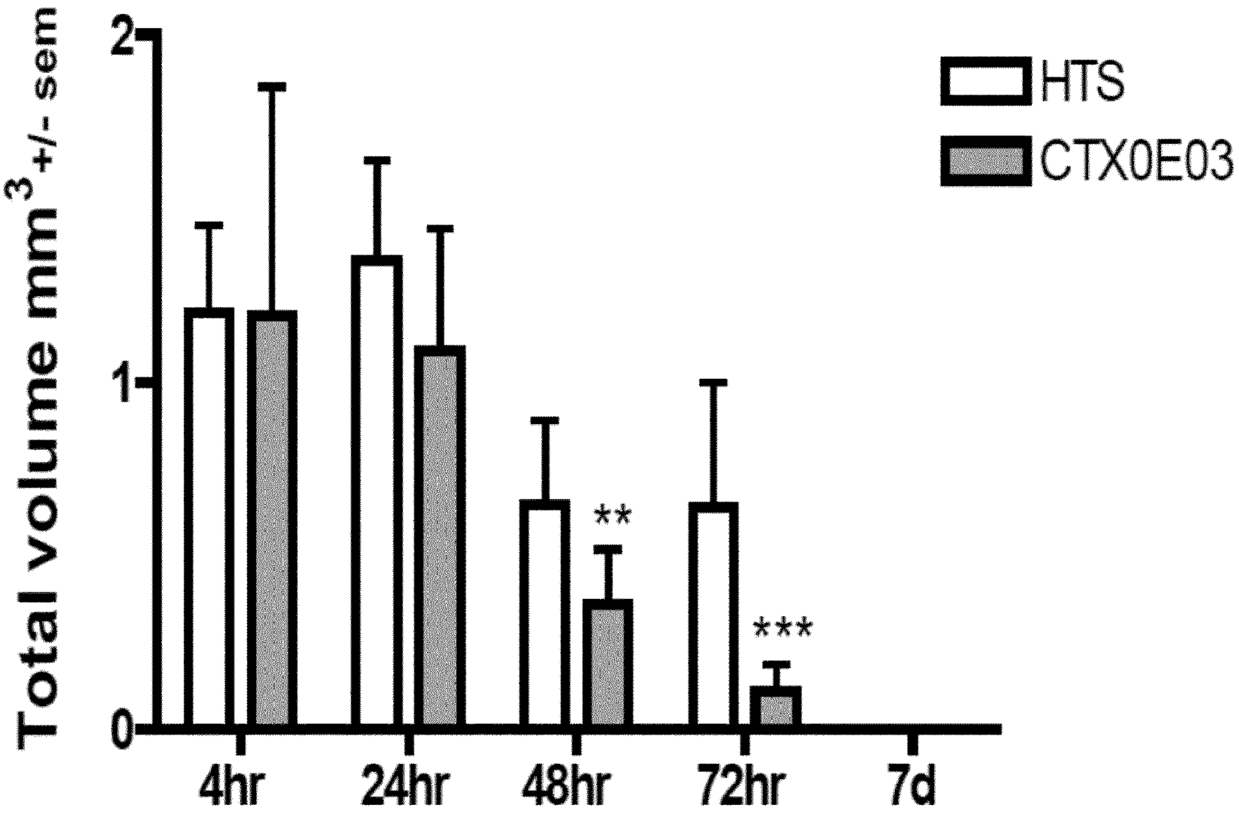

Figure 7

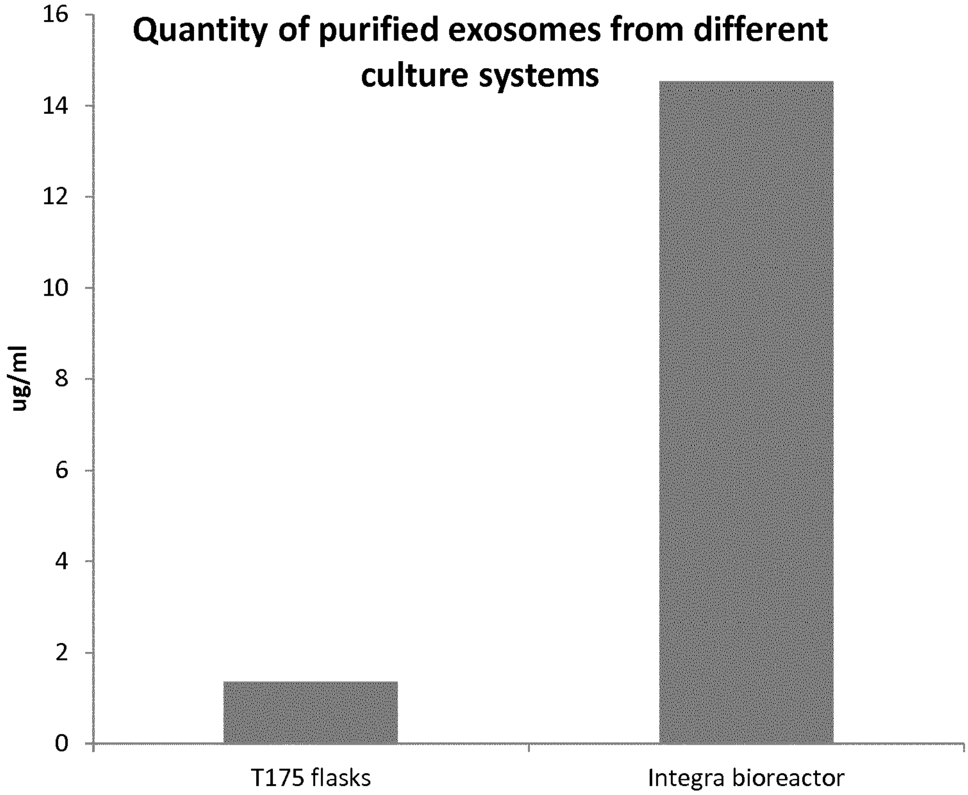
Quantity of purified exosomes from different culture systems
ug/ml
16
14
12
10
8
6
4
2
0
T175 flasks
Integra bioreactor

Figure 8A

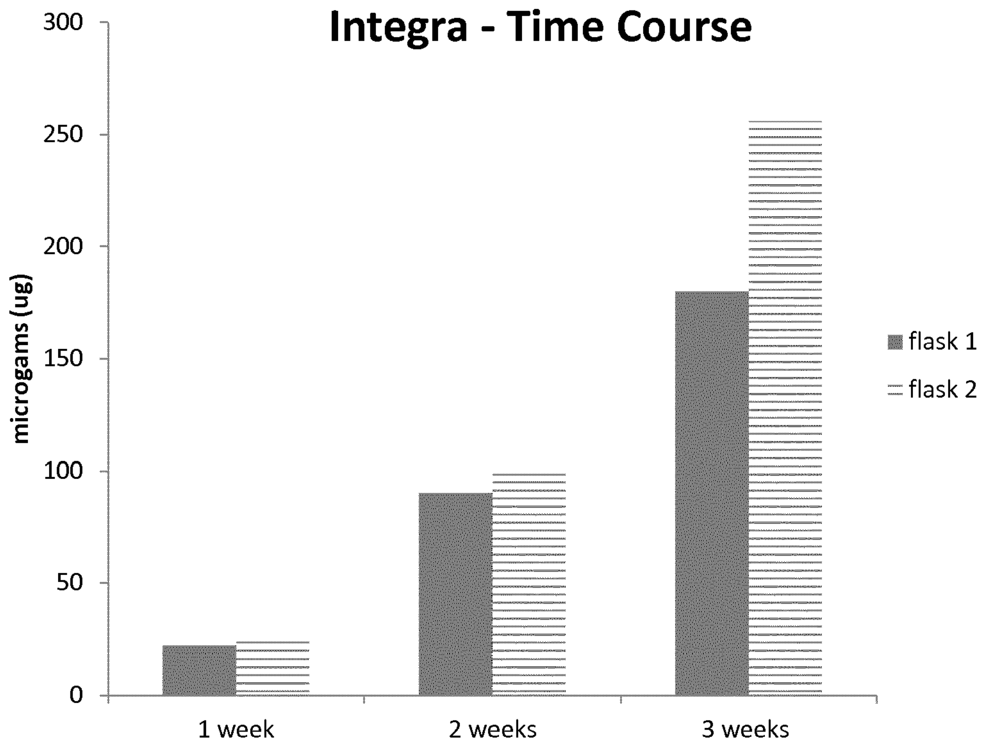


Figure 8B

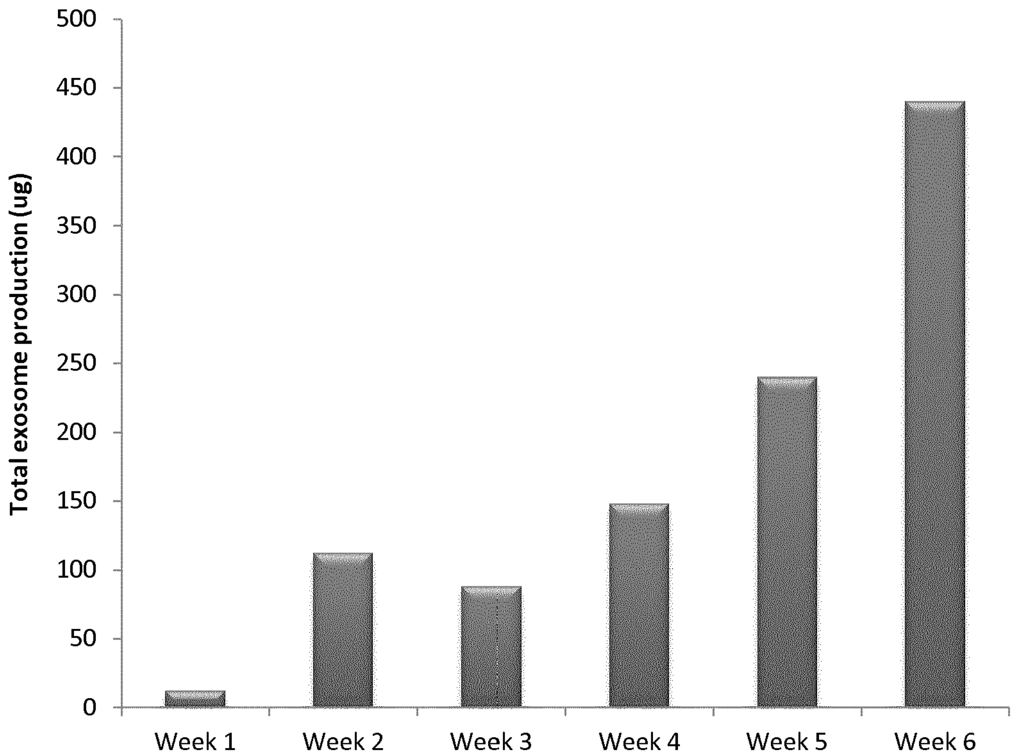

Figure 9

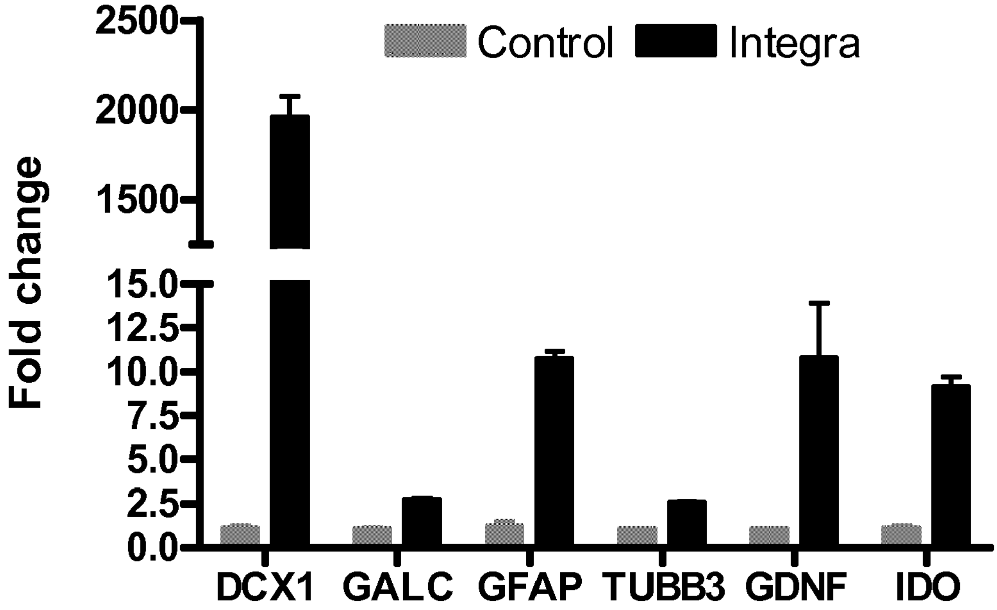
Control
Integra
Fold change
2500
2000
1500
15.0
12.5
10.0
7.5
5.0
2.5
0.0
DCX1
GALC
GFAP
TUBB3
GDNF
IDO

Figure 10

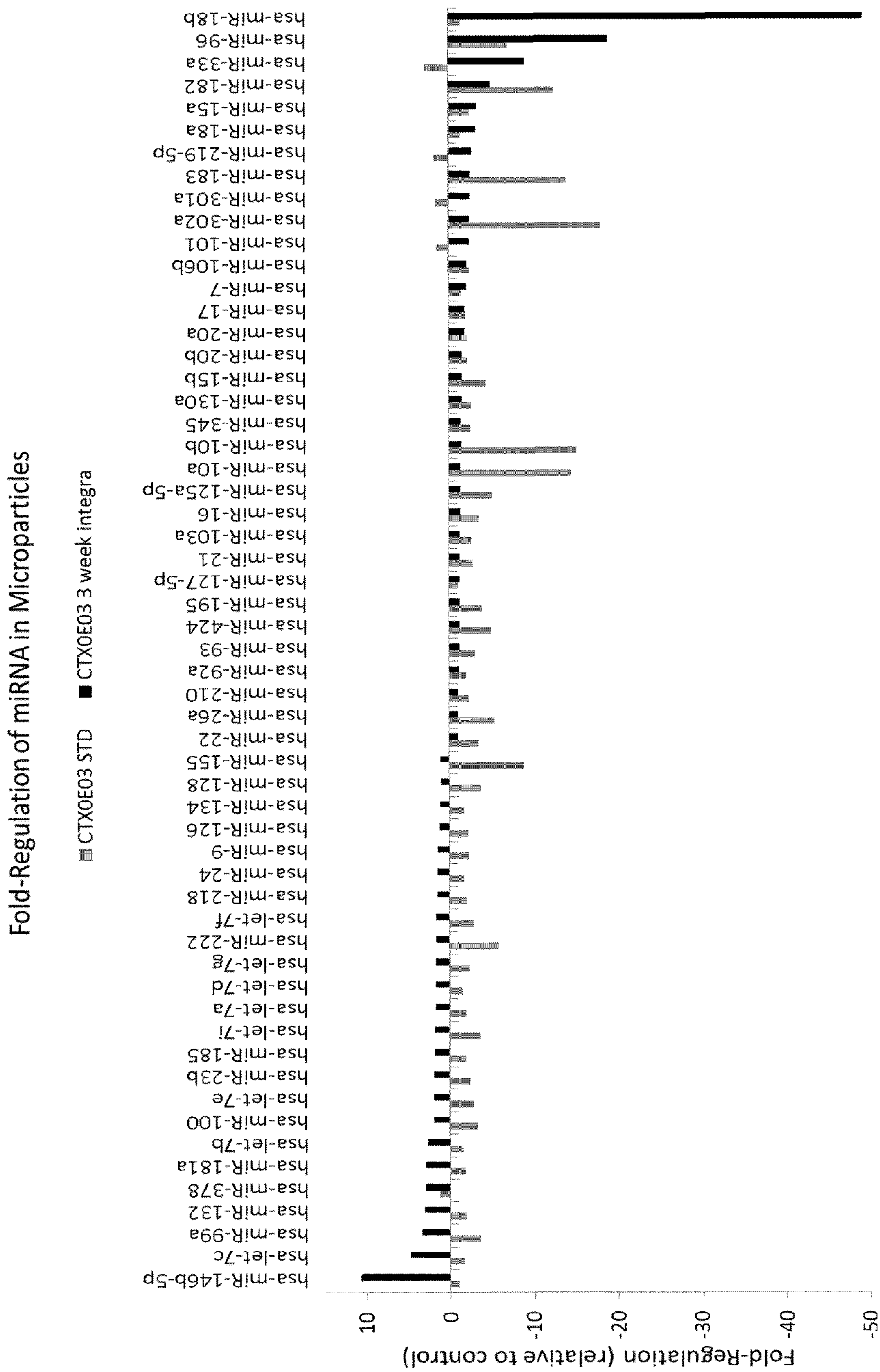
Fold-Regulation of miRNA in Microparticles
CTX0E03 STD
CTX0E03 3 week integra
hsa-miR-18b
hsa-miR-96
hsa-miR-33a
hsa-miR-182
hsa-miR-15a
hsa-miR-18a
hsa-miR-219-5p
hsa-miR-183
hsa-miR-301a
hsa-miR-302a
hsa-miR-101
hsa-miR-106b
hsa-miR-7
hsa-miR-17
hsa-miR-20a
hsa-miR-20b
hsa-miR-15b
hsa-miR-130a
hsa-miR-345
hsa-miR-10b
hsa-miR-10a
hsa-miR-125a-5p
hsa-miR-16
hsa-miR-103a
hsa-miR-21
hsa-miR-127-5p
hsa-miR-195
hsa-miR-424
hsa-miR-93
hsa-miR-92a
hsa-miR-210
hsa-miR-26a
hsa-miR-22
hsa-miR-155
hsa-miR-128
hsa-miR-134
hsa-miR-126
hsa-miR-9
hsa-miR-24
hsa-miR-218
hsa-let-7f
hsa-miR-222
hsa-let-7g
hsa-let-7d
hsa-let-7a
hsa-let-7i
hsa-miR-185
hsa-miR-23b
hsa-let-7e
hsa-miR-100
hsa-let-7b
hsa-miR-181a
hsa-miR-378
hsa-miR-132
hsa-miR-99a
hsa-let-7c
hsa-miR-146b-5p
10
0
-10
-20
-30
-40
-50
Fold-Regulation (relative to control)

Figure 11a

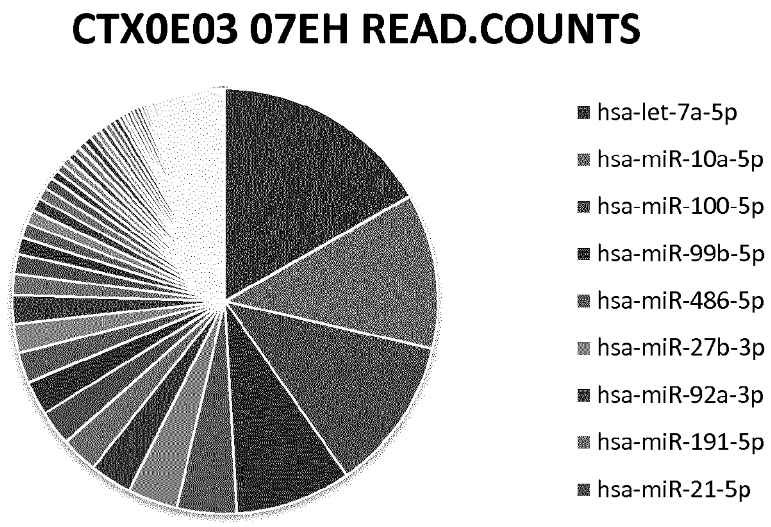
CTX0E03 07EH READ.COUNTS
hsa-let-7a-5p
hsa-miR-10a-5p
hsa-miR-100-5p
hsa-miR-99b-5p
hsa-miR-486-5p
hsa-miR-27b-3p
hsa-miR-92a-3p
hsa-miR-191-5p
hsa-miR-21-5p

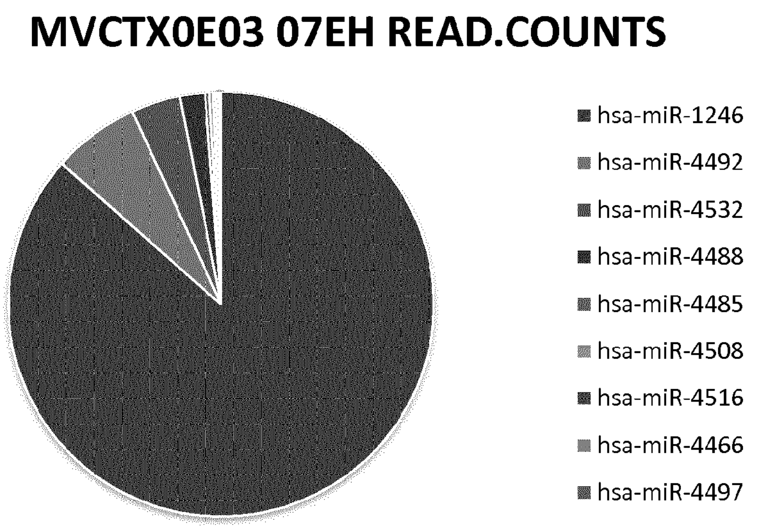
MVCTX0E03 07EH READ.COUNTS
hsa-miR-1246
hsa-miR-4492
hsa-miR-4532
hsa-miR-4488
hsa-miR-4485
hsa-miR-4508
hsa-miR-4516
hsa-miR-4466
hsa-miR-4497

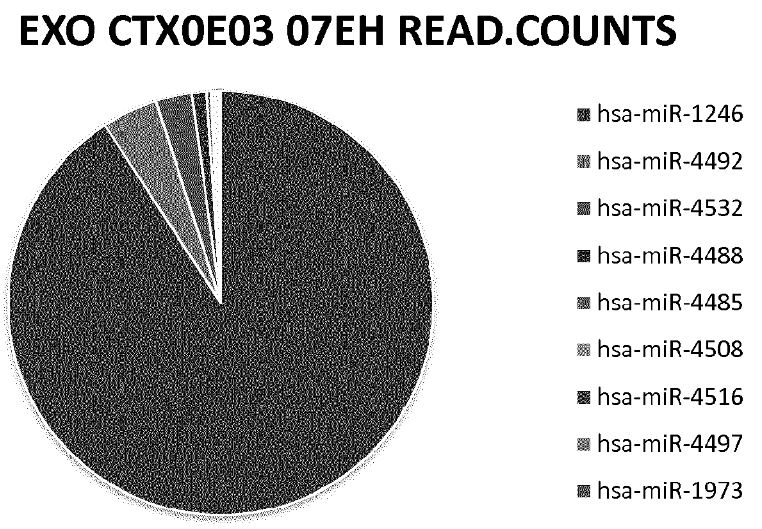
EXO CTX0E03 07EH READ.COUNTS
hsa-miR-1246
hsa-miR-4492
hsa-miR-4532
hsa-miR-4488
hsa-miR-4485
hsa-miR-4508
hsa-miR-4516
hsa-miR-4497
hsa-miR-1973

Figure 11b

## CTX0E03 07EI READ.COUNTS

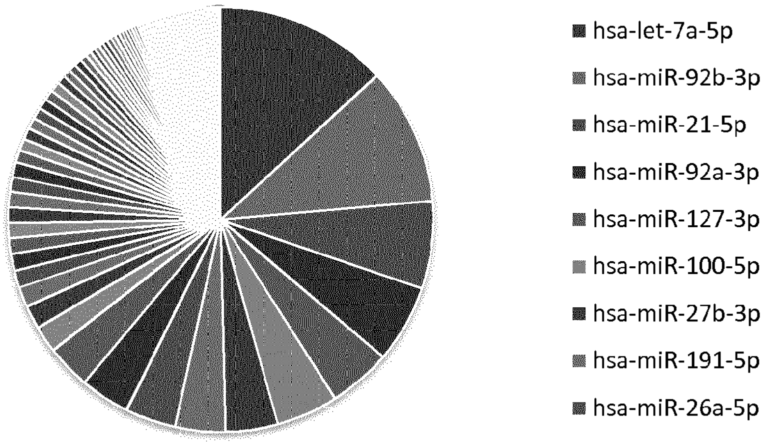


## MVCTX0E03 07EI READ.COUNTS

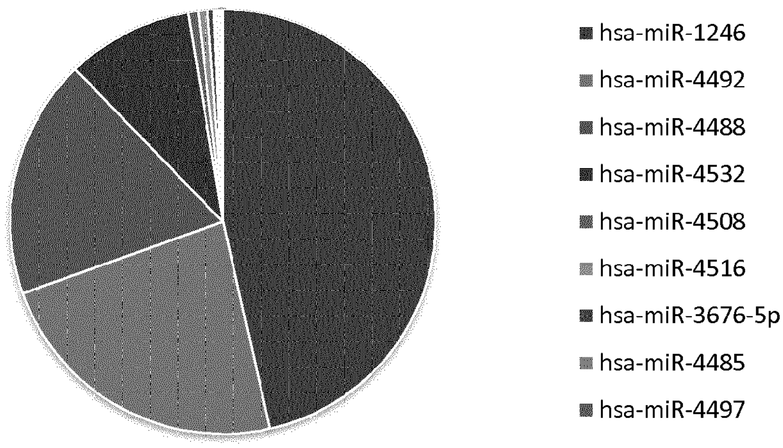


## EXO CTX0E03 07EI READ.COUNTS

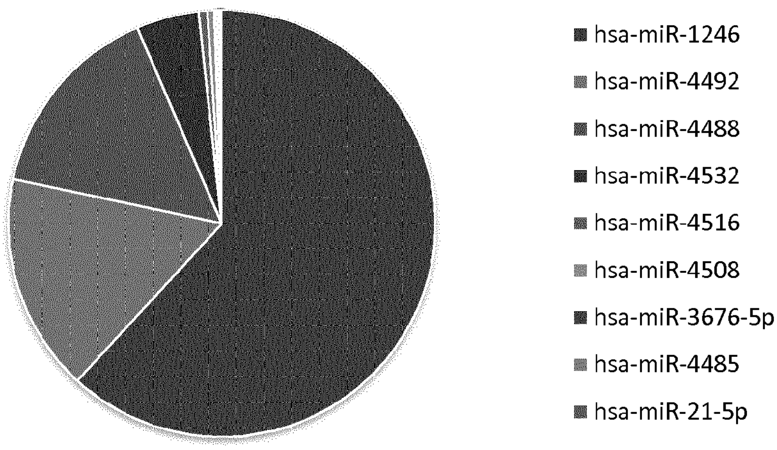

Figure 12A

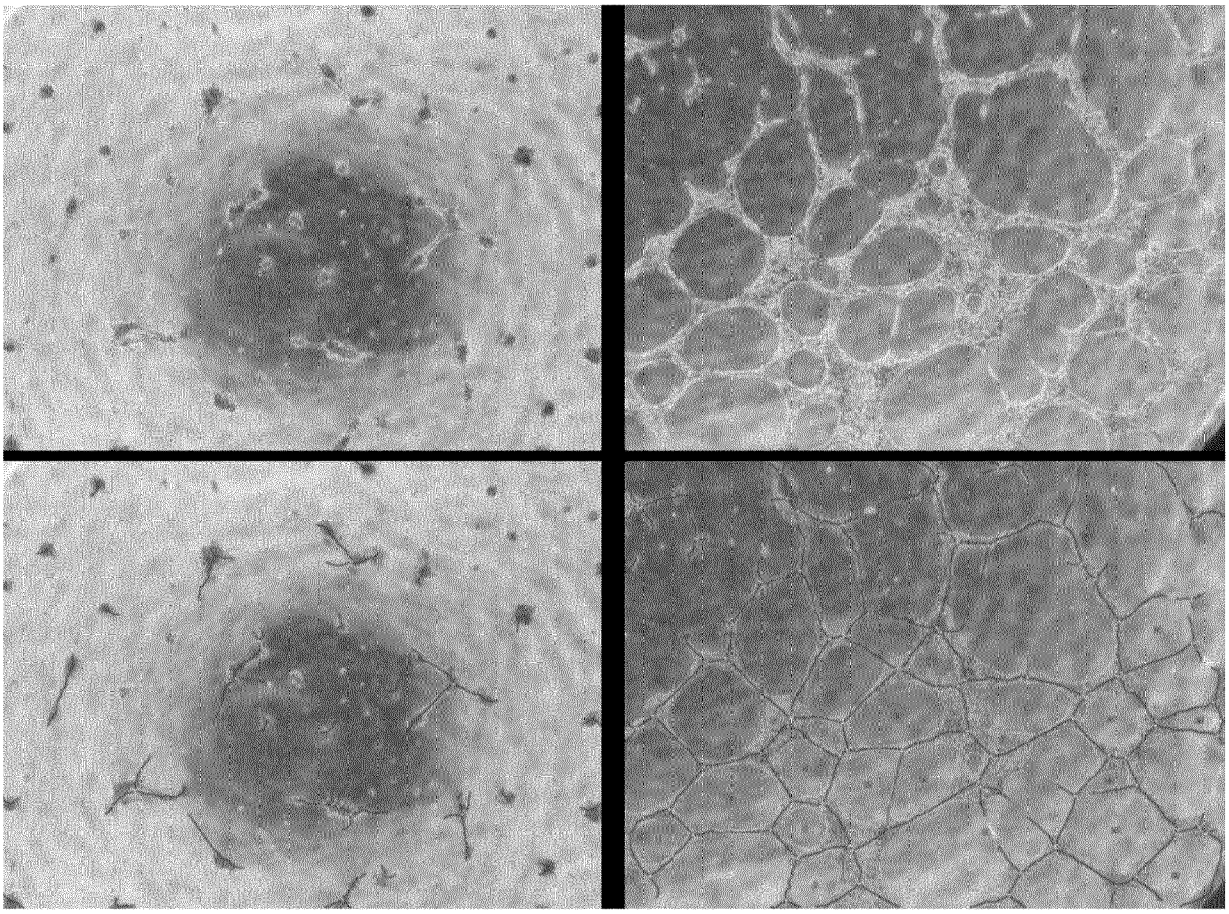

Figure 12B

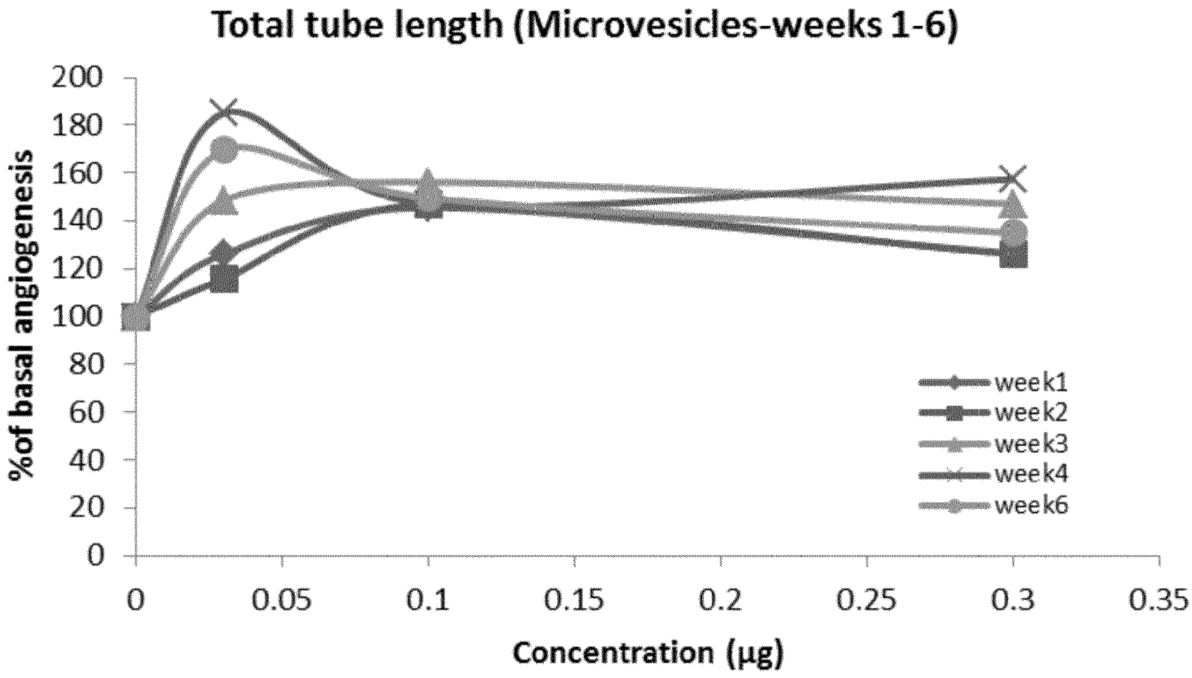

Figure 12C

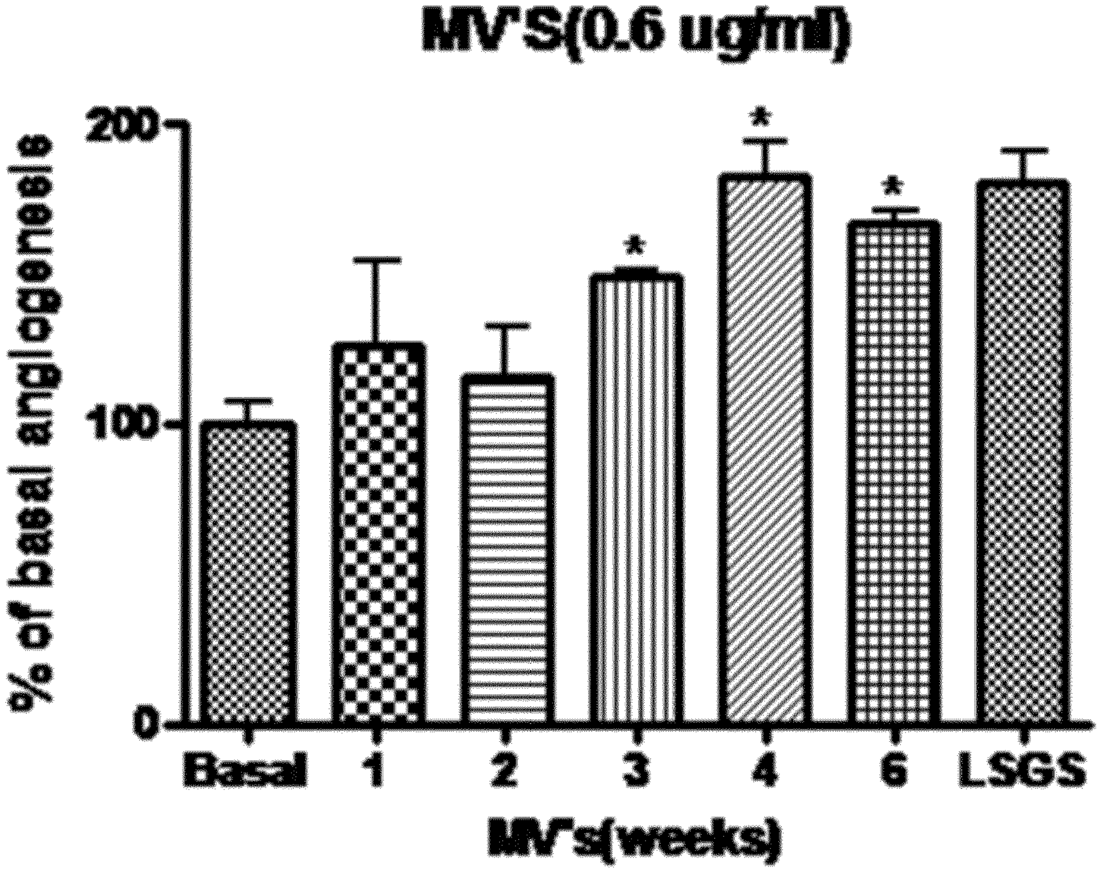
MV'S(0.6 ug/ml)
% of basal angiogenesis
200
100
0
*
*
*
Basal
1
2
3
4
6
LSGS
MV's(weeks)

Figure 13

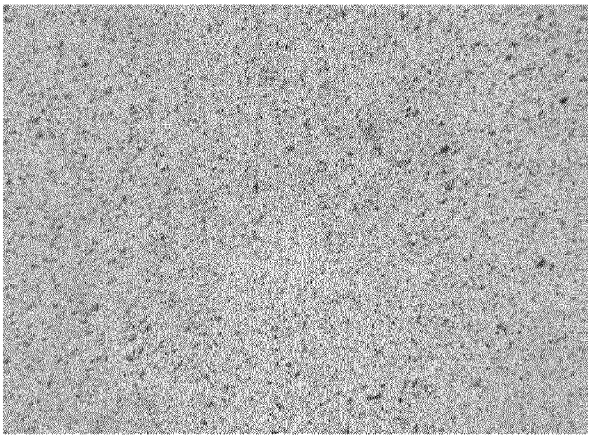

Basal

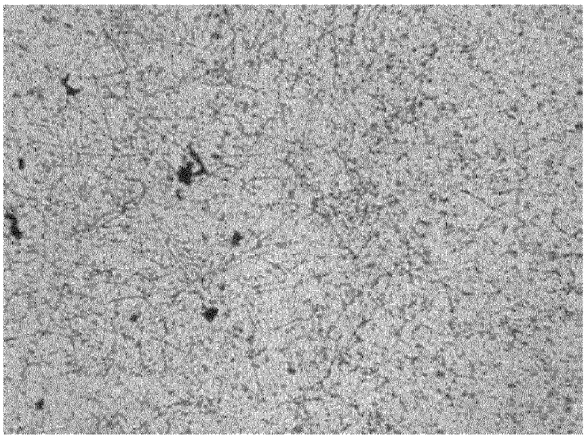

100ng/ml NGF

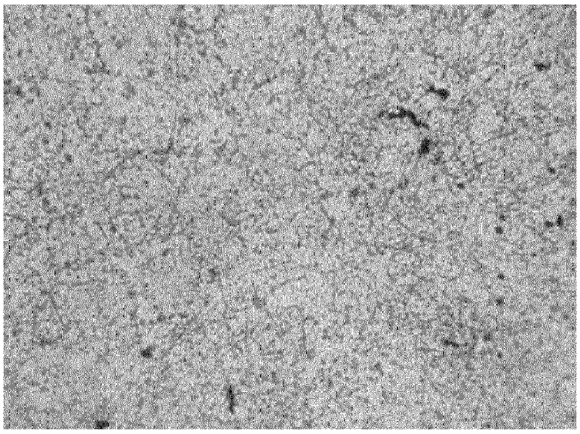

0.03µg week 2 Integra microvesicles
+ 100ng/ml NGF

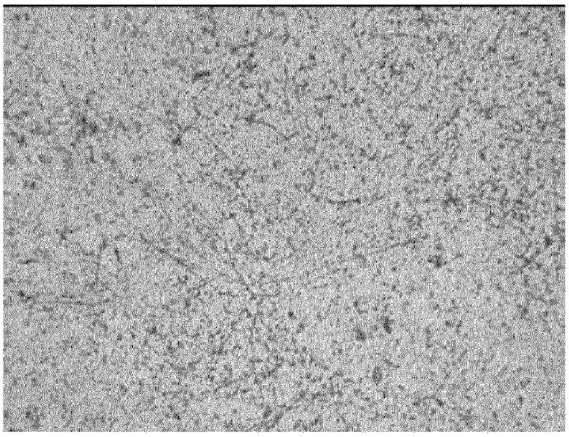

0.3µg week 2 Integra microvesicles
+ 100ng/ml NGF

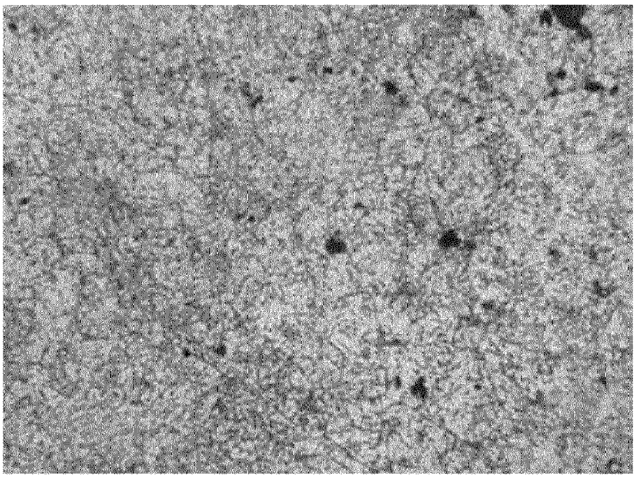

3µg week 2 Integra microvesicles + 100ng/ml NGF

Figure 14

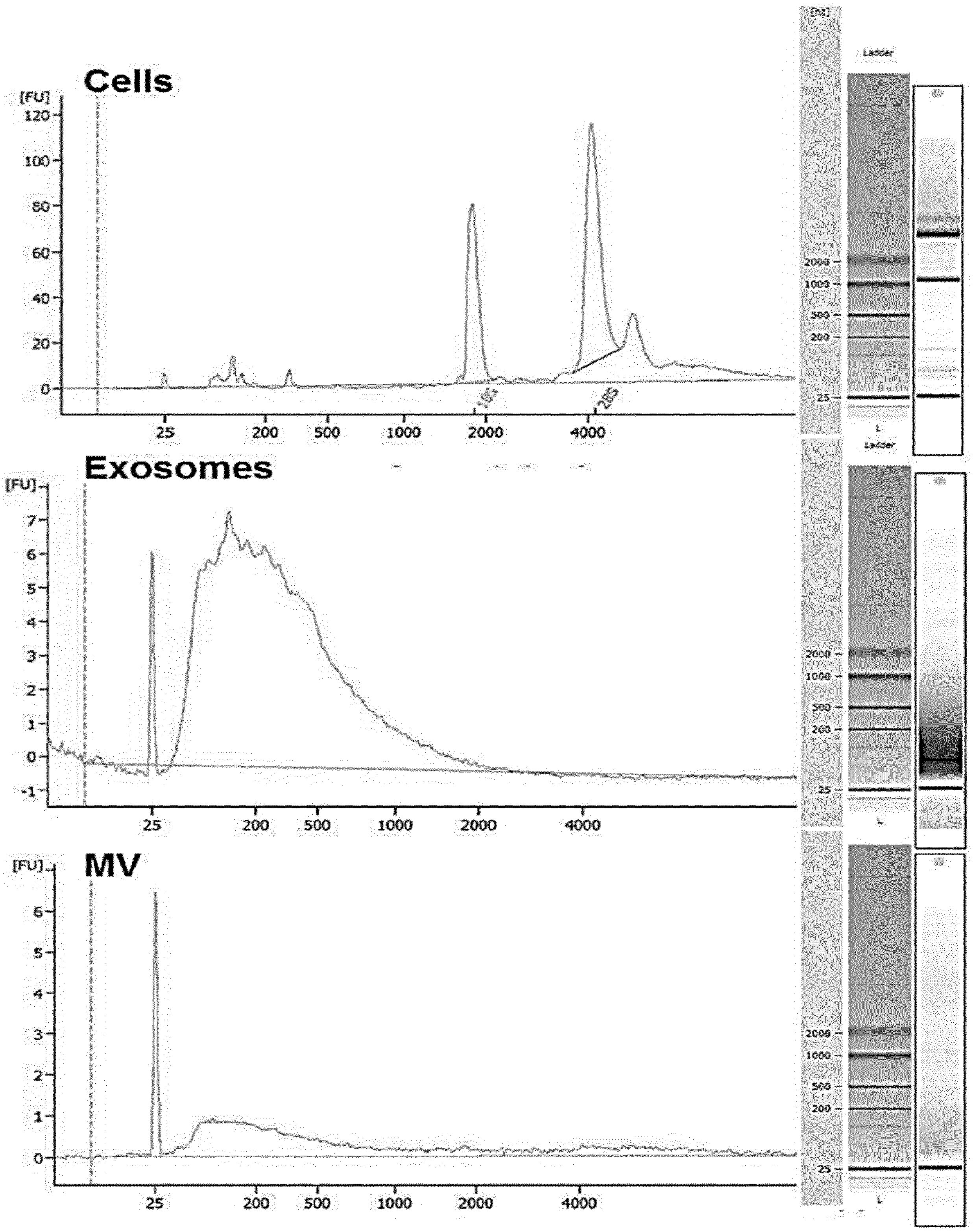
Cells
[FU]
120
100
80
60
40
20
0
25
200
500
1000
2000
4000
Exosomes
7
6
5
4
3
2
1
0
-1
MV
[nt]
Ladder
2000
1000
500
200
25
L

Figure 15

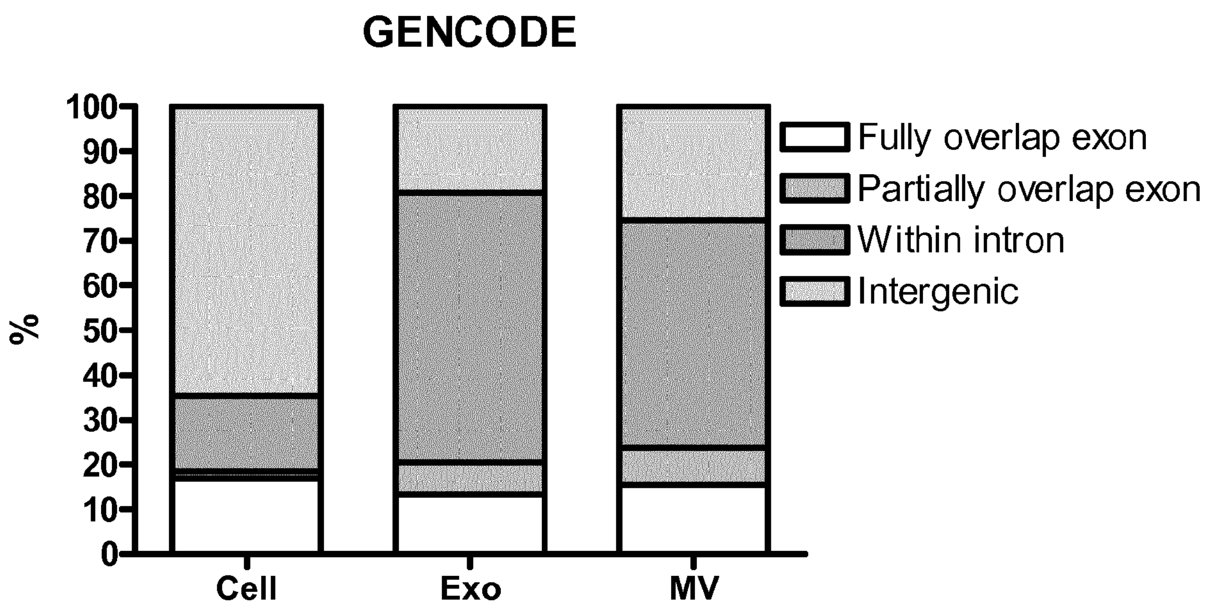


Figure 16

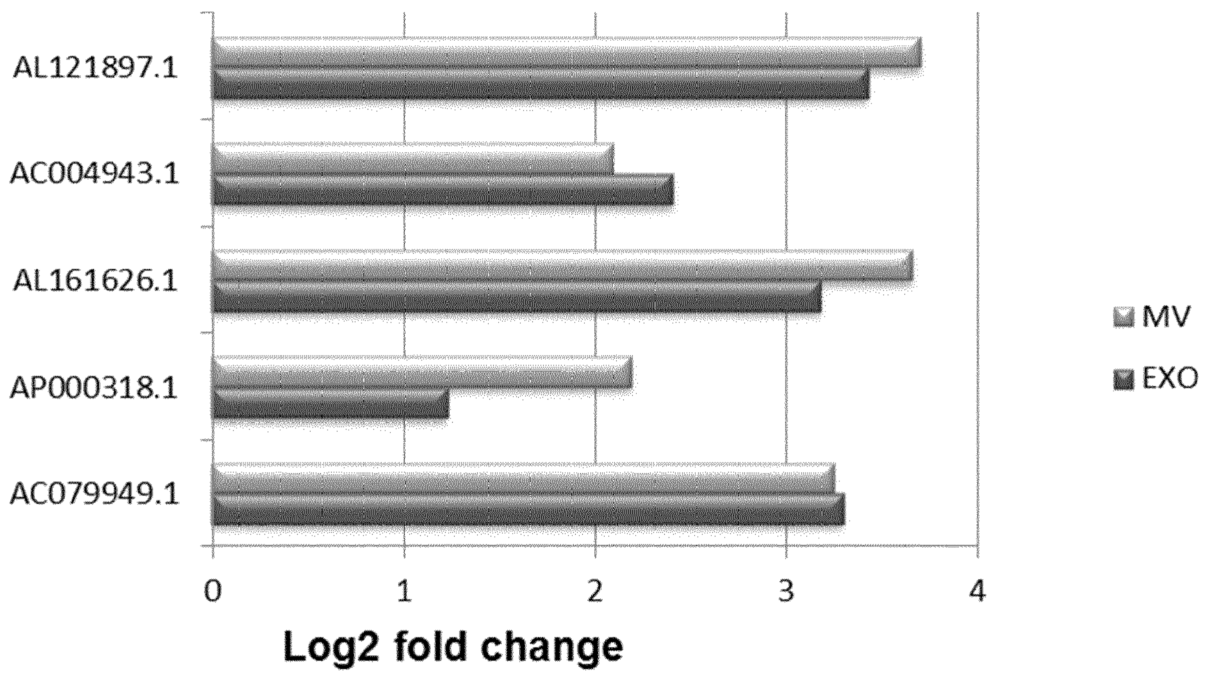

Figure 17A

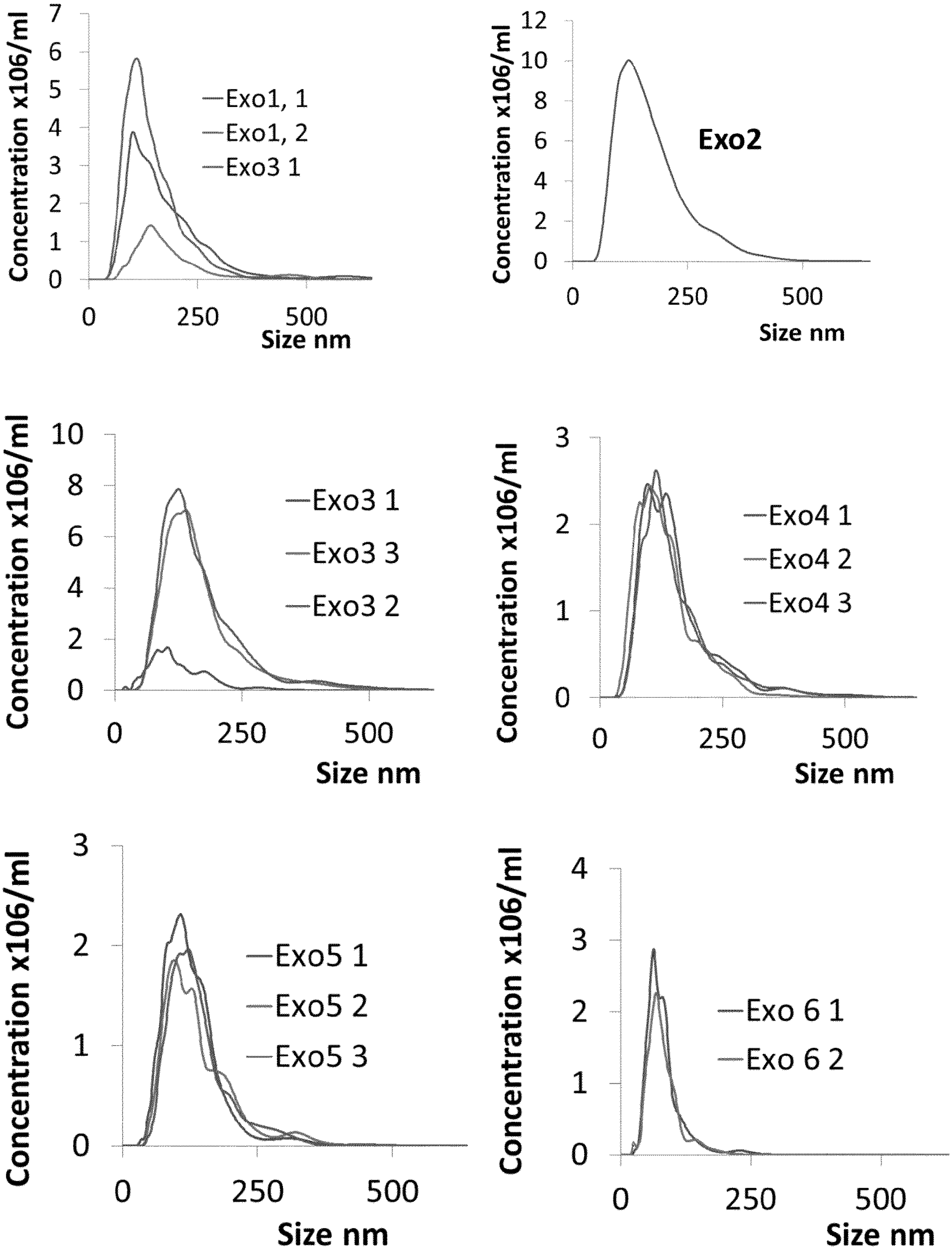
Concentration x106/ml
7
6
5
4
3
2
1
0
0
250
500
Size nm
Exo1, 1
Exo1, 2
Exo3 1
Concentration x106/ml
12
10
8
6
4
2
0
0
250
500
Size nm
Exo2
Concentration x106/ml
10
8
6
4
2
0
0
250
500
Size nm
Exo3 1
Exo3 3
Exo3 2
Concentration x106/ml
3
2
1
0
0
250
500
Size nm
Exo4 1
Exo4 2
Exo4 3
Concentration x106/ml
3
2
1
0
0
250
500
Size nm
Exo5 1
Exo5 2
Exo5 3
Concentration x106/ml
4
3
2
1
0
0
250
500
Size nm
Exo 6 1
Exo 6 2

Figure 17B

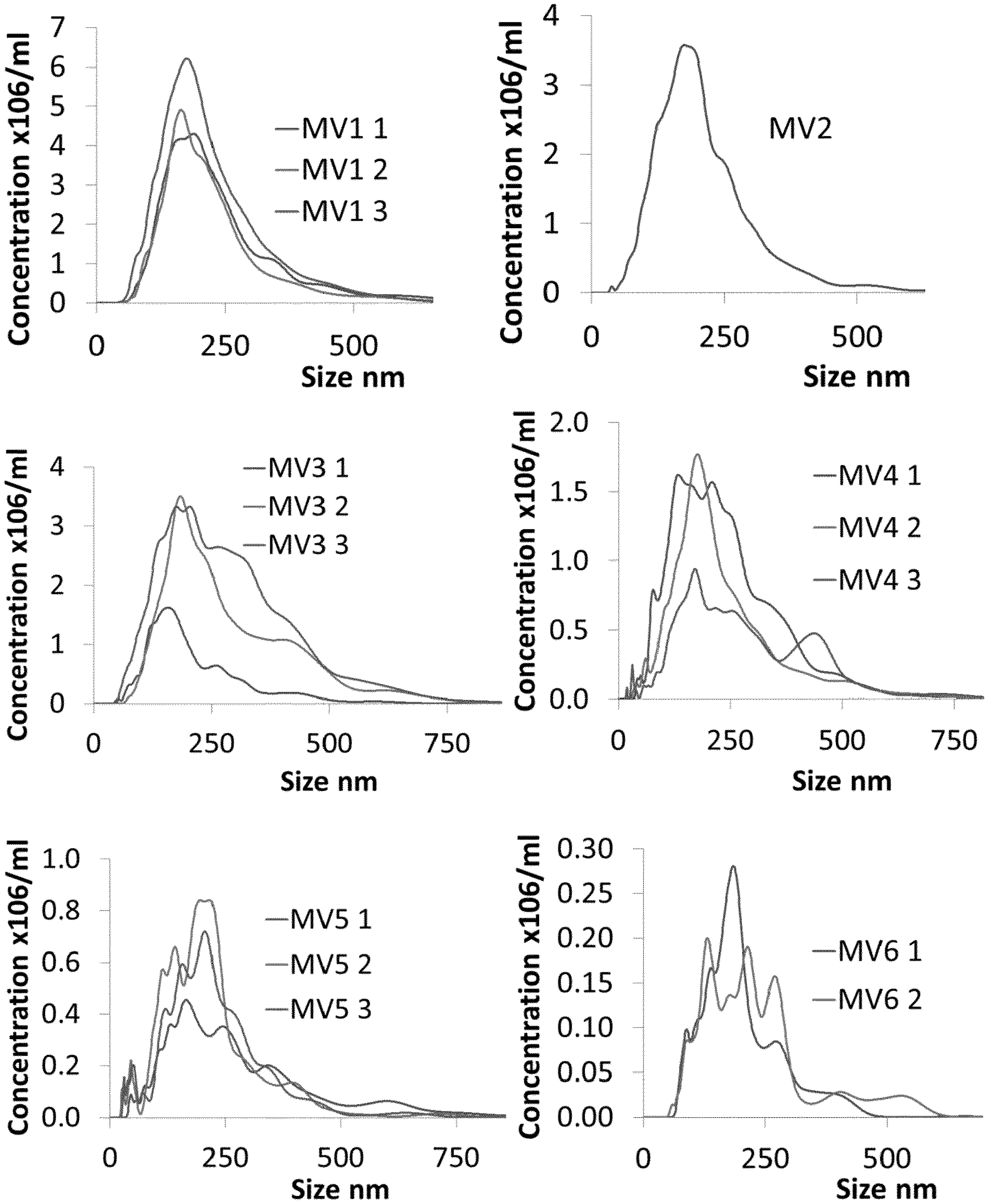
Concentration x106/ml
Size nm
MV1 1
MV1 2
MV1 3
MV2
MV3 1
MV3 2
MV3 3
MV4 1
MV4 2
MV4 3
MV5 1
MV5 2
MV5 3
MV6 1
MV6 2

Figure 18A

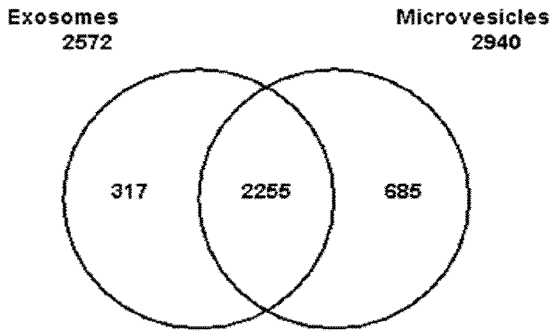


Figure 18B

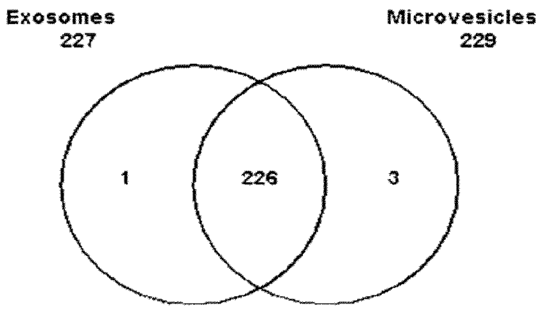


Figure 18C

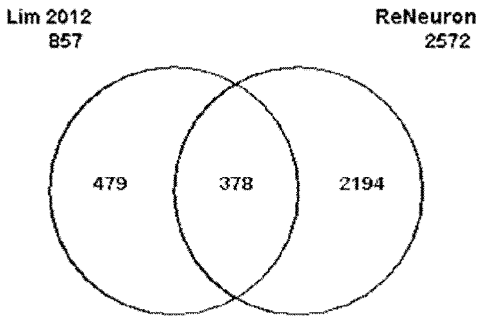


Figure 18D

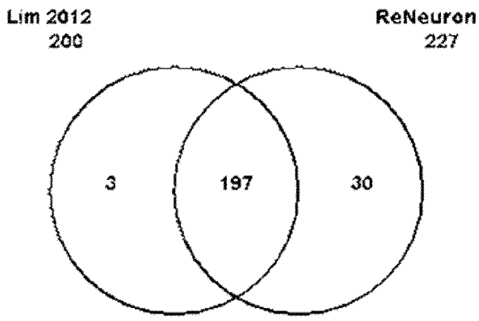

Figure 19

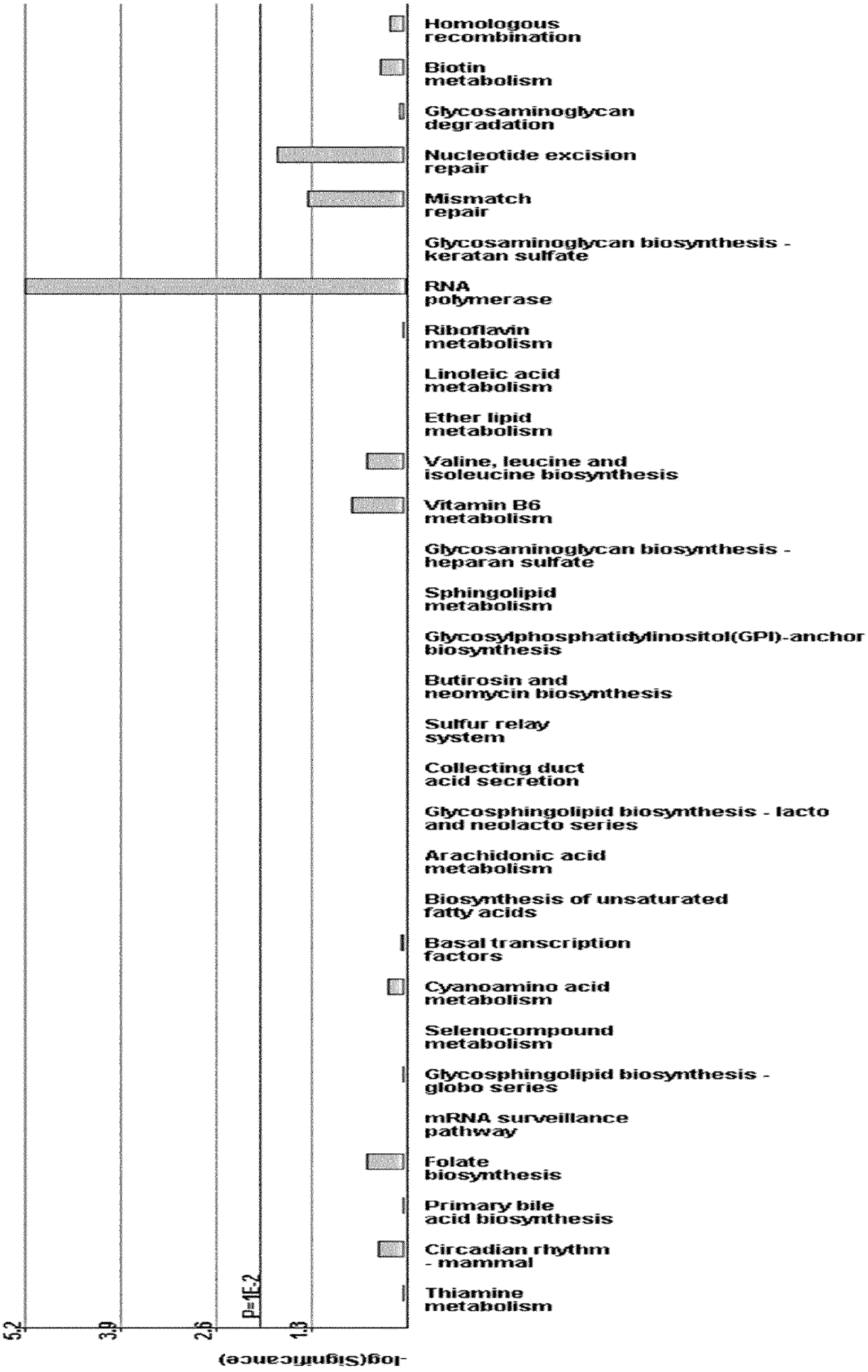
Homologous recombination
Biotin metabolism
Glycosaminoglycan degradation
Nucleotide excision repair
Mismatch repair
Glycosaminoglycan biosynthesis - keratan sulfate
RNA polymerase
Riboflavin metabolism
Linoleic acid metabolism
Ether lipid metabolism
Valine, leucine and isoleucine biosynthesis
Vitamin B6 metabolism
Glycosaminoglycan biosynthesis - heparan sulfate
Sphingolipid metabolism
Glycosylphosphatidylinositol(GPI)-anchor biosynthesis
Butirosin and neomycin biosynthesis
Sulfur relay system
Collecting duct acid secretion
Glycosphingolipid biosynthesis - lacto and neolacto series
Arachidonic acid metabolism
Biosynthesis of unsaturated fatty acids
Basal transcription factors
Cyanoamino acid metabolism
Selenocompound metabolism
Glycosphingolipid biosynthesis - globo series
mRNA surveillance pathway
Folate biosynthesis
Primary bile acid biosynthesis
Circadian rhythm - mammal
Thiamine metabolism
P=1E-2
5.2
3.9
2.6
1.3
-log(Significance)

Europäisches Patentamt
European Patent Office
Office européen des brevets

# EUROPEAN SEARCH REPORT

Application Number
EP 18 19 0631

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2011/062244 A1 (KURODA MASAHIKO [JP]; TANAKA MASAMI; MIZUTANI TAKAYUKI; TAKANASHI MASA) 26 May 2011 (2011-05-26)<br>* abstract * | 1-7,10, 11<br><br><br>12-15 | INV.<br>A61K31/711<br>C12N5/0797<br>A61P25/28<br>A61P11/00<br>A61P9/00 |
| X<br><br>Y | KR 2010 0081003 A (SNU R&DB FOUNDATION [KR]) 14 July 2010 (2010-07-14)<br>* abstract * | 1-4,7-11<br><br>12-15 | |
| X | WO 2006/087233 A2 (MAX PLANCK GESELLSCHAFT [DE]; CORBEIL DENIS [DE]; HUTTNER WIELAND B [D) 24 August 2006 (2006-08-24)<br>* claim 1; example 2 * | 1,2,10 | |
| X | DUKJIN KANG ET AL: "Proteomic Analysis of Exosomes from Human Neural Stem Cells by Flow Field-Flow Fractionation and Nanoflow Liquid Chromatography-Tandem Mass Spectrometry",<br>JOURNAL OF PROTEOME RESEARCH,<br>vol. 7, no. 8, 1 August 2008 (2008-08-01), pages 3475-3480, XP055066064,<br>ISSN: 1535-3893, DOI: 10.1021/pr800225z<br>* page 3477 - page 3478; table 2 * | 1-4,10 | TECHNICAL FIELDS SEARCHED (IPC)<br>A61K<br>C12N |
| Y | LYDIA ALVAREZ-ERVITI ET AL: "Delivery of siRNA to the mouse brain by systemic injection of targeted exosomes",<br>NATURE BIOTECHNOLOGY,<br>vol. 29, no. 4, 20 March 2011 (2011-03-20), pages 341-345, XP055554900,<br>ISSN: 1087-0156, DOI: 10.1038/nbt.1807<br>* pages 341-342 *<br>-/-- | 12-15 | |

The present search report has been drawn up for all claims

| Place of search | Date of completion of the search | Examiner |
|---|---|---|
| Munich | 11 February 2019 | Bochelen, Damien |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 19 0631

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | POLLOCK K ET AL: "A conditionally immortal clonal stem cell line from human cortical neuroepithelium for the treatment of ischemic stroke", EXPERIMENTAL NEUROLOGY, ACADEMIC PRESS, NEW YORK, NY, US, vol. 199, no. 1, 1 May 2006 (2006-05-01), pages 143-155, XP024946020, ISSN: 0014-4886, DOI: 10.1016/J.EXPNEUROL.2005.12.011 [retrieved on 2006-05-01] * page 147 - page 149 * ----- | 1-15 | |
| A | PARK DONG-HYUK ET AL: "Increased neuronal proliferation in the dentate gyrus of aged rats following neural stem cell implantation", STEM CELLS AND DEVELOPMENT, MARY ANN LIEBERT, INC, NEW ROCHELLE, NY, US, vol. 19, no. 2, 1 February 2010 (2010-02-01), pages 175-180, XP009152757, ISSN: 1557-8534 * pages 176-177 * ----- | 1-15 | TECHNICAL FIELDS SEARCHED (IPC) |

The present search report has been drawn up for all claims

| Place of search | Date of completion of the search | Examiner |
|---|---|---|
| Munich | 11 February 2019 | Bochelen, Damien |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 18 19 0631

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2019

| Patent document cited in search report | Publication date | Patent family member(s) | Publication date |
|---|---|---|---|
| WO 2011062244 A1 | 26-05-2011 | NONE | |
| KR 20100081003 A | 14-07-2010 | NONE | |
| WO 2006087233 A2 | 24-08-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009105044 A, Timmers
- US 5851832 A
- US 6777233 B
- US 6468794 B
- US 5753506 A
- WO 2005121318 A
- US 7416888 B
- EP 1645626 B1
- US 7514259 B
- WO 2012004611 A


### Non-patent literature cited in the description


- **HASSANI ; O'REILLY ; PEARSE ; STROEMER et al.** *PLoS One,* 2012, vol. 7, 11
- **GENNARO.** Remington: The Science and Practice of Pharmacy. 2000
- **E W MARTIN.** *Remington's Pharmaceutical Sciences*
- **AMBROS et al.** *RNA,* 2003, vol. 9, 277-279
- **BANERJEE, S. ; WILLIAMSON, D. ; HABIB, N. ; GORDON, M. ; CHATAWAY, J.** *Age and Ageing,* 2011, vol. 40, 7-13
- **CHUNG et al.** *Cell Stem Cell,* 2008, vol. 2, 113-117
- **DING, D. C. ; SHYU, W. C. ; LIN S. Z.** *Cell Transplant,* 2011, vol. 20, 5-14
- **EINSTEIN, O. ; BEN-HUR, T.** *Arch Neurol,* 2008, vol. 65, 452-456
- **HASSANI Z ; O'REILLY J ; PEARSE Y ; STROEMER P ; TANG E ; SINDEN J ; PRICE J ; THURET S.** Human neural progenitor cell engraftment increases neurogenesis and microglial recruitment in the brain of rats with stroke. *PLoS One,* 2012, vol. 7 (11), e50444
- **HODGES et al.** *Cell Transplant,* 2007, vol. 16 (2), 101-15
- **HORIE, N. ; PEREIRA, N.P. ; NIIZUMA, K. SUN, G et al.** *Stem Cells,* 2011, vol. 29, 274-285
- **KATSUDA ; KOSAKA ; TAKESHITA ; OCHIYA.** *Proteomics,* 2013, vol. 00, 1-17
- **KATSUDA ; TSUCHIYA ; KOSAKA ; YOSHIOKA ; TAKAGAKI ; OKI ; TAKESHITA ; SAKAI ; KURODA ; OCHIYA.** *Scientific Reports,* 2013, vol. 3 (1197), 1-11
- **KLIMANSKAYA et al.** *Nature,* 2006, vol. 444, 481-485
- **KORNBLUM.** *Stroke,* 2007, vol. 38, 810-816
- **LAI et al.** Proteolytic Potential of the MSC Exosome Proteome: Implications for an Exosome-Mediated Delivery of Therapeutic Proteasome. *International Journal of Proteomics,* 2012, 14
- **LITTLEWOOD, T. D. ; HANCOCK, D. C. ; DANIELIAN, P. S. et al.** *Nucleic Acid Research,* 1995, vol. 23, 1686-1690
- **MILJAN, E.A. ; SINDEN, J.D.** *Current Opinion in Molecular Therapeutics,* 2009, vol. 4, 394-403
- **MILJAN EA ; HINES SJ ; PANDE P ; CORTELING RL ; HICKS C ; ZBARSKY V ; UMACHANDRAN, M ; SOWINSKI P ; RICHARDSON S ; TANG E.** Implantation of c-mycER TAM immortalized human mesencephalic-derived clonal cell lines ameliorates behavior dysfunction in a rat model of Parkinson's disease. *Stem Cells Dev,* March 2009, vol. 18 (2), 307-19
- **MITCHELL et al.** *Journal of Immunological Methods,* 2008, vol. 335, 98-105
- **POLLOCK et al.** *Exp Neurol,* May 2006, vol. 199 (1), 143-55
- **MARK F PITTENGER ; ALASTAIR M MACKAY ; STEPHEN C BECK ; RAMA K JAISWAL et al.** *Science,* 02 April 1999, vol. 284, 5411
- **SMITH, E. J. ; STROEMER, R.P. ; GORENKOVA, N. ; NAKAJIMA, M. et al.** *Stem Cells,* 2012, vol. 30, 785-796
- **STEVENATO, L. ; CORTELING, R. ; STROEMER, P. ; HOPE, A. et al.** *BMC Neuroscience,* 2009, vol. 10, 86
- **STROEMER, P. ; PATEL, S. ; HOPE, A. ; OLIVEIRA, C. ; POLLOCK, K. ; SINDEN, J.** *Neurorehabil Neural Repair,* 2009, vol. 23, 895-909
- **THÉRY, C. ; OSTROWSKI, M. ; SEGURA, E. et al.** *Nature Reviews Immunology,* 2009, vol. 9, 581-593
- **THEIR et al.** Direct Conversion of Fibroblasts into Stably Expandable Neural Stem Cells. *Cell Stem Cell,* 20 March 2012
- **TIMMERS, L. ; LIM, S. K. ; ARSLAN, F. ; ARMSTRONG, J. S. et al.** *Stem Cell Res,* 2007, vol. 1, 129-137
- **YUAN, S.J. ; MARTIN, J ; ELIA, J. ; FLIPPIN, J. et al.** *PLoS ONE,* 2011, vol. 6, e17540

SEQUENCE LISTING

<110> Reneuron Limited

<120> Stem Cell Microparticles

<130> P059388WO

<141> 2013-04-03

<150> GB 1205972.1
<151> 2012-04-03

<150> GB 1212848.4
<151> 2012-09-19

<150> GB 1302468.2
<151> 2013-02-12

<160> 752

<170> SeqWin2010, version 1.0

<210> 1
<211> 22
<212> RNA
<213> Homo sapiens

<400> 1
ugagguagua gguuguauag uu 22

<210> 2
<211> 23
<212> RNA
<213> Homo sapiens

<400> 2
uacccuguag auccgaauuu gug 23

<210> 3
<211> 22
<212> RNA
<213> Homo sapiens

<400> 3
aacccguaga uccgaacuug ug 22

<210> 4
<211> 22
<212> RNA
<213> Homo sapiens

<400> 4
cacccguaga accgaccuug cg 22

<210> 5
<211> 22
<212> RNA
<213> Homo sapiens

<400> 5
uccuguacug agcugccccg ag 22

<210> 6
<211> 21
<212> RNA
<213> Homo sapiens

<400> 6
uucacagugg cuaaguucug c 21

<210> 7
<211> 22
<212> RNA
<213> Homo sapiens

<400> 7
uauugcacuu gucccggccu gu 22

<210> 8
<211> 23
<212> RNA
<213> Homo sapiens

<400> 8
caacggaauc ccaaaagcag cug 23

<210> 9
<211> 22
<212> RNA
<213> Homo sapiens

<400> 9
uagcuuauca gacugauguu ga 22

<210> 10
<211> 22
<212> RNA
<213> Homo sapiens

<400> 10
ugagguagua aguuguauug uu 22

<210> 11
<211> 22
<212> RNA
<213> Homo sapiens

<400> 11
ugagguagua gauuguauag uu 22

<210> 12
<211> 22
<212> RNA
<213> Homo sapiens

<400> 12
uucaaguaau ccaggauagg cu 22

<210> 13
<211> 22
<212> RNA
<213> Homo sapiens

<400> 13
uauugcacuc gucccggccu cc 22

<210> 14
<211> 22
<212> RNA
<213> Homo sapiens

<400> 14
ucggauccgu cugagcuugg cu 22

<210> 15
<211> 23
<212> RNA
<213> Homo sapiens

<400> 15
aacauucaac gcugucggug agu 23

<210> 16
<211> 24
<212> RNA
<213> Homo sapiens

<400> 16
uuuggcaaug guagaacuca cacu 24

<210> 17
<211> 22
<212> RNA
<213> Homo sapiens

<400> 17
ugagguagua gguuguaugg uu 22

<210> 18
<211> 21
<212> RNA
<213> Homo sapiens

<400> 18
ugguagacua uggaacguag g 21

<210> 19
<211> 22
<212> RNA
<213> Homo sapiens

<400> 19
ugagaacuga auuccauagg cu 22

<210> 20
<211> 21
<212> RNA
<213> Homo sapiens

<400> 20
caacaccagu cgaugggcug u 21

<210> 21
<211> 19
<212> RNA
<213> Homo sapiens

<400> 21

aauggauuuu uggagcagg 19

<210> 22
<211> 22
<212> RNA
<213> Homo sapiens

<400> 22
ugagguagua guuugugcug uu 22

<210> 23
<211> 17
<212> RNA
<213> Homo sapiens

<400> 23
ccccggggag cccggcg 17

<210> 24
<211> 22
<212> RNA
<213> Homo sapiens

<400> 24
uauggcacug guagaauuca cu 22

<210> 25
<211> 21
<212> RNA
<213> Homo sapiens

<400> 25
cuagacugaa gcuccuugag g 21

<210> 26
<211> 22
<212> RNA
<213> Homo sapiens

<400> 26
aaugcacccg ggcaaggauu cu 22

<210> 27
<211> 22
<212> RNA
<213> Homo sapiens

<400> 27
ugagguagga gguuguauag uu 22

<210> 28
<211> 22
<212> RNA
<213> Homo sapiens

<400> 28
ugagguagua gguugugugg uu 22

<210> 29
<211> 22
<212> RNA
<213> Homo sapiens

<400> 29
uagcagcacg uaaauauugg cg 22

<210> 30
<211> 22
<212> RNA
<213> Homo sapiens

<400> 30
uguaaacauc cucgacugga ag 22

<210> 31
<211> 22
<212> RNA
<213> Homo sapiens

<400> 31
uguaaacauc cccgacugga ag 22

<210> 32
<211> 23
<212> RNA
<213> Homo sapiens

<400> 32
agguuacccg agcaacuuug cau 23

<210> 33
<211> 22
<212> RNA
<213> Homo sapiens

<400> 33
aagcugccag uugaagaacu gu 22

<210> 34
<211> 17
<212> RNA
<213> Homo sapiens

<400> 34
ggggcugggc gcgcgcc 17

<210> 35
<211> 24
<212> RNA
<213> Homo sapiens

<400> 35
ucccugagac ccuuuaaccu guga 24

<210> 36
<211> 21
<212> RNA
<213> Homo sapiens

<400> 36
agcuacaucu ggcuacuggg u 21

<210> 37
<211> 21
<212> RNA
<213> Homo sapiens

<400> 37
ucgaggagcu cacagucuag u 21

<210> 38
<211> 23
<212> RNA
<213> Homo sapiens

<400> 38
aacauucauu gcugucggug ggu 23

<210> 39
<211> 22
<212> RNA
<213> Homo sapiens

<400> 39
accuggcaua caauguagau uu 22

<210> 40
<211> 22
<212> RNA
<213> Homo sapiens

<400> 40
caaagaauuc uccuuuuggg cu 22

<210> 41
<211> 23
<212> RNA
<213> Homo sapiens

<400> 41
ugaggggcag agagcgagac uuu 23

<210> 42
<211> 22
<212> RNA
<213> Homo sapiens

<400> 42
ucccugagac ccuaacuugu ga 22

<210> 43
<211> 22
<212> RNA
<213> Homo sapiens

<400> 43
ugagguagua guuuguacag uu 22

<210> 44
<211> 22
<212> RNA
<213> Homo sapiens

<400> 44
augcaccugg gcaaggauuc ug 22

<210> 45
<211> 22
<212> RNA

<213> Homo sapiens

<400> 45
uguaaacauc cuugacugga ag 22

<210> 46
<211> 21
<212> RNA
<213> Homo sapiens

<400> 46
uucacagugg cuaaguuccg c 21

<210> 47
<211> 22
<212> RNA
<213> Homo sapiens

<400> 47
gaauguugcu cggugaaccc cu 22

<210> 48
<211> 22
<212> RNA
<213> Homo sapiens

<400> 48
ucagugcauc acagaacuuu gu 22

<210> 49
<211> 22
<212> RNA
<213> Homo sapiens

<400> 49
acggguuagg cucuugggag cu 22

<210> 50
<211> 21
<212> RNA
<213> Homo sapiens

<400> 50
aauauaacac agauggccug u 21

<210> 51
<211> 22
<212> RNA
<213> Homo sapiens

<400> 51
uauacaaggg caagcucucu gu 22

<210> 52
<211> 22
<212> RNA
<213> Homo sapiens

<400> 52
aacccguaga uccgaucuug ug 22

<210> 53
<211> 22

<212> RNA
<213> Homo sapiens

<400> 53
agagguagua gguugcauag uu 22

<210> 54
<211> 22
<212> RNA
<213> Homo sapiens

<400> 54
ucagugcacu acagaacuuu gu 22

<210> 55
<211> 21
<212> RNA
<213> Homo sapiens

<400> 55
aucacauugc cagggauuuc c 21

<210> 56
<211> 22
<212> RNA
<213> Homo sapiens

<400> 56
cacuagauug ugagcuccug ga 22

<210> 57
<211> 23
<212> RNA
<213> Homo sapiens

<400> 57
agcucggucu gaggccccuc agu 23

<210> 58
<211> 23
<212> RNA
<213> Homo sapiens

<400> 58
ucuuugguua ucuagcugua uga 23

<210> 59
<211> 21
<212> RNA
<213> Homo sapiens

<400> 59
aucacauugc cagggauuac c 21

<210> 60
<211> 23
<212> RNA
<213> Homo sapiens

<400> 60
cacccggcug ugugcacaug ugc 23

<210> 61

<211> 18
<212> RNA
<213> Homo sapiens

<400> 61
agggggcggg cuccggcg 18

<210> 62
<211> 23
<212> RNA
<213> Homo sapiens

<400> 62
agcagcauug uacagggcua uga 23

<210> 63
<211> 22
<212> RNA
<213> Homo sapiens

<400> 63
cauugcacuu gucucggucu ga 22

<210> 64
<211> 21
<212> RNA
<213> Homo sapiens

<400> 64
uuaauaucgg acaaccauug u 21

<210> 65
<211> 21
<212> RNA
<213> Homo sapiens

<400> 65
acuggacuug gagucagaag g 21

<210> 66
<211> 23
<212> RNA
<213> Homo sapiens

<400> 66
uguaaacauc cuacacucuc agc 23

<210> 67
<211> 20
<212> RNA
<213> Homo sapiens

<400> 67
uaacggccgc gguacccuaa 20

<210> 68
<211> 22
<212> RNA
<213> Homo sapiens

<400> 68
ucacaaguca ggcucuuggg ac 22

<210> 69
<211> 21
<212> RNA
<213> Homo sapiens

<400> 69
uccgguucuc agggcuccac c 21

<210> 70
<211> 22
<212> RNA
<213> Homo sapiens

<400> 70
uuaucagaau cuccaggggu ac 22

<210> 71
<211> 22
<212> RNA
<213> Homo sapiens

<400> 71
cuuucagucg gauguuuaca gc 22

<210> 72
<211> 22
<212> RNA
<213> Homo sapiens

<400> 72
cuuggcaccu agcaagcacu ca 22

<210> 73
<211> 22
<212> RNA
<213> Homo sapiens

<400> 73
ugagaaccac gucugcucug ag 22

<210> 74
<211> 22
<212> RNA
<213> Homo sapiens

<400> 74
uuauaauaca accugauaag ug 22

<210> 75
<211> 22
<212> RNA
<213> Homo sapiens

<400> 75
ugagaccucu ggguucugag cu 22

<210> 76
<211> 22
<212> RNA
<213> Homo sapiens

<400> 76
gcugacuccu aguccagggc uc 22

<210> 77
<211> 22
<212> RNA
<213> Homo sapiens

<400> 77
cuuucagucg gauguuugca gc 22

<210> 78
<211> 22
<212> RNA
<213> Homo sapiens

<400> 78
uagcagcaca ucaugguuua ca 22

<210> 79
<211> 23
<212> RNA
<213> Homo sapiens

<400> 79
agcuacauug ucugcugggu uuc 23

<210> 80
<211> 21
<212> RNA
<213> Homo sapiens

<400> 80
aggcaagaug cuggcauagc u 21

<210> 81
<211> 23
<212> RNA
<213> Homo sapiens

<400> 81
ucucacacag aaaucgcacc cgu 23

<210> 82
<211> 22
<212> RNA
<213> Homo sapiens

<400> 82
caucaucguc ucaaaugagu cu 22

<210> 83
<211> 22
<212> RNA
<213> Homo sapiens

<400> 83
ugaaggucua cugugugcca gg 22

<210> 84
<211> 17
<212> RNA
<213> Homo sapiens

<400> 84

ucucgcuggg gcccucca 17

<210> 85
<211> 23
<212> RNA
<213> Homo sapiens

<400> 85
uggaagacua gugauuuugu ugu 23

<210> 86
<211> 22
<212> RNA
<213> Homo sapiens

<400> 86
cagugcaaug augaaagggc au 22

<210> 87
<211> 21
<212> RNA
<213> Homo sapiens

<400> 87
cugaccuaug aauugacagc c 21

<210> 88
<211> 22
<212> RNA
<213> Homo sapiens

<400> 88
uuguacaugg uaggcuuuca uu 22

<210> 89
<211> 22
<212> RNA
<213> Homo sapiens

<400> 89
uucccuuugu cauccuaugc cu 22

<210> 90
<211> 21
<212> RNA
<213> Homo sapiens

<400> 90
uucaaguaau ucaggauagg u 21

<210> 91
<211> 21
<212> RNA
<213> Homo sapiens

<400> 91
ucgaccggac cucgaccggc u 21

<210> 92
<211> 22
<212> RNA
<213> Homo sapiens

<400> 92
cuauacgacc ugcugccuuu cu 22

<210> 93
<211> 22
<212> RNA
<213> Homo sapiens

<400> 93
uuauaaagca augagacuga uu 22

<210> 94
<211> 22
<212> RNA
<213> Homo sapiens

<400> 94
ugugacuggu ugaccagagg gg 22

<210> 95
<211> 23
<212> RNA
<213> Homo sapiens

<400> 95
ucuuggagua ggucauuggg ugg 23

<210> 96
<211> 22
<212> RNA
<213> Homo sapiens

<400> 96
uguaaacauc cuacacucag cu 22

<210> 97
<211> 22
<212> RNA
<213> Homo sapiens

<400> 97
aaaagcuggg uugagagggc ga 22

<210> 98
<211> 22
<212> RNA
<213> Homo sapiens

<400> 98
caagcuugua ucuauaggua ug 22

<210> 99
<211> 22
<212> RNA
<213> Homo sapiens

<400> 99
ugcggggcua gggcuaacag ca 22

<210> 100
<211> 22
<212> RNA
<213> Homo sapiens

<400> 100
accaucgacc guugauugua cc 22

<210> 101
<211> 22
<212> RNA
<213> Homo sapiens

<400> 101
uggcaguguc uuagcugguu gu 22

<210> 102
<211> 22
<212> RNA
<213> Homo sapiens

<400> 102
accacugacc guugacugua cc 22

<210> 103
<211> 22
<212> RNA
<213> Homo sapiens

<400> 103
ugauauguuu gauauauuag gu 22

<210> 104
<211> 22
<212> RNA
<213> Homo sapiens

<400> 104
uaacagucua cagccauggu cg 22

<210> 105
<211> 22
<212> RNA
<213> Homo sapiens

<400> 105
aacauucaac cugucgguga gu 22

<210> 106
<211> 22
<212> RNA
<213> Homo sapiens

<400> 106
uagcaccauc ugaaaucggu ua 22

<210> 107
<211> 23
<212> RNA
<213> Homo sapiens

<400> 107
cagugcaaua guauugucaa agc 23

<210> 108
<211> 21
<212> RNA

<213> Homo sapiens

<400> 108
uaguagaccg uauagcguac g 21

<210> 109
<211> 21
<212> RNA
<213> Homo sapiens

<400> 109
ucacagugaa ccggucucuu u 21

<210> 110
<211> 17
<212> RNA
<213> Homo sapiens

<400> 110
gggagaaggg ucggggc 17

<210> 111
<211> 23
<212> RNA
<213> Homo sapiens

<400> 111
aaugacacga ucacucccgu uga 23

<210> 112
<211> 21
<212> RNA
<213> Homo sapiens

<400> 112
acucuuuccc uguugcacua c 21

<210> 113
<211> 22
<212> RNA
<213> Homo sapiens

<400> 113
cagugcaaug uuaaaagggc au 22

<210> 114
<211> 22
<212> RNA
<213> Homo sapiens

<400> 114
cuuucaguca gauguuugcu gc 22

<210> 115
<211> 22
<212> RNA
<213> Homo sapiens

<400> 115
uggugggccg cagaacaugu gc 22

<210> 116
<211> 23

<212> RNA
<213> Homo sapiens

<400> 116
caaagugcug uucgugcagg uag 23

<210> 117
<211> 22
<212> RNA
<213> Homo sapiens

<400> 117
aaucguacag ggucauccac uu 22

<210> 118
<211> 22
<212> RNA
<213> Homo sapiens

<400> 118
ucaggcucag uccccucccg au 22

<210> 119
<211> 22
<212> RNA
<213> Homo sapiens

<400> 119
uggcucaguu cagcaggaac ag 22

<210> 120
<211> 22
<212> RNA
<213> Homo sapiens

<400> 120
ucugugagac caaagaacua cu 22

<210> 121
<211> 23
<212> RNA
<213> Homo sapiens

<400> 121
ucuggcuccg ugucuucacu ccc 23

<210> 122
<211> 22
<212> RNA
<213> Homo sapiens

<400> 122
uucaccaccu ucuccaccca gc 22

<210> 123
<211> 23
<212> RNA
<213> Homo sapiens

<400> 123
uuuggcacua gcacauuuuu gcu 23

<210> 124

<211> 22
<212> RNA
<213> Homo sapiens

<400> 124
acucggcgug gcgucggucg ug 22

<210> 125
<211> 23
<212> RNA
<213> Homo sapiens

<400> 125
aggcagugua guuagcugau ugc 23

<210> 126
<211> 22
<212> RNA
<213> Homo sapiens

<400> 126
gccugcuggg guggaaccug gu 22

<210> 127
<211> 22
<212> RNA
<213> Homo sapiens

<400> 127
aaguucuguu auacacucag gc 22

<210> 128
<211> 23
<212> RNA
<213> Homo sapiens

<400> 128
ucaagagcaa uaacgaaaaa ugu 23

<210> 129
<211> 23
<212> RNA
<213> Homo sapiens

<400> 129
gauugagacu aguagggcua ggc 23

<210> 130
<211> 22
<212> RNA
<213> Homo sapiens

<400> 130
agggcuuagc ugcuugugag ca 22

<210> 131
<211> 22
<212> RNA
<213> Homo sapiens

<400> 131
aauugcacgg uauccaucug ua 22

<210> 132
<211> 21
<212> RNA
<213> Homo sapiens

<400> 132
ugucuugcag gccgucaugc a 21

<210> 133
<211> 20
<212> RNA
<213> Homo sapiens

<400> 133
guagaggaga uggcgcaggg 20

<210> 134
<211> 23
<212> RNA
<213> Homo sapiens

<400> 134
agugccugag ggaguaagag ccc 23

<210> 135
<211> 17
<212> RNA
<213> Homo sapiens

<400> 135
gcggggcugg gcgcgcg 17

<210> 136
<211> 22
<212> RNA
<213> Homo sapiens

<400> 136
uggugggcac agaaucugga cu 22

<210> 137
<211> 23
<212> RNA
<213> Homo sapiens

<400> 137
uauggcuuuu cauuccuaug uga 23

<210> 138
<211> 21
<212> RNA
<213> Homo sapiens

<400> 138
uaccacaggg uagaaccacg g 21

<210> 139
<211> 24
<212> RNA
<213> Homo sapiens

<400> 139
aauccuugga accuaggugu gagu 24

<210> 140
<211> 21
<212> RNA
<213> Homo sapiens

<400> 140
gcaguccaug ggcauauaca c 21

<210> 141
<211> 22
<212> RNA
<213> Homo sapiens

<400> 141
uuugugaccu gguccacuaa cc 22

<210> 142
<211> 21
<212> RNA
<213> Homo sapiens

<400> 142
uacaguacug ugauaacuga a 21

<210> 143
<211> 22
<212> RNA
<213> Homo sapiens

<400> 143
auauaauaca accugcuaag ug 22

<210> 144
<211> 22
<212> RNA
<213> Homo sapiens

<400> 144
aaaguucuga gacacuccga cu 22

<210> 145
<211> 23
<212> RNA
<213> Homo sapiens

<400> 145
caaagugcuu acagugcagg uag 23

<210> 146
<211> 23
<212> RNA
<213> Homo sapiens

<400> 146
uaaagugcuu auagugcagg uag 23

<210> 147
<211> 22
<212> RNA
<213> Homo sapiens

<400> 147

cugcccuggc ccgagggacc ga 22

<210> 148
<211> 22
<212> RNA
<213> Homo sapiens

<400> 148
aacuggcccu caaagucccg cu 22

<210> 149
<211> 22
<212> RNA
<213> Homo sapiens

<400> 149
caaaaacugc aguuacuuuu gc 22

<210> 150
<211> 22
<212> RNA
<213> Homo sapiens

<400> 150
aucauacaag gacaauuucu uu 22

<210> 151
<211> 23
<212> RNA
<213> Homo sapiens

<400> 151
aucaacagac auuaauuggg cgc 23

<210> 152
<211> 22
<212> RNA
<213> Homo sapiens

<400> 152
aaggagcuca cagucuauug ag 22

<210> 153
<211> 22
<212> RNA
<213> Homo sapiens

<400> 153
gucauacacg gcucuccucu cu 22

<210> 154
<211> 23
<212> RNA
<213> Homo sapiens

<400> 154
ugaggcucug uuagccuugg cuc 23

<210> 155
<211> 22
<212> RNA
<213> Homo sapiens

<400> 155
cgucccgggg cugcgcgagg ca 22

<210> 156
<211> 22
<212> RNA
<213> Homo sapiens

<400> 156
ugccuacuga gcugaaacac ag 22

<210> 157
<211> 23
<212> RNA
<213> Homo sapiens

<400> 157
aacauucauu guugucggug ggu 23

<210> 158
<211> 21
<212> RNA
<213> Homo sapiens

<400> 158
cacauuacac ggucgaccuc u 21

<210> 159
<211> 22
<212> RNA
<213> Homo sapiens

<400> 159
ccgcacugug gguacuugcu gc 22

<210> 160
<211> 22
<212> RNA
<213> Homo sapiens

<400> 160
acaggugagg uucuugggag cc 22

<210> 161
<211> 22
<212> RNA
<213> Homo sapiens

<400> 161
ucucugggcc ugugucuuag gc 22

<210> 162
<211> 17
<212> RNA
<213> Homo sapiens

<400> 162
guggggggaga ggcuguc 17

<210> 163
<211> 23
<212> RNA
<213> Homo sapiens

<400> 163
ugugcaaauc caugcaaaac uga 23

<210> 164
<211> 23
<212> RNA
<213> Homo sapiens

<400> 164
cagugcaaug auauugucaa agc 23

<210> 165
<211> 22
<212> RNA
<213> Homo sapiens

<400> 165
agaggcuggc cgugaugaau uc 22

<210> 166
<211> 23
<212> RNA
<213> Homo sapiens

<400> 166
uagcaccauu ugaaaucagu guu 23

<210> 167
<211> 22
<212> RNA
<213> Homo sapiens

<400> 167
aagggcuucc ucucugcagg ac 22

<210> 168
<211> 22
<212> RNA
<213> Homo sapiens

<400> 168
caggucgucu ugcagggcuu cu 22

<210> 169
<211> 23
<212> RNA
<213> Homo sapiens

<400> 169
uagugcaaua uugcuuauag ggu 23

<210> 170
<211> 21
<212> RNA
<213> Homo sapiens

<400> 170
uaaagugcug acagugcaga u 21

<210> 171
<211> 19
<212> RNA

<213> Homo sapiens

<400> 171
accgugcaaa gguagcaua 19

<210> 172
<211> 22
<212> RNA
<213> Homo sapiens

<400> 172
ugcuaugcca acauauugcc au 22

<210> 173
<211> 22
<212> RNA
<213> Homo sapiens

<400> 173
cuuaucagau uguauuguaa uu 22

<210> 174
<211> 22
<212> RNA
<213> Homo sapiens

<400> 174
aucaugaugg gcuccucggu gu 22

<210> 175
<211> 18
<212> RNA
<213> Homo sapiens

<400> 175
ggugggcuuc ccggaggg 18

<210> 176
<211> 21
<212> RNA
<213> Homo sapiens

<400> 176
ugagaugaag cacuguagcu c 21

<210> 177
<211> 23
<212> RNA
<213> Homo sapiens

<400> 177
ugugcaaauc uaugcaaaac uga 23

<210> 178
<211> 22
<212> RNA
<213> Homo sapiens

<400> 178
caugccuuga guguaggacc gu 22

<210> 179
<211> 22

<212> RNA
<213> Homo sapiens

<400> 179
aucaaggauc uuaaacuuug cc 22

<210> 180
<211> 22
<212> RNA
<213> Homo sapiens

<400> 180
gguggcccgg ccgugccuga gg 22

<210> 181
<211> 24
<212> RNA
<213> Homo sapiens

<400> 181
uugcagcugc cugggaguga cuuc 24

<210> 182
<211> 22
<212> RNA
<213> Homo sapiens

<400> 182
uaugugggau gguaaaccgc uu 22

<210> 183
<211> 22
<212> RNA
<213> Homo sapiens

<400> 183
auaaagcuag auaaccgaaa gu 22

<210> 184
<211> 22
<212> RNA
<213> Homo sapiens

<400> 184
acugcaguga aggcacuugu ag 22

<210> 185
<211> 21
<212> RNA
<213> Homo sapiens

<400> 185
aacauagagg aaauuccacg u 21

<210> 186
<211> 22
<212> RNA
<213> Homo sapiens

<400> 186
cagcagcaau ucauguuuug aa 22

<210> 187

<211> 22
<212> RNA
<213> Homo sapiens

<400> 187
uacccauugc auaucggagu ug 22

<210> 188
<211> 22
<212> RNA
<213> Homo sapiens

<400> 188
uugcauaguc acaaaaguga uc 22

<210> 189
<211> 23
<212> RNA
<213> Homo sapiens

<400> 189
ugaggaugga uagcaaggaa gcc 23

<210> 190
<211> 24
<212> RNA
<213> Homo sapiens

<400> 190
cccggacagg cguucgugcg acgu 24

<210> 191
<211> 18
<212> RNA
<213> Homo sapiens

<400> 191
gggcucacau caccccau 18

<210> 192
<211> 22
<212> RNA
<213> Homo sapiens

<400> 192
uaugugccuu uggacuacau cg 22

<210> 193
<211> 22
<212> RNA
<213> Homo sapiens

<400> 193
aaacauucgc ggugcacuuc uu 22

<210> 194
<211> 20
<212> RNA
<213> Homo sapiens

<400> 194
uaaagagccc uguggagaca 20

<210> 195
<211> 23
<212> RNA
<213> Homo sapiens

<400> 195
gcaaagcaca cggccugcag aga 23

<210> 196
<211> 21
<212> RNA
<213> Homo sapiens

<400> 196
aauaauacau gguugaucuu u 21

<210> 197
<211> 22
<212> RNA
<213> Homo sapiens

<400> 197
ugagaccagg acuggaugca cc 22

<210> 198
<211> 22
<212> RNA
<213> Homo sapiens

<400> 198
aaaaguacuu gcggauuuug cu 22

<210> 199
<211> 22
<212> RNA
<213> Homo sapiens

<400> 199
ucaccagccc uguguucccu ag 22

<210> 200
<211> 21
<212> RNA
<213> Homo sapiens

<400> 200
cucccacaug cagguuugc a 21

<210> 201
<211> 22
<212> RNA
<213> Homo sapiens

<400> 201
agagcuuagc ugauugguga ac 22

<210> 202
<211> 22
<212> RNA
<213> Homo sapiens

<400> 202
agagguugcc cuuggugaau uc 22

<210> 203
<211> 22
<212> RNA
<213> Homo sapiens

<400> 203
aaucauacag ggacauccag uu 22

<210> 204
<211> 23
<212> RNA
<213> Homo sapiens

<400> 204
agguugggau cgguugcaau gcu 23

<210> 205
<211> 22
<212> RNA
<213> Homo sapiens

<400> 205
auguagggcu aaaagccaug gg 22

<210> 206
<211> 21
<212> RNA
<213> Homo sapiens

<400> 206
uugugcuuga ucuaaccaug u 21

<210> 207
<211> 23
<212> RNA
<213> Homo sapiens

<400> 207
uagcccccag gcuucacuug gcg 23

<210> 208
<211> 22
<212> RNA
<213> Homo sapiens

<400> 208
agggacggga cgcggugcag ug 22

<210> 209
<211> 22
<212> RNA
<213> Homo sapiens

<400> 209
auauacaggg ggagacucuu au 22

<210> 210
<211> 21
<212> RNA
<213> Homo sapiens

<400> 210

accacugcac uccagccuga g 21

<210> 211
<211> 21
<212> RNA
<213> Homo sapiens

<400> 211
auccccagau acaauggaca a 21

<210> 212
<211> 22
<212> RNA
<213> Homo sapiens

<400> 212
gggguuccug gggaugggau uu 22

<210> 213
<211> 22
<212> RNA
<213> Homo sapiens

<400> 213
cugggagagg guuguuuacu cc 22

<210> 214
<211> 22
<212> RNA
<213> Homo sapiens

<400> 214
aacacaccug guuaaccucu uu 22

<210> 215
<211> 22
<212> RNA
<213> Homo sapiens

<400> 215
cuccuauaug augccuuucu uc 22

<210> 216
<211> 24
<212> RNA
<213> Homo sapiens

<400> 216
caaagugaug aguaauacug gcug 24

<210> 217
<211> 22
<212> RNA
<213> Homo sapiens

<400> 217
cuccugacuc cagguccugu gu 22

<210> 218
<211> 23
<212> RNA
<213> Homo sapiens

<400> 218
gaggcugaug ugaguagacc acu 23

<210> 219
<211> 23
<212> RNA
<213> Homo sapiens

<400> 219
ugaguggggc ucccgggacg gcg 23

<210> 220
<211> 21
<212> RNA
<213> Homo sapiens

<400> 220
guuucaccau guuggucagg c 21

<210> 221
<211> 21
<212> RNA
<213> Homo sapiens

<400> 221
aauauuauac agucaaccuc u 21

<210> 222
<211> 21
<212> RNA
<213> Homo sapiens

<400> 222
cuauacaauc uacugucuuu c 21

<210> 223
<211> 22
<212> RNA
<213> Homo sapiens

<400> 223
uagcagcaca uaaugguuug ug 22

<210> 224
<211> 22
<212> RNA
<213> Homo sapiens

<400> 224
uggagagaaa ggcaguuccu ga 22

<210> 225
<211> 21
<212> RNA
<213> Homo sapiens

<400> 225
aggcggagac uugggcaauu g 21

<210> 226
<211> 23
<212> RNA
<213> Homo sapiens

<400> 226
ucaacaaaau cacugaugcu gga 23

<210> 227
<211> 19
<212> RNA
<213> Homo sapiens

<400> 227
acuggccugg gacuaccgg 19

<210> 228
<211> 23
<212> RNA
<213> Homo sapiens

<400> 228
ugagcgccuc gacgacagag ccg 23

<210> 229
<211> 22
<212> RNA
<213> Homo sapiens

<400> 229
cuuagcaggu uguauuauca uu 22

<210> 230
<211> 22
<212> RNA
<213> Homo sapiens

<400> 230
auggccagag cucacacaga gg 22

<210> 231
<211> 20
<212> RNA
<213> Homo sapiens

<400> 231
ggcuccuugg ucuaggggua 20

<210> 232
<211> 17
<212> RNA
<213> Homo sapiens

<400> 232
cuccgggacg gcugggc 17

<210> 233
<211> 22
<212> RNA
<213> Homo sapiens

<400> 233
gcuaaggaag uccugugcuc ag 22

<210> 234
<211> 22
<212> RNA

<213> Homo sapiens

<400> 234
ggagaaauua uccuuggugu gu 22

<210> 235
<211> 20
<212> RNA
<213> Homo sapiens

<400> 235
aaaagugauu gcaguguuug 20

<210> 236
<211> 21
<212> RNA
<213> Homo sapiens

<400> 236
ccaguccugu gccugccgcc u 21

<210> 237
<211> 21
<212> RNA
<213> Homo sapiens

<400> 237
aucauagagg aaaauccacg u 21

<210> 238
<211> 22
<212> RNA
<213> Homo sapiens

<400> 238
gaaguuguuc gugguggauu cg 22

<210> 239
<211> 22
<212> RNA
<213> Homo sapiens

<400> 239
cagcccggau cccagcccac uu 22

<210> 240
<211> 22
<212> RNA
<213> Homo sapiens

<400> 240
ugaaacauac acgggaaacc uc 22

<210> 241
<211> 22
<212> RNA
<213> Homo sapiens

<400> 241
aaacaaacau ggugcacuuc uu 22

<210> 242
<211> 22

<212> RNA
<213> Homo sapiens

<400> 242
ucagcaaaca uuuauugugu gc 22

<210> 243
<211> 22
<212> RNA
<213> Homo sapiens

<400> 243
caagcucgug ucugugggu cg 22

<210> 244
<211> 21
<212> RNA
<213> Homo sapiens

<400> 244
uaggacacau ggucuacuuc u 21

<210> 245
<211> 21
<212> RNA
<213> Homo sapiens

<400> 245
cucacugaac aaugaaugca a 21

<210> 246
<211> 23
<212> RNA
<213> Homo sapiens

<400> 246
accuuggcuc uagacugcuu acu 23

<210> 247
<211> 22
<212> RNA
<213> Homo sapiens

<400> 247
caccuugcgc uacucagguc ug 22

<210> 248
<211> 22
<212> RNA
<213> Homo sapiens

<400> 248
uccgucucag uuacuuuaua gc 22

<210> 249
<211> 22
<212> RNA
<213> Homo sapiens

<400> 249
gcaugugaug aagcaaauca gu 22

<210> 250

<211> 23
<212> RNA
<213> Homo sapiens

<400> 250
uccccaggu gugauucuga uuu 23

<210> 251
<211> 17
<212> RNA
<213> Homo sapiens

<400> 251
ggcgggugcg gggugg 17

<210> 252
<211> 22
<212> RNA
<213> Homo sapiens

<400> 252
ccucccacac ccaaggcuug ca 22

<210> 253
<211> 22
<212> RNA
<213> Homo sapiens

<400> 253
cacgcucaug cacacaccca ca 22

<210> 254
<211> 23
<212> RNA
<213> Homo sapiens

<400> 254
agcagcauug uacagggcua uca 23

<210> 255
<211> 22
<212> RNA
<213> Homo sapiens

<400> 255
cugaagcuca gagggcucug au 22

<210> 256
<211> 23
<212> RNA
<213> Homo sapiens

<400> 256
uaaggugcau cuagugcaga uag 23

<210> 257
<211> 22
<212> RNA
<213> Homo sapiens

<400> 257
ccuauucuug auuacuuguu uc 22

<210> 258
<211> 21
<212> RNA
<213> Homo sapiens

<400> 258
agggcccccc cucaauccug u 21

<210> 259
<211> 21
<212> RNA
<213> Homo sapiens

<400> 259
agccgcgggg aucgccgagg g 21

<210> 260
<211> 21
<212> RNA
<213> Homo sapiens

<400> 260
aaucauucac ggacaacacu u 21

<210> 261
<211> 22
<212> RNA
<213> Homo sapiens

<400> 261
uacgcgcaga ccacaggaug uc 22

<210> 262
<211> 21
<212> RNA
<213> Homo sapiens

<400> 262
cuggauggcu ccuccauguc u 21

<210> 263
<211> 22
<212> RNA
<213> Homo sapiens

<400> 263
aguugccuuu uuguucccau gc 22

<210> 264
<211> 18
<212> RNA
<213> Homo sapiens

<400> 264
gggugcgggc cggcgggg 18

<210> 265
<211> 22
<212> RNA
<213> Homo sapiens

<400> 265
acccuaucaa uauugucucu gc 22

<210> 266
<211> 23
<212> RNA
<213> Homo sapiens

<400> 266
ccggucccag gagaaccugc aga 23

<210> 267
<211> 22
<212> RNA
<213> Homo sapiens

<400> 267
ugaguauuac auggccaauc uc 22

<210> 268
<211> 22
<212> RNA
<213> Homo sapiens

<400> 268
ccaaaacugc aguuacuuuu gc 22

<210> 269
<211> 27
<212> RNA
<213> Homo sapiens

<400> 269
accuucuugu auaagcacug ugcuaaa 27

<210> 270
<211> 24
<212> RNA
<213> Homo sapiens

<400> 270
agccuggaag cuggagccug cagu 24

<210> 271
<211> 22
<212> RNA
<213> Homo sapiens

<400> 271
uuagggcccu ggcuccaucu cc 22

<210> 272
<211> 23
<212> RNA
<213> Homo sapiens

<400> 272
acuccauuug uuuugaugau gga 23

<210> 273
<211> 23
<212> RNA
<213> Homo sapiens

<400> 273

cccaguguuc agacuaccug uuc 23

<210> 274
<211> 22
<212> RNA
<213> Homo sapiens

<400> 274
gaggguuggg uggaggcucu cc 22

<210> 275
<211> 21
<212> RNA
<213> Homo sapiens

<400> 275
ugcacggcac uggggacacg u 21

<210> 276
<211> 20
<212> RNA
<213> Homo sapiens

<400> 276
acugccccag gugcugcugg 20

<210> 277
<211> 21
<212> RNA
<213> Homo sapiens

<400> 277
gaacggcuuc auacaggagu u 21

<210> 278
<211> 21
<212> RNA
<213> Homo sapiens

<400> 278
aggggugcua ucugugauug a 21

<210> 279
<211> 22
<212> RNA
<213> Homo sapiens

<400> 279
uaaugccccu aaaaauccuu au 22

<210> 280
<211> 15
<212> RNA
<213> Homo sapiens

<400> 280
aggagauccu ggguu 15

<210> 281
<211> 22
<212> RNA
<213> Homo sapiens

<400> 281
aaugcaccug ggcaaggauu ca 22

<210> 282
<211> 22
<212> RNA
<213> Homo sapiens

<400> 282
cgucaacacu ugcugguuuc cu 22

<210> 283
<211> 22
<212> RNA
<213> Homo sapiens

<400> 283
ugucuuacuc ccucaggcac au 22

<210> 284
<211> 21
<212> RNA
<213> Homo sapiens

<400> 284
gccccgggca gugugaucau c 21

<210> 285
<211> 24
<212> RNA
<213> Homo sapiens

<400> 285
aaagacauag gauagaguca ccuc 24

<210> 286
<211> 22
<212> RNA
<213> Homo sapiens

<400> 286
auaauacaug guuaaccucu uu 22

<210> 287
<211> 23
<212> RNA
<213> Homo sapiens

<400> 287
uauucauuua uccccagccu aca 23

<210> 288
<211> 23
<212> RNA
<213> Homo sapiens

<400> 288
aggaagcccu ggaggggcug gag 23

<210> 289
<211> 20
<212> RNA
<213> Homo sapiens

<400> 289
cggcucuggg ucugugggga 20

<210> 290
<211> 22
<212> RNA
<213> Homo sapiens

<400> 290
cuauacggcc uccuagcuuu cc 22

<210> 291
<211> 18
<212> RNA
<213> Homo sapiens

<400> 291
cgggcguggu ggugggg 18

<210> 292
<211> 19
<212> RNA
<213> Homo sapiens

<400> 292
ggagauggag guugcagug 19

<210> 293
<211> 21
<212> RNA
<213> Homo sapiens

<400> 293
ugcaggacca agaugagccc u 21

<210> 294
<211> 24
<212> RNA
<213> Homo sapiens

<400> 294
uggcccugac ugaagaccag cagu 24

<210> 295
<211> 22
<212> RNA
<213> Homo sapiens

<400> 295
uaacacuguc ugguaaagau gg 22

<210> 296
<211> 21
<212> RNA
<213> Homo sapiens

<400> 296
cuccguuugc cuguuucgcu g 21

<210> 297
<211> 22
<212> RNA

<213> Homo sapiens

<400> 297
cuggcccucu cugcccuucc gu 22

<210> 298
<211> 21
<212> RNA
<213> Homo sapiens

<400> 298
caaaacguga ggcgcugcua u 21

<210> 299
<211> 20
<212> RNA
<213> Homo sapiens

<400> 299
ggauccgagu cacggcacca 20

<210> 300
<211> 17
<212> RNA
<213> Homo sapiens

<400> 300
gagacugggg uggggcc 17

<210> 301
<211> 21
<212> RNA
<213> Homo sapiens

<400> 301
uuagugcaua gucuuugguc u 21

<210> 302
<211> 22
<212> RNA
<213> Homo sapiens

<400> 302
uuaauuuuuu guuucgguca cu 22

<210> 303
<211> 23
<212> RNA
<213> Homo sapiens

<400> 303
uaauccuugc uaccugggug aga 23

<210> 304
<211> 22
<212> RNA
<213> Homo sapiens

<400> 304
aaaaguaauu gugguuuugg cc 22

<210> 305
<211> 24

<212> RNA
<213> Homo sapiens

<400> 305
cugaagugau guguaacuga ucag 24

<210> 306
<211> 22
<212> RNA
<213> Homo sapiens

<400> 306
auucuaauuu cuccacgucu uu 22

<210> 307
<211> 22
<212> RNA
<213> Homo sapiens

<400> 307
gacuauagaa cuuucccccu ca 22

<210> 308
<211> 21
<212> RNA
<213> Homo sapiens

<400> 308
aauggcgcca cuaggguugu g 21

<210> 309
<211> 20
<212> RNA
<213> Homo sapiens

<400> 309
accaggaggc ugaggccccu 20

<210> 310
<211> 22
<212> RNA
<213> Homo sapiens

<400> 310
acuccagccc cacagccuca gc 22

<210> 311
<211> 23
<212> RNA
<213> Homo sapiens

<400> 311
ccaguuaccg cuuccgcuac cgc 23

<210> 312
<211> 22
<212> RNA
<213> Homo sapiens

<400> 312
auccgcgcuc ugacucucug cc 22

<210> 313

<211> 22
<212> RNA
<213> Homo sapiens

<400> 313
uuuccggcuc gcgugggugu gu 22

<210> 314
<211> 22
<212> RNA
<213> Homo sapiens

<400> 314
auauacaggg ggagacucuc au 22

<210> 315
<211> 22
<212> RNA
<213> Homo sapiens

<400> 315
accguggcuu ucgauuguua cu 22

<210> 316
<211> 22
<212> RNA
<213> Homo sapiens

<400> 316
ccaauauuac ugugcugcuu ua 22

<210> 317
<211> 23
<212> RNA
<213> Homo sapiens

<400> 317
caaagugcuc auagugcagg uag 23

<210> 318
<211> 21
<212> RNA
<213> Homo sapiens

<400> 318
ccucccaugc caagaacucc c 21

<210> 319
<211> 22
<212> RNA
<213> Homo sapiens

<400> 319
ugguuuaccg ucccacauac au 22

<210> 320
<211> 22
<212> RNA
<213> Homo sapiens

<400> 320
cugggaggug gauguuuacu uc 22

<210> 321
<211> 22
<212> RNA
<213> Homo sapiens

<400> 321
cugggagaag gcuguuuacu cu 22

<210> 322
<211> 20
<212> RNA
<213> Homo sapiens

<400> 322
uuggccaugg ggcugcgcgg 20

<210> 323
<211> 21
<212> RNA
<213> Homo sapiens

<400> 323
ucucucggcu ccucgcggcu c 21

<210> 324
<211> 22
<212> RNA
<213> Homo sapiens

<400> 324
ucacccugca ucccgcaccc ag 22

<210> 325
<211> 19
<212> RNA
<213> Homo sapiens

<400> 325
gacuggacaa gcugaggaa 19

<210> 326
<211> 24
<212> RNA
<213> Homo sapiens

<400> 326
gaaaaugaug aguagugacu gaug 24

<210> 327
<211> 22
<212> RNA
<213> Homo sapiens

<400> 327
uaguggauga ugcacucugu gc 22

<210> 328
<211> 17
<212> RNA
<213> Homo sapiens

<400> 328
accccacucc ugguacc 17

<210> 329
<211> 22
<212> RNA
<213> Homo sapiens

<400> 329
cacccccugu uuccuggccc ac 22

<210> 330
<211> 21
<212> RNA
<213> Homo sapiens

<400> 330
acacaugggu ggcuguggcc u 21

<210> 331
<211> 22
<212> RNA
<213> Homo sapiens

<400> 331
ugugacagau ugauaacuga aa 22

<210> 332
<211> 22
<212> RNA
<213> Homo sapiens

<400> 332
cagggaaaug ggaagaacua ga 22

<210> 333
<211> 22
<212> RNA
<213> Homo sapiens

<400> 333
cgaaaacagc aauuaccuuu gc 22

<210> 334
<211> 23
<212> RNA
<213> Homo sapiens

<400> 334
ugagugugug ugugugagug ugu 23

<210> 335
<211> 21
<212> RNA
<213> Homo sapiens

<400> 335
ucuaguaaga guggcagucg a 21

<210> 336
<211> 22
<212> RNA
<213> Homo sapiens

<400> 336

uaugucugcu gaccaucacc uu 22

<210> 337
<211> 23
<212> RNA
<213> Homo sapiens

<400> 337
cugggaucuc cggggucuug guu 23

<210> 338
<211> 21
<212> RNA
<213> Homo sapiens

<400> 338
cugacuguug ccguccucca g 21

<210> 339
<211> 22
<212> RNA
<213> Homo sapiens

<400> 339
cuauacaacc uacugccuuc cc 22

<210> 340
<211> 26
<212> RNA
<213> Homo sapiens

<400> 340
aaguaguugg uuuguaugag augguu 26

<210> 341
<211> 23
<212> RNA
<213> Homo sapiens

<400> 341
aggaugagca aagaaaguag auu 23

<210> 342
<211> 22
<212> RNA
<213> Homo sapiens

<400> 342
uugcuugaac ccaggaagug ga 22

<210> 343
<211> 23
<212> RNA
<213> Homo sapiens

<400> 343
uggaguccag gaaucugcau uuu 23

<210> 344
<211> 21
<212> RNA
<213> Homo sapiens

<400> 344
ucagugcaug acagaacuug g 21

<210> 345
<211> 22
<212> RNA
<213> Homo sapiens

<400> 345
uguaacagca acuccaugug ga 22

<210> 346
<211> 21
<212> RNA
<213> Homo sapiens

<400> 346
uagcagcaca gaaauauugg c 21

<210> 347
<211> 23
<212> RNA
<213> Homo sapiens

<400> 347
uaauacugcc ggguaaugau gga 23

<210> 348
<211> 21
<212> RNA
<213> Homo sapiens

<400> 348
uaacagucuc cagucacggc c 21

<210> 349
<211> 22
<212> RNA
<213> Homo sapiens

<400> 349
cucaguagcc aguguagauc cu 22

<210> 350
<211> 23
<212> RNA
<213> Homo sapiens

<400> 350
ucagcaccag gauauuguug gag 23

<210> 351
<211> 20
<212> RNA
<213> Homo sapiens

<400> 351
auaugggguuu acuaguuggu 20

<210> 352
<211> 22
<212> RNA
<213> Homo sapiens

<400> 352
ugagggacag augccagaag ca 22

<210> 353
<211> 23
<212> RNA
<213> Homo sapiens

<400> 353
uagugaguua gagaugcaga gcc 23

<210> 354
<211> 23
<212> RNA
<213> Homo sapiens

<400> 354
cgcaucccu agggcauugg ugu 23

<210> 355
<211> 21
<212> RNA
<213> Homo sapiens

<400> 355
gugcauugua guugcauugc a 21

<210> 356
<211> 22
<212> RNA
<213> Homo sapiens

<400> 356
cucgugggcu cuggccacgg cc 22

<210> 357
<211> 22
<212> RNA
<213> Homo sapiens

<400> 357
agaucgaccg uguuauauuc gc 22

<210> 358
<211> 22
<212> RNA
<213> Homo sapiens

<400> 358
aucgggaaug ucguguccgc cc 22

<210> 359
<211> 17
<212> RNA
<213> Homo sapiens

<400> 359
gaagauggac guacuuu 17

<210> 360
<211> 22
<212> RNA

<213> Homo sapiens

<400> 360
uggcggcggu aguuaugggc uu 22

<210> 361
<211> 22
<212> RNA
<213> Homo sapiens

<400> 361
uuucuauuuc ucagugggc uc 22

<210> 362
<211> 22
<212> RNA
<213> Homo sapiens

<400> 362
aggacuggac ucccggcagc cc 22

<210> 363
<211> 18
<212> RNA
<213> Homo sapiens

<400> 363
cggugagcgc ucgcuggc 18

<210> 364
<211> 22
<212> RNA
<213> Homo sapiens

<400> 364
aggaccuucc cugaaccaag ga 22

<210> 365
<211> 22
<212> RNA
<213> Homo sapiens

<400> 365
aggcggggcg ccgcgggacc gc 22

<210> 366
<211> 21
<212> RNA
<213> Homo sapiens

<400> 366
aaggcagggc ccccgcuccc c 21

<210> 367
<211> 22
<212> RNA
<213> Homo sapiens

<400> 367
caagcucgcu ucuaugggc ug 22

<210> 368
<211> 21

<212> RNA
<213> Homo sapiens

<400> 368
agaggauacc cuuuguaugu u 21

<210> 369
<211> 22
<212> RNA
<213> Homo sapiens

<400> 369
ugagccccug ugccgccccc ag 22

<210> 370
<211> 21
<212> RNA
<213> Homo sapiens

<400> 370
uccuucugcu ccgucccca g 21

<210> 371
<211> 22
<212> RNA
<213> Homo sapiens

<400> 371
agaaggaaau ugaauucauu ua 22

<210> 372
<211> 21
<212> RNA
<213> Homo sapiens

<400> 372
agugaaugau ggguucugac c 21

<210> 373
<211> 19
<212> RNA
<213> Homo sapiens

<400> 373
aucccaccac ugccaccau 19

<210> 374
<211> 25
<212> RNA
<213> Homo sapiens

<400> 374
gaacccauga gguugaggcu gcagu 25

<210> 375
<211> 19
<212> RNA
<213> Homo sapiens

<400> 375
uggauuuuug gaucaggga 19

<210> 376

<211> 18
<212> RNA
<213> Homo sapiens

<400> 376
acguuggcuc ugguggug 18

<210> 377
<211> 18
<212> RNA
<213> Homo sapiens

<400> 377
cagggaggug aaugugau 18

<210> 378
<211> 22
<212> RNA
<213> Homo sapiens

<400> 378
cuccuggggc ccgcacucuc gc 22

<210> 379
<211> 23
<212> RNA
<213> Homo sapiens

<400> 379
agcuggugu gugaaucagg ccg 23

<210> 380
<211> 22
<212> RNA
<213> Homo sapiens

<400> 380
caguggguuuu acccuauggu ag 22

<210> 381
<211> 22
<212> RNA
<213> Homo sapiens

<400> 381
ugcccugugg acucaguucu gg 22

<210> 382
<211> 22
<212> RNA
<213> Homo sapiens

<400> 382
gcccaaaggu gaauuuuuug gg 22

<210> 383
<211> 21
<212> RNA
<213> Homo sapiens

<400> 383
cggcggggac ggcgauuggu c 21

<210> 384
<211> 20
<212> RNA
<213> Homo sapiens

<400> 384
ccccagggcg acgcggcggg 20

<210> 385
<211> 22
<212> RNA
<213> Homo sapiens

<400> 385
accuugccuu gcugcccggg cc 22

<210> 386
<211> 22
<212> RNA
<213> Homo sapiens

<400> 386
aacuggccua caaaguccca gu 22

<210> 387
<211> 23
<212> RNA
<213> Homo sapiens

<400> 387
aguuuugcag guuugcaucc agc 23

<210> 388
<211> 22
<212> RNA
<213> Homo sapiens

<400> 388
auaagacgaa caaaagguuu gu 22

<210> 389
<211> 22
<212> RNA
<213> Homo sapiens

<400> 389
uuggggaaac ggccgcugag ug 22

<210> 390
<211> 22
<212> RNA
<213> Homo sapiens

<400> 390
agaguugagu cuggacgucc cg 22

<210> 391
<211> 22
<212> RNA
<213> Homo sapiens

<400> 391
ccuguucucc auuacuuggc uc 22

<210> 392
<211> 23
<212> RNA
<213> Homo sapiens

<400> 392
agaauugcgu uuggacaauc agu 23

<210> 393
<211> 22
<212> RNA
<213> Homo sapiens

<400> 393
acugauuucu uuugguguuc ag 22

<210> 394
<211> 22
<212> RNA
<213> Homo sapiens

<400> 394
caucuggcau ccgucacaca ga 22

<210> 395
<211> 21
<212> RNA
<213> Homo sapiens

<400> 395
gcugcaccgg agacugggua a 21

<210> 396
<211> 21
<212> RNA
<213> Homo sapiens

<400> 396
uacccagucu ccggugcagc c 21

<210> 397
<211> 21
<212> RNA
<213> Homo sapiens

<400> 397
accugaauua ccaaaagcuu u 21

<210> 398
<211> 21
<212> RNA
<213> Homo sapiens

<400> 398
ccaggcucug cagugggaac u 21

<210> 399
<211> 22
<212> RNA
<213> Homo sapiens

<400> 399

cugcccuagu cuagcugaag cu 22

<210> 400
<211> 22
<212> RNA
<213> Homo sapiens

<400> 400
uggggcggag cuuccggagg cc 22

<210> 401
<211> 23
<212> RNA
<213> Homo sapiens

<400> 401
agguuguccg uggugaguuc gca 23

<210> 402
<211> 23
<212> RNA
<213> Homo sapiens

<400> 402
ucccuguccu ccaggagcuc acg 23

<210> 403
<211> 22
<212> RNA
<213> Homo sapiens

<400> 403
caaucagcaa guauacugcc cu 22

<210> 404
<211> 22
<212> RNA
<213> Homo sapiens

<400> 404
caaucacuaa cuccacugcc au 22

<210> 405
<211> 22
<212> RNA
<213> Homo sapiens

<400> 405
aaucacuaac cacacggcca gg 22

<210> 406
<211> 22
<212> RNA
<213> Homo sapiens

<400> 406
uuuaagaaaa caccauggag au 22

<210> 407
<211> 23
<212> RNA
<213> Homo sapiens

<400> 407
agggacuuuu gggggcagau gug 23

<210> 408
<211> 20
<212> RNA
<213> Homo sapiens

<400> 408
ccguguuucc cccacgcuuu 20

<210> 409
<211> 23
<212> RNA
<213> Homo sapiens

<400> 409
aguggaugau ggagacucgg uac 23

<210> 410
<211> 22
<212> RNA
<213> Homo sapiens

<400> 410
guagauucuc cuucuaugag ua 22

<210> 411
<211> 20
<212> RNA
<213> Homo sapiens

<400> 411
acugggcuug gagucagaag 20

<210> 412
<211> 22
<212> RNA
<213> Homo sapiens

<400> 412
uguccucuag ggccugcagu cu 22

<210> 413
<211> 22
<212> RNA
<213> Homo sapiens

<400> 413
ggaggaaccu uggagcuucg gc 22

<210> 414
<211> 21
<212> RNA
<213> Homo sapiens

<400> 414
uuucagauaa caguauuaca u 21

<210> 415
<211> 21
<212> RNA
<213> Homo sapiens

<400> 415
ugugcagcag gccaaccgag a 21

<210> 416
<211> 20
<212> RNA
<213> Homo sapiens

<400> 416
ggcggcggcg gaggcggggg 20

<210> 417
<211> 20
<212> RNA
<213> Homo sapiens

<400> 417
uguuccucug ucucccagac 20

<210> 418
<211> 18
<212> RNA
<213> Homo sapiens

<400> 418
cucgaguugg aagaggcg 18

<210> 419
<211> 22
<212> RNA
<213> Homo sapiens

<400> 419
uggggauuug gagaaguggu ga 22

<210> 420
<211> 22
<212> RNA
<213> Homo sapiens

<400> 420
auggcaucgu ccccuggugg cu 22

<210> 421
<211> 23
<212> RNA
<213> Homo sapiens

<400> 421
agggaaaaaa aaaaggauuu guc 23

<210> 422
<211> 22
<212> RNA
<213> Homo sapiens

<400> 422
uccaggcagg agccggacug ga 22

<210> 423
<211> 22
<212> RNA

<213> Homo sapiens

<400> 423
uaggggcagc agaggaccug gg 22

<210> 424
<211> 26
<212> RNA
<213> Homo sapiens

<400> 424
cacaggacug acucccucacc ccagug 26

<210> 425
<211> 22
<212> RNA
<213> Homo sapiens

<400> 425
cacacaagug gcccccaaca cu 22

<210> 426
<211> 20
<212> RNA
<213> Homo sapiens

<400> 426
cgccccuccu gcccccacag 20

<210> 427
<211> 24
<212> RNA
<213> Homo sapiens

<400> 427
ggugggaugg agagaaggua ugag 24

<210> 428
<211> 21
<212> RNA
<213> Homo sapiens

<400> 428
aguggaccga ggaaggaagg a 21

<210> 429
<211> 22
<212> RNA
<213> Homo sapiens

<400> 429
aguggggaac ccuuccauga gg 22

<210> 430
<211> 22
<212> RNA
<213> Homo sapiens

<400> 430
aauccuuugu cccugggguga ga 22

<210> 431
<211> 22

<212> RNA
<213> Homo sapiens

<400> 431
aauccacgcu gagcuuggca uc 22

<210> 432
<211> 25
<212> RNA
<213> Homo sapiens

<400> 432
aaaggauucu gcugucgguc ccacu 25

<210> 433
<211> 23
<212> RNA
<213> Homo sapiens

<400> 433
ucggggauca ucaugucacg aga 23

<210> 434
<211> 21
<212> RNA
<213> Homo sapiens

<400> 434
gcgacccaua cuugguuuca g 21

<210> 435
<211> 21
<212> RNA
<213> Homo sapiens

<400> 435
ucagcuacua ccucuauuag g 21

<210> 436
<211> 21
<212> RNA
<213> Homo sapiens

<400> 436
uagauaaaau auugguaccu g 21

<210> 437
<211> 22
<212> RNA
<213> Homo sapiens

<400> 437
ucaguuccag gccaaccagg cu 22

<210> 438
<211> 24
<212> RNA
<213> Homo sapiens

<400> 438
ucagaacaaa ugccgguucc caga 24

<210> 439

<211> 22
<212> RNA
<213> Homo sapiens

<400> 439
acucaaaacc cuucagugac uu 22

<210> 440
<211> 22
<212> RNA
<213> Homo sapiens

<400> 440
augcugacau auuuacuaga gg 22

<210> 441
<211> 21
<212> RNA
<213> Homo sapiens

<400> 441
uggguuuacg uugggagaac u 21

<210> 442
<211> 22
<212> RNA
<213> Homo sapiens

<400> 442
uucauuugcc ucccagccua ca 22

<210> 443
<211> 24
<212> RNA
<213> Homo sapiens

<400> 443
acuggcuagg gaaaaugauu ggau 24

<210> 444
<211> 23
<212> RNA
<213> Homo sapiens

<400> 444
gcagcagaga auaggacuac guc 23

<210> 445
<211> 22
<212> RNA
<213> Homo sapiens

<400> 445
uauguaauau gguccacauc uu 22

<210> 446
<211> 22
<212> RNA
<213> Homo sapiens

<400> 446
uauguaacau gguccacuaa cu 22

<210> 447
<211> 22
<212> RNA
<213> Homo sapiens

<400> 447
cgaaucauua uuugcugcuc ua 22

<210> 448
<211> 23
<212> RNA
<213> Homo sapiens

<400> 448
uuaaugcuaa ucgugauagg ggu 23

<210> 449
<211> 22
<212> RNA
<213> Homo sapiens

<400> 449
aucacacaaa ggcaacuuuu gu 22

<210> 450
<211> 21
<212> RNA
<213> Homo sapiens

<400> 450
gccccugggc cuauccuaga a 21

<210> 451
<211> 21
<212> RNA
<213> Homo sapiens

<400> 451
augaccuaug aauugacaga c 21

<210> 452
<211> 22
<212> RNA
<213> Homo sapiens

<400> 452
aucauagagg aaaauccaug uu 22

<210> 453
<211> 21
<212> RNA
<213> Homo sapiens

<400> 453
cggggcagcu caguacagga u 21

<210> 454
<211> 22
<212> RNA
<213> Homo sapiens

<400> 454
acugcugagc uagcacuucc cg 22

<210> 455
<211> 18
<212> RNA
<213> Homo sapiens

<400> 455
ucgaggagcu cacagucu 18

<210> 456
<211> 22
<212> RNA
<213> Homo sapiens

<400> 456
gugaacgggc gccaucccga gg 22

<210> 457
<211> 23
<212> RNA
<213> Homo sapiens

<400> 457
ccucagggcu guagaacagg gcu 23

<210> 458
<211> 23
<212> RNA
<213> Homo sapiens

<400> 458
uaagugcuuc cauguuuuag uag 23

<210> 459
<211> 22
<212> RNA
<213> Homo sapiens

<400> 459
aaaaacugag acuacuuuug ca 22

<210> 460
<211> 23
<212> RNA
<213> Homo sapiens

<400> 460
uaagugcuuc cauguuugag ugu 23

<210> 461
<211> 22
<212> RNA
<213> Homo sapiens

<400> 461
uacgucaucg uugucaucgu ca 22

<210> 462
<211> 21
<212> RNA
<213> Homo sapiens

<400> 462

cauuauuacu uuugguacgc g 21

<210> 463
<211> 21
<212> RNA
<213> Homo sapiens

<400> 463
uaauuuuaug uauaagcuag u 21

<210> 464
<211> 22
<212> RNA
<213> Homo sapiens

<400> 464
ucugggcaac aaagugagac cu 22

<210> 465
<211> 23
<212> RNA
<213> Homo sapiens

<400> 465
uacccuguag aaccgaauuu gug 23

<210> 466
<211> 23
<212> RNA
<213> Homo sapiens

<400> 466
cugauaagaa cagaggccca gau 23

<210> 467
<211> 22
<212> RNA
<213> Homo sapiens

<400> 467
agaauugugg cuggacaucu gu 22

<210> 468
<211> 24
<212> RNA
<213> Homo sapiens

<400> 468
agcgcgggcu gagcgcugcc aguc 24

<210> 469
<211> 22
<212> RNA
<213> Homo sapiens

<400> 469
uguuguacuu uuuuuuugu uc 22

<210> 470
<211> 22
<212> RNA
<213> Homo sapiens

<400> 470
uaacugguug aacaacugaa cc 22

<210> 471
<211> 23
<212> RNA
<213> Homo sapiens

<400> 471
aaaagugcuu acagugcagg uag 23

<210> 472
<211> 22
<212> RNA
<213> Homo sapiens

<400> 472
aaucauacac gguugaccua uu 22

<210> 473
<211> 22
<212> RNA
<213> Homo sapiens

<400> 473
uagcaccauu ugaaaucggu ua 22

<210> 474
<211> 22
<212> RNA
<213> Homo sapiens

<400> 474
gcugcgcuug gauuucgucc cc 22

<210> 475
<211> 22
<212> RNA
<213> Homo sapiens

<400> 475
uaauacugcc ugguaaugau ga 22

<210> 476
<211> 23
<212> RNA
<213> Homo sapiens

<400> 476
ugucacucgg cucggcccac uac 23

<210> 477
<211> 22
<212> RNA
<213> Homo sapiens

<400> 477
ugagaacuga auuccauggg uu 22

<210> 478
<211> 22
<212> RNA
<213> Homo sapiens

<400> 478
cugugcgugu gacagcggcu ga 22

<210> 479
<211> 21
<212> RNA
<213> Homo sapiens

<400> 479
cagcagcaca cugugguuug u 21

<210> 480
<211> 22
<212> RNA
<213> Homo sapiens

<400> 480
caacaaauca cagucugcca ua 22

<210> 481
<211> 22
<212> RNA
<213> Homo sapiens

<400> 481
ugaccuggga cucggacagc ug 22

<210> 482
<211> 22
<212> RNA
<213> Homo sapiens

<400> 482
uauguaacac gguccacuaa cc 22

<210> 483
<211> 22
<212> RNA
<213> Homo sapiens

<400> 483
cugcgcaagc uacugccuug cu 22

<210> 484
<211> 22
<212> RNA
<213> Homo sapiens

<400> 484
aacuagcucu guggauccug ac 22

<210> 485
<211> 22
<212> RNA
<213> Homo sapiens

<400> 485
uuaugguuug ccugggacug ag 22

<210> 486
<211> 22
<212> RNA

<213> Homo sapiens

<400> 486
cuguugccac uaaccucaac cu 22

<210> 487
<211> 23
<212> RNA
<213> Homo sapiens

<400> 487
uccaguacca cgugucaggg cca 23

<210> 488
<211> 22
<212> RNA
<213> Homo sapiens

<400> 488
uccugucuuu ccuuguugga gc 22

<210> 489
<211> 23
<212> RNA
<213> Homo sapiens

<400> 489
uuacaguugu ucaaccaguu acu 23

<210> 490
<211> 22
<212> RNA
<213> Homo sapiens

<400> 490
acgcccuucc cccccuucuu ca 22

<210> 491
<211> 22
<212> RNA
<213> Homo sapiens

<400> 491
gcucugacuu uauugcacua cu 22

<210> 492
<211> 22
<212> RNA
<213> Homo sapiens

<400> 492
uucugccucu guccaggucc uu 22

<210> 493
<211> 22
<212> RNA
<213> Homo sapiens

<400> 493
aaucagugaa ugccuugaac cu 22

<210> 494
<211> 22

<212> RNA
<213> Homo sapiens

<400> 494
acaguagucu gcacauuggu ua 22

<210> 495
<211> 22
<212> RNA
<213> Homo sapiens

<400> 495
aguucuucag uggcaagcuu ua 22

<210> 496
<211> 23
<212> RNA
<213> Homo sapiens

<400> 496
ccuccguguu accuguccuc uag 23

<210> 497
<211> 22
<212> RNA
<213> Homo sapiens

<400> 497
agacccuggu cugcacucua uc 22

<210> 498
<211> 22
<212> RNA
<213> Homo sapiens

<400> 498
uucuggaauu cugugugagg ga 22

<210> 499
<211> 21
<212> RNA
<213> Homo sapiens

<400> 499
caucccuugc augguggagg g 21

<210> 500
<211> 22
<212> RNA
<213> Homo sapiens

<400> 500
cuagguaugg ucccaggau cc 22

<210> 501
<211> 22
<212> RNA
<213> Homo sapiens

<400> 501
uagguuaucc guguugccuu cg 22

<210> 502

<211> 23
<212> RNA
<213> Homo sapiens

<400> 502
acugcccuaa gugcuccuuc ugg 23

<210> 503
<211> 23
<212> RNA
<213> Homo sapiens

<400> 503
uaagugcuuc cauguuuugg uga 23

<210> 504
<211> 20
<212> RNA
<213> Homo sapiens

<400> 504
ccucugggcc cuuccuccag 20

<210> 505
<211> 21
<212> RNA
<213> Homo sapiens

<400> 505
cuguacaggc cacugccuug c 21

<210> 506
<211> 22
<212> RNA
<213> Homo sapiens

<400> 506
ugccuacuga gcugauauca gu 22

<210> 507
<211> 25
<212> RNA
<213> Homo sapiens

<400> 507
aaauaugaug aaacucacag cugag 25

<210> 508
<211> 22
<212> RNA
<213> Homo sapiens

<400> 508
aagaugugga aaaauuggaa uc 22

<210> 509
<211> 23
<212> RNA
<213> Homo sapiens

<400> 509
cucucaccac ugcccuccca cag 23

<210> 510
<211> 22
<212> RNA
<213> Homo sapiens

<400> 510
uggguuccug gcaugcugau uu 22

<210> 511
<211> 23
<212> RNA
<213> Homo sapiens

<400> 511
acuuaaacgu ggauguacuu gcu 23

<210> 512
<211> 22
<212> RNA
<213> Homo sapiens

<400> 512
aauucccuug uagauaaccc gg 22

<210> 513
<211> 22
<212> RNA
<213> Homo sapiens

<400> 513
cuguacagcc uccuagcuuu cc 22

<210> 514
<211> 22
<212> RNA
<213> Homo sapiens

<400> 514
caaauucgua ucuaggggaa ua 22

<210> 515
<211> 22
<212> RNA
<213> Homo sapiens

<400> 515
ugcuggauca gugguucgag uc 22

<210> 516
<211> 23
<212> RNA
<213> Homo sapiens

<400> 516
guccaguuuu cccaggaauc ccu 23

<210> 517
<211> 24
<212> RNA
<213> Homo sapiens

<400> 517
gcugguuuca uaugguggu uaga 24

<210> 518
<211> 23
<212> RNA
<213> Homo sapiens

<400> 518
ucuggcaagu aaaaaacucu cau 23

<210> 519
<211> 20
<212> RNA
<213> Homo sapiens

<400> 519
gugcauugcu guugcauugc 20

<210> 520
<211> 22
<212> RNA
<213> Homo sapiens

<400> 520
uuuccuaccc uaccugaaga cu 22

<210> 521
<211> 21
<212> RNA
<213> Homo sapiens

<400> 521
acagggccgc agauggagac u 21

<210> 522
<211> 22
<212> RNA
<213> Homo sapiens

<400> 522
gaaaucaagc gugggugaga cc 22

<210> 523
<211> 22
<212> RNA
<213> Homo sapiens

<400> 523
uuugaggcua cagugagaug ug 22

<210> 524
<211> 23
<212> RNA
<213> Homo sapiens

<400> 524
cggcccgggc ugcugcuguu ccu 23

<210> 525
<211> 22
<212> RNA
<213> Homo sapiens

<400> 525

aaccaucgac cguugagugg ac 22

<210> 526
<211> 22
<212> RNA
<213> Homo sapiens

<400> 526
ugggucuuug cgggcgagau ga 22

<210> 527
<211> 21
<212> RNA
<213> Homo sapiens

<400> 527
ugauugucca aacgcaauuc u 21

<210> 528
<211> 22
<212> RNA
<213> Homo sapiens

<400> 528
uaggauuaca agugucggcc ac 22

<210> 529
<211> 22
<212> RNA
<213> Homo sapiens

<400> 529
ugcccugccu guuuucuccu uu 22

<210> 530
<211> 22
<212> RNA
<213> Homo sapiens

<400> 530
cggggagaga acgcagugac gu 22

<210> 531
<211> 22
<212> RNA
<213> Homo sapiens

<400> 531
aaucugagaa ggcgcacaag gu 22

<210> 532
<211> 23
<212> RNA
<213> Homo sapiens

<400> 532
uugaagagga ggugcucugu agc 23

<210> 533
<211> 24
<212> RNA
<213> Homo sapiens

<400> 533
gaugcgccgc ccacugcccc gcgc 24

<210> 534
<211> 21
<212> RNA
<213> Homo sapiens

<400> 534
uuaagacuug cagugauguu u 21

<210> 535
<211> 23
<212> RNA
<213> Homo sapiens

<400> 535
caaagacugc aauuacuuuu gcg 23

<210> 536
<211> 25
<212> RNA
<213> Homo sapiens

<400> 536
ugggaacggg uuccggcaga cgcug 25

<210> 537
<211> 22
<212> RNA
<213> Homo sapiens

<400> 537
cccugugccc ggcccacuuc ug 22

<210> 538
<211> 22
<212> RNA
<213> Homo sapiens

<400> 538
ccaauauugg cugugcugcu cc 22

<210> 539
<211> 22
<212> RNA
<213> Homo sapiens

<400> 539
uuuaacaugg ggguaccugc ug 22

<210> 540
<211> 22
<212> RNA
<213> Homo sapiens

<400> 540
caaccucgac gaucuccuca gc 22

<210> 541
<211> 22
<212> RNA
<213> Homo sapiens

<400> 541
cccaauacac ggucgaccuc uu 22

<210> 542
<211> 22
<212> RNA
<213> Homo sapiens

<400> 542
uuuuucauua uugcuccuga cc 22

<210> 543
<211> 20
<212> RNA
<213> Homo sapiens

<400> 543
acuguaaacg cuuucugaug 20

<210> 544
<211> 18
<212> RNA
<213> Homo sapiens

<400> 544
cuaagaaguu gacugaag 18

<210> 545
<211> 23
<212> RNA
<213> Homo sapiens

<400> 545
ugagcaccac acaggccggg cgc 23

<210> 546
<211> 21
<212> RNA
<213> Homo sapiens

<400> 546
auaggcacca aaaagcaaca a 21

<210> 547
<211> 22
<212> RNA
<213> Homo sapiens

<400> 547
uggcagugua uuguuagcug gu 22

<210> 548
<211> 22
<212> RNA
<213> Homo sapiens

<400> 548
gaagaacugu ugcauuugcc cu 22

<210> 549
<211> 19
<212> RNA

<213> Homo sapiens

<400> 549
caucccguccg ucuguccac 19

<210> 550
<211> 22
<212> RNA
<213> Homo sapiens

<400> 550
gggagccagg aaguauugau gu 22

<210> 551
<211> 22
<212> RNA
<213> Homo sapiens

<400> 551
caaaacuggc aauuacuuuu gc 22

<210> 552
<211> 21
<212> RNA
<213> Homo sapiens

<400> 552
uccucuucuc ccuccuccca g 21

<210> 553
<211> 23
<212> RNA
<213> Homo sapiens

<400> 553
agcuucuuua cagugcugcc uug 23

<210> 554
<211> 20
<212> RNA
<213> Homo sapiens

<400> 554
cgggcguggu gguggggug 20

<210> 555
<211> 23
<212> RNA
<213> Homo sapiens

<400> 555
cuggagauau ggaagagcug ugu 23

<210> 556
<211> 22
<212> RNA
<213> Homo sapiens

<400> 556
ugggugucu ggagauuugu gc 22

<210> 557
<211> 19

<212> RNA
<213> Homo sapiens

<400> 557
gaugaugcug cugaugcug 19

<210> 558
<211> 22
<212> RNA
<213> Homo sapiens

<400> 558
ucucccaacc cuuguaccag ug 22

<210> 559
<211> 21
<212> RNA
<213> Homo sapiens

<400> 559
ugauauguuu gauauugggu u 21

<210> 560
<211> 22
<212> RNA
<213> Homo sapiens

<400> 560
cgggguuuug agggcgagau ga 22

<210> 561
<211> 22
<212> RNA
<213> Homo sapiens

<400> 561
acugcauuau gagcacuuaa ag 22

<210> 562
<211> 22
<212> RNA
<213> Homo sapiens

<400> 562
uaaucucagc uggcaacugu ga 22

<210> 563
<211> 23
<212> RNA
<213> Homo sapiens

<400> 563
caggcaguga cuguucagac guc 23

<210> 564
<211> 22
<212> RNA
<213> Homo sapiens

<400> 564
agauguccag ccacaauucu cg 22

<210> 565

<211> 23
<212> RNA
<213> Homo sapiens

<400> 565
uguguacaca cgugccaggc gcu 23

<210> 566
<211> 20
<212> RNA
<213> Homo sapiens

<400> 566
aaaagcuggg uugagagggu 20

<210> 567
<211> 22
<212> RNA
<213> Homo sapiens

<400> 567
agguggucog uggcgcguuc gc 22

<210> 568
<211> 22
<212> RNA
<213> Homo sapiens

<400> 568
caggcacggg agcucaggug ag 22

<210> 569
<211> 21
<212> RNA
<213> Homo sapiens

<400> 569
uaacgcauaa uauggacaug u 21

<210> 570
<211> 22
<212> RNA
<213> Homo sapiens

<400> 570
ucaggugugg aaacugaggc ag 22

<210> 571
<211> 22
<212> RNA
<213> Homo sapiens

<400> 571
aagcaauacu guuaccugaa au 22

<210> 572
<211> 21
<212> RNA
<213> Homo sapiens

<400> 572
gaccgagagg gccucggcug u 21

<210> 573
<211> 23
<212> RNA
<213> Homo sapiens

<400> 573
ugggccaggg agcagcuggu ggg 23

<210> 574
<211> 22
<212> RNA
<213> Homo sapiens

<400> 574
accgaagacu gugcgcuaau cu 22

<210> 575
<211> 22
<212> RNA
<213> Homo sapiens

<400> 575
acaacaguga cuugcucucc aa 22

<210> 576
<211> 22
<212> RNA
<213> Homo sapiens

<400> 576
ugcuggggc cacaugagug ug 22

<210> 577
<211> 21
<212> RNA
<213> Homo sapiens

<400> 577
ccaaaucuug aucagaagcc u 21

<210> 578
<211> 22
<212> RNA
<213> Homo sapiens

<400> 578
agggggaugg cagagcaaaa uu 22

<210> 579
<211> 21
<212> RNA
<213> Homo sapiens

<400> 579
auccuugcua ucugggugcu a 21

<210> 580
<211> 22
<212> RNA
<213> Homo sapiens

<400> 580
aaaaguaauu gugguuuuug cc 22

<210> 581
<211> 22
<212> RNA
<213> Homo sapiens

<400> 581
aaaaguaauu gcggauuuug cc 22

<210> 582
<211> 22
<212> RNA
<213> Homo sapiens

<400> 582
aaaaguaauu gcggucuuug gu 22

<210> 583
<211> 20
<212> RNA
<213> Homo sapiens

<400> 583
cgugccaccc uuuuccccag 20

<210> 584
<211> 17
<212> RNA
<213> Homo sapiens

<400> 584
ucccaccgcu gccaccc 17

<210> 585
<211> 21
<212> RNA
<213> Homo sapiens

<400> 585
uggacugccc ugaucuggag a 21

<210> 586
<211> 22
<212> RNA
<213> Homo sapiens

<400> 586
uuuagagacg gggucuugcu cu 22

<210> 587
<211> 22
<212> RNA
<213> Homo sapiens

<400> 587
ucuacagugc acgugucucc ag 22

<210> 588
<211> 21
<212> RNA
<213> Homo sapiens

<400> 588

agggagggac gggggcugug c 21

<210> 589
<211> 22
<212> RNA
<213> Homo sapiens

<400> 589
ccaguauuaa cugugcugcu ga 22

<210> 590
<211> 21
<212> RNA
<213> Homo sapiens

<400> 590
ugagugccgg ugccugcccu g 21

<210> 591
<211> 21
<212> RNA
<213> Homo sapiens

<400> 591
caagucacua gugguuccgu u 21

<210> 592
<211> 22
<212> RNA
<213> Homo sapiens

<400> 592
ucugcaagug ucagaggcga gg 22

<210> 593
<211> 22
<212> RNA
<213> Homo sapiens

<400> 593
auuguccuug cuguuuggag au 22

<210> 594
<211> 22
<212> RNA
<213> Homo sapiens

<400> 594
ugaccgauuu cuccuggugu uc 22

<210> 595
<211> 22
<212> RNA
<213> Homo sapiens

<400> 595
gauaucagcu caguaggcac cg 22

<210> 596
<211> 21
<212> RNA
<213> Homo sapiens

<400> 596
cacagcaagu guagacaggc a 21

<210> 597
<211> 22
<212> RNA
<213> Homo sapiens

<400> 597
agcuuuuggg aauucaggua gu 22

<210> 598
<211> 22
<212> RNA
<213> Homo sapiens

<400> 598
uauaaaauga gggcaguaag ac 22

<210> 599
<211> 22
<212> RNA
<213> Homo sapiens

<400> 599
aggauuucag aaauacuggu gu 22

<210> 600
<211> 22
<212> RNA
<213> Homo sapiens

<400> 600
cggguggauc acgaugcaau uu 22

<210> 601
<211> 22
<212> RNA
<213> Homo sapiens

<400> 601
ugggcuggca gggcaagugc ug 22

<210> 602
<211> 22
<212> RNA
<213> Homo sapiens

<400> 602
ugugggaucu ggaggcaucu gg 22

<210> 603
<211> 22
<212> RNA
<213> Homo sapiens

<400> 603
uuucuucuua gacauggcaa cg 22

<210> 604
<211> 22
<212> RNA
<213> Homo sapiens

<400> 604
uuagccaauu guccaucuuu ag 22

<210> 605
<211> 23
<212> RNA
<213> Homo sapiens

<400> 605
uggagaucca gugcucgccc gau 23

<210> 606
<211> 22
<212> RNA
<213> Homo sapiens

<400> 606
ugaaacugga gcgccuggag ga 22

<210> 607
<211> 21
<212> RNA
<213> Homo sapiens

<400> 607
agcggugcuc cugcgggccg a 21

<210> 608
<211> 21
<212> RNA
<213> Homo sapiens

<400> 608
gagguuuggg gaggauuugc u 21

<210> 609
<211> 22
<212> RNA
<213> Homo sapiens

<400> 609
ucaggacacu ucugaacuug ga 22

<210> 610
<211> 23
<212> RNA
<213> Homo sapiens

<400> 610
uagcagcggg aacaguucug cag 23

<210> 611
<211> 24
<212> RNA
<213> Homo sapiens

<400> 611
ucugggcaca ggcggaugga cagg 24

<210> 612
<211> 22
<212> RNA

<213> Homo sapiens

<400> 612
caaaaacugc aauuacuuuu gc 22

<210> 613
<211> 20
<212> RNA
<213> Homo sapiens

<400> 613
aaaacugcag uuacuuuugc 20

<210> 614
<211> 22
<212> RNA
<213> Homo sapiens

<400> 614
ugugucacuc gaugaccacu gu 22

<210> 615
<211> 21
<212> RNA
<213> Homo sapiens

<400> 615
acagucugcu gagguuggag c 21

<210> 616
<211> 23
<212> RNA
<213> Homo sapiens

<400> 616
ugugcuugcu cgucccgccc gca 23

<210> 617
<211> 23
<212> RNA
<213> Homo sapiens

<400> 617
gggaugguag accggugacg ugc 23

<210> 618
<211> 21
<212> RNA
<213> Homo sapiens

<400> 618
ccccaccucc ucucuccuca g 21

<210> 619
<211> 22
<212> RNA
<213> Homo sapiens

<400> 619
uggaguguga caaugguguu ug 22

<210> 620
<211> 21

<212> RNA
<213> Homo sapiens

<400> 620
gugggcgggg gcaggugugu g 21

<210> 621
<211> 22
<212> RNA
<213> Homo sapiens

<400> 621
acccgucccg uucgucccccg ga 22

<210> 622
<211> 22
<212> RNA
<213> Homo sapiens

<400> 622
cggaugagca aagaaagugg uu 22

<210> 623
<211> 22
<212> RNA
<213> Homo sapiens

<400> 623
cuggacugag ccaugcuacu gg 22

<210> 624
<211> 26
<212> RNA
<213> Homo sapiens

<400> 624
gaugaugaug gcagcaaauu cugaaa 26

<210> 625
<211> 22
<212> RNA
<213> Homo sapiens

<400> 625
ggcgacaaaa cgagacccug uc 22

<210> 626
<211> 20
<212> RNA
<213> Homo sapiens

<400> 626
ucguuugccu uuuucugcuu 20

<210> 627
<211> 22
<212> RNA
<213> Homo sapiens

<400> 627
ugugagguug gcauuguugu cu 22

<210> 628

<211> 22
<212> RNA
<213> Homo sapiens

<400> 628
ccaccucccc ugcaaacguc ca 22

<210> 629
<211> 23
<212> RNA
<213> Homo sapiens

<400> 629
uauggcuuuu uauuccuaug uga 23

<210> 630
<211> 23
<212> RNA
<213> Homo sapiens

<400> 630
uuauugcuua agaauacgcg uag 23

<210> 631
<211> 21
<212> RNA
<213> Homo sapiens

<400> 631
cauaaaguag aaagcacuac u 21

<210> 632
<211> 22
<212> RNA
<213> Homo sapiens

<400> 632
ggugcagugc ugcaucucug gu 22

<210> 633
<211> 22
<212> RNA
<213> Homo sapiens

<400> 633
caggccauau ugugcugccu ca 22

<210> 634
<211> 22
<212> RNA
<213> Homo sapiens

<400> 634
cugccaauuc cauaggucac ag 22

<210> 635
<211> 22
<212> RNA
<213> Homo sapiens

<400> 635
uaacacuguc ugguaacgau gu 22

<210> 636
<211> 21
<212> RNA
<213> Homo sapiens

<400> 636
gcugggaagg caaagggacg u 21

<210> 637
<211> 22
<212> RNA
<213> Homo sapiens

<400> 637
acagcaggca cagacaggca gu 22

<210> 638
<211> 21
<212> RNA
<213> Homo sapiens

<400> 638
ucuggcuguu guggugugca a 21

<210> 639
<211> 22
<212> RNA
<213> Homo sapiens

<400> 639
ugccuggaac auaguaggga cu 22

<210> 640
<211> 20
<212> RNA
<213> Homo sapiens

<400> 640
uagaggaagc uguggagaga 20

<210> 641
<211> 22
<212> RNA
<213> Homo sapiens

<400> 641
uccccuucug caggccugcu gg 22

<210> 642
<211> 22
<212> RNA
<213> Homo sapiens

<400> 642
uucagccagg cuagugcagu cu 22

<210> 643
<211> 22
<212> RNA
<213> Homo sapiens

<400> 643
agaaggggug aaauuuaaac gu 22

<210> 644
<211> 19
<212> RNA
<213> Homo sapiens

<400> 644
aucgggcccu cggcgccgg 19

<210> 645
<211> 23
<212> RNA
<213> Homo sapiens

<400> 645
ccugggcagc guguggcuga agg 23

<210> 646
<211> 20
<212> RNA
<213> Homo sapiens

<400> 646
ucuggccagc uacgucccca 20

<210> 647
<211> 22
<212> RNA
<213> Homo sapiens

<400> 647
guggaguccu ggggaaugga ga 22

<210> 648
<211> 22
<212> RNA
<213> Homo sapiens

<400> 648
aaaagcuggg uugagagggc aa 22

<210> 649
<211> 23
<212> RNA
<213> Homo sapiens

<400> 649
uuuaguguga uaauggcguu uga 23

<210> 650
<211> 22
<212> RNA
<213> Homo sapiens

<400> 650
ucagcaggca ggcuggugca gc 22

<210> 651
<211> 21
<212> RNA
<213> Homo sapiens

<400> 651

ugaguguugu cuacgagggc a 21

<210> 652
<211> 22
<212> RNA
<213> Homo sapiens

<400> 652
auuguagaac cuaagauugg cc 22

<210> 653
<211> 22
<212> RNA
<213> Homo sapiens

<400> 653
cuuccccca guaaucuuca uc 22

<210> 654
<211> 22
<212> RNA
<213> Homo sapiens

<400> 654
uuuguucguu cggcucgcgu ga 22

<210> 655
<211> 19
<212> RNA
<213> Homo sapiens

<400> 655
acuggacuug gaggcagaa 19

<210> 656
<211> 22
<212> RNA
<213> Homo sapiens

<400> 656
gagcaaugua gguagacugu uu 22

<210> 657
<211> 22
<212> RNA
<213> Homo sapiens

<400> 657
uguguggauc cuggaggagg ca 22

<210> 658
<211> 22
<212> RNA
<213> Homo sapiens

<400> 658
uuugggacug aucuugaugu cu 22

<210> 659
<211> 20
<212> RNA
<213> Homo sapiens

<400> 659
gcucggacug agcagguggg 20

<210> 660
<211> 23
<212> RNA
<213> Homo sapiens

<400> 660
uucgggcugg ccugcugcuc cgg 23

<210> 661
<211> 22
<212> RNA
<213> Homo sapiens

<400> 661
uaauacuguc ugguaaaacc gu 22

<210> 662
<211> 22
<212> RNA
<213> Homo sapiens

<400> 662
accugucugu ggaaaggagc ua 22

<210> 663
<211> 17
<212> RNA
<213> Homo sapiens

<400> 663
uuggaggcgu ggguuuu 17

<210> 664
<211> 22
<212> RNA
<213> Homo sapiens

<400> 664
ccaggaggcg gaggaggugg ag 22

<210> 665
<211> 22
<212> RNA
<213> Homo sapiens

<400> 665
cuagugcucu ccguuacaag ua 22

<210> 666
<211> 22
<212> RNA
<213> Homo sapiens

<400> 666
cgcgcggccg ugcucggagc ag 22

<210> 667
<211> 22
<212> RNA
<213> Homo sapiens

<400> 667
ugugacaaua gagaugaaca ug 22

<210> 668
<211> 22
<212> RNA
<213> Homo sapiens

<400> 668
uuuggacaga aaacacgcag gu 22

<210> 669
<211> 22
<212> RNA
<213> Homo sapiens

<400> 669
aacuguuugc agaggaaacu ga 22

<210> 670
<211> 22
<212> RNA
<213> Homo sapiens

<400> 670
aacucguguu caaagccuuu ag 22

<210> 671
<211> 22
<212> RNA
<213> Homo sapiens

<400> 671
uuucuucuua gacauggcag cu 22

<210> 672
<211> 22
<212> RNA
<213> Homo sapiens

<400> 672
cuuccggucu gugagccccg uc 22

<210> 673
<211> 23
<212> RNA
<213> Homo sapiens

<400> 673
cuggggggacg cgugagcgcg agc 23

<210> 674
<211> 21
<212> RNA
<213> Homo sapiens

<400> 674
auacauguca gauuguaugc c 21

<210> 675
<211> 22
<212> RNA

<213> Homo sapiens

<400> 675
aacgggaaug caggcuguau cu 22

<210> 676
<211> 23
<212> RNA
<213> Homo sapiens

<400> 676
ucugaguucc uggagccugg ucu 23

<210> 677
<211> 22
<212> RNA
<213> Homo sapiens

<400> 677
gagcaggcga ggcugggcug aa 22

<210> 678
<211> 22
<212> RNA
<213> Homo sapiens

<400> 678
agaagauugc agaguaaguu cc 22

<210> 679
<211> 25
<212> RNA
<213> Homo sapiens

<400> 679
agcggggagg aagugggcgc ugcuu 25

<210> 680
<211> 22
<212> RNA
<213> Homo sapiens

<400> 680
ugagggcucc aggugacggu gg 22

<210> 681
<211> 25
<212> RNA
<213> Homo sapiens

<400> 681
caugcugacc ucccuccugc cccag 25

<210> 682
<211> 23
<212> RNA
<213> Homo sapiens

<400> 682
ucaggcaaag ggauauuuac aga 23

<210> 683
<211> 22

<212> RNA
<213> Homo sapiens

<400> 683
aaggcccggg cuuuccuccc ag 22

<210> 684
<211> 22
<212> RNA
<213> Homo sapiens

<400> 684
ugcggggaca ggccagggca uc 22

<210> 685
<211> 22
<212> RNA
<213> Homo sapiens

<400> 685
agccaggcuc ugaagggaaa gu 22

<210> 686
<211> 21
<212> RNA
<213> Homo sapiens

<400> 686
cgccugccca gcccuccugc u 21

<210> 687
<211> 21
<212> RNA
<213> Homo sapiens

<400> 687
auagcaauug cucuuuugga a 21

<210> 688
<211> 22
<212> RNA
<213> Homo sapiens

<400> 688
aauguuggaa uccucgcuag ag 22

<210> 689
<211> 23
<212> RNA
<213> Homo sapiens

<400> 689
ucugcacugu gaguuggcug gcu 23

<210> 690
<211> 21
<212> RNA
<213> Homo sapiens

<400> 690
uugaaaggcu auuucuuggu c 21

<210> 691

<211> 21
<212> RNA
<213> Homo sapiens

<400> 691
ugcuguauug ucagguagug a 21

<210> 692
<211> 24
<212> RNA
<213> Homo sapiens

<400> 692
agggcuggac ucagcggcgg agcu 24

<210> 693
<211> 24
<212> RNA
<213> Homo sapiens

<400> 693
aauuugguuu cugaggcacu uagu 24

<210> 694
<211> 22
<212> RNA
<213> Homo sapiens

<400> 694
ugaggacagg gcaaauucac ga 22

<210> 695
<211> 21
<212> RNA
<213> Homo sapiens

<400> 695
uuucccuuuc cauccuggca g 21

<210> 696
<211> 23
<212> RNA
<213> Homo sapiens

<400> 696
cagggcucag ggauuggaug gag 23

<210> 697
<211> 22
<212> RNA
<213> Homo sapiens

<400> 697
auucugcauu uuuagcaagu uc 22

<210> 698
<211> 22
<212> RNA
<213> Homo sapiens

<400> 698
aacggcaaug acuuuuguac ca 22

<210> 699
<211> 22
<212> RNA
<213> Homo sapiens

<400> 699
gaaaguaauu gcuguuuuug cc 22

<210> 700
<211> 22
<212> RNA
<213> Homo sapiens

<400> 700
aaaaguuauu gcgguuuugg cu 22

<210> 701
<211> 21
<212> RNA
<213> Homo sapiens

<400> 701
aaaaguaauu gcgguuuuug c 21

<210> 702
<211> 22
<212> RNA
<213> Homo sapiens

<400> 702
caagaaccuc aguugcuuuu gu 22

<210> 703
<211> 22
<212> RNA
<213> Homo sapiens

<400> 703
auauuaccau uagcucaucu uu 22

<210> 704
<211> 22
<212> RNA
<213> Homo sapiens

<400> 704
uaaaacuuua agugugccua gg 22

<210> 705
<211> 22
<212> RNA
<213> Homo sapiens

<400> 705
cagagugaca agcugguuaa ag 22

<210> 706
<211> 21
<212> RNA
<213> Homo sapiens

<400> 706
acuggcauua gugggacuuu u 21

<210> 707
<211> 24
<212> RNA
<213> Homo sapiens

<400> 707
uacagaugca gauucucuga cuuc 24

<210> 708
<211> 22
<212> RNA
<213> Homo sapiens

<400> 708
cucauuuaag uagucugaug cc 22

<210> 709
<211> 19
<212> RNA
<213> Homo sapiens

<400> 709
uuauugucac guucugauu 19

<210> 710
<211> 22
<212> RNA
<213> Homo sapiens

<400> 710
uucuggauaa caugcugaag cu 22

<210> 711
<211> 22
<212> RNA
<213> Homo sapiens

<400> 711
uugugucaau augcgaugau gu 22

<210> 712
<211> 22
<212> RNA
<213> Homo sapiens

<400> 712
cacacacugc aauuacuuuu gc 22

<210> 713
<211> 21
<212> RNA
<213> Homo sapiens

<400> 713
cacaagguau ugguauuacc u 21

<210> 714
<211> 22
<212> RNA
<213> Homo sapiens

<400> 714

uccauuacac uacccugccu cu 22

<210> 715
<211> 22
<212> RNA
<213> Homo sapiens

<400> 715
ugugcgcagg gagaccucuc cc 22

<210> 716
<211> 17
<212> RNA
<213> Homo sapiens

<400> 716
cgcgccgggc ccggguu 17

<210> 717
<211> 18
<212> RNA
<213> Homo sapiens

<400> 717
cggggcggca ggggccuc 18

<210> 718
<211> 18
<212> RNA
<213> Homo sapiens

<400> 718
gaggcugaag gaagaugg 18

<210> 719
<211> 17
<212> RNA
<213> Homo sapiens

<400> 719
gcugggcgag gcuggca 17

<210> 720
<211> 22
<212> RNA
<213> Homo sapiens

<400> 720
guggguacgg cccagugggg gg 22

<210> 721
<211> 17
<212> RNA
<213> Homo sapiens

<400> 721
gcuucuguag uguaguc 17

<210> 722
<211> 18
<212> RNA
<213> Homo sapiens

<400> 722
cggcuggagg ugugagga 18

<210> 723
<211> 21
<212> RNA
<213> Homo sapiens

<400> 723
uuacaggcgu gaaccaccgc g 21

<210> 724
<211> 24
<212> RNA
<213> Homo sapiens

<400> 724
ugggcuaagg gagaugauug ggua 24

<210> 725
<211> 21
<212> RNA
<213> Homo sapiens

<400> 725
ccgucgccgc cacccgagcc g 21

<210> 726
<211> 22
<212> RNA
<213> Homo sapiens

<400> 726
gguccagagg ggagauaggu uc 22

<210> 727
<211> 24
<212> RNA
<213> Homo sapiens

<400> 727
cauagcccgg ucgcugguac auga 24

<210> 728
<211> 17
<212> RNA
<213> Homo sapiens

<400> 728
ccccgccacc gccuugg 17

<210> 729
<211> 24
<212> RNA
<213> Homo sapiens

<400> 729
ugucagugac uccugccccu uggu 24

<210> 730
<211> 22
<212> RNA
<213> Homo sapiens

<400> 730
ucuggggaug aggacagugu gu 22

<210> 731
<211> 21
<212> RNA
<213> Homo sapiens

<400> 731
ucucaguaag uggcacucug u 21

<210> 732
<211> 18
<212> RNA
<213> Homo sapiens

<400> 732
agagcagaag gaugagau 18

<210> 733
<211> 22
<212> RNA
<213> Homo sapiens

<400> 733
ucaggccagg cacaguggcu ca 22

<210> 734
<211> 22
<212> RNA
<213> Homo sapiens

<400> 734
acuuuccuca cucccgugaa gu 22

<210> 735
<211> 21
<212> RNA
<213> Homo sapiens

<400> 735
ugacagcgcc cugccuggcu c 21

<210> 736
<211> 17
<212> RNA
<213> Homo sapiens

<400> 736
aagacugaga ggaggga 17

<210> 737
<211> 23
<212> RNA
<213> Homo sapiens

<400> 737
agaacucuug cagucuuaga ugu 23

<210> 738
<211> 57
<212> DNA

<213> Homo sapiens

<220>
<223> genomic sequence of miRNA precursor

<400> 738
ggccgcgccc cgtttcccag gacaaagggc actccgcacc ggaccctggt cccagcg 57

<210> 739
<211> 64
<212> DNA
<213> Homo sapiens

<220>
<223> genomic sequence of miRNA precursor

<400> 739
cccactccct ggcgccgctt gtggagggcc caagtccttc tgattgaggc ccaacccgtg 60
gaag 64

<210> 740
<211> 56
<212> DNA
<213> Homo sapiens

<220>
<223> genomic sequence of miRNA precursor

<400> 740
cgccgggacc ggggtccggg gcggagtgcc cttcctcctg ggaaacgggg tgcggc 56

<210> 741
<211> 81
<212> DNA
<213> Homo sapiens

<220>
<223> genomic sequence of miRNA precursor

<400> 741
gcttcacgtc cccaccggcg gcggcggcgg tggcagtggc ggcggcggcg gcggtggcgg 60
cggcggcggc ggcggcggct c 81

<210> 742
<211> 89
<212> DNA
<213> Homo sapiens

<220>
<223> genomic sequence of miRNA precursor

<400> 742
gccgcccccg ccgccgccgc cgccgccgcc gccgccgccg ccgcccgctt tcggctcggg 60
cctcaggtga gtcggagggg ccgggcgcc 89

<210> 743
<211> 22
<212> RNA
<213> Homo sapiens

<400> 743
ggcggagugc ccuucuuccu gg 22

<210> 744
<211> 22
<212> RNA
<213> Homo sapiens

<400> 744
ggagggccca aguccuucug au 22

<210> 745
<211> 22
<212> RNA
<213> Homo sapiens

<400> 745
gaccaggguc cggugcggag ug 22

<210> 746
<211> 17
<212> DNA
<213> Homo sapiens

<220>
<223> genomic primer sequence

<400> 746
agggtccggt gcggagt 17

<210> 747
<211> 12
<212> RNA
<213> Bos taurus

<400> 747
ggguccggug cg 12

<210> 748
<211> 20
<212> DNA
<213> Homo sapiens

<220>
<223> genomic primer sequence

<400> 748
tgcggagtgc cctttgtcct 20

<210> 749
<211> 22
<212> DNA
<213> Homo sapiens

<220>
<223> genomic primer sequence

<400> 749
ggagggccca agtccttctg at 22

<210> 750
<211> 14
<212> RNA
<213> Caenorhabditis remanei

<400> 750

```
cccaagugcu ucug                                                   14

<210>   751
<211>   18
<212>   DNA
<213>   Homo sapiens

<220>
<223>   genomic primer sequence

<400>   751
cggagtgccc ttcttcct                                               18

<210>   752
<211>   12
<212>   RNA
<213>   Zea mays

<400>   752
gugcccuucu uc                                                     12
```